# EUROPÄISCHE PATENTSCHRIFT

(11) **EP 2 501 761 B1**
(45) Veröffentlichungstag und Bekanntmachung des Hinweises auf die Patenterteilung: **17.06.2020**
(21) Anmeldenummer: 10787717.7
(22) Anmeldetag: 22.11.2010
(51) Int. Cl.: C09D 5/16, A61K 31/165, A61K 31/167, A61K 31/36, A61K 31/426, A61K 31/4402, A61K 31/443, A61K 31/4741, A61K 31/4743, A61K 31/506, A61K 31/513, C07C 233/11, C07C 235/36, C07C 235/38, C07D 213/75, A61K 8/49, A61Q 19/00, A23L 2/52, A23G 3/36, D06M 15/03, A24B 13/00

(54) **VERWENDUNG PHYSIOLOGISCHER KÜHLWIRKSTOFFE UND MITTEL ENTHALTEND SOLCHE WIRKSTOFFE**
USE OF PHYSIOLOGICAL COOLING ACTIVE INGREDIENTS, AND AGENTS CONTAINING SUCH ACTIVE INGREDIENTS
UTILISATION D'AGENTS RAFRAÎCHISSANTS PHYSIOLOGIQUES ET PRODUITS CONTENANT DE TELS AGENTS

(30) Priorität: 20.11.2009 EP 09176698; 03.03.2010 DE 102010002558
(43) Veröffentlichungstag der Anmeldung: 26.09.2012
(60) Teilanmeldung: 19194016.2
(73) Patentinhaber: Symrise AG, 37603 Holzminden (DE); BASF SE, 67056 Ludwigshafen am Rhein (DE)
(72) Erfinder: SURBURG, Horst, 37603 Holzminden (DE); BACKES, Michael, 37603 Holzminden (DE); OERTLING, Heiko, 1012 Lausanne (CH); MACHINEK, Arnold, 37603 Holzminden (DE); LOGES, Hubert, 37671 Höxter (DE); SIMCHEN, Ulrike, 37603 Holzminden (DE); SUBKOWSKI, Thomas, 69198 Schriesheim (DE); BOLLSCHWEILER, Claus, 69118 Heidelberg (DE); WITTENBERG, Jens, 67117 Limburgerhof (DE); SIEGEL, Wolfgang, 67117 Limburgerhof (DE)
(74) Vertreter: Eisenführ Speiser
(86) Internationale Anmeldenummer: PCT/EP2010/067936
(87) Internationale Veröffentlichungsnummer: WO 2011/061330

(56) Entgegenhaltungen:
- WO-A1-02/00590
- WO-A1-99/56548
- WO-A1-2006/040136
- WO-A1-2007/017093
- WO-A1-2007/048265
- WO-A1-2008/015403
- WO-A1-2010/026094
- WO-A2-2010/010435
- CH-A5- 651 445
- DATABASE WPI Week 198406 Thomson Scientific, London, GB; AN 1984-035077 XP002616829, & SU 776 048 A (AS BELO BIOORG CHEM) 23. September 1983 (1983-09-23) & SU 776 048 A1 (INST BISORGANICHESKOJ KHIM AN [SU]; MI G MED INST [SU]) 23. September 1983 (1983-09-23)
- CHEMICAL ABSTRACTS, Bd. 054, Nr. 01, 1995, Columbus, Ohio, US; abstract no.: 581648, GULYAKEVICH, O. V. ET AL: "Synthesis and properties of dithioacetals of conformationally restricted 8-azasteroid .alpha.-acyl-.beta.-aminovinyl ketones", XP002616826 & GULYAKEVICH, O. V. ET AL: "Synthesis and properties of dithioacetals of conformationally restricted 8-azasteroid .alpha.-acyl-.beta.-aminovinyl ketones", KHIMIYA GETEROTSIKLICHESKIKH SOEDINENII , (2), 187-94 CODEN: KGSSAQ; ISSN: 0132-6244, 1995,
- CHEMICAL ABSTRACTS, Bd. 054, Nr. 01, 1986, Columbus, Ohio, US; abstract no.: 34224, AKHREM, A. A. ET AL: "Heterocyclic steroid analogs. XIV. Synthesis and some properties of conjugated enamino lactones of the 8-aza-16-oxa steroid series", XP002616827 & AKHREM, A. A. ET AL: "Heterocyclic steroid analogs. XIV. Synthesis and some properties of conjugated enamino lactones of the 8-aza-16-oxa steroid series", ZHURNAL ORGANICHESKOI KHIMII ( 1985 ), 21(6), 1348-54 CODEN: ZORKAE; ISSN: 0514-7492, 1985,
- DATABASE WPI Week 199026 Thomson Scientific, London, GB; AN 1990-199211 XP002616831, & SU 704 082 A (AS BELO BIOORG CHEM) 23. Oktober 1989 (1989-10-23) & SU 704 082 A1 (INST BIOORG CHIMII [SU]; MI G MED INST [SU]) 23. Oktober 1989 (1989-10-23)
- DATABASE WPI Week 199409 Thomson Scientific, London, GB; AN 1994-073464 XP002616830, & SU 704 083 A1 (AS BELO BIOORG CHEM INST) 30. Oktober 1993 (1993-10-30) & SU 704 083 A1 (INSTITUT BIOORGANICHESKOJ KHIMII, BELARUS; MINSKIJ GOSUDAPSTVENNYJ MED) 30. Oktober 1993 (1993-10-30)
- KUZ'MITSKII B B ET AL: "8,16-Diheterosteroids as immunomodulators", STUDIES IN ORGANIC CHEMISTRY, ELSEVIER BV, NL, Bd. 18, Nr. Bio-Org. Heterocycl, 1. Januar 1984 (1984-01-01), Seiten 385-388, XP008131588, ISSN: 0165-3253 ISBN: 978-0-444-42691-8
- CHEMICAL ABSTRACTS, Bd. 054, Nr. 01, 1993, Columbus, Ohio, US; abstract no.: 255179, MIKHAL'CHUK, A. L. ET AL: "Dithiolene derivative of 8-aza-D-homogona-1,3,5(10),13-tetraene-12, 17a- dione", XP002616864 & MIKHAL'CHUK, A. L. ET AL: "Dithiolene derivative of 8-aza-D-homogona-1,3,5(10),13-tetraene-12, 17a- dione", ZHURNAL ORGANICHESKOI KHIMII , 28(8), 1771-2 CODEN: ZORKAE; ISSN: 0514-7492, 1992,
- DATABASE WPI Week 197937 Thomson Scientific, London, GB; AN 1979-67490B XP002617232, & SU 636 236 A (AS BELO BIOORGANIC) 15. Dezember 1978 (1978-12-15) & SU 636 236 A1 (INST BIOORG KHIM AN BRUSS SSR [SU]; MI G MED INST [SU]) 5. Dezember 1978 (1978-12-05)
- YESILADA AKGUL ET AL: "3,4-Dimethoxycinnamic acid tertiary amides: synthesis and evaluation of antiinflammatory and analgesic activities", FARMACO, SOCIETA CHIMICA ITALIANA, PAVIA, IT, Bd. 51, Nr. 8 - 9, 1. Januar 1996 (1996-01-01), Seiten 595-599, XP008136702, ISSN: 0014-827X
- DOHERTY ELIZABETH M ET AL: "Discovery of potent, orally available vanilloid receptor-1 antagonists. Structure-activity relationship of N-aryl cinnamides", JOURNAL OF MEDICINAL CHEMISTRY, AMERICAN CHEMICAL SOCIETY, Bd. 48, Nr. 1, 13. Januar 2005 (2005-01-13), Seiten 71-90, XP002408838, ISSN: 0022-2623, DOI: DOI:10.1021/JM049485I
- ANDERSSON D A ET AL: "TRPM8 Activation by Menthol, Icilin, and Cold Is Differentially Modulated by Intracellular pH", JOURNAL OF NEUROSCIENCE, NEW YORK, NY, US, Bd. 24, Nr. 23, 9. Juni 2004 (2004-06-09), Seiten 5364-5369, XP003015676, ISSN: 0270-6474, DOI: DOI:10.1523/JNEUROSCI.0890-04.2004
- WEI E T ET AL: "AG-3-5: A CHEMICAL PRODUCING SENSATIONS OF COLD", JOURNAL OF PHARMACY AND PHARMACOLOGY, ROYAL PHARMACEUTICAL SOCIETY OF GREAT BRITAIN, GB, Bd. 35, Nr. 2, 1. Januar 1983 (1983-01-01) , Seiten 110-112, XP009029151, ISSN: 0022-3573
- Youval Shvo ET AL: "Chemical Shift Nonequivalence of Diastereotopic Protons Due to Restricted Rotation around Aryl-Nitrogen Bonds in Substituted Amides", Journal of the American Chemical Society, 89:19, September 13, 1967, 1 January 1967 (1967-01-01), pages 4910-4917, XP055059523, Retrieved from the Internet: URL:http://pubs.acs.org/doi/pdf/10.1021/ja 00995a015 [retrieved on 2013-04-15]

## Beschreibung

Die Erfindung betrifft neuartige Modulatoren des Kälte-Menthol-Rezeptors TRPM8, Verfahren zur Modulation des TRPM8-Rezeptors unter Verwendung dieser Modulatoren; die Verwendung der Modulatoren zur Induktion von Kältegefühl; sowie die unter Verwendung dieser Modulatoren hergestellten Gegenstände und Mittel.

In einem speziellen Aspekt betrifft die Erfindung ein Mittel umfassend wenigstens einen ausgewählten solchen Modulatoren zum Erzielen einer Kühlwirkung auf Haut oder Schleimhaut, wobei die Kühlwirkung verglichen dem bekannten Kühlwirkstoff Menthan-3-carbonsäure-N-ethylamid (WS3) länger anhaltend ist.

Ferner betrifft der spezielle Aspekt der Erfindung Mittel, die solche ausgewählte TRPM8-Rezeptor-Modulatoren umfassen, wobei die Mittel zu bestimmten Zwecken dienen. Außerdem betrifft der spezielle Aspekt der Erfindung ein Verfahren zum Erreichen einer physiologischen Kühlwirkung auf Haut oder Schleimhaut, bei dem die zuletzt genannten Mittel eingesetzt werden.

### Hintergrund der Erfindung

Der Kälte-Menthol-Rezeptor TRPM8 (auch bezeichnet als Cold-Membrane Receptor (CMR)1) gehört zur Familie der "Transient Receptor Potential Ion Channels", wird spezifisch in einer speziellen Gruppe von Neuronen exprimiert und bildet in der Zellmembran Poren aus (jeweils 4 Einheiten lagern sich dabei zu einem Tetramer zusammen), die selektiv Ca²⁺ Ionen passieren lassen. Das Protein weist 6 Transmembrandomänen auf und einen cytoplasmatischen C- sowie N-Terminus. Durch niedrige Temperaturen (bevorzugt 10-25°C) wird dieser Rezeptor stimuliert, es kommt zu einer Signaltransduktion, die vom Nervensystem als Kältegefühl interpretiert wird. Der Rezeptor ist erstmals 2002 als Kälterezeptor in mehreren Publikationen beschrieben worden (Peier AM et al, .A TRP channel that senses cold stimuli and menthol.Cell. 2002 Mar 8;108(5):705-15; McKemy DD et al. Identification of a cold receptor reveals a general role for TRP channels in thermosensation Nature 2002 Mar 7; 416 (6876): 52-8; Zuker CS. Neurobiology: A cool ion channel Nature 2002 Mar 7; 416 (6876): 27-8)

Kühlende Verbindungen, wie z.B. Menthol, spielen bereits seit langem eine wichtige Rolle in der Geschmacks- und Riechstoffindustrie, um eine Assoziation mit Frische und Sauberkeit zu erzeugen. Für die Verbindung Menthol ist gezeigt worden, dass sie als ein natürlicher Modulator des Rezeptors TRPM8 wirkt (McKemy D.D., Molecular Pain 1, 2005, 16; McKemy D.D., Nature 416, 2002, 52-58; Peier A.M., Cell 108, 2002, 705-715; Dhaka A., Annu. Rev. Neurosci. 29, 2006, 135-161). Durch Applikation von Menthol wird TRPM8 aktiviert, wodurch ein Ca²⁺-Einstrom in die Kälte-sensitiven Neuronen bewirkt wird. Das dadurch erzeugte elektrische Signal wird schließlich als Kältegefühl wahrgenommen. Überhöhte Menthol-Konzentrationen führen zu Irritation und einer anästhetischen Wirkung. Darüber hinaus sind in verschiedenen Publikationen Mentholderivate mit ähnlicher Wirkung beschrieben worden (British Patent 1971 # 1315761 Watson H.R., J. Soc. Cosmet. Chem. 29, 1978, 185-200; Furrer S.M., Chem. Percept. 1, 2008, 119-126). Es gibt auch vereinzelte, strukturell mit Menthol nicht verwandte Verbindungen, die eine signifikante TRPM8-Modulation bewirken, wie z. B. Icilin (Wei E.T., J. Pharm. Pharmacol. 35, 1983, 110-112; WO 2004/026840), WS-23 oder in der Patentanmeldung WO 2007/019719 aufgeführte Verbindungen.

Weitere Wirkungen von Substanzen, die den TRPM8-Rezeptor bzw. seine Insektenanaloga modulieren, sind eine Repellent-Wirkung auf Insekten (WO 2002/015692; WO 2004/000023, US 2004/0028714), sowie Aktivität in der Antitumortherapie (z.B. eine Beeinflussung von Prostatatumoren), Aktivität bei der Behandlung von inflammatorischen Schmerzen / Hyperalgesie und eine Wirkung als TRPM8-Antagonisten in der Therapie von Blasensyndrom bzw. überaktiver Blase (Beck B. Cell Calcium, 41, 2007, 285-294; Levine J.D. Biochim. Biophys. Acta, Mol. Basis Dis. 1772, 2007, 989-1003; Mukerji G., BMC Urology 6, 2006, 6; US 2003/0207904; US 2005/6893626, Dissertation Behrendt H.J. 2004, Universität Bochum; Lashinger E.S.R. Am. J. Physiol. Renal Physiol. Am J Physiol Renal Physiol. 2008 Jun 18. [Epub ahead of print]; PMID: 18562636).

Viele der bisher gefundenen Modulatoren von TRPM8 weisen jedoch Mängel in Bezug auf Wirkstärke, Wirkdauer, Haut-/Schleimhautreizung, Geruch, Geschmack, Löslichkeit und /oder Flüchtigkeit auf.

In der älteren Internationalen Patentanmeldung PCT/EP2009/061019 der Anmelderin Anmeldetag 26, August 2009, werden einzelne Verbindungen zur Modulation des TRPM8-Rezeptors vorgeschlagen. Dies sind im einzelnen die folgenden, konkret offenbarten Verbindungen: 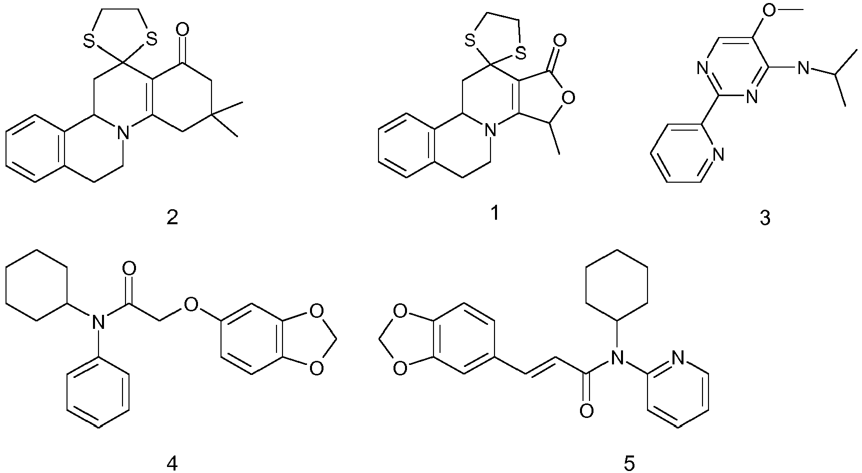 wobei die Verbindung in chemisch reiner oder angereicherter Form, als einzelnes Stereoisomer oder in Form von Stereoisomeren-Gemischen vorliegen kann.

Diese Verbindungen sind an sich bekannt und zwar:
Verbindung 1 unter CAS Nummer: 99602-94-5 (3R-cis-Form)
Verbindung 2 unter CAS Nummer: 165753-08-2
Verbindung 3 unter CAS Nummer: 338771-57-6
Verbindung 4 unter CAS Nummer: 878942-21-3
Verbindung 5 unter CAS Nummer: 748783-13-3 (ohne Stereochemie)

Diese konkret offenbarte Verwendung der Verbindungen 1 bis 5 der PCT/EP2009/061019 ist von vorliegender Anmeldung explizit ausgenommen.

Weiterhin findet sich in der PCT/EP2009/061019 die generische Offenbarung, dass die dort beschriebenen Verbindungen gegebenenfalls ungeladen oder in Form ihrer Salze, wie z.B. als Säureadditionssalz, vorliegen können. Konkret offenbart sind jedoch keine derartigen Salze. Weiterhin findet sich in der PCT/EP2009/061019 die generischen Offenbarung, dass in den dort beschriebenen Verbindungen gegebenenfalls vorhandene funktionelle Gruppen gegebenenfalls durch äquivalente chemische Gruppen ersetzt sein können; so können z.B. Sauerstoffgruppen (wie z.B. Ethergruppen) durch entsprechende Schwefelgruppen ersetzt sein und umgekehrt; Ketogruppen können durch entsprechende Thionylgruppen ersetzt sein. Konkret offenbart sind jedoch keine derartigen Abwandlungen.

Die Offenbarung dieser Internationalen Patentanmeldung PCT/EP2009/061019 gilt hiermit, soweit als zur Abgrenzung davon erforderlich, explizit als ausgenommen vom Umfang der vorliegenden Erfindung. Weiterhin offenbart die WO2007/048265 A1 Kühlwirkstoffe. Die WO2010/010435 A2 ist nachveröffentlichter Stand der Technik. Das Dokument WO99/56548 A1 betrifft Insektizide, das Dokument CH651445 A5 Unkrautbekämpfungsmittel. Dokument WO02/00590 A1 betrifft die Synthese von Olefinen, das Dokument Youval Shvo et al. "Chemical Shift Nonequivalence of Diastereotopic Protons Due to Restricted Rotation around Aryl-Nitrogen Bonds in Substituted Amides", Journal of the American Chemical Society, 89:19. September 13, 1967, 1. Januar 1967 (1967-01-01), Seiten 4910-4917 betrifft stereochemische Fragestellungen und das Dokument Yesilada Akgul et al. "3,4-Dimethoxycinnamic acid tertiary amides: synthesis and evaluation of anti-inflammatory and analgesic activities", FARMACO, SOCIETA CHIMICA ITALIANA, PAVIA, IT, Bd. 51, Nr. 8 - 9, 1. Januar 1996 (1996-01-01), Seiten 595-599, betrifft antiinflammatorische Mittel bzw. analgetische Verbindungen. Alle in diesem Absatz genannten Dokumente sind entweder nachveröffentlichter Stand der Technik oder technisch weit von der Aufgabenstellung der vorliegenden Patentanmeldung entfernt (siehe unten). Dementsprechend wurde gegen zufällige Vorwegnahmen in diesen Dokumenten eine Abgrenzung mittels Disclaimer - sofern erforderlich - durchgeführt.

### Kurzfassung der Haupterfindunq (allgemeiner Teil)

Aufgabe der vorliegenden Erfindung war es daher, neue Substanzen zu identifizieren, die zu einer Modulation des TRPM8-Rezeptors führen, welche als Alternativen zu den bisher bekannten Modulatoren einsetzbar sind. Solche Verbindungen sollten sich insbesondere auch für Anwendungen im Bereich Kosmetik (z.B. hair care, skin care, oral care), Ernährung (feed/food), Textil, OTC-Produkte (z.B. Brandsalbe), Pharmaka (z.B. Tumorbehandlung, Blasenschwäche) (nicht erfindungsgemäß) oder Verpackungen.

### Figurenbeschreibung (allgemeiner Teil):

Figur 1 zeigt (a) die mRNA-Sequenz (SEQ ID NO:1) bzw. (b) die davon abgeleitete Aminosäuresequenz (SEQ ID NO:2) des hTRPM8-Rezeptors gemäß Sequenzdatenbank-Eintrag NM 024080.
Figur 2 zeigt die Vektorkarte des mit hTRPM8 kodierenden Plasmids plnd_M8, welches zur Transfektion der HEK293 Zellen verwendet wurde.

### Detaillierte Beschreibung der Erfindung:

### 1. Definitionen

### 1.1 Allgemeine Begriffe

In der Literatur gibt es verschiedene Synonyme für "TRPM8": TRPP8, LTRPC6, CMR1, MGC2849, transient receptor potential cation channel subfamily M member 8. Im Sinne der vorliegenden Erfindung sind alle Bezeichnungen mit umfasst. Mit umfasst sind auch alle funktionalen Modifikationen des Rezeptors, wie insbesondere Splicevarianten, Isoformen, wie z.B. TRPM8 CRA_a, TRPM8 CRA_b und alle analogen Rezeptoren aus verschiedenen Organismen, wie Mensch, Maus, Ratte. Die Nukleotid- bzw. Aminosäuresequenzen der verschiedenen Rezeptoren sind an sich bekannt und in Sequenzdatenbanken hinterlegt. So ist z.B. die Sequenzinformation für hTRPM8 unter der Nummer NM_024080 eingetragen.

Ein "Modulator" im Sinne der Erfindung stellt eine Verbindung dar, die als Agonist und/oder Antagonist des TRPM8-Rezeptors *in vivo* und/oder *in vitro,* insbesondere *in vivo* wirken kann.

Geeignete Modulatoren können dabei entweder nur als Antagonist oder Agonist, insbesondere nur als Agonist, oder sowohl als Antagonist als auch als Agonist agieren. Dabei können sich insbesondere eine agonistische oder eine antagonistische Wirkung in Abhängigkeit von der jeweiligen gewählten Modulatorkonzentration einstellen.

Ein "Agonist" ist dabei eine Verbindung, welche eine Aktivierung des TRPM8-Rezeptors vermittelt, also einen Ca²⁺ -Einstrom in die kälte-sensitiven Neuronen induziert und damit ein Kältegefühl vermittelt. Ein "Antagonist" ist dagegen eine Verbindung, welche dieser Aktivierung des TRPM8-Rezeptors entgegenwirken kann.

Die erfindungsgemäßen Mediatoren können ihre Wirkung dadurch ausüben, dass sie reversibel oder irreversibel, spezifisch oder unspezifisch an ein TRPM8-Rezeptormolekül binden. Gewöhnlich erfolgt die Bindung nicht-kovalent über ionische und/oder nichtionische, wie z.B. hydrophobe, Wechselwirkungen mit dem Rezeptormolekül. "Spezifisch" umfasst dabei sowohl ausschließliche Wechselwirkung mit einem oder mehreren verschiedenen TRPM8-Rezeptormolekülen (wie z.B. TRPM8-Molekülen verschiedenen Ursprungs oder verschiedenen Isoformen). "Unspezifisch" ist dagegen eine Wechselwirkung des Modulators mit mehreren verschiedenen Rezeptormolekülen unterschiedlicher Funktion und/oder Sequenz, wobei sich jedoch als Folge eine gewünschte agonistische und/oder antagonistische Modulation (wie oben beschrieben) des TRPM8-Rezeptors feststellen lässt.

Unter "Standardbedingungen" in einem zellularen Aktivitätstest für erfindungsgemäße Modulatoren versteht man in diesem Zusammenhang einen Aktivitätstest, durchgeführt mit HEK293-Zellen, welche transformiert wurden mit humanem TRPM8 und beladen sind mit Calciumsensitivem Farbstoff (wie z.B. Fluo-4AM, d.h. Fluo-4-Acetoxymethylester), anschließende Zugabe der Testverbindung und Nachweis der Farbänderung, wobei Versuchsdurchführung bei 37 °C erfolgt; wie z.B. beschrieben in Referenzbeispiel 3, unten, oder bei Behrendt et al (2004) a.a.O).

Ein erfindungsgemäßer Modulator dient insbesondere zur Induktion von Kältegefühl, bei Mensch und/oder Tier. Eine "Induktion von Kältegefühl" liegt vor, wenn die Verbindung im oben beschriebenen zellulären Aktivitätstest einen agonistischen Effekt auf hTRPM8 zeigt. Erfindungsgemäße Mittel enthalten neben den für das jeweilige Mittel üblichen Bestandteilen eine wirksame Menge wenigstens eines erfindungsgemäßen Modulators. "Wirksam" bedeutet in diesem Zusammenhang eine Konzentration des Modulators, die ausreicht, um bei Applikation des Mittels (z.B. Auftragung auf die Haut) den gewünschten Effekt, wie z.B. pharmakologischen Effekt (nicht erfindungsgemäß), oder sensorischen Effekt wie z.B. den olfaktorischen Kälteeffekt zu vermitteln.

Eine "topische" Anwendung umfasst insbesondere, cutane oder orale Anwendungsformen.

### 1.2 Chemische Begriffe

Werden keine anderslautenden Angaben gemacht, so gelten im Rahmen der vorliegenden Erfindung die folgenden allgemeinen Bedeutungen:
**Halogen:** F, Cl, Br oder J
**Alkyl sowie alle Alkylteile in davon abgeleiteten Resten, wie z,B. Alkoxy, Alkylthio, Alkoxyalkyl, Alkoxyalkoxy, Alkylamino und Dialkylamino:** gesättigte, geradkettige oder verzweigte Kohlenwasserstoffreste mit 1 bis 4, 1 bis 6, 1 bis 8, 1 bis 10 oder 1 bis 10 Kohlenstoffatomen, z. B.
   - **C₁-C₆-Alkyl** wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.
   - **C₁-C₆-Alkoxy,**umfassend **C₁-C₄-Alkoxy,** wie z.B. Methoxy, Ethoxy, n-Propoxy, 1-Methylethoxy, Butoxy, 1-Methylpropoxy, 2-Methylpropoxy oder 1,1-Dimethylethoxy;
      sowie z. B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
**Alkenyl:** ein- oder mehrfach, insbesondere einfach ungesättigte, geradkettige oder verzweigte Kohlenwasserstoffreste mit 2 bis 4, 2 bis 6, 2 bis 8 2 bis 10 oder 2 oder bis 20 Kohlenstoffatomen und einer Doppelbindung in einer beliebigen Position, z. B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl,
   1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
**Halo(gen)alkyl:** geradkettige oder verzweigte Alkylgruppen mit 1 bis 4, 1 bis 6, 1 bis 8, 1 bis 10 oder 1 bis 20 Kohlenstoffatomen (wie vorstehend genannt), wobei in diesen Gruppen teilweise oder vollständig die Wasserstoffatome durch Halogenatome wie vorstehend genannt ersetzt sein können, z. B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 1,1,1-Trifluorprop-2-yl.
**Halogenalkoxy:** für einen Alkoxyrest mit 1 bis 8, insbesondere 1 bis 6 und speziell 1 bis 4 C-Atomen wie vorstehend genannt, der partiell oder vollständig durch Fluor, Chlor, Brom und/oder lod, vorzugsweise durch Fluor substituiert ist, also z. B. OCH₂F, OCHF₂, OCF₃, OCH₂Cl, OCHCl₂, OCCl₃, Chlorfluormethoxy, Dichlorfluormethoxy, Chlordifluormethoxy, 2-Fluorethoxy, 2-Chlorethoxy, 2-Bromethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy;
**Cycloalkyl:** carbocyclische Reste mit 3 bis 20 Kohlenstoffatomen, wie z.B. C₃-C₁₂-Cycloalkyl, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl, Cycloheptyl, sowie Cyclopropyl-methyl, Cyclopropyl-ethyl, Cyclobutyl-methyl, Cyclobutyl-ethyl, Cyclopentyl-methyl, Cyclopentyl-ethyl, Cyclohexyl-methyl oder C₃-C₇-Cycloalkyl, wie Cyclopropyl, Cyclobutyl, Cyclopentyl, Cyclohexyl, Cycloheptyl, Cyclopropyl-methyl, Cyclopropyl-ethyl, Cyclobutyl-methyl, Cyclopentyl-ethyl, Cyclohexyl-methyl, wobei die Anbindung an den Rest des Moleküls über jegliches geeignetes C-Atom erfolgen kann.
**Cycloalkenyl:** monocyclische, einfach ungesättigte Kohlenwasserstoffgruppen mit 5 bis 8, vorzugsweise bis 6 Kohlenstoffringgliedern, wie Cyclopenten-1-yl, Cyclopenten-3-yl, Cyclohexen-1-yl, Cyclohexen-3-yl und Cyclohexen-4-yl;
**Alkylen:** geradkettige oder ein- oder mehrfach verzweigte Kohlenwasserstoff-Brückengruppen mit 1 bis 20 Kohlenstoffatomen, wie z.B. C₁-C₇-Alkylengruppen ausgewählt unter -CH₂-, - (CH₂)₂-, -(CH₂)₃-,-(CH₂)₄-, -(CH₂)₂-CH(CH₃)-, -CH₂-CH(CH₃)-CH₂-, (CH₂)₄-, -(CH₂)₅-, -(CH₂)₆, - (CH₂)₇-, -CH(CH₃)-CH₂-CH₂-CH(CH₃)- oder -CH(CH₃)-CH₂-CH₂-CH₂-CH(CH₃)- oder C₁-C₄-Alkylengruppen ausgewählt unter -CH₂-, -(CH₂)₂-, -(CH₂)₃-,-(CH₂)₄-, -(CH₂)₂-CH(CH₃)-, -CH₂-CH(CH₃)-CH₂-.
**Alkenylen:** die ein- oder mehrfach, insbesondere einfach ungesättigten Analoga obiger Alkylengruppen mit 2 bis 20 Kohlenstoffatomen, insbesondere für C₂-C₇-Alkenylene oder C₂-C₄-Alkenylen, wie -CH=CH-, -CH=CH-CH₂-, - CH₂-CH=CH-, -CH=CH-CH₂-CH₂-, -CH₂-CH=CH-CH₂-, -CH₂-CH₂-CH=CH-, -CH(CH₃)-CH=CH-, -CH₂-C(CH₃)=CH-.
**Aryl:** ein- oder mehrkernige, vorzugsweise ein- oder zweikernige, gegebenenfalls substituierte aromatische Restemit 6 bbis 20 wie z.B. 6 bis 10 Ring-Kohlenstoffatomen, wie z.B. Phenyl, Biphenyl, Naphthyl wie 1- oder 2-Naphthyl, Tetrahydronaphthyl, Fluorenyl, Indenyl und Phenanthrenyl. Diese Arylreste können gegebenenfalls 1, 2, 3, 4, 5 oder 6 gleiche oder verschiedene Substituenten tragen.
**Arylalkyl:** die Aryl-substituierten Analoga obiger Alkylreste, wobei Aryl ebenfalls die oben angegebenen Bedeutungen besitzt, wie z.B. Phenyl-C₁-C₄-Alkylreste ausgewählt unter Phenylmethyl oder Phenyl-ethyl.
**Aryloxy:** die Sauerstoff-verknüpften Analoga obiger gegebenenfalls substituierter Arylreste.
**Heterocyclyl:** fünf- bis siebengliedriger gesättigter, partiell ungesättigter oder aromatischer Heterocyclen bzw. Heterocyclylreste, enthaltend ein, zwei , drei oder vier Heteroatome aus der Gruppe O, N oder S. Beispielsweise können folgende Untergruppen genannt werden
   - 5- oder 6-gliedriges gesättigtes oder einfach ungesättigtes Heterocyclyl, enthaltend ein bis zwei Stickstoffatome und/oder ein Sauerstoff- oder Schwefelatom oder ein oder zwei Sauerstoff- und/oder Schwefelatome als Ringglieder, z. B. 2-Tetrahydrofuranyl, 3-Tetrahydrofuranyl, 2-Tetrahydrothienyl, 3-Tetrahydrothienyl, 1-Pyrrolidinyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 1-Piperidinyl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
   - 5-gliedriges aromatisches Heterocyclyl (= **Heteroaryl** bzw. **Hetaryl),** enthaltend neben Kohlenstoffatomen ein, zwei oder drei Stickstoffatome oder ein oder zwei Stickstoffatome und ein Schwefel- oder Sauerstoffatom als Ringglieder, z. B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
   - 5-gliedriges aromatisches Heterocyclyl (= **Heteroaryl** bzw. **Hetaryl),** das 1, 2, 3 oder 4 Stickstoffatome als Ringglieder aufweist, wie 1-, 2- oder 3-Pyrrolyl, 1-, 3- oder 4-Pyrazolyl, 1-, 2-oder 4-Imidazolyl, 1,2,3-[1H]-Triazol-1-yl, 1,2,3-[2H]-Triazol-2-yl, 1,2,3-[1H]-Triazol-4-yl, 1,2,3-[1H]-Triazol-5-yl, 1,2,3-[2H]-Triazol-4-yl, 1,2,4-[1H]-Triazol-1-yl, 1,2,4-[1H]-Triazol-3-yl, 1,2,4-[1H]-Triazol-5-yl, 1,2,4-[4H]-Triazol-4-yl, 1,2,4-[4H]-Triazol-3-yl, [1H]-Tetrazol-1-yl, [1H]-Tetrazol-5-yl, [2H]-Tetrazol-2-yl und [2H]-Tetrazol-5-yl;
   - 5-gliedriges aromatisches Heterocyclyl (= **Heteroaryl** bzw. **Hetaryl),** das 1 unter Sauerstoff und Schwefel ausgewähltes Heteroatome und gegebenenfalls 1, 2 oder 3 Stickstoffatome als Ringglieder aufweist, beispielsweise 2-Furyl, 3-Furyl, 2-Thienyl, 3-Thienyl, 3- oder 4-Isoxazolyl, 3- oder 4- Isothiazolyl, 2-, 4- oder 5-Oxazolyl, 2-, 4 oder 5-Thiazolyl, 1,2,4-Thiadiazol-3-yl, 1,2,4-Thiadiazol-5-yl, 1,3,4-Thiadiazol-2-yl, 1,2,4-Oxadiazol-3-yl, 1,2,4-Oxadiazol-5-yl und 1,3,4-Oxadiazol-2-yl;
   - 6-gliedriges Heterocyclyl (= **Heteroaryl** bzw. **Hetaryl),** enthaltend neben Kohlenstoffatomen ein oder zwei bzw. ein, zwei oder drei Stickstoffatome als Ringglieder, z. B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,2,4-Triazin-3-yl; 1,2,4-Triazin-5-yl, 1,2,4-Triazin-6-yl und 1,3,5-Triazin-2-yl;
**Heteroaryloxy** bzw**.Heteroaryloxy** steht für die Sauerstoff-verknüpften Analoga obigerr Heterocyclyl- bzw. Heteroarylreste.
**Substituenten,** wie insbesondere für obige Reste, sind insbesondere ausgewählt sind unter Ketogruppen, - COOH, -COO-Alkyl, -OH, -SH, -CN, Amino, -NO₂, Alkyl, oder Alkenylgruppen, wobei in den Alkyl- oder Alkenylgruppen ein oder mehrere H-Atome durch Halogen, ersetzt sein können.

Die in diesem Kapitel ausgeführten Definitionen gelten auch für den speziellen Aspekt der Erfindung, (siehe Kapitel 6) sofern nicht anders ausgeführt.

### 2. Besondere Ausführungsformen der Erfindung (allgemeiner Teil):

Die Erfindung betrifft insbesondere folgende besondere Ausführungsformen, sofern sie unter die Ansprüche fallen:
1. Nicht-therapeutisches Verfahren zur In-vitro- oder In-vivo-Modulation des Kälte-Menthol-Rezeptors TRPM8, wobei man den Rezeptor mit wenigstens einem Modulator in Kontakt bringt, der ausgewählt ist unter Verbindungen der folgenden Strukturtypen 1 bis 3; wobei nur die nachfolgenden Verbindungen des Strukturtypes 3 zur Erfindung gehören, ggf. wie in den Ansprüchen weiter eingeschränkt:
a) Strukturtyp 1: (nicht erfindungsgemäß) 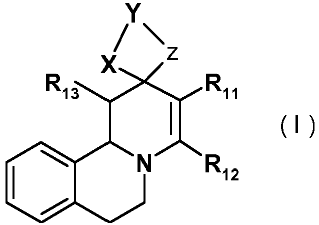 worin
R₁₁, R₁₂ und R₁₃ unabgängig voneinander ausgewählt sind unter:
   H;
   geradkettigen oder verzweigten C₁-C₆-Alkylgruppen, die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen; und
   geradkettigen oder verzweigten C₁-C₆-Alkyloxygruppen, die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen; oder
   R₁₁ und R₁₂ zusammen mit den Kohlenstoffatomen, an die sie gebunden sind, einen 4-, 5-, 6- oder 7-gliedrigen, ein- oder mehrfach ungesättigten, carbo- oder heterocyclischen Ring bilden, der gegebenenfalls 1, 2, 3, 4 oder 5 gleiche oder verschiedene Substituenten trägt, die ausgewählt sind unter geradkettigen oder verzweigten C₁-C₆-Alkylgruppen, und Oxogruppen (=O), und die Ring-Heteroatome gleich oder verschieden sind und ausgewählt sind unter O, N und S;
X und Z unabhängig voneinander ausgewählt sind unter -O-, -S-, -NH-, -S(=O)-, oder -S(=O)₂- Gruppen; und
Y ausgewählt ist unter
   geradkettigen oder verzweigten C₁-C₈-Alkylengruppen, die gegebenenfalls 1, 2, 3 oder 4 gleichen oder verschiedenen Substituenten tragen, die ausgewählt sind unter NH₂, OH, SH, Halogen oder geradkettigen oder verzweigten C₁-C₆-Alkoxygruppen; oder
X-Y-Z zusammen mit dem Kohlenstoffatom an das sie gebunden sind, eine Ketogruppe bilden,
sowie Salze dieser Verbindungen, insbesondere Säureadditionssalze mit anorganischen oder insbesondere organischen, ein- oder insbesondere mehrwertigen Carbonsäuren;
gegebenenfalls in Stereoisomeren-reiner Form oder als Gemisch von Stereoisomeren;
und wobei gegebenenfalls die Verbindungen mit der Struktur 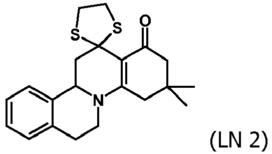 und 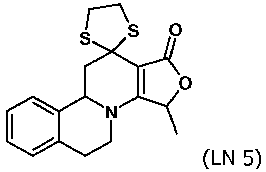 ausgenommen sind;

b) Strukturtyp 2: (nicht erfindungsgemäß) 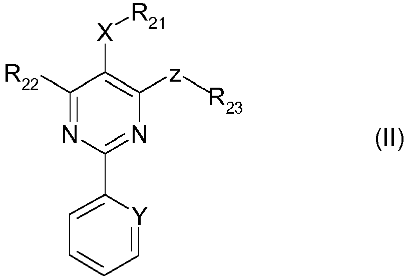 worin
R₂₁ und R₂₂ unabhängig voneinander ausgewählt sind unter:
   H;
   geradkettigen oder verzweigten C₁-C₆-Alkylgruppen, die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen; geradkettigen oder verzweigten C₁-C₆-Alkyloxygruppen, die gegebenenfalls 1, 2, 3 oder 4 gleiche oder verschiedenen Substituenten tragen, die ausgewählt sind unter NH₂, OH, SH, Halogen oder geradkettigen oder verzweigten C₁-C₆-Alkoxygruppen; ein- oder mehrkernige Aryl-, Arylalkyl- und Heteroarylgruppen, die gegebenenfalls 1, 2, 3 oder 4 gleiche oder verschiedene Substituenten tragen, die ausgewählt sind unter NH₂, OH, SH, Halogen, geradkettigen oder verzweigten C₁-C₆-Alkylgruppen und geradkettigen oder verzweigten C₁-C₆-Alkyloxygruppen; wobei die Heteroarylgruppen 1, 2, 3 oder 4 Ring-Heteroatome aufweisen, die gleich oder verschieden sind und ausgewählt sind unter O, N und S;
R₂₃ ausgewählt ist unter:
   H;
   geradkettigen oder verzweigten C₁-C₆-Alkylgruppen die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen;
   C₃-C₇-Cycloalkylgruppen, die gegebenenfalls 1,2, 3 oder 4 gleiche oder verschiedene Substituenten tragen, die ausgewählt sind unter NH₂, OH, SH, Halogen, geradkettigen oder verzweigten C₁-C₆-Alkylgruppen, oder geradkettigen oder verzweigten C₁-C₆-Alkoxygruppen; wobei die Cycloalkylgruppe gegebenenfalls über eine C₁-C₄-Alkylengruppe an Z gebunden ist; und wobei gegebenenfalls 1, 2 oder 3 Ring-Kohlenstoffatome durch gleiche oder verschiedene Heteroatome, ausgewählt unter O, N und S ersetzt sein können;
   ein- oder mehrkernige Aryl-, Arylalkyl- und Heteroarylgruppen, die gegebenenfalls 1, 2, 3 oder 4 gleiche oder verschiedene Substituenten tragen, die ausgewählt sind unter NH₂, OH, SH, Halogen, geradkettigen oder verzweigten C₁-C₆-Alkylgruppen und geradkettigen oder verzweigten C₁-C₆-Alkyloxygruppen; wobei die Heteroarylgruppen 1, 2, 3 oder 4 Ring-Heteroatome aufweisen, die gleich oder verschieden sind und ausgewählt sind unter O, N und S;
   X ausgewählt ist unter O, S oder Methylen;
Y ausgewählt ist unter N oder CH; und
Z ausgewählt ist unter O, S oder NR₂₄ , wobei
   R₂₄ für H; oder eine geradkettige oder verzweigte C₁-C₆-Alkylgruppe steht, die gegebenenfalls 1, 2, 3 oder 4 gleiche oder verschiedene Substituenten trägt, die ausgewählt sind unter NH₂, OH, SH, Halogen oder geradkettigen oder verzweigten C₁-C₆-Alkoxygruppen; oder
   R₂₄ und R₂₃ zusammen mit der Z-Gruppe, an die sie gebunden sind, einen 4-, 5-, 6-oder 7-gliedrigen, gesättigten, oder ein- oder mehrfach ungesättigten heterocyclischen Ring bilden, der gegebenenfalls 1, 2, 3, 4 oder 5 gleiche oder verschiedene Substituenten trägt, die ausgewählt sind unter geradkettigen oder verzweigten C₁-C₆-Alkylgruppen, und der 1, 2 oder 3 zusätzliche Ring-Heteroatome aufweist, die gleich oder verschieden sind und ausgewählt sind unter O, N und S;
sowie Salze dieser Verbindungen, insbesondere Säureadditionssalze mit anorganischen oder insbesondere organischen, ein- oder insbesondere mehrwertigen Carbonsäuren;
gegebenenfalls in Stereoisomeren-reiner Form oder als Gemisch von Stereoisomeren;
und wobei gegebenenfalls die Verbindung mit der Struktur 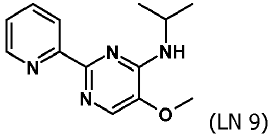 ausgenommen ist;
   und

c) Strukturtyp 3: 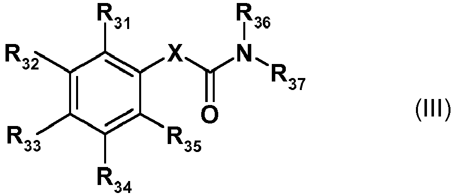 worin
R₃₁, R₃₂, R₃₃, R₃₄ und R₃₅ gleich oder verschieden sind und ausgewählt sind unter
H;
Halogen;
geradkettigen oder verzweigten C₁-C₆-Alkylgruppen die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen;
geradkettigen oder verzweigten C₁-C₆-Alkoxygruppen die gegebenenfalls 1, 2, 3 oder 4 gleiche oder verschiedene Substituenten tragen, die ausgewählt sind unter NH₂, OH, SH, Halogen oder C₁-C₆-Alkoxygruppen;
ein- oder mehrkernige Aryl-, Arylalkyl- und Heteroarylgruppen, die gegebenenfalls 1, 2, 3 oder 4 gleiche oder verschiedene Substituenten tragen, die ausgewählt sind unter NH₂, OH, SH, Halogen, geradkettigen oder verzweigten C₁-C₆-Alkylgruppen und geradkettigen oder verzweigten C₁-C₆-Alkyloxygruppen; wobei die Heteroarylgruppen 1, 2, 3 oder 4 Ring-Heteroatome aufweisen, die gleich oder verschieden sind und ausgewählt sind unter O, N und S; oder

zwei benachbarte Reste R₃₁, R₃₂, R₃₃, R₃₄ und R₃₅ zusammen mit den Kohlenstoffatomen, an die sie gebunden sind, einen 4-, 5-, 6- oder 7-gliedrigen, ein- oder mehrfach ungesättigten heterocyclischen Ring bilden, der gegebenenfalls 1, 2, 3, 4 oder 5 gleiche oder verschiedene Substituenten trägt, die ausgewählt sind unter geradkettigen oder verzweigten C₁-C₆-Alkylgruppen, und der 1, 2 oder 3 Ring-Heteroatome aufweist, die gleich oder verschieden sind und ausgewählt sind unter O, N und S;
R₃₆ und R₃₇ gleich oder verschieden sind und ausgewählt ist unter:
geradkettigen oder verzweigten C₁-C₆-Alkylgruppen die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen;
ein- oder mehrkernige Aryl-, Arylalkyl-, Aryloxy-, Heteroaryl- und Heteroaryloxygruppen, die gegebenenfalls 1, 2, 3 oder 4 gleiche oder verschiedene Substituenten tragen, die ausgewählt sind unter NH₂, OH, SH, Halogen, geradkettigen oder verzweigten C₁-C₆-Alkylgruppen und geradkettigen oder verzweigten C₁-C₆-Alkyloxygruppen; wobei die Heteroarylgruppen 1, 2, 3 oder 4 Ring-Heteroatome aufweisen, die gleich oder verschieden sind und ausgewählt sind unter O, N und S; und C₃-C₇-Cycloalkylgruppen, die gegebenenfalls 1, 2, 3 oder 4 gleiche oder verschiedene Substituenten tragen, die ausgewählt sind unter NH₂, OH, SH, Halogen, geradkettigen oder verzweigten C₁-C₆-Alkylgruppen, oder geradkettigen oder verzweigten C₁-C₆-Alkoxygruppen; wobei die Cycloalkylgruppe gegebenenfalls über eine C₁-C₄-Alkylengruppe gebunden ist; und wobei gegebenenfalls 1, 2 oder 3 Ring-Kohlenstoffatome durch gleiche oder verschiedene Heteroatome, ausgewählt unter O, N und S ersetzt sein können;

X ausgewählt ist unter
-C₁-C₄-Alkylengruppen; -C₂-C₄-Alkenylengruppen, sowie -Z-C₁-C₄- oder -C₁-C₄-Z-Alkylengruppen oder -Z-C₂-C₄- oder - C₂-C₄-Z-Alkenylengruppen, worin Z für O, S oder NH steht; oder für eine chemische Einfachbindung steht;
sowie Salze dieser Verbindungen, insbesondere Säureadditionssalze mit anorganischen oder insbesondere organischen, ein- oder insbesondere mehrwertigen Carbonsäuren;
gegebenenfalls in Stereoisomeren-reiner Form oder als Gemisch von Stereoisomeren;
und wobei die Verbindungen mit der Struktur 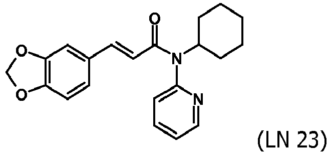 und 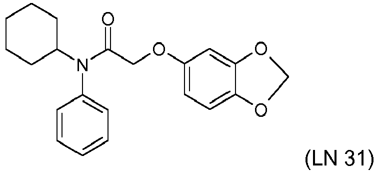
sowie die weiteren im Anspruch 1 als explizit ausgenommen beschreibenden Verbindungen ausgenommen sind.
sowie bevorzugt ausgewählt unter den weiter unten aufgeführten spezielleren Gruppen von Strukturtyp 3;
insbesondere solche Verbindungen obiger Formeln, welche in einem zellulären Aktivitätstest, insbesondere unter Standardbedingungen, unter Verwendung von Zellen, welche den humanen TRPM8-Rezeptor rekombinant exprimieren, die Permeabilität dieser Zellen für Ca²⁺ Ionen modulieren;
oder insbesondere solche Verbindungen, die auf die zelluläre Ca²⁺-Ionen-Permeabilität agonistisch oder antagonistisch wirken, wobei jeweils die jeweiligen Verbindungen der
Tabelle V

**(Tabelle V)**

| LN | Struktur |
|---|---|
| 23 | 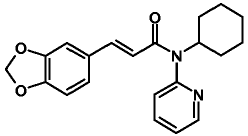 |
| 31 | 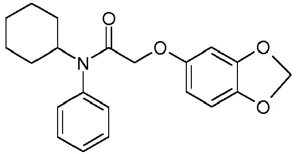 |

ausgenommen sind.
2. Verwendung einer erfindungsgemäßenVerbindung gemäß der Definition der Ausführungsform 1, zur nicht-therapeutischen Induktion von Kältegefühl bei Mensch und/oder Tier, wobei zusätzlich die Maßgabe nicht gilt, dass die Verbindung der Formel (III) nicht 1-(2-Aminoethyl)-1-(1,3-benzothiazol-2-yl)-3-[(1S)-1-phenylethyl]urea hydrochlorid und 1-(2-Aminoethyl)-1-(1,3-benzothiazol-2-yl)-3-[(1R)-1-phenylethyl]urea hydrochlorid ist.
3. Verwendung einer Verbindung gemäß der Definition in Ausführungsform 1, als aktiven Bestandteil eines pharmazeutischen Mittels. (nicht erfindungsgemäß)
4. Verwendung einer Verbindung gemäß der Definition in Ausführungsform 1, zur Behandlung von Prostatakarzinomen, zur Behandlung von Blasenschwäche oder in der Schmerztherapie. (nicht erfindungsgemäß)
5. Verwendung einer erfindungsgemäßen Verbindung gemäß der Definition in Ausführungsform 1, wobei zusätzlich die Maßgabe nicht gilt, dass die Verbindung der Formel (III) nicht 1-(2-Aminoethyl)-1-(1,3-benzothiazol-2-yl)-3-[(1S)-1-phenylethyl]urea hydrochlorid und 1-(2-Aminoethyl)-1-(1,3-benzothiazol-2-yl)-3-[(1R)-1-phenylethyl]urea hydrochlorid ist, zur nicht-therapeutischen Induktion eines Kältegefühls durch eine Verpackung, z.B. aus Papier oder Kunststoff in verschiedensten Verarbeitungsformen (wie z.B. Fasern, Geweben, Formteilen), wobei sich das Kältegefühl insbesondere bei Kontakt mit dem Verpackungsmaterial bemerkbar macht. Dabei können die Substanzen in unterschiedlichster Art mit dem Verpackungsmaterial assoziiert sein: z.B. durch Spincoating, Aufdrucken, in Form von Mikroverkapselung, direkte Einarbeitung in das Verpackungsmaterial (z.B. extrudieren), kovalente Kopplung von geeigneten Derivaten der Modulatoren (über geeignete Spacer/ Linker-Gruppen, mit deren Hilfe das Molekül reversibel oder irreversibel an das Verpackungsmaterial gebunden wird). Geeignete Arbeitsweisen sind dem Fachmann bekannt.
6. Verwendung einer erfindungsgemäßen Verbindung gemäß der Definition in Ausführungsform 1, zur nicht-therapeutischen Induktion eines Kältegefühls durch ein Textil. Dabei können die Substanzen in unterschiedlichster Art mit dem Textil assoziiert sein: z.B. durch Spincoating, Aufdrucken, in Form von Mikroverkapselung, direkte Einarbeitung in das Textilmaterial (z.B. extrudieren), kovalente Kopplung von geeigneten Derivaten der Modulatoren (über geeignete Spacer/Linker-Gruppen, mit deren Hilfe das Molekül reversibel oder irreversibel an das Verpackungsmaterial gebunden wird). Geeignete Arbeitsweisen sind dem Fachmann bekannt.
7. Erfindungsgemäße Verbindung gemäß der Definition nach Ausführungsform 1 zur Verwendung als Mediator des TRPM8-Rezeptors und insbesondere als Agonist davon, und vor allem zur nicht-therapeutischen Induzierung von Kältegefühl bei Mensch und/oder Tier, wobei die gemäß Anspruch 8 ausgenommenen Verbindungen ausgenommen sind. Aufgrund eines erweiterten Eigenschaftsprofils erfindungsgemäßer Verbindungen kann deren Verwendung mehreren Zwecken gleichzeitig dienen. So eignen sich beispielsweise Verbindungen des Strukturtyps 3 als UV-Absorber, so dass z.B. eine kombinierte Verwendung dieser Verbindung als UV-Absorber und Cooling Agent, bespielsweise in Sonnecremes, besonders vorteilhaft ist.
8. Mittel enthaltend wenigstens eine erfindungsgemäße Verbindung wie in Anspruch 8 definiert.
9. Mittel nach Ausführungsform 8, wobei statt der in Anspruch 8 definierten Verbindungen (oder zusätzlich dazu) auch die weiteren, in Anspruch 10 definierten Verbindungen enthalten sein können, ausgewählt unter
a) pharmazeutischen Mitteln, wie Antitumormittel, Mittel zur Behandlung von Erkrankungen der Blase, Schmerzmittel; (nicht erfindungsgemäß)
b) Nahrungsmitteln, wie Eis, Mousse, Creme, Getränke, Süßwaren;
c) Mundpflegemitteln, wie Zahnpasta, Mundwasser, Kaugummi; Atemerfrischungsmittel
d) Körperpflegemitteln, wie Haut- oder Haarpflegemitteln, wie Sonnencreme, Sonnenbrandcreme, Lotionen, Shampoos, Rasiercreme, Conditioner, Gesichtsreiniger, Seifen, Badeöle und Badeschäume, Antitranspirationsmittel, desodorierende Mittel,
e) Schäumen und Gelen.

10. Produkt enthaltend wenigstens eine erfindungsgemäße Verbindung nach Ausführungsform 1, wobei zusätzlich die Maßgabe nicht gilt, dass die Verbindung der Formel (III) nicht 1-(2-Aminoethyl)-1-(1,3-benzothiazol-2-yl)-3-[(1S)-1-phenylethyl]urea hydrochlorid und 1-(2-Aminoethyl)-1-(1,3-benzothiazol-2-yl)-3-[(1R)-1-phenylethyl]urea hydrochlorid ist,
zur Verwendung als Mediator des TRPM8-Rezeptors ausgewählt unter
a) Textilprodukten, wie z.B. Hemden, Hosen, Socken, Handtücher,
b) Verpackungsmaterialien,
c) Tabakprodukten;
d) Heilmitteln (Pflaster, Verbandmaterialien) (nicht erfindungsgemäß)
e) Hygieneprodukten (Schwämme, Windeln, Slipeinlagen, Reinigungstücher)
f) Erfrischungstüchern

11. Verbindungen gemäß der Definition in Anspruch 8.
12. Verbindungen wie in Anspruch 14 defniert, gegebenenfalls in stereoisomerenreiner Form, als Gemisch von Stereoisomeren, sowie Salze dieser Verbindungen.

Bei allen erfindungsgemäßen Ausführungsformen können gegebenenfalls die Verbindungen der obenstehenden Tabelle V ausgenommen sein. Dies gilt insbesondere auch für de im nachfolgenden Kapitel beschriebenen Ausgestaltungen.

Die in diesem Kapitel beschriebenen Ausführungsformen sind unter Anderem im Kapitel 6 (spezieller Aspekt der Erfindung) weiter spezifiziert.

### 3. Weitere Ausgestaltungen der Verfahren, Verwendungen und Wirkstoffe. Diese Ausgestaltungen sind nur erfindungsgemäß, sofern sie unter die Ansprüche fallen.

Folgende spezielle Ausgestaltungen erfindungsgemäßer Wirkstoffe gelten sinngemäß sowohl für die Wirkstoffe per se als auch deren Anwendungen, wie z.B. in Mitteln, Verfahren sowie Verwendungen.

### 3.1 Verbindungen der Formel I (Strukturtyp 1): (nicht erfindungsgemäß)

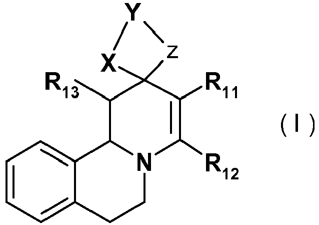

ausgewählt unter Verbindungen der folgenden Gruppen (1) bis (18):
(1) Verbindungen der Formel I, worin R₁₃ für H, geradkettige oder verzweigte C₁-C₆-Alkylgruppen, insbesondere für H steht;
(2) Verbindungen der Formel I, worin R₁₁ und R₁₂ zusammen mit den Kohlenstoffatomen, an die sie gebunden sind, einen 5- oder 6-gliedrigen, einfach ungesättigten carbo- oder heterocyclischen Ring bilden, der gegebenenfalls 1, 2, oder 3 gleiche oder verschiedene Substituenten trägt, die ausgewählt sind unter geradkettigen oder verzweigten C₁-C₆-Alkylgruppen, und Oxogruppen (=O); und die Ring-Heteroatome O-Atome sind;
(3) Verbindungen der Formel I, worin R₁₁ und R₁₂ zusammen mit den Kohlenstoffatomen, an die sie gebunden sind, einen 5- oder 6-gliedrigen, einfach ungesättigten, carbo- oder heterocyclischen Ring bilden, der gegebenenfalls 1, 2 oder 3 gleiche oder verschiedene Substituenten trägt, die ausgewählt sind unter geradkettigen C₁-C₄-Alkylgruppen, und eine Oxogruppen (=O) trägt; und das Ring-Heteroatom ein O-Atom sind;
(4) Verbindungen der Formel I, worin R₁₁ und R₁₂ zusammen Verbrückungsgruppen bilden, ausgewählt unter
   -C(=O)-O-C*H(CH₃)- in beiden stereoisomeren Formen
   -C(=O)-CH₂-C(CH₃)₂-CH₂-
   -C(=O)-CH₂-CH₂-CH₂- wobei die Ketogruppe über die R12 oder insbesondere über die R₁₁-Position an das Molekül gebunden ist.
(5) Verbindungen der Formel I, worin X und Z gleich oder verschieden sind und ausgewählt sind unter -S-, -S(=O)-, oder -S(=O)₂- Gruppen;
(6) Verbindungen der Formel I, worin X und Z gleich sind und jeweils für -S- stehen;
(7) Verbindungen der Formel I, worin X und Z verschieden sind und ausgewählt sind - S(=O)-, oder -S(=O)₂- Gruppen;
(8) Verbindungen der Formel I, worin Y ausgewählt ist unter geradkettigen C₂- oder C₃-Alkylengruppen, insbesondere -CH₂-CH₂- oder -CH₂-CH₂-CH₂-,
(9) Verbindungen der Formel I, worin X-Y-Z zusammen mit dem Kohlenstoffatom an das sie gebunden sind eine Ketogruppe bilden;
(10) Kombinationen der Ausführungsformen: (1)+(2), (1)+(3), (1)+(4)
(11) Kombinationen der Ausführungsformen: (1)+(5), (1)+(6), (1)+(7)
(12) Kombinationen der Ausführungsformen: (1)+(8),
(13) Kombinationen der Ausführungsformen: (1)+(9),
(14) Kombinationen der Ausführungsformen: (10)+(5), (10)+(6), (10)+(7)
(15) Kombinationen der Ausführungsformen: (10)+(8)
(16) Kombinationen der Ausführungsformen: (10)+(9)
(17) Kombinationen der Ausführungsformen: (14)+(8)
(18) Kombinationen der Ausführungsformen: (11)+(8)

### 3.2 Verbindungen der Formel II (Strukturtyp 2): (nicht erfindungsgemäß)

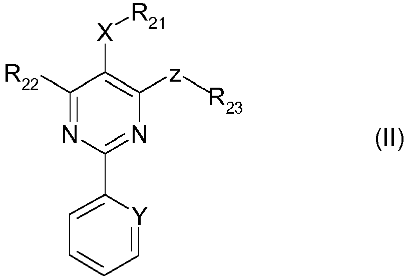

ausgewählt unter Verbindungen den folgenden Gruppen (1) bis (61):
(1) Verbindungen der Formel II, worin X für O steht;
(2) Verbindungen der Formel II, worin X für Methylen steht;
(3) Verbindungen der Formel II, worin Z für O steht;
(4) Verbindungen der Formel II, worin Z für NH steht;
(5) Verbindungen der Formel II, worin Y für N steht;
(6) Verbindungen der Formel II, worin Y für CH steht;
(7) Verbindungen der Formel II, worin R₂₁ für eine geradkettige oder verzweigte C₁-C₆-Alkylgruppe steht; oder für eine einkernige Aryl-, Arylalkyl- und Heteroarylgruppe steht, wobei die Heteroarylgruppe 1 oder 2 Ring-Heteroatome aufweist, die gleich oder verschieden sind und ausgewählt sind unter O, N und S;
(8) Verbindungen der Formel II, worin R₂₁ für eine geradkettige oder verzweigte C₁-C₆-Alkylgruppe, oder eine einkernige Arylgruppe steht,
(9) Verbindungen der Formel II, worin R₂₁ für Methyl, Ethyl, n-Propyl, i-Propyl, n-Butyl, sec. Butyl, i-Buty, tert-Butyl oder Phenyl, insbesondere Methyl steht;
(10) Verbindungen der Formel II, worin R₂₂ für H, eine geradkettige oder verzweigte C₁-C₆-Alkylgruppe steht; oder für eine einkernige Aryl-, Arylalkyl- und Heteroarylgruppe steht, wobei die Heteroarylgruppe 1 oder 2 Ring-Heteroatome aufweist, die gleich oder verschieden sind und ausgewählt sind unter O, N und S;
(11) Verbindungen der Formel II, worin R₂₂ für H, eine geradkettige oder verzweigte C₁-C₆-Alkylgruppe steht,
(12) Verbindungen der Formel II, worin R₂₂ für H, Methyl, Ethyl, n-Propyl, i-Propyl, n-Butyl, sec. Butyl, i-Buty, tert-Butyl oder Phenyl, insbesondere für H steht;
(13) Verbindungen der Formel II, worin R₂₃ ausgewählt ist untergeradkettigen oder verzweigten C₁-C₆-Alkylgruppen die gegebenenfalls 1 oder 2 gleichen oder verschiedenen Substituenten tragen, die ausgewählt sind unter NH₂, OH, SH , Halogen oder geradkettigen oder verzweigten C₁-C₆-Alkoxygruppen; C₃-C₇-Cycloalkylgruppen, wobei die Cycloalkylgruppe gegebenenfalls über eine C₁-C₄-Alkylengruppe an Z gebunden ist, und wobei gegebenenfalls 1, 2 oder 3 Ring-Kohlenstoffatome durch gleiche oder verschiedene Heteroatome, ausgewählt unter O, N und S ersetzt sein können; einkernige Aryl-, Arylalkyl- und Heteroarylgruppen, wobei die Heteroarylgruppen 1, oder 2 Ring-Heteroatome aufweisen, die gleich oder verschieden sind und ausgewählt sind unter O, N und S;
(14) Verbindungen der Formel II, worin R₂₃ ausgewählt ist unter geradkettigen oder verzweigten C₁-C₆-Alkylgruppen, die gegebenenfalls 1 Substituenten tragen, der ausgewählt ist unter OH, oder geradkettigen oder verzweigten C₁-C₃-Alkoxygruppen; C₃-C₇-Cycloalkylgruppen, wobei die Cycloalkylgruppe gegebenenfalls über eine C₁-C₄-Alkylengruppe an Z gebunden ist, und wobei gegebenenfalls 1 oder 2 Ring-Kohlenstoffatome durch gleiche oder verschiedene Heteroatome, ausgewählt unter O und N ersetzt sein können;
(15) Verbindungen der Formel II, worin R₂₃ ausgewählt ist unter: Methyl, Ethyl, n-Propyl, i-Propyl, n-Butyl, sec.-Butyl, i-Butyl, tert-Butyl, n-Pentyl (Amyl), 2-Pentyl (sec-Pentyl), 3-Pentyl, 2-Methylbutyl, 3-Methylbutyl (iso-Pentyl oder iso-Amyl), 3-Methylbut-2-yl, 2-Methylbut-2-yl; 2,2-Dimethylpropyl (Neopentyl); 1-Methoxy-prop-2-yl; 1-Methoxy-prop-3yl, 1-Hydroxy-prop-2-yl; 1-Hydroxy-prop-3yl; 1-Hydroxy-but-4-yl, 1-Hydroxy-but-3-yl, 1-Hydroxy-but-2-yl, 1-Hydroxy-but-1-yl, 2-Hydroxy-but-4-yl, 2-Hydroxy-but-3-yl, 2-Hydroxy-but-3-yl, 2-Hydroxy-but-4-yl; 1-Methoxy-but-4-yl, 1-Methoxy-but-3-yl, 1-Methoxy-but-2-yl, 1-Methoxy-but-1-yl, 2-Methoxy-but-4-yl, 2-Methoxy-but-3-yl, 2-Methoxy-but-3-yl, 2-Methoxy-but-4-yl, Cyclopropyl, Cyclopropyl-methyl, Cyclopropyl-ethyl, Cyclobutyl, Cyclobutyl-methyl, Cyclopentyl, Cyclopentyl-methyl, Cyclohexyl, Cyclohexyl-methyl; insbesondere Cyclobutyl, Cyclopentyl, i-Propyl, sec .Butyl, 3-Methylbut-2-yl, 1-Methoxy-prop-2-yl; 1-Hydroxy-but-3-yl, Cyclopropyl-methyl,
(16) Verbindungen der Formel II, worin R₂₄ und R₂₃ zusammen mit der Z-Gruppe, an die sie gebunden sind, einen 5-, oder 6-gliedrigen, gesättigten, oder ein- oder mehrfach ungesättigten heterocyclischen Ring bilden, der 1 oder 2 zusätzliche Ring-Heteroatome aufweist, die gleich oder verschieden sind und ausgewählt sind unter O, N und S;
(17) Verbindungen der Formel II, worin R₂₄ und R₂₃ zusammen mit der Z-Gruppe, an die sie gebunden sind, einen 6-gliedrigen, gesättigten oder ein- oder mehrfach ungesättigten heterocyclischen Ring bilden, der 1 zusätzliches Ring-Heteroatom aufweist, das ausgewählt ist unter O, N und S;
(18) Verbindungen der Formel II, worin R₂₄ und R₂₃ zusammen mit der Z-Gruppe, an die sie gebunden sind, für Pyridyl oder Morpholinyl stehen;
(19) Kombinationen der Ausführungsformen: (1)+(3), (1)+(4), (2)+(3), (2)+(4);
(20) Kombinationen der Ausführungsformen: (1)+(5), (1)+(6), (2)+(5), (2)+(6);
(21) Kombinationen der Ausführungsformen: (1)+(7), (1)+(8), (1)+(9), (2)+(7); (2)+(8), (2)+(9);
(22) Kombinationen der Ausführungsformen: (1)+(10), (1)+(11), (1)+ (12), (2)+(10), (2)+(11), (2)+(12);
(23) Kombinationen der Ausführungsformen (1)+(13), (1)+(14), (1)+(15), (2)+(13), (2)+(14), (2)+(15);
(24) Kombinationen der Ausführungsformen: (1)+(16), (1)+(17), (1)+(18), (2)+(16), (2)+(17), (2)+(18);
(25) Kombinationen der Ausführungsformen: (19)+(5), (19)+(6);
(26) Kombinationen der Ausführungsformen: (19)+(7), (19)+(8), (19)+(9);
(27) Kombinationen der Ausführungsformen: (19)+(10), (19)+(11), (19)+(12);
(28) Kombinationen der Ausführungsformen: (19)+(13), (19)+(14), (19)+(15);
(29) Kombinationen der Ausführungsformen: (25)+(7), (25)+(8), (25)+(9);
(30) Kombinationen der Ausführungsformen: (25)+(10), (25)+(11), (25)+(12);
(31) Kombinationen der Ausführungsformen: (25)+(13), (25)+(14), (25)+(15);
(32) Kombinationen der Ausführungsformen: (26)+(10), (26)+(11), (26)+(12);
(33) Kombinationen der Ausführungsformen: (26)+(13), (26)+(14), (26)+(15);
(34) Kombinationen der Ausführungsformen: (27)+(13), (27)+(14), (27)+(15);
(35) Kombinationen der Ausführungsformen: (29)+(10), (29)+(11), (29)+(12)
(36) Kombinationen der Ausführungsformen: (29)+(13), (29)+(14), (29)+(15);
(37) Kombinationen der Ausführungsformen: (30)+(13), (30)+(14), (30)+(15);
(38) Kombinationen der Ausführungsformen: (35)+(13), (35)+(14), (35)+(15);
(39) Kombinationen der Ausführungsformen: (20)+(7), (20)+(8), (20)+(9);
(40) Kombinationen der Ausführungsformen: (20)+(10), (20)+(11), (20)+(12);
(41) Kombinationen der Ausführungsformen: (20)+(13), (20)+(14), (20)+(15);
(42) Kombinationen der Ausführungsformen: (20)+(16), (20)+(17), (20)+(18);
(43) Kombinationen der Ausführungsformen: (39)+(10), (39)+(11), (39)+(12);
(44) Kombinationen der Ausführungsformen: (39)+(13), (39)+(14), (39)+(15);
(45) Kombinationen der Ausführungsformen: (39)+(16), (39)+(17), (39)+(18);
(46) Kombinationen der Ausführungsformen: (40)+(13), (40)+(14), (40)+(15);
(47) Kombinationen der Ausführungsformen: (40)+(16), (40)+(17), (40)+(18);
(48) Kombinationen der Ausführungsformen: (43)+(13), (43)+(14), (43)+(15);
(49) Kombinationen der Ausführungsformen: (43)+(16), (43)+(17), (43)+(18);
(50) Kombinationen der Ausführungsformen: (21)+(10), (21)+(11), (21)+(12);
(51) Kombinationen der Ausführungsformen: (21)+(13), (21)+(14), (21)+(15);
(52) Kombinationen der Ausführungsformen: (21)+(16), (21)+(17), (21)+(18);
(53) Kombinationen der Ausführungsformen: (50)+(13), (50)+(14), (50)+(15);
(54) Kombinationen der Ausführungsformen: (50)+(16), (50)+(17), (50)+(18);
(55) Kombinationen der Ausführungsformen: (22)+(13), (22)+(14), (22)+(15);
(56) Kombinationen der Ausführungsformen: (22)+(16), (22)+(17), (22)+(18);
(57) Stereoisomere Formen von Verbindungen der Formel (II), wenn Z für NH steht;
(58) Säureadditionssalze von Verbindungen der Formel II, insbesondere der Ausführungsformen (1) bis (57), wenn Z ein protonierbares N-Atom umfasst;
(59) Säureadditionssalze von Verbindungen der Formel II, insbesondere der Ausführungsformen (1) bis (57), wenn Z ein protonierbartes N-Atom umfasst, mit organischen Mono-oder Polycarbonsäuren,
(60) Säureadditionssalze von Verbindungen der Formel II, insbesondere der Ausführungsformen (1) bis (57), wenn Z ein protonierbartes N-Atom umfasst, mit organischen Mono-oder Polycarbonsäuren, wobei die Carbonsäure ausgewählt ist unter gesättigten oder ein- oder mehrfach ungesättigten C₁-C₃₀-Monocarbonsäuren, gesättigten oder ein- oder mehrfach ungesättigten C₃-C₁₀-Di oder Tricarbonsäuren, wobei die Carbonsäure ein oder mehrfach mit Hydroxygruppen substituiert sein kann;
(61) Säureadditionssalze von Verbindungen der Formel II, insbesondere der Ausführungsformen (1) bis (57), wenn Z ein protonierbartes N-Atom umfasst, mit Carbonsäuren, ausgewählt unter Fumarsäure, Zitronensäure, Äpfelsäure, Weinsäure, Bernsteinsäure, Laurin-, Myristin-, Palmitin- oder Stearinsäure und deren stereoisomeren Formen.

### 3.3 Verbindungen der allgemeinen Formel III (Strukturtyp 3):

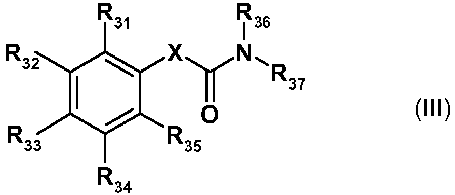

ausgewählt unter Verbindungen der folgenden Gruppen (1) bis (63):
(1) Verbindungen der Formel III, worin R₃₄ für H steht;
(2) Verbindungen der Formel III, worin R₃₅ für H oder Halogen steht;
(3) Verbindungen der Formel III, worin R₃₁ für H, Halogen, eine geradkettige oder verzweigte C₁-C₆-Alkylgruppe, die gegebenenfalls 1, 2, 3 oder 4 gleiche oder verschiedene Substituenten trägt, die ausgewählt sind unter NH₂, OH, SH , Halogen oder geradkettigen oder verzweigten C₁-C₆-Alkoxygruppen; oder eine geradkettige oder verzweigte C₁-C₆-Alkoxygruppe steht, die gegebenenfalls 1, 2, 3 oder 4 gleiche oder verschiedene Substituenten trägt, die ausgewählt sind unter NH₂, OH, SH , Halogen oder C₁-C₆-Alkoxygruppen;
(4) Verbindungen der Formel III, worin R₃₁ für H, Halogen, eine geradkettige oder verzweigte C₁-C₆-Alkylgruppe; oder eine geradkettige oder verzweigte C₁-C₆-Alkoxygruppe steht;
(5) Verbindungen der Formel III, worin R₃₁ für H, Methyl, Ethyl, Methoxy, Ethoxy, oder Halogen, insbesondere H, Fluor, Chlor, Brom, Methyl oder Methoxy steht;
(6) Verbindungen der Formel III, worin die Reste R₃₂ und R₃₃ gleich oder verschieden sind und ausgewählt sind unter H; Halogen, geradkettigen oder verzweigten C₁-C₆-Alkylgruppen, die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen; oder geradkettigen oder verzweigten C₁-C₆-Alkoxygruppen steht, die gegebenenfalls 1, 2, 3 oder 4 gleiche oder verschiedene Substituenten tragen, die ausgewählt sind unter NH₂, OH, SH, Halogen oder C₁-C₆-Alkoxygruppen;
(7) Verbindungen der Formel III, worin die Reste R₃₂ und R₃₃ gleich oder verschieden sind und ausgewählt sind unter H, Halogen, geradkettigen oder verzweigten C₁-C₆-Alkylgruppen; oder geradkettigen oder verzweigten C₁-C₆-Alkoxygruppen;
(8) Verbindungen der Formel III, worin die Reste R₃₂ und R₃₃ gleich oder verschieden sind und ausgewählt sind unter H, Halogen, Methyl oder Methoxy
(9) Verbindungen der Formel III, worin die benachbarten Reste R₃₂ und R₃₃, zusammen mit den Kohlenstoffatomen, an die sie gebunden sind, einen 4-, 5-, 6- oder 7-gliedrigen, ein- oder mehrfach ungesättigten heterocyclischen Ring bilden, der gegebenenfalls 1, 2, 3, 4 oder 5 gleiche oder verschiedene Substituenten trägt, die ausgewählt sind unter geradkettigen oder verzweigten C₁-C₆-Alkylgruppen, und der 1, 2 oder 3 Ring-Heteroatome aufweist, die gleich oder verschieden sind und ausgewählt sind unter O, N und S;
(10) Verbindungen der Formel III, worin die benachbarten Reste R₃₂ und R₃₃, zusammen mit den Kohlenstoffatomen, an die sie gebunden sind, einen 5-oder 6-gliedrigen, einfach ungesättigten heterocyclischen Ring bilden, der 1 oder 2 Ring-Heteroatome aufweist, die gleich oder verschieden sind und ausgewählt sind unter O, N und S;
(11) Verbindungen der Formel III, worin die benachbarten Reste R₃₂ und R₃₃, zusammen eine der Gruppen -O-CH₂O- oder -O-CH₂-CH₂-O-, bilden;
(12) Verbindungen der Formel III, worin die Reste R₃₆ und R₃₇ gleich oder verschieden sind und ausgewählt sind unter geradkettigen oder verzweigten C₁-C₆-Alkylgruppen die gegebenenfalls 1 oder 2 gleiche oder verschiedene Substituenten tragen, die ausgewählt sind unter NH₂, OH, SH , Halogen oder geradkettigen oder verzweigten C₁-C₆-Alkoxygruppen; einkernigen Aryl-, Arylalkyl- und Heteroarylgruppen, die gegebenenfalls 1 oder 2 gleiche oder verschiedene Substituenten tragen, die ausgewählt sind unter NH₂, OH, SH, Halogen geradkettigen oder verzweigten C₁-C₆-Alkylgruppen und geradkettigen oder verzweigten C₁-C₆-Alkyloxygruppen; wobei die Heteroarylgruppen 1, 2 oder 3 Ring-Heteroatome aufweisen, die gleich oder verschieden sind und ausgewählt sind unter O, N und S; und C₃-C₇-Cycloalkylgruppen, die gegebenenfalls 1 oder 2 gleiche oder verschiedene Substituenten tragen, die ausgewählt sind unter NH₂, OH, SH, Halogen geradkettigen oder verzweigten C₁-C₆-Alkylgruppen, oder geradkettigen oder verzweigten C₁-C₆-Alkoxygruppen; wobei die Cycloalkylgruppe gegebenenfalls über eine C₁-C₄-Alkylengruppe gebunden ist und wobei gegebenenfalls 1, 2 oder 3 Ring-Kohlenstoffatome durch gleiche oder verschiedene Heteroatome, ausgewählt unter O, N und S ersetzt sein können;
(13) Verbindungen der Formel III, worin die Reste R₃₆ und R₃₇ gleich oder verschieden sind und ausgewählt sind unter geradkettigen oder verzweigten C₁-C₆-Alkylgruppen; einkernigen Aryl-, Arylalkyl- und Heteroarylgruppen, die gegebenenfalls einen Substituenten tragen, der ausgewählt ist unter NH₂, OH, SH, Halogen, geradkettigen C₁-C₆-Alkylgruppen und geradkettigen C₁-C₆-Alkyloxygruppen; wobei die Heteroarylgruppen 1, 2 oder 3 Ring-Heteroatome aufweisen, die gleich oder verschieden sind und ausgewählt sind unter O, N und S; und C₃-C₇-Cycloalkylgruppen, die gegebenenfalls 1 oder 2 gleiche oder verschiedene Substituenten tragen, die ausgewählt sind unter NH₂, OH, SH, Halogen geradkettigen oder verzweigten C₁-C₆-Alkylgruppen, oder geradkettigen oder verzweigten C₁-C₆-Alkoxygruppen; wobei die Cycloalkylgruppe gegebenenfalls über eine C₁-C₄-Alkylengruppe gebunden ist; und wobei gegebenenfalls 1 oder 2 Ring-Kohlenstoffatome durch gleiche oder verschiedene Heteroatome, ausgewählt unter O und N ersetzt sein können;
(14) Verbindungen der Formel III, worin die Reste R₃₆ und R₃₇ gleich oder verschieden sind und ausgewählt sind unter
   Methyl, Ethyl, n-Prop-1-yl, n-Prop-2-yl, n-Butyl, sec.-Butyl, i-Butyl, tert-Butyln-Pentyl (Amyl), 2-Pentyl (sec-Pentyl), 3-Pentyl, 2-Methylbutyl, 3-Methylbutyl (iso-Pentyl oder iso-Amyl), 3-Methylbut-2-yl, 2-Methylbut-2-yl; 2,2-Dimethylpropyl (Neopentyl); Cyclopropyl, Cyclopropyl-methyl, Cyclopropyl-ethyl, Cyclobutyl, Cyclobutyl-methyl, Cyclopentyl, Cyclopentyl-methyl, Cyclohexyl, Cyclohexyl-methyl; Cycloheptyl; Benzyl; Phenyl; 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl; 2-Fluorbenzyl, 3-Fluorbenzyl, 4-Fluorbenzyl, 2-Chlorbenzyl, 3-Chlorbenzyl, 4-Chlorbenzyl, 2-Brombenzyl, 3-Brombenzyl, 4-Brombenzyl; 2-Methylphenyl, 3-Methylphenyl, 4-Methylphenyl, 2-Methylbenzyl, 3-Methylbenzyl, 4-Methylbenzyl;
   2-Pyridyl, 3-Pyridyl, 4-Pyridyl, Thiazolyl, Oxazolyl, Pyrazolyl, Furanyl, Morpholinyl, Pyranyl,
   insbesondere Cyclohexyl, Cyclopropylmethyl, Phenyl, Benzyl, 4-Chlorphenyl, 2-Methylphenyl, 2-Pyridyl, 2-Thiazolyl, Methyl, Ethyl, n-Propyl, i-Propyl, n-Butyl, i-Butyl;
(15) Verbindungen der Formel III, worin X ausgewählt ist unter -C₁-C₄-Alkylengruppen; -C₂-C₄-Alkenylengruppen, sowie -O-C₁-C₄-Alkylengruppen, oder für eine chemische Einfachbindung steht;
(16) Verbindungen der Formel III, worin X ausgewählt ist unter -CH₂-,-CH₂-CH₂-, -CH=CH-; -O-CH₂-; - CH₂-O-; und einer chemischen Bindung.
(17) Kombinationen der Ausführungsformen: (1)+(2);
(18) Kombinationen der Ausführungsformen: (1)+(3), (1)+(4), (1)+5);
(19) Kombinationen der Ausführungsformen: (1)+(6), (1)+(7), (1)+(8);
(20) Kombinationen der Ausführungsformen:(1)+(9), (1)+(10), (1)+(11);
(21) Kombinationen der Ausführungsformen: (1)+(12), (1)+(13), (1)+(14);
(22) Kombinationen der Ausführungsformen: (1)+(15), (1)+(16);
(23) Kombinationen der Ausführungsformen: (17)+(3), (17)+(4), (17)+(5)
(24) Kombinationen der Ausführungsformen: (17)+(6), (17)+(7), (17)+(8);
(25) Kombinationen der Ausführungsformen: (17)+(9), (17)+(10), (17)+(11);
(26) Kombinationen der Ausführungsformen: (17)+(12), (17)+(13), (17)+(14);
(27) Kombinationen der Ausführungsformen: (17)+(15), (17)+(16);
(28) Kombinationen der Ausführungsformen: (23)+(6), (23)+(7), (23)+(8);
(29) Kombinationen der Ausführungsformen: (23)+(9), (23)+(10), (23)+(11);
(30) Kombinationen der Ausführungsformen: (23)+(12), (23)+(13), (23)+(14);
(31) Kombinationen der Ausführungsformen: (23)+(15), (23)+(16);
(32) Kombinationen der Ausführungsformen: (28)+(12), (28)+(13), (28)+(14);
(33) Kombinationen der Ausführungsformen: (28)+(15), (28)+(16);
(34) Kombinationen der Ausführungsformen: (32)+(15), (32)+(16);
(35) Kombinationen der Ausführungsformen: (29)+(12), (29)+(13), (29)+(14);
(36) Kombinationen der Ausführungsformen: (29)+(15), (29)+(16);
(37) Kombinationen der Ausführungsformen: (35)+(15), (35)+(16);
(38) Kombinationen der Ausführungsformen: (30)+(15), (30)+(16);
(39) Kombinationen der Ausführungsformen: (24)+(12), (24)+(13), (24)+(14);
(40) Kombinationen der Ausführungsformen: (24)+(15), (24)+(16);
(41) Kombinationen der Ausführungsformen: (39)+(15), (39)+(16);
(42) Kombinationen der Ausführungsformen: (25)+(12), (25)+(13), (25)+(14);
(43) Kombinationen der Ausführungsformen: (25)+(15), (25)+(16);
(44) Kombinationen der Ausführungsformen: (42)+(15), (42)+(16);
(45) Kombinationen der Ausführungsformen: (26)+(15), (26)+(16);
(46) Kombinationen der Ausführungsformen: (18)+(6), (18)+(7), (18)+(8);
(47) Kombinationen der Ausführungsformen: (18)+(9), (18)+(10), (18)+(11);
(48) Kombinationen der Ausführungsformen: (18)+(12), (18)+(13), (18)+(14);
(49) Kombinationen der Ausführungsformen: (18)+(15), (18)+(16);
(50) Kombinationen der Ausführungsformen: (46)+(12), (46)+(13), (46)+(14);
(51) Kombinationen der Ausführungsformen: (46)+(15), (46)+(16);
(52) Kombinationen der Ausführungsformen:; (50)+(15), (50)+(16)
(53) Kombinationen der Ausführungsformen: (47)+(12), (47)+(13), (47)+(14);
(54) Kombinationen der Ausführungsformen: (47)+(15), (47)+(16);
(55) Kombinationen der Ausführungsformen: (53)+(15), (53)+(16);
(56) Kombinationen der Ausführungsformen: (48)+(15), (48)+(16);
(57) Kombinationen der Ausführungsformen: (19)+(12), (19)+(13), (19)+(14);
(58) Kombinationen der Ausführungsformen: (19)+(15), (19)+(16);
(59) Kombinationen der Ausführungsformen: (57)+(15), (57)+(16);
(60) Kombinationen der Ausführungsformen: (20)+(12), (20)+(13), (20)+(14);
(61) Kombinationen der Ausführungsformen: (20)+(15), (20)+(16);
(62) Kombinationen der Ausführungsformen: (60)+(15), (60)+(16);
(63) Kombinationen der Ausführungsformen: (21)+(15), (21)+(16);

### 4. Weitere Ausgestaltungen der erfindungsgemäßen Mittel (allgemeiner Teil):

### 4.1 Allgemeine Angaben zu Anwendungsgebieten und Formulierungen der ggf. erfindungsgemäßen Wirkstoffe (erfindungsgemäß, soweit sie unter die Ansprüche fallen)

Die Wirkstoffe besitzen ein breites Anwendungsgebiet in der Humankosmetik- und -pflege, insbesondere der Haut- und Haarpflege, sind aber auch pharmakologisch einsetzbar, sowie in Nahrungsmitten und Textilprodukten, aber auch als Repellents und als Bestandteil von insektizid wirkenden Zusammensetzungen verwendbar.

Bei den Mitteln kann es sich insbesondere um hautkosmetische, haarkosmetische, dermatologische, hygienische oder pharmazeutische Mittel handeln. Insbesondere werden die kühlend wirkenden, oben beschriebenen Wirkstoffe für die Haut-, und/oder Haarkosmetik oder als Mundpflegemittel angewendet. Sofern nachfolgend von "oben beschriebenen" Wirkstoffen oder "oben beschriebenen" Kühlwirkstoffen die Rede ist, bezieht sich dieser Hinweis auf die Verbindungen der Strukturtypen 1, 2 und 3, so wie diese oben definiert sind.

Die haar- oder hautpflegenden Zubereitungen liegen insbesondere in Form einer Emulsion, einer Dispersion, einer Suspension, in Form einer wässrigen Tensidzubereitung, einer Milch, einer Lotion, einer Creme, eines Balsams, einer Salbe, eines Gels, eines Granulats, eines Puders, eines Stiftpräparates, wie z. B. eines Lippenstifts, eines Schaums, eines Aerosols oder eines Sprays vor. Solche Formulierungen sind gut geeignet für topische Zubereitungen. Als Emulsionen kommen Öl-in-Wasser-Emulsionen und Wasser-in-ÖI-Emulsionen oder Mikroemulsionen in Frage.

Im Regelfall wird die haar- oder hautkosmetische Zubereitung zur Applikation auf der Haut (topisch) oder dem Haar verwendet. Unter "topischen Zubereitungen" sind dabei solche Zubereitungen zu verstehen, die dazu geeignet sind, die Wirkstoffe in feiner Verteilung, wie z.B. in einer durch die Haut resorbierbaren Form auf die Haut aufzubringen. Hierfür eignen sich z. B. wässrige und wässrig-alkoholische Lösungen, Sprays, Schäume, Schaumaerosole, Salben, wässrige Gele, Emulsionen vom O/W- oder W/O-Typ, Mikroemulsionen oder kosmetische Stiftpräparate.

Nach einer Ausführungsform des kosmetischen Mittels enthält dieses einen Träger. Bevorzugt als Träger ist Wasser, ein Gas, eine Wasser-basierte Flüssigkeit, ein Öl, ein Gel, eine Emulsion oder Mikroemulsion, eine Dispersion oder eine Mischung davon. Die genannten Träger zeigen eine gute Hautverträglichkeit. Besonders vorteilhaft für topische Zubereitungen sind wässrige Gele, Emulsionen oder Mikroemulsionen.

Die Lehre umfasst auch die Verwendung der hierin beschriebenen Wirkstoffe in pharmazeutischen Mitteln zur Behandlung eines Individuums, vorzugsweise eines Säugers, insbesondere eines Menschen, Nutz- oder Haustieres. Die Wirkstoffe werden dazu in Form von pharmazeutischen Zusammensetzungen verabreicht, die einen pharmazeutisch verträglichen Exzipienten mit wenigstens einem erfindungsgemäßen Wirkstoff und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Inhibitoren verwendet werden. Ferner können auch Liposomen, Mikrosphären oder Polymermatrizes zur Anwendung kommen.

Bei der Herstellung von Zusammensetzungen werden oben beschriebene Wirkstoffe gewöhnlich mit einem Exzipienten vermischt oder verdünnt. Exzipienten können feste, halbfeste oder flüssige Materialien sein, die als Vehikel, Träger oder Medium für den Wirkstoff dienen. Der Wirkstoffgehalt (eines oder mehrerer gleichzeitig enthaltener erfindungsgemäßer Wirkstoffe) kann dabei in einem weiten Bereich variieren und liegt etwa, jeweils bezogen auf das Gesamtgewicht der Zusammensetzung, im ppm-Bereich von etwa 0,05 ppm - < 0,1 ppm und 0,1 bis 1000 ppm (d.h. 0,00001 bis 0,1 Gew.-%), wie z.B. 1 bis 800 ppm oder 100 bis 500 ppm oder im Bereich von 0,1 bis 50, 1 bis 30 oder 2 bis 10 Gew.-%.

Zu geeigneten Exzipienten gehören beispielsweise Lactose, Dextrose, Sucrose, Sorbitol, Mannitol, Stärken, Akaziengummi, Calciumphosphat, Alginate, Traganth, Gelatine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, oder Hager's Handbuch der Pharmazeutischen Praxis, Springer Verlag, Heidelberg dargestellt ist.

Die Mittel können neben üblichen Zusatz- oder Hilfsstoffen zusätzlich kosmetisch und/oder dermatologisch und/oder pharmakologisch aktive Wirkstoffe enthalten.

Als nicht-limitierende Beispiele geeigneter weiterer Wirkstoffe sind zu nennen:
Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, keratinhärtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe, verzweigte Fettsäuren, wie 18-Methyleicosansäure, und Mischungen davon.

Künstlich hautbräunende Wirkstoffe, die geeignet sind, die Haut ohne natürliche oder künstliche Bestrahlung mit UV-Strahlen zu bräunen; dies sind z. B. Dihydroxyaceton, Alloxan und Walnussschalenextrakt. Geeignete keratinhärtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat etc.

Antimikrobielle Wirkstoffe, die eingesetzt werden, um Mikroorganismen zu zerstören bzw. ihr Wachstum zu hemmen. Sie dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure etc. Derartige desodorierend wirkende Stoffe sind z. B. Zinkricinoleat, Triclosan, Undecylensäurealkylolamide, Citronensäuretriethylester, Chlorhexidin etc.

Geeignete Hilfs- und Zusatzstoffe für die Herstellung von haarkosmetischen oder hautkosmetischen Zubereitungen sind dem Fachmann geläufig und können aus Handbüchern der Kosmetik, beispielsweise Schrader, Grundlagen und Rezepturen der Kosmetika, Hüthig Verlag, Heidelberg, 1989, ISBN 3-7785-1491-1, entnommen werden. Die Hilfs- und Zusatzstoffe sind bevorzugt kosmetisch und/oder pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidanzien, Antireizstoffe, Chelatbildner, Emulsionsstabilisatoren, Filmbildner, Gelbildner, Geruchsmaskierungsmittel, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilanzien, Treibmittel, Trocknungsmittel, Trübungsmittel, Verdickungsmittel, Wachse, Weichmacher, Weißöl. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Weitere geeignete Zusatzstoffe sind ausgewählt unter Parfümölen, Haarpolymeren, Haar- und Hautkonditionern, Pfropfpolymeren, wasserlöslichen oder dispergierbaren silikonhaltigen Polymeren, Lichtschutzmitteln, Bleichmitteln, Pflegemitteln, Färbemitteln, Tönungsmitteln, Bräunungsmitteln, Farbstoffen, Konsistenzgebern, Feuchthaltemitteln, Rückfettern, Collagen, Eiweißhydrolysaten, Lipiden, Antioxidanzien, Entschäumern, Antistatika, Emollienzien, Weichmachern, Peroxidzersetzern.

Als Beispiele geeigneter Hilfs- und Zusatzstoffe sind zu nennen:
(1) Antioxidanzien, ausgewählt unter Aminosäuren (z.B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z. B. β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z. B. Buthioninsulfoximine, Homocysteinsulfoximine, Buthioninsulfone, Penta-, Hexa-, Heptathioninsulfoximin), insbesondere in sehr geringen, verträglichen Dosierungen (z. B. pmol bis µmol/kg Bereich), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und dessen Derivaten (z. B. Natriumascorbat, Ascorbylpalmitat, Mg-Ascorbylphosphat, Ascorbylacetat), Tocopherol und Derivate (z. B. Vitamin-E-Acetat, Tocotrienol), Vitamin A und Derivate (Vitamin-A-Palmitat) sowie Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, ZnSO₄), Selen und dessen Derivaten (z. B. Selenmethionin), Stilbenen und deren Derivaten (z. B. Stilbenoxid, Trans-Stilbenoxid).
(2) Peroxidzersetzer, d. h. Verbindungen, die in der Lage sind Peroxide, besonders bevorzugt Lipidperoxide, zu zersetzen. Darunter sind organische Substanzen zu verstehen, wie z. B. Pyridin-2-thiol-3-carbonsäure, 2-Methoxy-pyrimidinol-carbonsäuren, 2-Methoxypyridincarbonsäuren, 2-Dimethylamino-pyrimidinolcarbonsäuren, 2-Dimethylaminopyridincarbonsäuren.
(3) Verdickungsmittel, wie vernetzte Polyacrylsäuren und deren Derivate, Polysaccharide und deren Derivate, wie Xanthangummi, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon. Insbesondere werden nichtionische Verdicker eingesetzt.
(4) Konservierungsmittel, die mit ihrer E-Nummer nachfolgend aufgeführt sind

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind in der Kosmetik gebräuchliche Konservierungsmittel oder Konservierungshilfsstoffe geeignet, wie Dibromdicyanobutan (2-Brom-2-brommethylglutarodinitril), 3-Jod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Formaldehydabspalter.
Ferner sind Phenylhydroxyalkylether, insbesondere die unter der Bezeichnung Phenoxyethanol bekannte Verbindung, aufgrund ihrer bakteriziden und fungiziden Wirkungen auf eine Anzahl von Mikroorganismen als Konservierungsmittel geeignet.
Auch andere keimhemmende Mittel sind ebenfalls geeignet, in die Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden. Ebenso können auch antimikrobielle Polypeptide eingesetzt werden.
(5) Lichtfilterwirkstoffe, die UV-Strahlen im UV-B- und/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl, und Mischungen davon. Geeignet sind weiterhin p-Aminobenzoesäureester, Zimtsäureester, Benzophenone, Kampferderivate sowie UV-Strahlen abhaltende Pigmente, wie Titandioxid, Talkum und Zinkoxid.
Als UV-Filtersubstanzen kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethyl-ester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethyl-1-[(trimethylsilyl)oxy]disiloxanyl]propyl]phenol | 155633-54-8 |
| 38 | 1,1-[(2,2'-Dimethylpropoxy)carbonyl]-4,4-diphenyl-1,3-butadien | 363602-15-7 |

Die kosmetischen und dermatologischen Zubereitungen können vorteilhafterweise außerdem UV-Strahlen abhaltende anorganische Pigmente auf Basis von Metalloxiden und/oder anderen in Wasser schwer löslichen oder unlöslichen Metallverbindungen, ausgewählt aus der Gruppe der Oxide des Zinks (ZnO), Titan (TiO₂), Eisens (z. B. Fe₂O₃), Zirkoniums (ZrO₂), Siliciums (SiO₂), Mangans (z.B. MnO), Aluminiums (Al₂O₃), Cers (z. B. Ce₂O₃), Mischoxiden der entsprechenden Metalle sowie Abmischungen aus solchen Oxiden enthalten.
Die anorganischen Pigmente können dabei in gecoateter Form vorliegen, d. h. dass sie oberflächlich behandelt sind. Diese Oberflächenbehandlung kann beispielsweise darin bestehen, dass die Pigmente nach an sich bekannter Weise, wie in DE-A-33 14 742 beschrieben, mit einer dünnen hydrophoben Schicht versehen sind.
(6) Repellentwirkstoffe, d. h. Verbindungen, die in der Lage sind, bestimmte Tiere, insbesondere Insekten, vom Menschen abzuhalten oder zu vertreiben. Dazu gehört z. B. 2-Ethyl-1, 3-hexandiol, N, N-Diethyl-m-toluamid etc.
(7) Geeignete hyperemisierend wirkende Stoffe, welche die Durchblutung der Haut anregen, sind z. B. ätherische Öle, wie Latschenkieferextrakt, Lavendelextrakt, Rosmarinextrakt, Wacholderbeerextrakt, Rosskastanienextrakt, Birkenblätterextrakt, Heublumenextrakt, Ethylacetat, Kampfer, Menthol, Pfefferminzöl, Rosmarinextrakt, Eukalyptusöl etc.
(8) Geeignete keratolytisch und keratoplastisch wirkende Stoffe sind z. B. Salicylsäure, Kalziumthioglykolat, Thioglykolsäure und ihre Salze, Schwefel etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion etc.
(9) Geeignete Antiphlogistika, die Hautreizungen entgegenwirken, sind z. B. Allantoin, Bisabolol, Dragosantol, Kamillenextrakt, Panthenol etc.
(10) Kosmetisch oder pharmazeutisch akzeptable Polymere, wie kationische, amphotere und neutrale Polymere.

Geeignete Polymere sind z. B. kationische Polymere mit der Bezeichnung Polyquaternium nach INCI, z. B. Copolymere aus Vinylpyrrolidon/N-Vinylimidazoliumsalzen (Luviquat FC, Luviquat HM, Luviquat MS, Luviquat Care), Copolymere aus N-Vinylpyrrolidon/Dimethylaminoethylmethacrylat, quaternisiert mit Diethylsulfat (Luviquat PQ 11), Copolymere aus N-Vinylcaprolactam/N-Vinylpyrrolidon/N-Vinylimidazoliumsalzen (Luviquat E Hold), kationische Cellulosederivate (Polyquaternium-4 und - 10), Acrylamidocopolymere (Polyquaternium-7) und Chitosan.

Geeignete kationische (quaternisierte) Polymere sind auch Merquat (Polymer auf Basis von Dimethyldiallylammoniumchlorid), Gafquat (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar-Marken der Firma Rhodia.

Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und deren Derivate. Dazu zählt beispielsweise Luviflex ® Swing (teilverseiftes Copolymerisat von Polyvinylacetat und Polyethylenglykol, Firma BASF).

Geeignete Polymere sind auch nichtionische, wasserlösliche bzw. wasserdispergierbare Polymere oder Oligomere, wie Polyvinylcaprolactam, z. B. Luviskol ® Plus (BASF), oder Polyvinylpyrrolidon und deren Copolymere, insbesondere mit Vinylestern, wie Vinylacetat, z. B. Luviskol ® VA 37 (BASF), Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer (National Starch) erhältlichen Octylacrylamid / Methylmethacrylat / tert.-Butylaminoethylmethacrylat-Hydroxypropylmethacrylat-Copolymere sowie zwitterionische Polymere, wie sie beispielsweise in den deutschen Patentanmeldungen DE39 29 973, DE 21 50 557, DE28 17 369 und DE 3708 451 offenbart sind. Acrylamidopropyltrimethylammoniumchlorid/Acrylsäure- bzw. Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette (AMERCHOL) im Handel erhältlich sind, und Copolymere aus Hydroxyethylmethacrylat, Methylmethacrylat, N, N-Dimethylaminoethylmethacrylat und Acrylsäure (Jordapon (D)).

Geeignete Polymere sind auch nichtionische, siloxanhaltige, wasserlösliche oder dispergierbare Polymere, z. B. Polyethersiloxane, wie Tegopren ® (Firma Goldschmidt) oder Besi (Firma Wacker).

Im Folgenden werden einzelne besondere Anwendungsformen erfindungsgemäßer Wirkstoffe beispielhaft näher erläutert.

### 4.2 Kühlende Haut- und Haarpflegemittel (erfindungsgemäß, sofern sie unter die Ansprüche fallen)

Nach einer bevorzugten Ausführungsform handelt es sich bei den Mitteln um ein kühlendes Haut- oder Haarpflege- oder -reinigungsmittel, sofern es unter die Ansprüche fällt, ist es erfindungsgemäß.

Bevorzugte Haut- oder Haarreinigungsmittel sind Seifen von flüssiger bis gelförmiger Konsistenz, wie Transparentseifen, Luxusseifen, Deoseifen, Cremeseifen, Babyseifen, Hautschutzseifen, abrasive Seifen und Syndets, pastose Seifen, Schmierseifen und Waschpasten, Peelingseifen, Feuchtigkeitstücher, flüssige Wasch-, Dusch- und Badepräparate, wie Waschlotionen, Duschbäder und -gele, Schaumbäder, Ölbäder und Scrub-Präparate, Rasierschäume, - lotionen und -cremes.

Nach einer weiteren bevorzugten Ausführungsform handelt es sich bei den Mitteln um ein Duschgel, eine Shampoo-Formulierung oder ein Badepräparat. Solche Formulierungen enthalten wenigstens einen oben beschriebenenWirkstoff sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Grundsätzlich kann der Wirkstoffgehalt über einen weiten Bereich, wie z.B. 0,00001 bis 50 Gew.-%, insbesondere 0,001 bis 10 Gew.-% oder 0,005 bis 1 Gew.-% variieren.

### i) Spezielle Ausgestaltungen für Mittel zur Auftragung auf die Haut:

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die dermatologischen Mittel verwendet werden in Nose-Strips zur Porenreinigung, in Antiaknemitteln, Repellents, Rasiermitteln, After- und Pre-Shave-Pflegemitteln, After-Sun-Pflegemitteln, Haarentfernungsmitteln, Haarfärbemitteln, Intimpflegemitteln, Fußpflegemitteln sowie in der Babypflege.

Bei den Hautpflegemitteln handelt es sich insbesondere um W/O- oder O/W-Hautcremes, Tag-und Nachtcremes, Augencremes, Gesichtscremes, Antifaltencremes, Sonnenschutzcremes, Feuchthaltecremes, Bleichcremes, Selbstbräunungscremes, Vitamincremes, Hautlotionen, Pflegelotionen und Feuchthaltelotionen.

Hautkosmetische und dermatologische Mittel enthalten insbesondere wenigstens einen oben beschriebenen Wirkstoff in einem Anteil von etwa 0,0001 bis 50 Gew.-%, wie z. B. 0,001 bis 10 Gew.-%, insbesondere 0,005 bis 0,1 Gew.-%, bezogen auf das Gesamtgewicht des Mittels.

Je nach Anwendungsgebiet können die Hautkosmetikmittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den oben beschriebenen Wirkstoffen und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Enzyme, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Silikonöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Die Herstellung der kosmetischen oder dermatologischen Zubereitungen erfolgt nach üblichen, dem Fachmann bekannten Verfahren.

Zur Herstellung der dermatologischen Mittel können die Wirkstoffe mit einem geeigneten Hilfsstoff (Exzipient) vermischt oder verdünnt werden. Exzipienten können feste, halbfeste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Weiterhin sind die Polymere und Dispersionen geeignet, als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

Bevorzugt liegen die kosmetischen und dermatologischen Mittel in Form von Emulsionen insbesondere als Wasser-in-ÖI (W/O)- oder Öl-in-Wasser (O/W)-Emulsionen vor. Es ist aber auch möglich, andere Formulierungsarten zu wählen, beispielsweise, Gele, Öle, Oleogele, multiple Emulsionen, beispielsweise in Form von W/O/W- oder O/W/O-Emulsionen, wasserfreie Salben bzw. Salbengrundlagen usw. Auch emulgatorfreie Formulierungen wie Hydrodispersionen, Hydrogele oder eine Pickering-Emulsion sind vorteilhafte Ausführungsformen.

Die Herstellung von Emulsionen erfolgt nach bekannten Methoden. Die Emulsionen enthalten neben wenigstens einem oben beschriebenen Wirkstoff in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion als W/O-Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein Polyelektrolytkomplex eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Rizinusöl, Sesamöl, Olivenöl, Jojobaöl, Karite-Öl, Hoplostethus-Öl, mineralische Öle, deren Destillationsbeginn unter Atmosphärendruck bei ca. 250°C und deren Destillationsendpunkt bei 410°C liegt, wie z. B. Vaselinöl, Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Silikonöle, wie Dimethylpolysiloxan, Methylphenylpolysiloxan und das Silikonglykol-Copolymer , Fettsäuren und Fettalkohole enthalten.

Neben den oben beschriebenen Wirkstoffen können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den Mitteln um ein Duschgel, eine Shampooformulierung oder ein Badepräparat.

Solche Formulierungen enthalten wenigstens einen oben beschriebenen Wirkstoff sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidanzien sowie Verdickern/Gelbildnern, Hautkonditioniermitteln und Feuchthaltemitteln.

Diese Formulierungen enthalten insbesondere 2 bis 50 Gew.-%, wie 5 bis 40 Gew.-%, oder 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten, im Molekül aufweisen.

Dazu zählen z. B. Natriumlaurylsulfat, Ammoniumtaurytsulfat, Natriumlaurylethersulfat, Ammoniumlaurylethersulfat, Natriumlaurylsarkosinat, Natriumoleylsuccinat, Ammoni-umlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt ca. 6 bis 60 Mole auf ein Mol Alkohol. Ferner sind Alkylaminoxide, Mono-oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampooformulierungen Verdicker, wie z. B. Kochsalz, PEG-55, Propylenglykol-Oleat, PEG-120-Methylglucosedioleat und andere sowie Konservierungsmittel, weitere Wirk- und Hilfsstoffe und Wasser enthalten.

### ii) Spezielle Ausgestaltungen für Mittel zur Auftragung auf das Haar

Nach einer weiteren bevorzugten Ausführungsform handelt es sich bei den Mitteln um ein Haarbehandlungsmittel, das erfindungsgemäß ist, soweit es unter die Ansprüche fällt.

Die Haarbehandlungsmittel enthalten insbesondere wenigstens einen oben beschriebenen Wirkstoff in einer Menge im Bereich von etwa 0,0001 bis 50 Gew.-%, wie z.B. 0,001 bis 10 Gew.-%, insbesondere 0,005 bis 0,1 Gew.-%, bezogen auf das Gesamtgewicht des Mittels.

Vorzugsweise liegen die Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-) Spray, (Aerosol-) Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die haarkosmetischen Formulierungen enthalten in einer speziellen Ausführungsform a) 0,0001 bis 50 Gew.-% oder 0,001 bis 10, oder 0,005 bis 1 Gew.-% wenigstens eines oben beschriebenen Wirkstoffs b) 20 bis 99,95 Gew.-% Wasser und/oder Alkohol, c) 0 bis 50 Gew.-% wenigstens eines Treibgases, d) 0 bis 5 Gew.-% wenigstens eines Emulgators, e) 0 bis 3 Gew.-% wenigstens eines Verdickers, sowie bis zu 25 Gew.-% weitere Bestandteile.

Unter Alkohol sind alle in der Kosmetik üblichen Alkohole zu verstehen, z. B. Ethanol, Isopropanol, n-Propanol.

Weiterhin zählen hierzu alle in der Kosmetik bekannten Styling- und Conditioner-Polymere, die in Kombination mit den oben beschriebenen Wirkstoffen eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon-Copolyole (CTFA) und aminofunktionelle Silikonverbindungen wie Amodimethicone (CTFA).

Die Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten Sprayzubereitungen a) 0,0001 bis 50 Gew.-% oder 0,001 bis 10, oder 0,005 bis 1 Gew.-% wenigstens eines oben beschriebenen Wirkstoffs, b) 20 bis 99,9 Gew.-% Wasser und/oder Alkohol, c) 0 bis 70 Gew.-% wenigstens eines Treibmittel, d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine bevorzugte Formulierung für Aerosolhaarschäume enthält a) 0,0001 bis 50 Gew.-% oder 0,001 bis 10, oder 0,005 bis 1 Gew.-% wenigstens eines oben beschriebenen Wirkstoffs, b) 55 bis 99,8 Gew.-% Wasser und/oder Alkohol, c) 5 bis 20 Gew.-% eines Treibmittel, d) 0,1 bis 5 Gew.-% eines Emulgators, e) 0 bis 10 Gew.-% weitere Bestandteile.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4 ; Cetethe, z. B. Cetheth-1, Polyethylenglycolcetylether, Cetearethe, z. B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine für Styling-Gele geeignete Zubereitung kann beispielsweise wie folgt zusammengesetzt sein: a) 0,0001 bis 50 Gew.-% oder 0,001 bis 10, oder 0,005 bis 1 Gew.-% wenigstens eines oben beschriebenen Wirkstoffs b) 80 bis 99,85 Gew.-% Wasser und/oder Alkohol, c) 0 bis 3 Gew.-%, bevorzugt 0,05 bis 2 Gew.-%, eines Gelbildners, d) 0 bis 20 Gew.-% weitere Bestandteile.

Der Einsatz von Gelbildnern kann von Vorteil sein, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid / Acrylamid-Copolymere, Steareth-10-Allylether, AcrylatCopolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44.

Spezielle Shampooformulierungen enthalten a) 0,0001 bis 50 Gew.-% oder 0,001 bis 10, oder 0,005 bis 1 Gew.-% wenigstens eines oben beschriebenen Wirkstoffs, b) 25 bis 94,95 Gew.-% Wasser, c) 5 bis 50 Gew.-% Tenside, c) 0 bis 5 Gew.-% eines weiteren Konditioniermittels, d) 0 bis 10 Gew.-% weitere kosmetische Bestandteile.

In den Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxid-Einheiten, bevorzugt 1 bis 3 Ethylenoxid-Einheiten, im Molekül aufweisen.

Geeignet sind zum Beispiel Natriumlaurylsulfat, Ammoniumlaurysulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den oben beschriebenen Wirkstoffen eingesetzt werden.

Hierzu zählen beispielsweise die zuvor genannten kationischen Polymere mit der Bezeichnung Polyquaternium nach INCI, insbesondere Copolymere aus Vinylpyrrolidon/ N-Vinylimidazoliumsalzen (Luviquat FC, Luviquat HM, Luviquat MS, Luviquat Care), Copolymere aus N-Vinylpyrrolidon/Dimethylaminoethylmethacrylat, quaternisiert mit Diethylsulfat (Luviquat D PQ 11), Copolymere aus N-Vinylcaprolactam/N-Vinylpyrrolidon/N-Vinylimidazoliumsalzen (Luviquat D Hold), kationische Cellulosederivate (Polyquaternium-4 und -10), Acrylamidcopolymere (Polyquaternium-7). Ferner können Eiweißhydrolysate verwendet werden sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon-Kopolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

### 4.3 Kühlende Mundpflegemittel (erfindungsgemäß, soweit sie unter die Ansprüche fallen)

Die Mundpflegemittel können in an sich bekannter Weise, z.B. als Zahnpasta, Zahngel, oder wässrige oder wässrig-alkoholische Mundpflegemittel (Mundwasser) formuliert sein.

Erfindungsgemäße Mundpflegezusammensetzungen enthalten, bezogen auf das Gewicht der Zusammensetzung, 0,00001 bis 50 Gew.-%, 0,0001 bis 10 Gew.-%, 0,001 bis 5 Gew.-%, 0,005 bis 1 Gew.-% oder 0,1 bis 20 Gew.-%, 0,5 bis 15 Gew.-% oder 1 bis 5 Gew.-% der Gesamtmenge wenigstens eines oben beschriebenen Wirkstoffs

Darüber hinaus können die Mundpflegemittel, insbesondere Zahnpasten, auch Abrasiva enthalten, wie Siliziumoxidhydrat, Dicalciumphosphatdihydrat, Calciumcarbonat, Natriumhydrogencarbonat, Calciumpyrophosphat und Aluminiumoxid. Beispielsweise kann auch eine Mischung aus zur Zähflüssigkeit neigendem gefällten Silizium und scheuernden gefällten Silizium [Handbook of Pharmaceutical Excipients, The Pharmaceutical Society of Great Britain, 1 Lambeth High Street, London SE 1 7JN, England, pages 253-256] verwendet werden. Das zuerst genannte wird auf Grund seiner thixotropen Eigenschaften, das zweite wegen seiner besseren Wirksamkeit beim Beseitigen der an den Zahnflächen anhaftenden Substanzen verwendet. Der Einsatz dieser Produkte gewährleistet eine geringe Scheuerwirkung, denn es handelt sich um amorphe Feststoffe mittlerer Härte, die gleichzeitig voll und ganz mit dem als Mineralisierungsmittel benutzten Fluorid kompatibel sind, da sie keine Kalksalze enthalten, welche ihre Unlöslichkeit bewirken und ihre Bioverfügbarkeit verringern würden.

Die Rezeptur der Mundpflegemittel, wie z.B. Zahnpasta, kann auch geeignete Zusätze und Vehikel enthalten, um deren Eigenschaften zu verbessern und die Herstellung zu erleichtern. Diese sind z.B. unter Bindemitteln, Verdickungsmittel, Duftstoffen, Farbstoffen, Konservierungsmitteln, Benetzungsmitteln oder Feuchthaltemittel, Tensiden, Schmiermitteln, Trübungsmitteln, Remineralisierungsstoffen, Tensiden, Puffern, Alkoholen, Vitaminen, Wasser, zusätzlichen Wirkstoffen und deren Mischungen ausgewählt.

Als Bindemittel kann jedes normalerweise bei der Herstellung dieser Art von Rezepturen verwendete Mittel eingesetzt werden, z. B. Tragantgummi. Das Bindemittel kann in der Rezeptur in einer Menge von 0,5-1,5 Gew.-% der Gesamtmenge enthalten sein.

In dem Mundpflegemittel können auch organische Verdickungsmittel eingearbeitet werden, wie Natriumcarboxymethylcellulose, Celluloseether, Xanthangummi, Karageenane, Natriumalginat und Carbopole. Es können auch anorganische Verdickungsmittel, wie Siliziumoxid-Verdickungsmittel, Natriumaluminiumsilikate und Tone, zur Bereitstellung der entsprechenden Rheologie verwendet werden. Das Verdickungsmittel kann in der Rezeptur in einer Menge von 0,5-5 Gew.-% der Gesamtmenge enthalten sein.

Die Zahnpasta kann durch Zusatz eines geeigneten üblichen Duftstoffes, z. B. eines Pfefferminzaromas, aromatisiert werden. Ätherische Öle einschließlich u.a. Nelkenöl, Zimtöl, Pfefferminzöl und Krauseminzöl sind ebenfalls geeignet. Der Duftstoff kann in der Rezeptur in einer Menge von 0,5-15 Gew.-% der Gesamtmenge enthalten sein.

Als Farbstoff kann jeder der üblicherweise bei der Herstellung von Zahnpasta verwendete Farbstoff eingesetzt werden zum Beispiel Brillantblau FCF, C.42090 [KIRSCH PHARMA]. Der Farbstoff kann in der Rezeptur in einer Menge von 0,001 - 0,005 Gew.-% der Gesamtmenge enthalten sein.

Bei dem Konservierungsmittel kann es sich um jedes übliche Mittel, wie etwa ein Derivat der Benzoesäure, z. B. p-Hydroxy-Methylbenzoat handeln. Das Konservierungsmittel kann in der Rezeptur in einer Menge von 0,1- 0,3 Gew.-% der Gesamtmenge enthalten sein.

Als Süßstoff kann man z. B. Natriumsaccharin oder Zyklaminsäure und deren Derivate, z. B. Natriumcyclamat, verwenden. Der Süßstoff kann in der Rezeptur in einer Menge von 0,08-0,15 Gew.-% der Gesamtmenge enthalten sein.

Das zur Verhinderung des Austrocknens und der Härtung der Zahnpasta verwendete Benetzungs- oder Feuchthaltemittel ist insbesondere ausgewählt unter Glycerin, Sorbit, Propylenglykol, Xylit und flüssige Polyethylenglykolen, insbesondere eine Mischung aus Sorbit, Glycerin und Xylit, z.B in einem Anteil von 1-60 Gew.-% der Gesamtmenge.

Als Schmiermittel kann jedes der üblicherweise in der Rezeptur einer Zahnpasta verwendeten Mittel sein, z. B. Dimethikon (Polymer des Dimethylpolysiloxans), bei dem es sich um ein oberflächenaktives Mittel handelt, das dazu beiträgt, der Zahnpasta gute rheologische Eigenschaften zu verleihen. Das Schmiermittel kann in der Rezeptur in einer Menge von 0,25 bis 0,75 Gew.-% der Gesamtmenge enthalten sein.

Als Trübungsmittel kann jedes der üblicherweise eingesetzten Mittel verwendet werden, z. B. Titandioxyd. Das Trübungsmittel kann in der Rezeptur in einer Menge von 0,05 bis 1 Gew.-% der Gesamtmenge enthalten sein.

Als Reminalisierungsmittel verwendet man eine Fluoridquelle, z. B. Natriumfluorid, Zinn(II)-fluorid und Natriummonofluorophosphat, da man auf diese Weise 100% eines aktiven Fluorids als Mittel zur Remineralisierung der weißen Läsionen, verursacht durch die organischen Säuren, die eine Folge des bakteriellen Stoffwechsels sind. Das Remineralisierungsmittel kann in der Rezeptur in einer Menge von 0,2 bis 0,4 Gew.-% der Gesamtmenge enthalten sein.

Typischerweise können des Weiteren übliche Bestandteile enthalten sein, wie anionische Tenside, wie z.B. Natriumlaurylsulphat, Natrium-N-Iaurylsarcosinat, Natriumlaurylsulphoacetat und Natriumalkylglycerylethersulphonat. Das Tensid kann in der Rezeptur in einer Menge von 0,05 bis 5 Gew.-% der Gesamtmenge enthalten sein

Wenn gewünscht, kann die vorgeschlagene Zahnpasta auch Vitamin enthalten, das aus der von Vitamin A, Vitamin B5, Vitamin C und Vitamin E gebildeten Gruppe und deren Mischungen ausgewählt wird. Im Fall einer Verwendung kann jedes Vitamin in der Rezeptur in einer Menge von 0,1 bis 5 Gew.-% der Gesamtmenge enthalten sein. Diese Vitamine können als solche, in Form von Provitaminen oder in Form akzeptierbarer pharmazeutischer Salze eingesetzt werden. Das Vitamin A, das in der Regel in Form seines Palmitatsalzes verwendet wird, fördert die Epithelisierung der Mundschleimhaut und schützt das Zahnfleisch. Das Vitamin B5, genauer gesagt das D- Panthenol, wirkt schmerzstillend, heilend und entzündungshemmend, schützt die Epithelschleimhaut, fördert die Epithelisierung der Verletzungen und glättet die Narben; es ist für die Behandlung von Verletzungen geeignet, welche infolge von Zahn-extraktionen, Gingivitis, Stomatitis, Schmerzen nach dem Einsetzen von Zahnprothesen, Geschwüren, traumatischen Lädierungen der Schleimhaut, chronischen und rückläufigen Aphthen entstehen. Das Vitamin C regeneriert das Epithel der Mundschleimhaut, fördert die Kollagensynthese und das Immunsystem (Entzündungs-mechanismus) und erhöht die Schutzfähigkeit der Phagozyten gegen Bakterien. Das Vitamin E, das normalerweise in Form seines Azetatsalzes eingesetzt wird, wirkt schmerzstillend und entzündungshemmend, schützt die Mundschleimhaut gegen die Fettüberoxidierung infolge der Bildung freier Radikale und gegen kontaminisierende Substanzen der Umwelt (Ozon, Zigarettenrauch usw.) und begünstigt die Heilung der Verletzungen. Durch die Zugabe eines oder einiger dieser Vitamine bietet diese Beschreibung eine Zahnpasta, die neben den zuvor genannten Eigenschaften auch entzündungshemmende Merkmale und schmerzstillende Auswirkungen besitzt, welche die Schutzfähigkeit der Membranen der Mundschleimhaut erhöhen und den Index der Zahnbelags- und Zahnsteinbildung sowie den der bakteriellen Kontamination verringern.

Zusätzliche Wirkstoffe sind z.B. antimikrobielle und Plaque-penetrierende Mittel, wie beta-Naphthol, Thymol, Chlorthymol und Hexylresorcin; oder keimtötende Verbindungen, wie quaternäre Ammoniumverbindungen.; Zahnsteinbekämpfungsmittel, wie Tetranatriumpyrophosphat, GANTREZ-Polymer® S-70, Natriumtripolyphosphat und Zinkcitrat; Peroxidverbindungen, wie Wasserstoffperoxid und anorganischen Peroxide.

Gegebenenfalls kann auch ein Puffer verwendet werden, der in für die Aufrechterhaltung eines pH-Werts von etwa 6-8 geeigneten Konzentrationen enthalten ist, wie z.B Alkalimetallphosphatpuffer. Die Anwesenheit von Kaliumionen übt zudem eine die Überempflindichkeit lindernde Wirkung aus.

Wasser oder Alkohol können in einem Anteil von 1 bis 20 Gew.-% der Gesamtmenge des Mittels enthalten sein.

In Kombination mit dem Alkohol oder anstelle des Alkohols können auch Glykolverbindungen verwendet werden, wie Glycerin, Sorbit oder Propylenglykol.

Das Mundpflegemittel kann leicht durch Mischung geeigneter Mengen der verschiedenen Bestandteile in einem z.B. mit Rührschaufeln ausgestatteten Reaktor hergestellt werden.

### 4.4. Kühlende Pflaster (erfindungsgemäß soweit sie unter die Ansprüche fallen)

Grundsätzlich kann der Wirkstoffgehalt über einen weiten Bereich, wie z.B. 0,00001 bis 50 Gew.-%, insbesondere 0,001 bis 10 Gew.-% oder 0,005 bis 1 Gew.-% variieren.

Die Pflaster können in beliebiger Weise aufgebaut sein, beispielsweise nach dem Matrixsystem, dem Membransystem oder dem Vliessystem (Drug Dev. Ind. Pharm. 14 (1988), 183-209; Drug Dev. Ind. Pharm. 13 (1987), 589-651; Drugs of Today 23 (1987), 625-646.

Das Matrixsystem besteht in einfachster Weise aus 3 Teilen: der flexiblen Stützfolie, der den Wirkstoff enthaltenden Klebematrix und einer Abziehfolie. Falls eine nichtklebende Matrix verwendet wird, muss zur Haftung auf der Haut eine Randzone der Stützfolie mit Klebstoff versehen werden.

Ein Membransystem weist hingegen mindestens 5 Teile auf: eine flexible Stützfolie, ein Reservoir mit gelöstem oder suspendiertem Wirkstoff, eine Membran zur Steuerung der Wirkstofffreigabe, eine auf die Membran aufgezogene Klebeschicht und eine Abziehfolie.

Beim Vliessystem besteht die den Wirkstoff enthaltende Schicht aus einem saugfähigen Vlies oder porösen Polymer, das mit einer Wirkstofflösung oder -suspension getränkt ist. Diese Schicht, die fest mit der Stützfolie verbunden ist, wird durch eine Abziehfolie abgedeckt. Der Rand der Stützfolie ist, zur Applikation auf die Haut, mit Klebstoff versehen.

Grundsätzlich sind alle oben beschriebenen Wirkstoffe in dieser Weise formulierbar.

Die zu verwendenden Hilfsstoffe sind die für die Herstellung von Pflastern üblichen. Neben dem klebenden Agens, in der Regel ein Polymer mit einer Glastemperatur zwischen -70 und -10, insbesondere -55 und -25 °C, sowie einer Trägerfolie, die mit diesem klebenden Agens beschichtet wird, und dem Wirkstoff werden häufig Emulgatoren, Verdickungsmittel sowie Stoffe, die die Wirkstofffreigabe beeinflussen sollen, und andere Hilfsmittel zugesetzt.

Die klebrigen Polymeren mit den oben genannten niedrigen Glastemperaturen sind bekannt, beispielsweise aus den US-Patenten 2 973 282 und 3 307 544. Die selbstklebenden Bänder und Folien sollen auf bloßen Kontakt auf der menschlichen Haut kleben, doch soll die Kohäsion der Klebschicht und deren Adhäsion an der Trägerfolie größer sein als die Adhäsion an der Haut, so dass sie sich weitgehend rückstandslos wieder abziehen lassen. Es handelt sich in der Regel um Copolymerisate auf der Basis von Acryl- und Methacrylsäureestern von Alkoholen mit 2 bis 12, insbesondere 4 bis 8 Kohlenstoffatomen, die zahlreiche andere Comonomere einpolymerisiert enthalten können, beispielsweise (Meth)acrylsäure, (Meth)acrylnitril, (Meth)acrylamid, N-tert.-Butyl-(meth-)acrylamid, Vinylester wie Vinylacetat, -propionat oder - butyrat, andere Vinylverbindungen wie Styrol, ferner Butadien. Besonders hervorgehoben seien Butylacrylat und 2-Ethylhexylacrylat. Die Polymeren können durch Zusatz geringer Mengen Comonomerer mit 2 oder mehr copolymerisierbaren Doppelbindungen, also beispielsweise von Diacrylaten, wie Butandioldiacrylat, oder Divinylverbindungen, wie Divinylbenzol, oder durch Zusatz anderer Vernetzungsmittel, z.B. Melamin-Formaldehydharze, vernetzt sein. Als klebrige Polymere können ferner Polyisobutylene und Polyvinylether unterschiedlichen Molekulargewichts verwendet werden.

Die Teilchengröße der Dispersionen soll zwischen 50 und 500 nm, insbesondere zwischen 50 und 200 nm liegen. Die Teilchengröße und der Vernetzungsgrad können in bekannter Weise in Abhängigkeit von den Polymerisationsbedingungen und den Comonomeren eingestellt werden. Kleinere Teilchengrößen und ein erhöhter Vernetzungsgrad können eine Steigerung der Wirkstofffreisetzung bewirken.

Matrix-Pflaster können in üblicher Weise hergestellt werden, indem man den Wirkstoff in einer geeigneten Polymerlösung löst oder fein dispergiert und anschließend diese wirkstoffhaltige Selbstklebemasse mittels Walzen- oder Rakelauftragsverfahren zum Film auszieht. In einigen Fällen ist es zweckmäßig, den Wirkstoff vor der Zugabe zu der Polymerlösung in einem organischen Lösungsmittel, z.B. Ethanol oder Aceton, aufzulösen oder feinst zu dispergieren. Dadurch kann eine bessere Verteilung des Wirkstoffes im Polymer erzielt werden.

Die Pflaster können auch nach der deutschen Patentanmeldung P 38 07 283.1 hergestellt werden, indem man den Wirkstoff in feinpulvriger Form (Teilchengröße unter 200, insbesondere unter 50 µm) in die wässrige Latexdispersion einarbeitet, oder in einer wässrigen Emulgatorlösung dispergiert oder löst und diese Mischung der wässrigen Latexdispersion bei einer Temperatur von 10 bis 80, insbesondere 30 bis 70 °C zumischt. Daneben kann auch das Salz eines Wirkstoffs in wässriger Lösung mit der Polymerdispersion bei einem pH-Wert gemischt werden, bei dem der Wirkstoff vorwiegend in der wasserlöslichen ionisierten Form vorliegt. Durch pH-Verschiebung wird dann der Wirkstoff in die ungeladene wasserunlösliche Form gebracht und simultan in die Dispersion einemulgiert.

Zweckmäßig legt man den Wirkstoff vor, gibt den Emulgator und Wasser zu und mischt dann mit der Polymerdispersion. Die so erhaltene wirkstoffhaltige Dispersion wird gegebenenfalls mit weiteren Hilfsstoffen versehen und, wie erwähnt, in an sich bekannter Weise auf einer Stützfolie zu einem Film ausgezogen und getrocknet. Die Trocknungstemperatur kann hierbei zwischen Raumtemperatur und 100°C liegen, wobei ein Optimum zwischen angestrebter schneller Trocknung und zu vermeidender Blasenbildung im Film sowie thermischer Belastung des Wirkstoffs im Allgemeinen bei 35 bis 45°C liegt.

Dieses Verfahren hat den großen Vorteil der Vermeidung von organischen Lösungsmitteln. Jedoch kommen im Prinzip auch alle anderen üblichen Herstellverfahren für Matrix-Pflaster in Betracht.

Die resultierenden Filme haben Dicken von 10 bis 800, bevorzugt 50 bis 300 µm. Die Filmherstellung kann kontinuierlich oder diskontinuierlich erfolgen. Das Auftrageverfahren kann mehrmals wiederholt werden, bis der Film die gewünschte Dicke erreicht hat. Die klebrige Polymerschicht enthält den Wirkstoff in einer Konzentration im Bereich von 1 bis 40, insbesondere 5 bis 25 Gew.-%. Die gleiche Konzentration gilt auch für die Reservoirflüssigkeit beim Membransystem und für die Wirkstofflösung oder -Dispersion, mit der beim Vliessystem das Vlies oder das poröse Polymere getränkt wird.

Als Emulgatoren sowohl für die Wirkstoffe wie auch die Polymeren werden die hierfür üblichen Tenside verwendet, wie das Natriumsalz von längerkettigen Fettsäuren und des Schwefelsäurehalbesters eines (gegebenenfalls oxethylierten) Fettalkohols als Beispiele für anionische Tenside sowie polyoxethylierte Alkylphenole und längerkettige Fettalkohole (z.B. Hexadecan-(1)-ol) und Glycerin-Fettsäurepartialester als Beispiele für nichtionische Tenside und Coemulgatoren.

Die gewünschte Viskosität der ausziehfertigen Masse kann z.B. mit Polyacrylsäuren oder Cellulosederivaten eingestellt werden.

Als zusätzliche Vernetzungsmittel die die Kohäsion und damit die Klebeeigenschaften der Filme verbessern, können z.B. Melamin-Formaldehydharze verwendet werden.

Im Sinne einer Verbesserung der Wirkstofffreisetzung wirken Quellstoffe wie Polyvinylpyrrolidon, Cellulosederivate oder Polyacrylate, da der Film vermehrt Wasser aufnehmen kann und dadurch der Diffusionswiderstand sinkt. Die Freisetzung der Wirkstoffe kann ferner verbessert werden durch den Zusatz von hydrophilen Weichmachern wie Glycerin, 1, 2-Propandiol der Polyethylenglykolen und lipophilen Weichmachern wie Triacetin, Dibutylphthalat oder Isopropylmyristat.

Matrixpflaster ergeben üblicherweise eine Wirkstofffreisetzung 1. Ordnung. Durch die Verwendung von Füllstoffen, die den Wirkstoff adsorbieren, wie Aerosil, mikrokristalline Cellulose oder Lactose, resultiert annähernd eine Freisetzung 0. Ordnung.

Die Stützfolie, auf die die wirkstoffhaltige Selbstklebemasse aufgetrocknet wird, ist zweckmäßig sowohl für den Wirkstoff wie für Wasserdampf praktisch undurchlässig. Sie kann z.B. aus einer Aluminium-Kunststoff-Verbundfolie, einer metallisierten Kunststofffolie, einer Kunststofffolie, die zur Wirkstoffseite hin mit einer Sperrschicht aus z.B. Polyvinylidenchlorid versehen ist, oder aus einer einfachen Kunststofffolie, z.B. Polyesterfolie, bestehen.

Die Pflaster, die nach dem Membransystem aufgebaut sind, werden ebenfalls in üblicher Weise hergestellt (z.B. EP 0 186 071 A2, US 4 262 003).

Die Herstellung der nach dem Vliessystem aufgebauten Pflaster erfolgt dadurch, dass auf der Stützfolie befestigte Vliese oder poröse Polymere mit einer Lösung oder Dispersion des Wirkstoffes in einem hydrophilen oder lipophilen Lösungsmittel oder Lösungsmittelgemisch getränkt werden. Anschließend wird die undurchlässige Abziehfolie aufgebracht.

### 4.5 Kühlende Nahrungsmittel (erfindungsgemäß, soweit sie unter die Ansprüche fallen)

Die kühlenden Nahrungsmittel können in (bei Umgebungstemperatur) fester, flüssiger, halbfester, pastöser, cremiger order geschäumter Form vorliegen. Sie enthalten neben herkömmlichen Nahrungsbestandteilen wenigstens eine wirksame (d.h. als kühlend wirkende) Menge wenigstens eines oben beschriebenen Wirkstoffs.

Typische Bestandteile sind dabei Fette, Kohlenhydrate, Proteine, Ballaststoffe, Wasser, Alkohol und dergleichen.

Der Proteinanteil kann z.B. 0 bis 50 Gew.-%, bezogen auf das Gesamtgewicht des Nahrungsmittels betragen;
der Fettanteil kann z.B. der 0 bis 50 Gew.-%, bezogen auf das Gesamtgewicht des Nahrungsmittels betragen;
der Kohlenhydratanteil kann z.B. 0 bis 90 Gew.-%, bezogen auf das Gesamtgewicht des Nahrungsmittels betragen;
der Ballaststoffanteil kann z.B. 0 bis 90 Gew.-%, bezogen auf das Gesamtgewicht des Nahrungsmittels betragen;
der Wasseranteil kann z.B. 0 bis 95 Gew.-%, bezogen auf das Gesamtgewicht des Nahrungsmittels betragen;
der Alkoholanteil kann z.B. 0 bis 15 Gew.-%, bezogen auf das Gesamtgewicht des Nahrungsmittels betragen;
der Anteil oben beschriebenen Wirkstoffe kann z.B. im Bereich von 0,0001 bis 50, 0,001 bis 20, 0,005 bis 1, oder 0,01 bis 10, insbesondere 0,1 bis 10 oder 1 bis 5 Gew.-%, bezogen auf das Gesamtgewicht des Nahrungsmittels liegen.

Beispiele für Kohlehydrate sind z.B. Mono- und Disaccharide, Glukose, Galaktose, Mannose, Laktose, Maltose, und Saccharose; Fruktose und Mannose; Polysaccharide wie z. B. Stärken. Maltodextrine, Mehl.

Der Begriff "Ballaststoff" bezieht sich auf lösliche, unlösliche, fermentierbare, nicht fermentierbare oder eine beliebige Kombination solcher Ballaststoffe. Bei dem Ballaststoff kann es sich z. B. um Sojafasern, Pektin, bestimmte resistente Stärken, Oligofruktose, Inuline, Haferfasern, Erbsenfasern, Guargummi, Gum acazia, modifizierte Cellulose handeln.

Bei dem Fettbestandteil kann es sich um ein beliebiges Lipid oder Fett handeln, dessen Eignung zur Verwendung in Nährmitteln bekannt ist. Typische Fette sind unter anderem Milchfett, Distelöl, Canolaöl, Eidotterlipid, Olivenöl, Baumwollsamenöl, Kokosnussöl, Palmöl, Palmkernöl, Sojaöl, Sonnenblumenöl, Fischöl und Fraktionen aller vorstehenden Öle, die sich davon ableiten, wie Palmolein, mittelkettige Triglyceride (MCT), und Fettsäureester, wobei es sich bei den Fettsäuren z. B. um Arachidonsäure, Linolsäure, Palmitinsäure, Stearinsäure, Docosahexaensäure, Eicosapentaensäure, Linolensäure, Ölsäure, Laurinsäure, Caprinsäure, Caprylsäure, Capronsäure handelt. Hochölsäurehaltige Formen verschiedener Öle werden auch als geeignet für die vorliegende Verwendung erachtet, wie hochölsäurehaltiges Sonnenblumenöl und hochölsäurehaltiges Distelöl.

Bei dem Protein kann es sich um ein beliebiges Protein und/oder Aminosäuregemisch handeln, dessen Eignung zur Verwendung in Nährmitteln bekannt ist. Typische Proteine sind tierische Proteine, pflanzliche Proteine wie Sojaprotein, Milchprotein wie Magermilchprotein, Molkenprotein und Casein, und Aminosäuren (oder Salze davon) wie Isoleucin, Phenylalanin, Leucin, Lysin, Methionin, Threonin, Tryptophan, Arginin, Glutamin, Taurin, Valin. Bevorzugte Proteinquellen sind Molkenprotein, Natriumcaseinat oder Calciumcaseinat, das gegebenenfalls mit Aminosäuren versetzt ist. Für einige Anwendungen ist eine bevorzugte Proteinquelle hydrolysiertes Protein (Proteinhydrolysat) das gegebenenfalls mit Aminosäuren versetzt ist.

Bei dem Proteinhydrolysat kann es sich um ein beliebiges geeignetes Proteinhydrolysat handeln, das in einem Nährmittel verwendet wird, wie Sojaproteinhydrolysat, Caseinhydrolysat, Molkenproteinhydrolysat, andere tierische und pflanzliche Proteinhydrolysate und Gemische davon. Das Proteinhydrolysat der Zusammensetzung ist vorzugsweise ein Sojaprotein-, Molkenprotein- oder ein Caseinproteinhydrolysat, das kurze Peptide und Aminosäuren umfasst und gegebenenfalls mit zusätzlichen Aminosäuren versetzt ist. In einer bevorzugten Ausführungsform enthält das geeignete Proteinhydrolysat einen hohen Anteil freier Aminosäuren (z. B. mehr als 40%) und niedermolekularer Peptidfragmente.

Das hydrolysierte Protein der Zusammensetzung ist auch vorzugsweise versetzt mit verschiedenen freien Aminosäuren, um einen ernährungsmäßig ausgewogenen Aminosäuregehalt bereitzustellen. Beispiele für solche freien Aminosäuren sind unter anderem L-Tryptophan, L-Methionin, L-Cystin, L-Tyrosin und L-Arginin.

Die Nährmittel enthalten gegebenenfalls auch Vitamine und Mineralien. Dem Fachmann ist geläufig, dass Minimalerfordernisse für bestimmte Vitamine und Mineralien aufgestellt worden sind, die für die normale physiologische Funktion notwendig sind. Der Fachmann weiß außerdem, dass den Nährmitteln angemessene zusätzliche Mengen von Vitamin- und Mineralbestandteilen zugesetzt werden müssen, um gewisse Verluste bei der Verarbeitung und Lagerung derartiger Zusammensetzungen zu kompensieren. Die Zusammensetzung enthält gegebenenfalls ernährungsmäßig signifikante Mengen Vitamine und Mineralien.

Beispiele für Mineralien, Vitamine und andere Nährstoffe, die gegebenenfalls in der hier beschriebenen Zusammensetzung vorliegen, sind unter anderem Vitamin A, Vitamin B₆, Vitamin B₁₂, Vitamin E, Vitamin K, Vitamin C, Vitamin D, Inositol, Taurin, Folsäure, Thiamin, Riboflavin, Niacin, Biotin, Pantothensäure, Cholin, Calcium, Phosphor, lod, Eisen, Magnesium, Kupfer, Zink, Mangan, Chlorid, Kalium, Natrium, Beta-Carotin, Nucleotide, Selen, Chrom, Molybdän und L-Carnitin. Mineralien werden üblicherweise in Salzform zugesetzt.

Die Zusammensetzung enthält gegebenenfalls auch üblicherweise Emulgatoren und/oder Stabilisatoren wie Lecithin (z. B. aus Ei oder Soja), modifiziertes Lecithin (z. B. enzymatisch oder acetyliert), Carrageenan, Xanthangummi, Mono- und Diglyceride, Guargummi, Carboxymethylcellulose, Stearoyllactylate, succinylierte Monoglyceride, Saccharoseester von Fettsäuren, Diacetylweinsäureester von Monoglyceriden, Polyglycerolester von Fettsäuren oder beliebige Gemische davon.

Die Zusammensetzung enthält wahlweise ein oder mehrere natürliche oder künstliche Geschmacksträger zur Verbesserung der Schmackhaftigkeit. Man kann jeden auf dem Sektor verwendeten Geschmacksträger verwenden, wie Erdbeer, Kirsch, Schokolade, Orange, Kokosnuss, Vanille; Gewürze wie Muskat und Zimt; oder Citronensäure. In einigen Fällen, in denen natürliche Geschmacksträger, wie Kokosnussstücke, verwendet werden, trägt der Bestandteil zum Gesamtnährwertprofil der Zusammensetzung bei, d. h. er trägt zur Qualität und Quantität des Fett-, Protein- und/oder Kohlenhydratbestandteils bei.

Die Zusammensetzung enthält wahlweise auch verschiedene andere Bestandteile, die zum Nährwertprofil der Zusammensetzung beitragen und/oder erwünschte Geschmackseigenschaften, wie Geschmacksverstärkung oder Mundgefühl, verleihen können. Derartige Bestandteile sind unter anderem Erdnüsse, Rosinen, Käsepulver, Essig, Salz, Natriumbicarbonat. Bei Riegeln wird die Zusammensetzung üblicherweise mit einem Schokoladen- oder einem aromatisierten Überzug (z. B. Schokolade, Vanille, Erdbeer usw.) versehen.

Die Zusammensetzung enthält wahlweise auch natürliche oder künstliche Farbstoffe, um den ästhetischen Anreiz zu verbessern.

Die Zusammensetzungen können in mehreren physikalischen Erscheinungsformen vorliegen, z. B. als flüssige enterale Nährmittel oder Getränke für Erwachsene oder Kinder, in einer halbfesten Form wie Pudding, Creme, Mousse, oder einer festen Form, wie einem Nährmittelriegel oder Keks.

Die Zusammensetzung kann nach bekannten Standardverfahren der Lebensmitteltechnik hergestellt werden, zum Beispiel nach analogen Verfahren zu den in folgenden Druckschriften beschriebenen: US-Patente 4,670,268; 4,497,800; 4,900,566; 5,104,677; 5,389,395; und 5,223,285; Chocolate, Cocoa and Confectionery: Science and Technology, 3. Auflage, Bernard W. Minifie, Van Nostrand Reinhold, New York, 1989, S. 502-506; worauf vollinhaltlich verwiesen wird.

Bei Nährmittelriegeln und Keksen ist es üblicherweise angestrebt, die Zusammensetzung nach der physikalischen Formung zu backen.

Die Zusammensetzung kann gewünschtenfalls nach bekannten Verfahren sterilisiert werden, zum Beispiel durch Wärmebehandlung wie Autoklavieren oder Sterilisieren oder Bestrahlung, oder mit sterilen Verfahren hergestellt und verpackt werden.

Die Zusammensetzung kann in eine beliebige Art von Behälter oder Verpackung verpackt werden, deren Eignung zur Aufbewahrung von Lebensmitteln bekannt ist, wie Papier, Glas, beschichteter Karton, Kunststoff oder beschichtete Metalldosen.

Die Zusammensetzung kann ernährungsmäßig ausgewogen sein. Unter dem Begriff "ernährungsmäßig ausgewogen" versteht man, dass die Zusammensetzung angemessene Nährstoffe enthält, um ein gesundes menschliches Leben über ausgedehnte Zeitspannen zu erhalten.

### 4.6. Textilprodukte, ausgerüstet mit oben beschriebenen Wirkstoffen (erfindungsgemäß, soweit sie unter die Ansprüche fallen).

Grundsätzlich kann der Wirkstoffgehalt über einen weiten Bereich, wie z.B. 0,00001 bis 50 Gew.-%, insbesondere 0,001 bis 10 Gew.-% oder 0,005 bis 1 Gew.-% variieren.

Die Ausrüstung von Textilien mit oben beschriebenen Wirkstoffen, ist in vielerlei Hinsicht von Interesse.

So findet die Ausrüstung von Textilien mit kühlend wirkenden Verbindungen insbesondere dort Verwendung wo Kleidungsstücke in direkten Kontakt mit der Haut gelangen können, so dass der Wirkstoff durch transdermale Übertragung seine Wirkungen, z.B. lokal oder systemisch, entfalten kann. Kürzlich wurde über Textilien berichtet, die mit sogenannten Wellness-Additiven, d.h. Substanzen, welche das Wohlbefinden fördern, ausgerüstet sind (R. Breier "Megatrend Wellness - Innovative Ideen für die Textilausrüstung", 31. Aachener Textiltage November 2004).

Eine insektizide Ausrüstung wiederum ist im Hinblick auf den Materialschutz, z.B. Ausrüstung des Textils gegen Mottenfraß etc., aber insbesondere auch zur Abwehr von parasitären Insekten, wie Mücken, von Interesse.

Grundlegendes Problem bei der Ausrüstung von Textilien mit Wirkstoffen ist die Anbindung des Wirkstoffs an den textilen Träger, die einerseits eine Permanenz der Ausrüstung gewährleisten muss und andererseits so gewählt werden muss, dass der Wirkstoff seine Wirkung nicht verliert. Hierzu werden im Stand der Technik verschiedene Ansätze vorgeschlagen.

So wurden z.B. Cyclodextrine zur Anbindung von Wirkstoffen an Textilien vorgeschlagen (siehe beispielsweise DE-A-19810951 und EP-A-0 392 608). Cyclodextrine sind cyclische Oligosaccharide, die durch enzymatischen Abbau von Stärke gebildet werden. Die häufigsten Cyclodextrine sind α-, β- und γ-Cyclodextrine, die aus sechs, sieben bzw. acht α-1,4-verknüpften Glucose-Einheiten bestehen. Eine charakteristische Eigenschaft der Cyclodextrin-Moleküle ist ihre Ringstruktur mit weitgehend unveränderlichen Abmessungen. Der Innendurchmesser der Ringe beträgt etwa 570 pm für α-Cyclodextrin, etwa 780 pm für β-Cyclodextrin und etwa 950 pm für γ-Cyclodextrin. Aufgrund ihrer Struktur sind Cyclodextrine in der Lage, Gastmoleküle, insbesondere hydrophobe Gastmoleküle, in wechselnden Mengen bis zur Sättigung einschließen zu können.

Die EP-A-1710345 beschreibt die Ausrüstung von Textilien mit Duftstoffen und anderen niedermolekularen organischen Wirkstoffen über eine amylosehaltige Substanz mit einem Amylosegehalt von wenigstens 30 % an das Textil gebunden sind.

Durch die Amyloseanteile der amylosehaltigen Substanz wird der Wirkstoff auf dem Textil gebunden und kontrolliert abgegeben, so dass die Wirkung über einen langen Zeitraum erhalten bleibt. Man nimmt an, dass der Wirkstoff ähnlich wie bei Cyclodextrinen in den durch die helikale Konformation der Amylose gebildeten Hohlräumen im Sinne einer Einschlussverbindung reversibel gebunden wird, wodurch einerseits eine Fixierung des Wirkstoffs auf der Oberfläche des textilen Trägers erreicht wird und andererseits eine kontrollierte Freisetzung möglich ist.

Für die Ausrüstung von Textilien sind neben Amylose grundsätzlich alle Substanzen, insbesondere amylosehaltigen Stärken, d.h. native Stärken, modifizierte Stärken und Stärkederivate, geeignet, deren Amylosegehalt wenigstens 30 Gew.-% und insbesondere wenigstens 40 Gew.-% beträgt. Die Stärke kann nativ sein, z.B. Maisstärke, Weizenstärke, Kartoffelstärke, Sorghumstärke, Reisstärke oder Marantastärke, durch Teilaufschluss nativer Stärke gewonnen oder chemisch modifiziert sein. Geeignet ist auch reine Amylose als solche, z.B. enzymatisch gewonnene Amylose, z. B. aus Sucrose gewonnne Amylose. Geeignet sind auch Mischungen von Amylose und Stärke, sofern der Gesamtgehalt an Amylose wenigstens 30 Gew.-%, bezogen auf das Gesamtgewicht der Mischung beträgt. Es versteht sich, dass hier und im Folgenden alle Angaben in Gew.-%, welche sich auf Amylose oder amylosehaltige Substanzen beziehen, bei Mischungen aus Amylose und Stärke stets auf das Gesamtgewicht von Amylose + Stärke bezogen sind, sofern nicht ausdrücklich anderes angegeben ist.

Besonders geeignet sind amylosehaltige Substanzen, insbesondere Amylose und amylosehaltige Stärken sowie Amylose/Stärkegemische, deren Amylosegehalt wenigstens 40 Gew.-% und insbesondere wenigstens 45 Gew.-%, bezogen auf das Gesamtgewicht der Substanz beträgt. In der Regel wird der Amylosegehalt 90 Gew.-% und insbesondere 80 Gew.-% nicht überschreiten. Derartige Substanzen sind bekannt und kommerziell erhältlich. Beispielsweise werden amylosehaltige Stärken von den Firmen Cerestar unter der Handelsmarke Amylogel® und National Starch unter den Handelsbezeichnungen HYLON® V und VII vertrieben.

Zur Erreichung der Anbindung des/der Wirkstoff(e) and das Textil, kann man das Textil mit der amylosehaltigen Substanz in der Regel in einer Menge von wenigstens 0,5 Gew.-%, vorzugsweise wenigstens 1 Gew.-% und insbesondere wenigstens 2 Gew.-%, jeweils bezogen auf das Gewicht des Textils, ausrüsten. In der Regel wird man die amylosehaltige Substanz in einer Menge von nicht mehr als 25 Gew.-%, häufig nicht mehr als 20 Gew.-% und insbesondere nicht mehr als 15 Gew.-%, bezogen auf das Gewicht des Textils, einsetzen um die taktilen Eigenschaften des Textils nicht nachteilig zu beeinflussen.

Zunächst wird das textile Material mit der amylosehaltigen Substanz als solcher ausgerüstet und anschließend das so ausgerüstete Textil mit einer geeigneten Aufbereitung des Wirkstoffs behandelt. Hierdurch wird die auf dem textilen Material befindliche amylosehaltige Substanz mit dem Wirkstoff beladen.

Man kann aber auch die amylosehaltige Substanz gemeinsam mit einem Wirkstoff einsetzen, um die Textilie auszurüsten. Hierbei können der Wirkstoff und die amylosehaltige Substanz sowohl als Mischung separater Komponenten als auch in der bereits vorgefertigten Form des Amylose-Wirkstoff-Komplexes angewandt werden.

In der Regel wird man den Wirkstoff in einer Menge einsetzen, die für den gewünschten Effekt ausreicht. Die Obergrenze wird durch die maximale Aufnahmekapazität der Amyloseeinheiten der eingesetzten amylosehaltigen Substanz bestimmt und wird in der Regel 20 Gew.-% und häufig 10 Gew.-%, bezogen auf den Amyloseanteil der Substanz, nicht überschreiten. Sofern erwünscht, setzt man den Wirkstoff in der Regel in einer Menge von 0,00001 bis 15 Gew.-%, 0,0001 bis 10 Gew.-%, 0,001 bis 5 Gew.-%, 0,005 bis 1 Gew.-% oder 0,1 bis 10 Gew.-% oder 0,5 bis 5 Gew.-%, bezogen auf den Amyloseanteil der amylosehaltigen Substanz ein.

Zur Textilausrüstung können auch Kombinationen oben beschriebener Wirkstoffe mit anderen an sich bekannten und zur Textilausrüstung geeigneten Wirkstoffen verwendet werden.

Als weitere Wirkstoffe sind grundsätzlich alle organischen Verbindungen und Mischungen organischer Verbindungen geeignet, die als Wirkstoffe bekannt sind und die in Lebewesen wie Menschen und Tieren, einschließlich Mikroorganismen, eine physiologische Wirkung induzieren. Zu nennen sind solche Wirkstoffe, die bekanntermaßen mit Cyclodextrinen Einschlussverbindungen bilden können. Besonders geeignet sind Wirkstoffe, die Kohlenwasserstoffgruppen und insbesondere aliphatische, cycloaliphatische und/oder aromatische Strukturen aufweisen. Das Molekulargewicht der Wirkstoffe liegt typischerweise unterhalb 1000 Dalton und häufig im Bereich von 100 bis 600 Dalton. Geeignet sind außerdem anorganische Verbindungen wie Wasserstoffperoxid, die bekanntermaßen in Cyclodextrinen gebunden werden können (siehe hierzu F. Vögtle, Supramolekulare Chemie, 2. Auflage, B. G. Teubner, Stuttgart 1992, Cyclodextrine und dort zitierte Literatur)

Zu den weiteren Wirkstoffen zählen insbesondere pharmazeutische Wirkstoffe und Wirkstoffe, die das Wohlbefinden von Lebewesen, insbesondere von Menschen fördern und die gemeinhin auch als "Wellness-Additive" bezeichnet werden. Anders als bei pharmazeutischen Wirkstoffen muss bei den Wellness-Additiven nicht zwingend eine therapeutische Wirkung gegeben sein. Vielmehr kann der das Wohlbefinden fördernde Effekt auf einer Vielzahl von Faktoren wie pflegenden, anregenden, kosmetischen oder sonstigen Wirkungen beruhen. Gleichermaßen geeignet sind organische Wirkstoffe, die gegen parasitäre Organismen wirken. Hierzu zählen beispielsweise Wirkstoffe, die gegen Pilze und/oder Mikroorganismen wirken, z.B. Fungizide und Bakterizide, oder die gegen tierische Schädlinge wie Schnecken, Würmer, Milben, Insekten und/oder Nager wirken, z.B. Nematizide, Molluskizide, Insektizide, Akarizide, Rodentizide und Repellent-Wirkstoffe, sowie weiterhin Wirkstoffe gegen Ungräser, d.h. Herbizide, oder Duftstoffe.

Bevorzugte pharmazeutische Wirkstoffe sind solche, die bekanntermaßen über die Haut resorbiert werden können. Hierzu zählen beispielsweise Ibuprofen, Flurbiprofen, Acetylsalicylsäure, Acetamidophen, Apomorphin, butyliertes Hydroxytoluol, Chamzulen, Gujazulen, Chlorthalidon, Cholecalciferol, Dicumarol, Digoxin, Diphenylhydantoin, Furosemid, Hydroflumethiazid, Indomethacin, Iproniazid-Phosphat, Nitroglycerin, Nicotin, Nicotinsäureamid, Oubain, Oxprenolol, Papaverin-Alkaloide wie Papaverin, Laudanosin, Ethaverin und Narcotin sowie Berberin, weiterhin Retionol, trans-Retinolsäure, Pretinol, Spironolacton, Sulpirid, Theophyllin, Theobromin, Corticosteroide und Derivate wie Testosteron, 17-Methyltestosteron, Cortison, Corticosteron, Dexamethason, Triamcinolon, Methylprednisolon, Fludrocortison, Fluocortolon, Prednison, Prednisolon, Progesteron, u.a. Estrogene und Gestagene wie Estradiol, Estriol, Ethinylestradiol-3-methylether, Norethisteron und Ethisteron, sowie Phenethylamin und Derivate wie Tyramin, Adrenalin, Noradrenalin und Dopamin.

Beispiele für geeignete Wirkstoffe mit einer Wirkung gegen parasitäre Organismen sind die unter www.reith-pfister.de/w.list.html sowie unter www.hclrss.demon.co.uk/class pesticides.html genannten Nematizide, Bakterizide, Fungizide, Insektizide, Insekten-Repellents, Akarizide und Molluskizide.

Beispiele für bakterizide und fungizide Substanzen umfassen:
- Antibiotioka, z.B. Cycloheximid, Griseofulvin, Kasugamycin, Natamycin, Polyoxin, Streptomycin, Penizillin oder Gentamycin;
- Organische Verbindungen und Komplexe biozider Metalle, z.B. Komplexe des Silbers, Kupfers, Zinns und/oder Zinks wie Bis-(tributyllzinn)oxid, Kupfer-, Zink- und Zinn-Naphthenate, Oxine-Kupfer wie Cu-8, Tris-N-(cyclohexyldiazeniumdioxy)-aluminium, N-(Cyclohexyldiazeniumdioxy)-tributylzinn, Bis-N-(cyclohexyldiazeniumdioxy)-kupfer;;
- Quarternäre Ammoniumsalze, z.B. Benzyl-C₈-C₁₈-alkyldimethylammoniumhalogenide, insbesondere Chloride (Benzalkoniumchloride);
- aliphatische Stickstofffungizide und Bactericide wie Cymoxanil, Dodin, Dodicin, Guazidine, Iminoctadin, Dodemorph, Fenpropimorph, Fenpropidin, Tridemorph,
- Substanzen mit Peroxidgruppen wie Wasserstoffperoxid, und organische Peroxide wie Dibenzoylperodid;
- Organische Chlorverbindungen wie z. B. Chlorhexidin;
- Triazolfungizide wie Azaconazol, Cyproconazol, Diclobutrazol, Difenoconazol, Diniconazol, Epoxiconazo, Fenbuconazol, Fluquinconazol, Flusilazol, Flutriafol, Hexaconazol, Metconazol, Propiconazol, Tetraconazol, Tebuconazol und Triticonazol;
- Strobilurine wie Dimoxystrobin, Fluoxastrobin, Kresoxim-methyl, Metominostrobin, Orysastrobin, Picoxystrobin, Pyraclostrobin und Trifloxystrobin
- Sulfonamide wie Tolylfluanid und Diclofluanid;
- lodverbindungen wie Diiodmethyl-p-tolylsulfon, Napcocide 3-lod-2-propinylalkohol, 4-Chlorphenyl-3-iodpropargylformal, 3-Brom-2,3-diiod-3-propenylethylcarbonat, 2,3,3-Triiodallylalkohol, 3-lod-2-propinyl-n-hexylcarbamat, 3-Brom-2,3-diiod-2-propenylalkohol, 3-lod-2-propinylphenylcarbamat, 3-lod-2-propinyl-n-butylcarbamat, O-1-(6-lod-3-oxohex-5-inyl)phenylcarbamat, O-1-(6-lod-3-oxohex-5-inyl)butylcarbamat;
- Isothiazolinone wie N-Methylisothiazolin-3-on, 5-Chloro-N-methylisothiazolin-3-on, 4,5-Dichloror-N-octylisothiazolin-3-on, 1,2-Benzisothiazol-3(2H)on, 4,5-Trimethylisothiazol-3-on und N-Octyl-isothiazolin-3-on.

Beispiele für Insektizide und Acarizide sind
- Organophosphate wie Acephate, Azamethiphos, Azinphos-methyl, Chlorpyrifos, Chlorpyriphos-methyl, Chlorfenvinphos, Diazinon, Dichlorvos, Dicrotophos, Dimethoate, Disulfoton, Ethion, Fenitrothion, Fenthion, Isoxathion, Malathion, Methamidophos, Methidathion, Methyl-Parathion, Mevinphos, Monocrotophos, Oxydemeton-methyl, Paraoxon, Parathion, Phenthoate, Phosalone, Phosmet, Phosphamidon, Phorate, Phoxim, Pirimiphos-methyl, Profenofos, Prothiofos, Sulprophos, Triazophos, Trichlorfon;
- insbesondere Pyrethroide wie Acrinatrin, Allethrin, Bioallethrin, Barthrin, Bifenthrin, Bioethanomethrin, Cyclethrin, Cycloprothrin, Cyfluthrin, beta-Cyfluthrin, Cyhalothrin, gamma-Cyhalothrin, lambda-Cyhalothrin, Cypermethrin, α-Cypermethrin, ß-Cypermethrin, λ-Cypermethrin, zeta-Cypermethrin, Cyphenothrin, Deltamethrin, Dimefluthrin, Dimethrin, Empenthrin, Fenfluthrin, Fenprithrin, Fenpropathrin, Fenvalerat, Esfenvalerat, Flucythrinat, Fluvinate, tau-Fluvinate, Furethrin, Permethrin, Biopermethrin, Trans-Permethrin, Phenothrin, Prallethrin, Profluthrin, Pyresmethrin, Resmethrin, Bioresmethrin, Cismethrin, Tefluthrin, Terallethrin, Tetramethrin, Tralomethrin, Transfluthrin, Etofenprox, Flufenprox, Halfenprox, Protrifenbute und Silafulfen.
- Pyrrol- und Pyrazol-Insektizide wie Acetoprole, Ethiprole, Fipronil, Tebufenpyrad, Tolfenpyrad, Chlorfenapyr und Vaniliprole.

Beispiele für Repellent-Wirkstoffe sind insbesondere Anthrachinon, Acridinbasen, Kupfernaphthenat, Butopyronoxyl, Dibutylphthalat, Dimethylphthalat, Dimethylcarbat, Ethohexadiol, Hexamide, Methoquin-Butyl, N-Methylneodecanamid, Kampfer, Bergamotte-Öl, Pyrethrum, Nelkenöl, Geraniumöl, Thymianöl und insbesondere Diethyl-m-toluamid und 1-Piperidincarboxylsäure-2-(2-hydroxyethyl)-1-methylpropylester (Picardin).

Beispiele für Wellness-Additive sind insbesondere die nachfolgend aufgeführten Substanzen und Substanzgemische, z.B.
- Fette, vorzugsweise pflanzlichen Ursprungs, z.B. Lecithine,
- Pflanzenöle wie Jojoba-Öl, Teebaumöl, Nelkenöl, Nachtkerzenöl, Mandelöl, Kokosöl, Avocadoöl, Sojaöl und dergleichen,
- Fettsäuren, z.B. ω-6-Fettsäuren, Linolensäure, Linolsäure,
- Wachse tierischen oder planzlichen Ursprungs wie Bienenwachs, Candelillawachs, Sheabutter, Shoreabutter, Mangokernbutter, Japanwachs und dergleichen,
- Vitamine, insbesondere fettlösliche Vitamine, z. B. Tocopherole, Vitamin E, Vitamin A und dergleichen,
- Cortico-Steroide wie Cortison, Corticosteron, Dexamethason, Triamcinolon, Methylprednisolon, Fludrocortison, Fluocortolon, Prednison, Prednisolon, Progesteron,
- Aminosäuren, z.B. Arginin, Methionin,
- Pflanzenextrakte wie Algenextrakt, Rosskastanienextrakt, Mangoextrakt und dergleichen.

Zur Verbesserung der Waschpermanenz der Ausrüstung hat es sich bewährt, wenn man die amylosehaltige Substanz mit einem Bindemittel auf dem Textil fixiert. Als Bindemittel kommen zum einen filmbildende, wasserunlösliche Polymere und zum anderen niedermolekulare reaktive Substanzen, die unter Erwärmen polymerisieren, in Betracht. In der Regel wird man das Bindemittel in einer Menge einsetzten, dass das Gewichtsverhältnis von amylosehaltiger Substanz zu wasserunlöslichem Polymer im Bereich von 1:1 bis 100:1, vorzugsweise im Bereich von 1,5:1 bis 50:1 und insbesondere im Bereich von 2:1 bis 20:1 liegt.

In der Regel werden die filmbildenden Polymere in Form einer wässrigen Dispersion von feinteiligen Polymerteilchen eingesetzt. Die Teilchengröße ist für den Erfolg von untergeordneter Bedeutung. Sie liegt jedoch in der Regel unterhalb 5 µm (Gewichtsmittel) und beträgt in der Regel 50 nm bis 2 µm.

Das filmbildende Polymer kann insbesondere eine Glasübergangstemperatur T_{G} im Bereich von -40 bis 100 °C, vorzugsweise -30 bis +60 °C, insbesondere -20 bis +40 °C, aufweist. Sofern das polymere Bindemittel mehrere Polymerkomponenten umfasst sollte zumindest der Hauptbestandteil eine Glasübergangstemperatur in diesem Bereich aufweisen. Insbesondere liegt die Glasübergangstemperatur des Hauptbestandteils im Bereich von -30 °C bis +60 °C und besonders bevorzugt im Bereich von -20 °C bis +40 °C. Vorzugsweise weisen alle polymeren Bestandteile eine Glasübergangstemperatur in diesen Bereichen auf. Die angegebenen Glasübergangstemperaturen beziehen sich dabei auf die gemäß ASTM-D 3418-82 mittels DSC bestimmte "midpoint temperature". Im Falle vernetzbare Bindemittel bezieht sich die Glasübergangstemperatur auf den unvernetzten Zustand.

Beispiele für geeignete filmbildende Polymere basieren auf den folgenden Polymerklassen:
(1) Polyurethan-Harze
(2) Acrylatharze (Reinacrylate: Copolymere aus Alkylacrylaten und Alkylmethacrylaten);
(3) Styrolacrylate (Copolymere aus Styrol und Alkylacrylaten);
(4) Styrol/Butadien-Copolymerisate;
(5) Polyvinylester, insbesondere Polyvinylacetate und Copolymere des Vinylacetats mit Vinylpropionat;
(6) Vinylester-Olefin-Copolymere, z. B. Vinylacetat/Ethylen-Copolymere;
(7) Vinylester-Acrylat-Copolymere, z. B. Vinylacetat/Alkylacrylat-Copolymere sowie Vinylacetat/Alkylacrylat/Ethylen-Terpolymere;

Derartige Polymere sind bekannt und im Handel erhältlich, z. B. Polymere der Klassen (2) bis (7) in Form wässriger Dispersionen unter den Bezeichnungen ACRONAL, STYROFAN, BUTOFAN (BASF-AG), MOWILITH, MOWIPLUS, APPRETAN (Clariant), VINNAPAS, VINNOL (WACKER). Wässrige, für das Verfahren geeignete Polyurethan-Dispersionen (1) sind insbesondere solche, die für die Beschichtung von Textilien verwendet werden (siehe z. B. J. Hemmrich, Int. Text. Bull. 39, 1993, Nr.2, S. 53-56; "Wässrige Polyurethan-Beschichtungssysteme" Chemiefasern/Textilind. 39 91 (1989) T149, T150; W. Schröer, Textilveredelung 22, 1987, S. 459-467). Wässrige Polyurethandispersionen sind im Handel erhältlich, z. B. unter den Handelsbezeichnungen Alberdingk® der Fa. Alberdingk, Impranil® der Fa. BAYER AG, Permutex® der Fa. Stahl, Waalwijk, Niederlande, der Fa. BASF SE oder können nach bekannten Verfahren hergestellt werden, wie sie beispielsweise in "Herstellverfahren für Polyurethane" in Houben-Weyl, "Methoden der organischen Chemie", Band E 20/Makromolekulare Stoffe, S. 1587, D. Dietrich et al., Angew. Chem. 82 (1970), S. 53 ff., Angew. Makrom. Chem. 76, 1972, 85 ff. und Angew. Makrom. Chem. 98, 1981, 133-165, Progress in Organic Coatings, 9, 1981, S. 281-240, bzw. Römpp Chemielexikon, 9. Auflage, Band 5, S. 3575 beschrieben werden.

Die filmbildenden Polymere können selbstvernetzend sein, d. h. die Polymere weisen funktionelle Gruppen (vernetzbare Gruppen) auf, die beim Trocknen der Zusammensetzung, gegebenenfalls beim Erwärmen, miteinander, mit den funktionellen Gruppen der Amylose oder mit einem niedermolekularen Vernetzer unter Bindungsbildung reagieren.

Beispiele für vernetzbare funktionelle Gruppen umfassen aliphatisch gebundene OH-Gruppen, NH-CH₂-OH-Gruppen, Carboxylat-Gruppen, Anhydrid-Gruppen, verkappte Isocyanatgruppen und Aminogruppen. Häufig wird man ein Polymer verwenden, dass noch freie OH-Gruppen als reaktive Gruppen aufweist. In der Regel beträgt der Anteil der reaktiven funktionellen Gruppen 0,1 bis 3 mol/kg Polymer. Die Vernetzung kann innerhalb des Polymers durch Reaktion komplementär-reaktiver funktioneller Gruppen bewirkt werden. Vorzugsweise bewirkt man die Vernetzung des Polymers durch Zugabe eines Vernetzers, der reaktive Gruppen aufweist, welche hinsichtlich ihrer Reaktivität zu den funktionellen Gruppen des Vernetzers komplementär sind. Geeignete Paare funktioneller Gruppen, die eine komplementäre Reaktivität aufweisen sind dem Fachmann bekannt. Beispiele für solche Paare sind OH/COOH, OH/NCO, NH₂/COOH, NH₂/NCO sowie M²⁺/COOH, wobei M²⁺ für ein zweiwertiges Metallion wie Zn²⁺, Ca²⁺, oder Mg²⁺ steht. Beispiele für geeignete Vernetzer sind die nachstehend bei den Polyurethanen genannten Di- oder Polyole; primäre oder sekundäre Diamine, vorzugsweise primäre Diamine, z. B. Alkylendiamine wie Hexamethylendiamin, Diethylentriamin, Triethylentetramin, Tetraethylenpentamin, N,N-Bis[(aminopropyl)amino]-ethan, 3,6-Dioxaoctandiamin, 3,7-Dioxanonandiamin, 3,6,9-Trioxaundecandiamin oder Jeffamine, (4,4'-Diaminodicyclohexyl)methan, (4,4'-Diamino-3,3-dimethyldicyclohexyl)methan; Aminoalkohole wie Ethanolamin, Hydroxypropylamin; ethoxylierte Di- und Oligoamine; Dihydrazide von aliphatischen oder aromatischen Dicarbonsäuren wie Adipinsäuredihydrazid; Dialdehyde wie Glyoxal; teilweise oder vollständig O-methylierte Melamine, sowie Verbindungen oder Oligomere, die im Mittel zwei oder mehr, vorzugsweise drei oder mehr Isocyanatgruppen oder reversibel z. B. Hydrogensulfit blockierte Isocyanatgruppen aufweisen. In diesem Fall wird das Mengenverhältnis von Vernetzer zu polymerem Bindemittel so bemessen, dass das Molverhältnis der reaktiven Gruppen im polymeren Bindemittel (Gesamtmenge der reaktiven Gruppen in den Polymeren) zu den reaktiven Gruppen im Vernetzer in der Regel im Bereich von 1:10 bis 10:1 und vorzugsweise im Bereich von 3:1 bis 1:3 liegt. Üblicherweise liegt das Gewichtsverhältnis von polymerem Bindemittel (gerechnet als Feststoff) zu Vernetzer im Bereich von 100:1 bis 1:1 und insbesondere im Bereich von 50:1 bis 5:1.

Alternativ zur Fixierung der amylosehaltigen Substanz mit wasserunlöslichen Polymeren kann man die Amylose bzw. die amylosehaltigen Substanz auch mit reaktiven Verbindungen, die wenigstens eine, gegenüber den OH-Gruppen der Amylose reaktive Gruppe und wenigstens eine weitere funktionelle Gruppe aufweisen, die gegenüber den funktionellen Gruppen auf den Fasern des textilen Materials, z.B. OH-Gruppen, NH₂-Gruppen oder COOH-Gruppen, reaktiv ist, auf dem textilen Material fixieren. Zu den reaktiven Verbindungen zählen die oben genannten Vernetzer sowie die in DE-A 40 35 378 für die Fixierung von Cyclodextrinen vorgeschlagenen Substanzen, z.B. N-Hydroxmethyl- und N-Alkoxymethylderivate von Harnstoff oder harnstoffartigen Verbindungen wie Dimethylolharnstoff (Bis(hydroxymethyl)harnstoff), Di(methoxymethyl)-harnstoff, Dimethylolalkandioldiurethane wie N,N-Dimethylolethylenharnstoff (N,N-Bis(hydroxymethyl)imidazolin-2-on), N,N-Dimethylol-dihydroxyethylenharnstoff (N,N-Bis(hydroxymethyl)-4,5-dihydroxyimidazolin-2-on), Dimethylolpropylenharnstoff und dergleichen. Derartige Materialien sind in Form wässrige Formulierungen für die Ausrüstung von Textilien im Handel, z.B. unter den Handelsbezeichnungen Fixapret® und Fixapret®-eco der BASF SE. Zu den reaktiven Materialien, die zur Fixierung der amylosehaltigen Substanz auf dem textilen Material genutzt werden können, zählen insbesondere auch Verbindungen mit 2, 3, 4 oder mehr (gegebenenfalls reversibel blockierten) Isocyanat-Gruppen, speziell die mit Bisulfit oder CH-aciden Verbindungen oder Oximene, z.B. Butanonoxim reversibel blockierten Polyisocyanat-Präpolymere auf Basis von Polyether- und Polyesterurethanen, die in DE 2837851, DE 19919816 und der älteren Europäischen Patentanmeldung 03015121 beschrieben werden. Derartige Produkte sind auch kommerziell erhältlich, beispielsweise unter den Handelsbezeichnungen PROTOLAN®367 und PROTOLAN®357 der Rotta GmbH, Mannheim.

Zur Fixierung der amylosehaltigen Substanz kann auch die für die Fixierung von Cyclodextrinen bekannte Vorgehensweise in analoger Weise genutzt werden, bei der man das Cyclodextrin bzw. im vorliegenden Fall die amylosehaltige Substanz mit Reaktivankern versieht, beispielsweise indem man sie mit Dicarbonsäuren oder Dicarbonsäureanhydriden wie Maleinsäure, Fumarsäure, Maleinsäureanhydrid, Bernsteinsäure, Bernsteinsäureanhydrid oder Adipinsäure, mit Diisocyanaten, z. B. Toluoldiisocyanat, Isophorondiisocyanat, Tetramethylendiisocyanat oder Hexamethylendiisocyanat, oder mit Aminocarbonsäuren in an sich bekannter Weise dergestalt umsetzt, dass nur eine der in diesen Verbindungen vorhandenen Funktionalitäten mit den OH-Gruppen der amylosehaltigen Substanz reagiert und die andere zur Anbindung an die reaktiven Gruppen des Fasermaterials erhalten bleibt. Reaktivanker lassen sich auf der amylosehaltigen Substanz auch durch Umsetzung mit 1,3,5-Trichlortriazin, 2,3-Dichlorchinoxalin-5,6-carbonsäurechlorid sowie mit Chlordifluorpyrimidin erzeugen.

Ferner können zur Fixierung der Amylose auch Alkoxysilane, wie Diethoxydimethylsilan, Dimethoxydimethylsilan, Triethoxyphenylsilan, Tetraethoxysilan sowie dimere, trimere und höhere Kondensationsprodukte dieser Verbindungen eingesetzt werden.

Auf diese Weise können grundsätzlich alle textilen Materialien ausgerüstet werden, d.h. nicht konfektionierte Ware als auch konfektionierte Ware. Textile Materialien umfassen hier und im Folgenden Gewebe, Gestricke, Gewirke und Vliese. Die textilen Materialen können aus Naturfasergarnen, Synthesefasergarnen und/oder Mischgarnen aufgebaut sein. Als Fasermaterialien kommen grundsätzlich alle für die Herstellung von Textilien üblicherweise eingesetzten Fasermaterialien in Betracht. Hierzu zählen Baumwolle, Wolle, Hanffaser, Sisalfasern, Flachs, Ramie, Polyacrylnitrilfasern, Polyesterfasern, Polyamidfasern, Viskosefasern, Seide, Acetatfasern, Triacetatfasern, Aramidfasern und dergleichen sowie Mischungen dieser Fasermaterialien.

Das Ausrüsten bzw. Behandeln der textilen Materialien mit der amylosehaltigen Substanz kann in an sich bekannter Weise, z.B. mittels der in DE-A 4035378 für die Ausrüstung von Textilien mit Cyclodextrinen beschriebenen Verfahren erfolgen.

Zu nennen sind beispielsweise Verfahren, bei denen die amylosehaltige Substanz, gegebenenfalls als Komplex mit dem Wirkstoff bereits in die Faser, das Filament und/oder das Garn eingesponnen ist, aus dem das Gewebe hergestellt ist.

Häufig wird man jedoch das textile Material vor oder nach Konfektionierung mit der amylosehaltigen Substanz oder einem Komplex aus amylosehaltiger Substanz und Wirkstoff behandeln. In der Regel wird man hierzu das Textil mit einer wässrigen Flotte behandeln, welche die amylosehaltige Substanz und gegebenenfalls den Wirkstoff in ausreichender Menge enthält. Je nach Art des Auftrags und der gewünschten Menge, in der die amylosehaltiger Substanz aufgebracht werden soll, liegt die Konzentration an amylosehaltiger Substanz in der Flotte im Bereich von 1 bis 40 Gew.-%, insbesondere im Bereich von 2 bis 20 Gew.-% und speziell im Bereich von 4 bis 15 Gew.-%.

Die Art der Behandlung ist von untergeordneter Bedeutung und kann beispielsweise als Minimalauftrag, z.B. durch Sprühauftrag, als Normalauftrag im Foulard oder als Hochfeuchteauftrag erfolgen. Hierbei wird das textile Material mit der wässrigen Flotte getränkt. Gegebenenfalls kann man danach überschüssige Flotte entfernen, z.B. durch Abquetschen auf eine Flottenaufnahme von etwa 30 bis 120 %.

Eine andere Möglichkeit zur Behandlung des Textils mit amylosehaltiger Substanz bzw. Komplex aus amylosehaltiger Substanz und Wirkstoff, ist eine Flotte mit Wasser anzusetzen, in der die gewünschten Menge an amylosehaltiger Substanz und gegebenenfalls Wirkstoff enthalten ist, z.B. 0,5 bis 20 Gew.-% (bezogen auf die Masse der auszurüstenden Textilie). Das textile Material wird über einen gewissen Zeitraum, z.B. 10-60 min mit der Behandlungsflotte in hierfür geeigneten Ausrüstungsaggregaten (z.B. Haspelkufe; Rollenkufe; Paddel; etc.) durchtränkt und danach wie oben angegeben abgequetscht und/oder abgeschleudert. Das Flottenverhältnis liegt hierbei in der Regel im Bereich von 1:2 bis 1:50 und insbesondere im Bereich von 1:3 bis 1:20.

Derartige Verfahren sind dem Fachmann bekannt, beispielsweise aus H.K Rouette, Lexikon der Textilveredlung, Laumann-Verlag, Dülmen 1995, S. 669 ff.

In der Regel schließt sich an die Behandlung mit der Flotte ein Trocknungsvorgang an. Die Temperaturen liegen dabei in der Regel im Bereich von 100 bis 200°C und vorzugsweise im Bereich von 120 bis 180°C. Das Trocknen kann in den hierfür üblichen Vorrichtungen durchführen, bei konfektionierter Ware beispielsweise durch trockentumblen bei den oben angegebenen Temperaturen. Bei nicht-konfektionierter Ware wird man in der Regel im Anschluss an den Auftrag das textile Material über ein oder mehrere Spannrahmen führen.

Sofern die amylosehaltige Substanz zusammen mit einem filmbildenden Polymer eingesetzt wird, führt das Trocknen zu einer Fixierung der amylosehaltigen Substanz auf den Textilfasern. In der Regel wird dann die Trocknungstemperatur 100 nicht unterschreiten und liegt vorzugsweise im Bereich von 120 bis 200°C und insbesondere im Bereich von 140 bis 180°C. Im Allgemeinen erfolgt das Trocknen während einer Zeitdauer von 1 bis 10 min, insbesondere 1 bis 2 min, wobei längere Trockenzeiten ebenfalls geeignet sind.

Für das Behandeln mit einer wässrigen Flotte hat es sich als vorteilhaft erwiesen, wenn die wässrige Flotte neben der amylosehaltigen Substanz und gegebenenfalls dem Wirkstoff wenigstens eine oberflächenaktive Substanz (bzw. grenzflächenaktive Substanz) enthält, welche zur Dispergierung der amylosehaltigen Substanz und dem Wirkstoff in der wässrigen Flotte geeignet ist. Vorzugsweise handelt es sich bei der oberflächenaktiven Substanz um ein oligomeres oder polymeres Dispergiermittel. Der Begriff oligomeres oder polymeres Dispergiermittel umfasst im Unterschied zu niedermolekularen oberflächenaktiven Substanzen solche Dispergiermittel, deren zahlenmittleres Molekulargewicht in der Regel wenigstens 2000 Dalton beträgt, z.B. 2000 bis etwa 100000 Dalton und insbesondere im Bereich von etwa 3000 bis 70000 Dalton liegt.

In der Regel enthält die wässrige Flotte das polymere oder oligomere Dispergiermittel in einer Menge von 0,5 bis 20 Gew.-%, vorzugsweise 1 bis 18 Gew.-% und insbesondere 5 bis 15 Gew.-%, bezogen auf die amylosehaltige Substanz.

Geeignete oligomeres oder polymeres Dispergiermittel sind in Wasser löslich und umfassen sowohl neutrale und amphotere wasserlösliche Polymere als auch kationische und anionische Polymere, wobei letztere bevorzugt sind.

Beispiele für neutrale polymere Dispergiermittel sind Polyethylenoxid, Ethylenoxid/ Propylenoxid-Copolymere, bevorzugt Blockcopolymere, Polyvinylpyrrolidon sowie Copolymere von Vinylacetat mit Vinylpyrrolidon.

Die bevorzugten anionischen oligomeren bzw. polymeren Dispergiermittel zeichnen sich dadurch aus, dass sie Carboxylgruppen und/oder Sulfonsäuregruppen aufweisen und üblicherweise als Salze, z.B. als Alkalimetallsalze oder Ammoniumsalze eingesetzt werden.

Bevorzugte anionische Dispergiermittel sind beispielsweise carboxylierte Derivate der Zellulose wie Carboxymethylcellulose, Homopolymere ethylenisch ungesättigter C₃-C₈-Mono- und C₄-C₈-Dicarbonsäuren, z.B. der Acrylsäure, der Methacrylsäure, der Maleinsäure, der Itaconsäure, Copolymere wenigstens zweier verschiedener ethylenisch ungesättigter C₃-C₈-Mono- und C₄-C₈-Dicarbonsäuren wie vorstehend genannt, und Copolymere wenigstens einer der vorgenannten ethylenisch ungesättigten C₃-C₈-Mono- oder C₄-C₈-Dicarbonsäure mit wenigstens einem neutralen Comonomeren. Beispiele für neutrale Comonomere sind N-Vinyllactame wie N-Vinylpyrrolidon, Vinylester aliphatischer C₂-C₁₆-Carbonsäuren wie Vinylacetat, Vinylpropionat, Amide der vorgenannten ethylenisch ungesättigten Carbonsäuren, wie Acrylamid, Methacrylamid und dergleichen, Hydroxy-C₁-C₄-alkyl(meth)acrylate wie Hydroxyethylacrylat und -methacrylat, Ester ethylenisch ungesättigter C₃-C₈-Mono- oder C₄-C₈-Dicarbonsäuren mit Polyethern, z.B. Ester der Acrylsäure oder der Methacrylsäure mit Polyethylenoxiden oder Ethylenoxid/Propylenoxid-Blockcopolymeren, Vinylaromaten wie Styrol und C₂-C₁₆-Olefine wie Ethylen, Propen, 1-Hexen, 1-Octen, 1-Decen, 1-Dodecen und dergleichen. Weiterhin bevorzugt sind Homopolymere ethylenisch ungesättigter Sulfonsäuren wie Styrolsulfonsäure und Acrylamidopropansulfonsäure und deren Copolymer mit den vorgenannten Comonomeren. In den Copolymeren wird der Anteil der ethylenisch ungesättigten Säure in der Regel wenigstens 20 Gew.-% betragen und einen Wert von 90 Gew.-% und insbesondere 80 Gew.-%, jeweils bezogen auf das Gesamtgewicht aller das Polymer konstituierenden Monomere, nicht überschreiten.

Copolymerisate aus mindestens einer der oben genannten Säuren und mindestens einem Comonomer sind für diesen Zweck bekannt und im Handel erhältlich, beispielsweise die Copolymere von Acrylsäure und Maleinsäure als Sokalan-Marken der BASF-AG.

Ebenfalls bevorzugte anionische Dispergiermittel sind Phenolsulfonsäure-Formaldehyd-Kondensate und Naphthalinsulfonsäure-Formaldehyd-Kondensate (z.B. die Tamol- und Setamol-Marken der BASF) und Ligninsulfonate.

Brauchbare Dispergiermittel sind außerdem niedermolekulare anionische, nicht-ionische, kationische, ampholytische und zwitterionische Tenside. Geeignete Tenside sind z.B. die Alkalimetall-, Ammonium- oder Aminsalze von C₈-C₁₈-Alkylsulfaten, wie Natriumlaurylsulfat; C₈-C₁₈-Alkylsulfonaten, wie Dodecylsulfonat; C₈-C₁₈-Alkylethersulfaten; sowie C₈-C₁₈-Alkylethoxylate; Polyoxyethylensorbitanester; C₈-C₁₈-Alkylglycinate; C₈-C₁₈-Alkyldimethylaminoxide; Betaine etc. Bevorzugt sind die Alkylsulfate und Alkylsulfonate.

Sofern die amylosehaltige Substanz nicht gemeinsam mit einem filmbildenden, wasserunlöslichen Polymer eingesetzt wird, kann das Textil in einem separaten Arbeitsschritt mit dem Polymer behandelt werden. Insbesondere erfolgt die Behandlung gemeinsam mit der amylosehaltigen Substanz. Dementsprechend betrifft eine besondere Ausführungsform ein Verfahren, bei dem die wässrige Flotte zusätzlich ein dispergiertes, filmbildendes, wasserunlösliches Polymer der oben bezeichneten Art umfasst. Die Menge an filmbildenden Polymer ist dabei so gewählt, dass das Gewichtsverhältnis von amylosehaltiger Substanz zu wasserunlöslichem Polymer im Bereich von 1:1 bis 100:1, vorzugsweise im Bereich von 1,5:1 bis 50:1 und insbesondere im Bereich von 2:1 bis 20:1 liegt.

Die Ausrüstung des Textils mit dem Wirkstoff kann in einem separaten Vorgang oder in einem Arbeitsgang zusammen mit der Ausrüstung mit der amylosehaltigen Substanz erfolgen.

Sofern man das Textil in einem separaten Arbeitsgang mit dem Wirkstoff ausrüstet, wird man das Textil zweckmäßigerweise ebenfalls mit einer wässrigen Flotte des Wirkstoffs behandeln. Hierzu wird man in der Regel den Wirkstoff, der üblicherweise in Wasser nicht löslich ist, in Wasser emulgieren oder dispergieren, gegebenenfalls unter Verwendung geeigneter oberflächenaktiver Substanzen. Geeignete oberflächenaktive Substanzen sind insbesondere die zuvor erwähnten niedermolekularen Tenside und hierunter bevorzugt die nichtionischen Tenside, insbesondere Polyoxyethylensorbitanester, Ester von Mono- oder Oligosacchariden mit C₆-C₁₈-Fettsäuren und besondere bevorzugt C₈-C₁₈-Alkylethoxylate, insbesondere solche mit einem Ethoxylierungsgrad im Bereich von 6 bis 50. In der Regel enthält die wässrige Flotte den Wirkstoff in einer Menge von 0,1 bis 10 Gew.-% und insbesondere in einer Menge von 0,2 bis 5 Gew.-%. Die Menge an oberflächenaktiver Substanz liegt in der Regel im Bereich von 0,5 bis 50 Gew.-% und insbesondere im Bereich von 3 bis 30 Gew.-%, bezogen auf den Wirkstoff. Das Aufbringen des Wirkstoffs aus wässriger Flotte kann mit den hierfür üblichen Methoden erfolgen, z.B. mittels eines Foulards.

Man kann aber auch die Ausrüstung mit Wirkstoff und amylosehaltiger Substanz in einem Arbeitsgang durchführen. Dabei kann man grundsätzlich wie für die Ausrüstung mit der amylosehaltigen Substanz beschrieben vorgehen, wobei die wässrige Flotte der amylosehaltigen Substanz nunmehr zusätzlich den wenigstens einen Wirkstoff enthält. Der Wirkstoff kann dabei separat der Flotte zugegeben werden oder in Form einer Einschlussverbindung, d.h. in Form eines Wirt-Gast-Komplexes mit der amylosehaltigen Substanz.

Das Verfahren kann zur Ausrüstung beliebiger Textilien, einschließlich Gewebe, Gewirke, Vliese und dergleichen eingesetzt werden. Die Art des textilen Materials richtet sich in erster Linie nach dem gewünschten Anwendungszweck.

Bei den auszurüstenden Textilien kann es sich um fertig konfektionierte Produkte wie Bekleidung, einschließlich Unterwäsche und Oberbekleidung, wie z.B. Hemden, Hosen, Jacken, Outdoor-, Trecking- und Militärausrüstungen, Dächer, Zelte, Netze, z.B. Insektenschutznetze und Vorhänge, Hand- und Badetücher, Bettwäsche und dergleichen handeln.

In gleicher Weise kann die Ausrüstung auf der Rohware in Ballen- oder Rollenform erfolgen.

Die mit Wirkstoffen gegen parasitäre Organismen wie Insekten und Acariden ausgerüsteten Textilien sind neben dem Schutz des Menschen auch besonders im Tierschutz zum Schutz gegen Zecken, Milben, Flöhe und dergleichen geeignet.

Die textilen Materialen können aus Naturfasergarnen, Synthesefasergarnen und/oder Mischgarnen aufgebaut sein, wobei die Gewebe üblicherweise ein Flächengewicht im Bereich von 10 bis 500 g/m² vorzugsweise 20 bis 250 g/m² aufweisen. Als Fasermaterialien kommen grundsätzlich alle für die Herstellung von Textilien üblicherweise eingesetzten Fasermaterialien in Betracht. Hierzu zählen Baumwolle, Wolle, Hanffaser, Sisalfasern, Flachs, Ramie, Polyacrylnitrilfasern, Polyesterfasern, Polyamidfasern, Viskosefasern, Seide, Acetatfasern, Triacetatfasern, Aramidfasern und dergleichen sowie Mischungen dieser Fasermaterialien. Geeignet sind auch Glasfasern sowie Mischungen der vorgenannten Fasermaterialien mit Glasfasern z. B. Glasfaser/Kevlar-Mischungen.

Mit einer oben beschriebenen amylose-basierten Wirkstoff-Ausrüstung verbleiben die Wirkstoffe auch nach mehreren Wäschen in den damit ausgerüsteten Textilien. Zudem zeichnen sich die so ausgerüsteten Textilien durch einen angenehmen Griff aus, was insbesondere für den Tragekomfort von aus diesen Textilien hergestellter Bekleidung von Vorteil ist.

### 4.7 Kühlende Tabakprodukte (erfindungsgemäß, soweit sie unter die Ansprüche fallen)

Grundsätzlich kann der Wirkstoffgehalt über einen weiten Bereich, wie z.B. 0,00001 bis 50 Gew.-%, insbesondere 0,001 bis 10 Gew.-% oder 0,005 bis 1 Gew.-% variieren.

Die oben beschriebenen Wirkstoffe können in vorteilhafter Weise auch zur Herstellung von Tabakprodukten verwendet werden. Beispiele für derartige Tabakprodukte umfassen, Zigarren, Zigaretten, Pfeifentabak, Kautabak, und Schnupftabak.

Die Herstellung von Tabakprodukten, welche mit kühlend wirkende Zusätzen ergänzt sind, ist an sich bekannt und z.B. beschrieben in US 3,111,127, US 5,752,529 und US 2005/0000529, worauf hiermit ausdrücklich Bezug genommen wird.

### 4.8 Kühlende Verpackungsmaterialien (erfindungsgemäß, sofern sie unter die Ansprüche fallen)

Die oben beschriebenen Wirkstoffe sind auch in vorteilhafter Weise zur Herstellung von Verpackungsmaterialien geeignet.

Die Herstellung erfolgt dabei ebenfalls in an sich bekannter Weise. Die Wirkstoffe können dabei in das Verpackungsmaterial, in freier oder z.B. verkapselter Form, eingearbeitet oder auf das Verpackungsmaterial, in freier oder verkapselter Form aufgebracht werden.

So sind entsprechend ausgerüstete Kunststoffverpackungsmaterialien entsprechend den Angaben in der Literatur zur Herstellung von Polymerfilmen herstellbar (z.B. Ullmann, 6th Edn, 2003. Vol. 36, S. 567). Die Herstellung in geeigneter Weise beschichteter Papiere ist ebenfalls bekannt und z.B. beschrieben in Ullmann, Vol. 25, S. 106 ff, 6th Edn, 2003.

### 5. Wirkstoffkombinationen (erfindungsgemäß, sofern sie unter die Ansprüche fallen)

Gegebenenfalls können die oben beschriebenen Wirkstoffe (Kühwirkstoffe) der Strukturtypen 1, 2 und 3 mit weiteren bekannten Wirkstoffen, insbesondere auch solchen mit vergleichbarer Wirkung, kombiniert werden. Beispielsweise können diese mit bekannten kühlenden Verbindungen, wie z.B. Menthol, Menthon, N-Ethyl-p-menthancarboxamid (WS-3, auch Menthan-3-carbonsäure-N-ethylamid genannt), N-2,3-Trimethyl-2-isopropylbutanamid (WS-23), Menthyllactat (Frescolat® ML), Menthonglycerinacetal (Frescolat® MGA), Mono-menthylsuccinat (Physcool ®), Mono-menthylglutarat, O-Menthyl-glycerin, Menthyl-N,N-dimethylsuccinamat kombiniert werden.

Die oben beschriebenen Kühlwirkstoffe, insbesondere die der Tabelle 0 (siehe unten) können bevorzugt mit den folgenden Kühlwirkstoffen kombiniert werden: Menthol und Mentholderivate (z.B. L-Menthol, D-Menthol, racemisches Menthol, Isomenthol, Neoisomenthol, Neomenthol) Menthylether (z.B. (I-Menthoxy)-1,2-propandiol, (1-Menthoxy)-2-methyl-1,2-propandiol, I-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Mischungen), die Halbester von Mentholen mit einer Dicarbonsäure oder deren Derivate (z.B. Mono-Menthylsuccinat, Mono-Menthylglutarat, Mono-Menthylmalonat, O-Menthyl-bernsteinsäureester-N,N-(dimethyl)amid, O-Menthylbernsteinsäureesteramid), Menthancarbonsäureamide (z.B. Menthancarbonsäure-N-ethylamid [WS3], Nα-(Menthan-carbonyl)glycinethylester [WS5], Menthancarbonsäure-N-(4-cyanophenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben).

Die oben beschriebenen Kühlwirkstoffe, insbesondere die der Tabelle 0 (siehe unten), können besonders bevorzugt mit den folgenden Kühlwirkstoffen kombiniert werden: Menthylether (z.B. (I-Menthoxy)-1,2-propandiol, (I-Menthoxy)-2-methyl-1,2-propandiol), polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), nicht erfindungsgemäße Menthancarbonsäureamide (z.B. Menthancarbonsäure-N-ethylamid [WS3], Nα-(Menthancarbonyl)glycinethylester [WS5], Menthancarbonsäure-N-(4-cyanophenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, die in WO 2004/026840 beschrieben sind).

Die im gesamten Kapitel 4 und 5 genannten Ausführungen gelten auch für den speziellen Teil der Erfindung (Kapitel 6) sofern nicht anders angegeben.

### 6. Spezieller Teil der Erfindung (erfindungsgemäß, sofern die Gegenstände unter die Ansprüche fallen)

Wie oben bereits angedeutet betrifft der spezielle Teil der Erfindung die Verwendung ganz spezieller Verbindungen (TRPM8-Rezeptor-Modulatoren) zur Erzeugung einer langanhaltenden physiologischen Kühlwirkung auf Haut oder Schleimhaut.

Der spezielle Teil der Erfindung betrifft ferner Mischungen und Zubereitungen (Mittel), die spezielle solche Verbindungen umfassen.

Sie betrifft außerdem ein Verfahren zur Herstellung eines Arzneimittels sowie ein Verfahren zum Erreichen einer physiologischen Kühlwirkung auf der Haut und/oder Schleimhäuten, wobei jeweils besonders geeignete Verbindungen eingesetzt werden.

Physiologische Kühlwirkstoffe werden regelmäßig eingesetzt, um einen kühlen sensorischen Eindruck auf der Haut bzw. Schleimhaut, zum Beispiel auf der Schleimhaut im Mund-, Nasen- und/oder Rachenraum hervorzurufen, wobei allerdings tatsächlich keine physikalische Abkühlung wie zum Beispiel bei der Verdunstung von Lösungsmitteln stattfindet. Als physiologische Kühlwirkstoffe können sowohl Einzelkomponenten als auch Mischungen verwendet werden. Dabei ist zu berücksichtigen, dass nicht alle Verbindungen, die *in vitro* Rezeptoren beeinflussen, die (auch) an der Vermittlung einer physiologischen Kühlwirkung beteiligt sind, tatsächlich einen solchen Effekt *in vivo* auf der Haut oder auf Schleimhäuten erzeugen. Insbesondere wird ein solcher Effekt nicht immer identisch verlaufen. Das bedeutet zum Beispiel, dass die Stärke der vermittelten physiologischen Kühlwirkung sowie der Verlauf der Stärke der Kühlwirkung gegen Zeitdauer nicht alleine aus der Tatsache geschlossen werden können, dass eine bestimmte Verbindung ein Argonist eines an der Vermittlung eines Kühleindruckes beteiligten Rezeptors ist.

Der bekannteste physiologisch wirksame Kühlwirkstoff ist L-Menthol, der aber einige Nachteile besitzt, z. B. starker Geruchseindruck, eine hohe Flüchtigkeit und in höheren Konzentrationen einen bitteren und/oder scharfen Eigengeschmack, bzw. eine Haut reizende Wirkung.

Es wurde daher schon früher nach starken Kühlwirkstoffen gesucht, die nicht die nachteiligen Eigenschaften des I-Menthols aufweisen. So wurden z.B. Milchsäureester von Menthol(en) gemäß DE 2 608 226 und gemischte Carbonate mit Menthol(en) und Polyolen gemäß DE 4 226 043 und Menthonketale gemäß EP 0 507 190 beschrieben.

Menthylmonoester von Disäuren nach US 5,725,865 und US 5,843,466 sind zwar interessante natürlich vorkommende Alternativen, können aber in sensorischen Tests nicht die Stärke der vorher beschriebenen Kühlwirkstoffe erreichen.

In J. Soc. Cosmet. Chem. 1978, 29, 185-200 wurden die Ergebnisse einer Studie an ca. 1200 Verbindungen präsentiert, bei der die Verbindungen L-Menthancarbonsäure-*N*-ethylamid ("WS3") und insbesondere *N*^{α}-(L-Menthancarbonyl)-glycinethylester ("WS5") als die stärksten Kühlwirkstoffe gefunden wurden. Letzterer hat bei starker Wirkung aber den Nachteil, hydrolyseempfindlich zu sein und dabei die entsprechende freie Säure *N^{α}*-(L-Menthancarbonyl)-glycin zu bilden, die selbst nur noch eine sehr schwache Kühlwirkung zeigt. Trotz der beschriebenen ausführlichen Untersuchungen ist eine systematische Vorhersage zu den Eigenschaften von potentiellen Kühlwirkstoffen, insbesondere zu deren Bitterkeit und/oder deren anderen trigeminalen Effekten nicht möglich und auch nicht beschrieben. So sind auch viele unter die Klasse der Menthancarbonsäureamide fallende Moleküle zwar stark kühlend, zeigen jedoch häufig gleichzeitig ausgeprägt bittere Noten (z.B. die Menthancarbonsäure-N-(alkyloxyalkyl)amide nach JP 2004059474) oder sind zusätzlich stark reizend (WS5: N-[[5-Methyl-2-(1-methylethyl)cyclohexyl]carbonyl]glycinethylester, US 2005/0222256).

*N^{α}*-(Menthancarbonyl)alkyloxyalkylamide wurden in JP 2004059474 beschrieben. Diese haben bei starker Kühlwirkung und hoher Hydrolysestabilität jedoch den Nachteil, stark bitter zu sein, und sie sind somit in Nahrungsmitteln und auch in der Gesichtspflege dienenden kosmetischen Produkten nicht einsetzbar.

Des Weiteren sind Menthylglyoxylate und ihre Hydrate in JP 2005343795 als Kühlsubstanzen beschrieben worden.

Übersichten zu den bisher hergestellten und verwendeten Kühlwirkstoffen findet man bei M. Erman, Perfumer & Flavorist 32(10), 20-35 (2007**)** und M. L. Dewis in D. J. Rowe, Chemistry and Technology of Flavors and Fragrances, Blackwell Publishing Ltd, Oxford 2005, p. 212 - 222.

Es war die primäre Aufgabe des vorliegenden speziellen Aspektes der Erfindung, neue Mittelanzugeben, die eine besondere physiologische Kühlwirkung besitzen und dabei in Nahrungs- und/oder Genussmitteln und/oder Mundpflegeprodukten und/oder (oralen) pharmazeutischen Zubereitungen und/oder kosmetischen Zubereitungen als Kühlsubstanzen (Kühlwirkstoffe) eingesetzt werden können. Die anzugebenden Verbindungen bzw. Mischungen von Verbindungen sollten vorzugsweise einen möglichst schwachen Eigengeschmack zeigen, insbesondere wenig oder gar nicht bitter schmecken sowie möglichst nicht reizend sein.

Gelöst wird diese Aufgabe erfindungsgemäß durch die Verwendung eines Mittels umfassend wenigstens eine, zwei, drei oder mehr der Verbindungen ausgewählt aus der Gruppe A (Tabelle 0) bestehend aus

**Tabelle 0**

| LN | Struktur | Benennung in den Tabellen A, B oder C gemäß Testbeispiel 1 (siehe unten) |
|---|---|---|
| 23 | 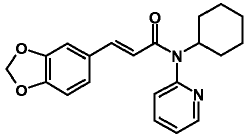 | 3-1 |
| 24 | 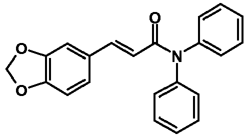 | 3-6 |
| | 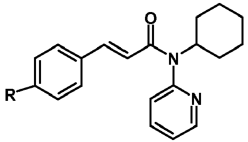 | |
| 25 | R = H | 3-25 |
| 26 | R = Me | 3-32 |
| 27 | R = MeO | 3-17 |
| | | |
| | 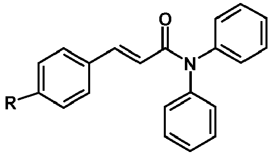 | |
| 28 | R = H | 3-27 |
| 29 | R = Me | 3-34 |
| 30 | R = MeO | 3-20 |

in einer Konzentration von 0,1 ppm bis 10 Gew-% bezogen auf das Gesamtgewicht des Mittels zum Erzielen einer Kühlwirkung auf Haut oder Schleimhaut, die verglichen mit der Kühlwirkung eines Mittels gleicher Zusammensetzung, bei dem lediglich die Verbindung oder die Verbindungen ausgewählt aus der Gruppe A gegen Menthancarbonsäure-N-ethylamid in gleicher Konzentration ausgetauscht sind, um wenigstens 10 Minuten verlängert ist, zu nichttherapeutischen Zwecken oder zur Herstellung eines Arzneimittels.

Die Benennung der Verbindungen lauten wie folgt:
LN 23
   3,4-Methylendioxyzimtsäure-N-cyclohexyl-N-2-pyridylamid
LN 24
   3,4-Methylendioxyzimtsäure-N,N-diphenylamid
LN 25
   Zimtsäure-N-cyclohexyl-N-2-pyridylamid
LN 26
   4-Methylzimtsäure N-cyclohexyl-N-2-pyridylamid
LN 27
   4-Methoxyzimtsäure-N-cyclohexyl-N-2-pyridylamid
LN 28
   Zimtsäure-N,N-diphenylamid
LN 29
   4-Methylzimtsäure-N,N-diphenylamid
LN 30
   4-Methoxyzimtsäure-N,N-diphenylamid

Sofern Differenzen zwischen der Namensbezeichnung und dem jeweiligen Formelbild bestehen sollten, ist die Aussage des Formelbildes maßgebend.

Für die Lösung der erfindungsgemäßen Aufgabe waren dabei vor allem Mittel mit Wirkstoffen gesucht, die eine besonders langandauernde Kühlempfindung vermitteln können. Bevorzugt sollten diese Mittel darüberhinaus besonders intensive und/oder schnell einsetzende Kühleindrücke vermitteln können.

Auf der Mundschleimhaut zeigen die oben erwähnten konventionellen, im Stand der Technik bekannten Kühlsubstanzen alle ein mehr oder weniger identisches Kühlverhalten. Das durch sie vermittelte kühlende Frischempfinden setzt nach etwa 0,5 Min. ein, flacht dann aber nach einem Höhepunkt bei 3 bis 5 Min wieder relativ schnell ab, wobei die Kühlung insgesamt für höchstens 30 min deutlich wahrnehmbar ist und erfahrungsgemäß in Intensität und Dauer nur wenig durch eine Veränderung der Dosierung zu beeinflussen ist. Auf Seiten der Verbraucher besteht aber der Wunsch nach einer insbesondere lange andauernden Kühlwirkung, die für den Verwender mit einem entsprechenden Frische- und Wohlgefühl verbunden ist.

Es hat sich überraschender Weise gezeigt, dass insbesondere in die in der Tabelle 0 aufgeführten Verbindungen die gemeinsame Eigenschaft haben, *in vivo* eine besonders lange Kühlwirkung auf Haut oder Schleimhaut zu erzielen. Dies war weder für die in dieser Anmeldung erwähnten TRPM8-Agonisten vorhersagbar, noch trifft es auf alle dieser Agonisten zu.

Bisher gab es im Stand der Technik keine Erkenntnisse darüber, dass die Verbindungen aus der Tabelle 0 überhaupt eine Kühlwirkung zu vermitteln in der Lage sind. Um die langandauernde Kühlwirkung zu quantifizieren, werden Vergleichversuche mit Menthan-3-carbonsäure-N-ethylamid durchgeführt. Für diese Vergleichsversuche tauscht der Fachmann die erfindungsgemäß einzusetzende Verbindung oder die Verbindungen durch Menthan-3-carbonsäure-N-ethylamid (auch WS3) in gleicher Konzentration aus. Dann werden die Kühlwirkungen der jeweiligen Mittel, wie anhand des Testbeispiels für Zahnpasta demonstriert, miteinander verglichen. Sofern die erfindungsgemäß einzusetzenden Verbindungen (die Verbindungen aus der Tabelle 0) im zu untersuchenden Mittel in einer Konzentration von höher als 100 ppm enthalten sind, ist es bevorzugt, dass für die Evaluierung, ob die Kühlwirkung gegenüber WS3 verlängert ist, das zu testende Mittel so zu verdünnen, dass die erfindungsgemäßen einzusetzenden Verbindungen (aus Tabelle 0) in einer Konzentration von 100 ppm vorliegen. Selbstverständlich ist der gleiche Verdünnungsschritt auch für das Vergleichsmittel vorzunehmen, das WS3 enthält.

In diesem Zusammenhang ist bevorzugt, dass in den entsprechenden Vergleichen die Kühlwirkung der Mittel mit den erfindungsgemäß einzusetzenden Verbindungen (aus Tabelle 0) um wenigstens 15 Minuten, weiter bevorzugt wenigstens 20 Minuten und besonders bevorzugt wenigstens 30 Minuten gegenüber den Vergleichsversuchen verlängert ist

Darüber hinaus ist bevorzugt, dass nach zehn Minuten die wahrgenommene Kühlintensität auf einer Skala von 0 - 9 um ≥ 1, bevorzugt ≥ 2, weiter bevorzugt ≥ 3 und besonders bevorzugt ≥ 4 gegenüber einem Mittel gleicher Zusammensetzung, bei dem lediglich die erfindungsgemäß einzusetzende Verbindung durch Menthan-3-carbonsäure-N-ethylamid in gleicher Konzentration ersetzt wurde, erhöht ist.

Alternativ oder zusätzlich bevorzugt ist, dass die wahrgenommene Intensität auf der gleichen Skala nach 20 Minuten um ≥ 1, bevorzugt ≥ 2, besonders bevorzugt ≥ 3 und ganz besonders bevorzugt größer gleich 4 entsprechend erhöht ist.

Alternativ oder ebenfalls zusätzlich bevorzugt ist, dass die wahrgenommene Intensität unter den vorbeschriebenen Bedingungen nach 30 Minuten um ≥ 1, weiter bevorzugt um ≥ 2 erhöht ist und/oder die wahrgenommene Intensität oder den vorbeschriebenen Bedingungen nach 45 Minuten nach ≥ 1, bevorzugt ≥ 2 erhöht ist.

Es sei noch einmal darauf hingewiesen, dass die Vergleichsversuche hinsichtlich der Verlängerung der Kühlwirkung zur Vergleichssubstanz WS3 im Zweifelsfall analog zum Testbeispiel 2 durchzuführen sind, wobei bevorzugt ist, dass bei Konzentrationen in der erfindungsgemäß einzusetzenden Verbindungen (Verbindungen der Tabelle 0) von > 100 ppm bezogen auf das gesamte Mittel ein Verdünnungsschritt stattfindet, so dass diese Verbindungen nur noch in einer Konzentration von 100 ppm in dem zu testenden Mittel enthalten sind und wobei der Verdünnungsschritt analog in dem Vergleichsmittel (das Mittel enthaltend WS3) durchgeführt wird.

Ebenfalls bevorzugt ist, dass das Testpanel (vergleiche Testbeispiel (2) wenigstens 6 Personen umfasst und die Intensitätseindruckswerte mathematisch gemittelt werden.

Die erfindungsgemäß zu verwendenden Verbindungen für den speziellen Aspekt der Erfindung (die Verbindung der Tabelle 0) können gegebenenfalls als Isomerengemische,, Racemate oder reine Enatiomere eingesetzt werden.

Die erfindungsgemäß zu verwendenden Verbindungen sind synthetisch in an sich bekannter Weise zugänglich. Eine Reihe von Herstellungsbeispielen finden sich weiter hinten in diesem Text. Überraschenderweise zeigen die erfindungsgemäß einzusetzenden Verbindungen (die Verbindungen der Tabelle 0) kaum andere trigeminale Effekte wie Schärfe, Tingling oder Betäubung und sind nicht bitter. Gleichzeitig sind die erfindungsgemäßen Verbindungen im Rahmen der üblichen Formulierungen und Zubereitungsbedingungen im Bereich von pH 1 bis pH 12, insbesondere im Bereich von pH 4 bis pH 9, bezogen auf wasserhaltige Zubereitungen, hydrolysestabil, so dass die erfindungsgemäßen Verbindungen und Mischungen in Zubereitungen lange haltbar sind, so dass auch die jeweilige Zubereitung selbst lange haltbar ist.

Die Erfindung betrifft auch ein Mittel ausgewählt aus der Gruppe bestehend aus Aromamischung und der Ernährung, der Mundhygiene oder dem Genuss dienende pharmazeutische oder kosmetische Zubereitung, umfassend eine zwei, drei oder mehr der Verbindungen ausgewählt aus der Gruppe B bestehend aus

| LN | Struktur |
|---|---|
| 24 | 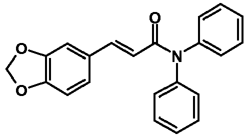 |
| 25 | 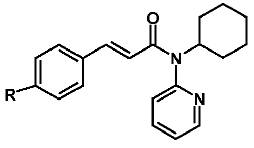 mit R = H |
| 26 | 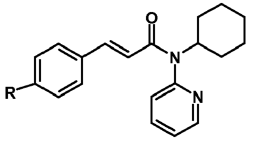 mit R = Me |
| 27 | 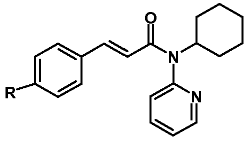 mit R = MeO |
| 28 | 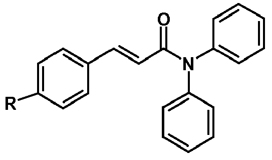 mit R = H |
| 29 | 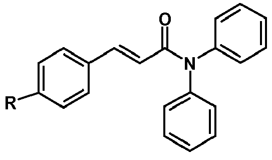 mit R = Me |
| 30 | 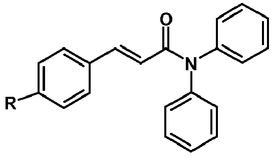 mit R = MeO |

Ferner betrifft die Erfindung bevorzugt ein i) vorbeschriebenes erfindungsgemäßes Mittel und/oder ein ii) Mittel ausgewählt aus der Gruppe bestehend aus Aromamischung und der Ernährung, der Mundhygiene oder dem Genuss dienende pharmazeutische oder kosmetische Zubereitung, umfassend eine zwei, drei oder mehr der Verbindungen ausgewählt aus der Gruppe C bestehend aus

| LN | Struktur |
|---|---|
| 23 | 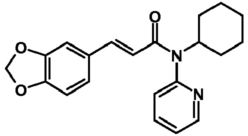 |

wobei die Verbindung der Gruppe C in einer Konzentration von 0,05 ppm - < 0,1 ppm oder 0,1 ppm bis 50 Gew-% bezogen auf das Gesamtgewicht der Zubereitung enthalten ist, bevorzugt mit der Maßgabe, dass im Falle (ii) die Zubereitung kein Mundwasser mit der Zusammensetzung von jeweils bezogen auf einen Liter

| | |
|---|---|
| Ethanol 95% | 177mL |
| Sorbit 70% | 250 g |
| Verbindung der Formel 2, 5, 9 oder 23 als 1%ige Lösung in Ethanol | 50mL |
| Pfefferminzöl, | 0.30 g |
| Methylsalicylat | 0.64 g |
| Eucalyptol | 0.922 g |
| Thymol | 0.639 g |
| Benzoesäure | 1.50 g |
| Pluronic® F127 nichtionisches Tensid | 5.00 g |
| Natrium-Saccharin | 0.60 g |
| Natriumcitrat | 0.30 g |
| Zitronensäure | 0.10 g |
| Wasser q.s. | 1 Liter |

ist.

Es sei darauf hingewiesen, dass die Differenzierung zwischen den Verbindungen der Gruppe B und der Verbindung der Gruppe C nur formalen patentrechtlichen Gründen geschuldet ist, nicht aber technischen Aspekten.

Bevorzugt sind in den für den speziellen Aspekt der Erfindung beschriebenen Mitteln die erfindungsgemäß einzusetzenden Verbindungen (Verbindungen der Tabelle 0) in einer (Gesamt-) Konzentration von 0,05 ppm bis 50 Gew.-% bezogen auf das Gesamtgewicht der Zubereitung bzw. des Mittels enthalten. Dabei setzt sich dieser Bereich insbesondere aus folgenden Unterbereichen zusammen: 0,05 ppm - < 0,1 ppm. 0,1 ppm - 1.000 ppm und 0,1 - 50 Gew.-%. Bevorzugte Konzentrationsbereiche, bezogen auf das Gesamtgewicht der Zubereitung bzw. des Mittels sind die folgenden, wobei die Konzentrationsbereiche in der aufgezählten Reihenfolge jeweils gegenüber dem davorgehenden Aufzählungsglied weiter bevorzugt sind: 0,05 ppm - 10 Gew.-%,. 0,5 ppm-5 Gew.-%, 1 ppm, - 2,5 Gew.-%.

Vorteilhaft an den erfindungsgemäßen Mitteln, insbesondere solchen in der bevorzugten Variante ist, dass sie in der Lage sind, einen langanhaltenden Kühleffekt auf der Haut bzw. Schleimhaut zu vermitteln, ohne dass die erfindungsgemäß einzusetzenden Verbindungen (die Verbindungen der Tabelle 0) den Einsatzzweck des jeweiligen Mittels verhindern bzw. einschränken.

In diesem Zusammenhang sei darauf hingewiesen, dass für die gemäß dem speziellen Teil der Erfindung erfindungsgemäßen Mittel das im Bereich der Beschreibung des allgemeinen Teils der Erfindung, (insbesondere den Kapiteln 3 - 5) beschriebene analog gilt. Besonders bevorzugte Varianten für die erfindungsgemäßen Mittel gemäß dem speziellen Teil der Erfindung werden zusätzlich in diesem Kapitel beschrieben.

Bevorzugt ist ein erfindungsgemäßes Mittel, insbesondere eines gemäß des speziellen Aspektes der Erfindung, umfassend
(1) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
   und/oder
(2) einen oder mehrere Aromastoffe ohne physiologische Kühlwirkung und/oder
(3) einen oder mehrere trigeminal oder mundwässernd wirksame Stoffe ohne physiologische Kühlwirkung
   und/oder
(4) (iii) eine oder
   (iv) mehrere Verbindungen, die im Falle (iv) unabhängig von einander oder gemeinsam
   zusätzlich einen geschmacksmodulierenden Effekt und/oder einen trigeminalen und/oder einen mundwässernden Reiz verursachen.

Weiter bevorzugt umfasst ein solches Mittel einen oder mehrere weitere Stoffe mit physiologischer Kühlwirkung ohne geschmacklichen Effekt. Hierdurch wird vermieden, dass mit dem erfindungsgemäßen Mittel beispielsweise nur Aromen mit einem minzigen Aroma-Charakter erhalten werden können.

Ganz besonders bevorzugt ist ein erfindungsgemäßes Mittel umfassend als Bestandteil (2) einen oder mehrere Aromastoffe ohne physiologische Kühlwirkung und/oder als Bestandteil (3) eine oder mehrere Verbindungen, der oder die unabhängig voneinander oder gemeinsam zusätzlich einen geschmacksmodulierenden Effekt und/oder einen trigiminalen und/oder einen mundwässernden Reiz verursachen, wobei der trigiminale Reiz bevorzugt keine physiologische Kühlwirkung darstellt. Insbesondere besitzen solche erfindungsgemäßen Mittel, die die letztgenannten Bestandteile (2) und (3) gleichzeitig enthalten eine angenehme Kühlwirkung und ein ausgeglichenes sensorisches Profil bei gleichzeitig hohem Impact, d.h. hohem geschmacklichen Ersteindruck.

Der spezielle Aspekt der Erfindung betrifft bevorzugt auch Mittel insbesondere als der Ernährung, der Mundhygiene oder dem Genuss dienende oder pharmazeutische oder kosmetische Zubereitungen, die eine zum Erreichen einer physiologischen Kühlwirkung auf der Haut und/oder Schleimhaut ausreichende Menge einer erfindungsgemäß einzusetzenden Verbindung (Verbindung aus der Tabelle 0) oder einer erfindungsgemäß einzusetzenden Mischung von solchen Verbindungen umfassen. Insbesondere soll die eingesetzte Menge dieser Verbindung oder dieser Mischung zum Erreichen einer physiologischen Kühlwirkung auf der Schleimhaut im Mund-, Nasen- und/oder Rachenraum ausreichen.

In diesem Zusammenhang sei darauf hingewiesen, dass die Begriffe "Mittel" und "Zubereitung" synonym verwendet werden können. Bevorzugt ist jedoch, dass eine Zubereitung mittels eines Arbeitsschrittes hergestellt werden muss, der über das ledigliche Mischen der Einzelverbindungen hinaus geht. Ein solcher Arbeitsschritt kann zum Beispiel zum Erzeugen einer Suspension oder einer Emulgierung dienen.

Bevorzugte erfindungsgemäße Mittel umfassen übliche Grund-, Hilfs- und Zusatzstoffe für der Ernährung, der Mundhygiene oder dem Genuss dienende oder pharmazeutische oder kosmetische Zubereitungen. Bevorzugte erfindungsgemäße Zubereitungen enthalten 0,000005 Gew.-% bis 20 Gew.-%, bevorzugt 0,00001 bis 10 Gew.-%, besonders bevorzugt 0,0001 Gew.-% bis 0,5 Gew.-% an erfindungsgemäßen einzusetzenden Verbindungen der Tabelle 0, bezogen auf das Gesamtgewicht der Zubereitung. Weitere Bestandteile, insbesondere Bestandteile (1) (weitere Stoffe mit physiologischer Kühlwirkung), (2) (Aromastoffe ohne physiologische Kühlwirkung) und/oder (3) (trigeminal oder mundwässernd wirksame Stoffe ohne physiologische Kühlwirkung) (wie oben beschrieben) sowie weitere übliche Grund-, Hilfs- und Zusatzstoffe können in Mengen von 0,0000001 bis 99,99 Gew.-%, vorzugsweise 10 bis 80 Gew.-%, bezogen auf das Gesamtgewicht der Zubereitung, enthalten sein. Ferner können die erfindungsgemäßen Zubereitungen Wasser in einer Menge bis zu 99,99 Gew.-%, vorzugsweise 5 bis 80 Gew.-%, bezogen auf das Gesamtgewicht der Zubereitung, enthalten.

Weiter bevorzugt ist ein erfindungsgemäßen Mittel, wobei wenigstens eine der Verbindungen ausgewählt aus der Gruppe A ausgewählt ist aus der Gruppe D, bestehend aus

| LN | Struktur |
|---|---|
| 23 | 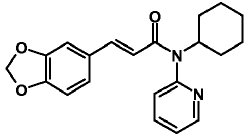 |
| 24 | 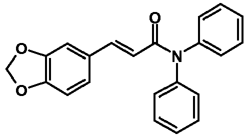 |
| 25 | 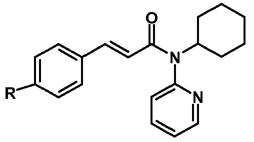 mit R = H |
| 26 | 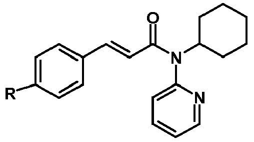 mit R = Me |
| 27 | 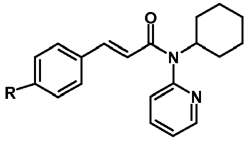 mit R = MeO |

Besonders bevorzugt ist ein erfindungsgemäßes Mittel, wobei wenigstens eine der Verbindungen ausgewählt aus der Gruppe A ausgewählt ist aus der Gruppe E, bestehend aus

| LN | Struktur |
|---|---|
| 23 | 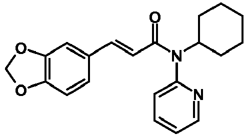 |
| 24 | 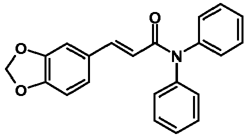 |

Bevorzugt werden die erfindungsgemäß einzusetzenden Verbindungen der Tabelle 0 und/oder deren Mischungen zum Herstellen eines Arzneimittels, das der Bekämpfung oder der Linderung von Husten-Schnupfen-, Mund-, Nasen-, Hals- oder Rachenentzündungs-, Halsschmerz- oder Heiserkeitssymptomen dient, verwendet.

Ein weiterer Aspekt des speziellen Aspektes der vorliegenden Erfindung betrifft ein therapeutisches (nicht erfindungsgemäßes) oder nicht-therapeutisches Verfahren zum Erreichen einer physiologischen Kühlwirkung auf der Haut und/oder einer Schleimhaut, mit folgendem Schritt:
- Applizieren einer zum Erreichen einer physiologischen Kühlwirkung ausreichenden Mengeeines erfindungsgemäßen Mittels
   auf die Haut und/oder eine Schleimhaut.

Bevorzugt ist im Sinne des speziellen Aspektes der Erfindung, dass das erfindungsgemäße Mittel eine Aromamischung ist, welche einen oder mehrere Aromastoffe und/oder einen oder mehrere weitere Kühlwirkstoffe (Kühlwirkstoffe, bei denen es sich nicht um eine Verbindung der Tabelle 0 handelt) umfasst, zur Aromatisierung von unter Verwendung der Aromamischung gefertigter Fertigwaren.

In Zubereitungen (Aromamischungen), die zu Aromatisierungen von Zahnpasten und -cremes eingesetzt werden, beträgt der Gehalt der erfindungsgemäß zu verwendenden Stoffe der Tabelle 0 0,001 bis 50 Gew.-%; bevorzugt ist ein Bereich von 0,005 bis 5 Gew.-% und besonders bevorzugt ein Bereich von 0,01 bis 2 Gew.-%. Bei üblichen Dosierungen der Aromen zwischen 0,5 und 1,5 Gew.-%, bezogen auf die gebrauchsfertigen Zahnpasten und -cremes beträgt dann der Gehalt der erfindungsgemäß zu verwendenden Stoffe der Formel I 0,000005 bis 0,75 Gew-% bezogen auf das Fertigprodukt; bevorzugt ist entsprechend ein Bereich von 0,000025 bis 0,075 Gew.-% und besonders bevorzugt entsprechend ein Gehalt von 0,00005 bis 0,03 Gew.-%.

In Zubereitungen(Aromamischungen), die zu Aromatisierungen von Kaugummis eingesetzt werden, beträgt der Gehalt der erfindungsgemäß zu verwendenden Stoffe der Tabelle 0 0,005 bis 10 Gew.-%; bevorzugt ist ein Bereich von 0,01 bis 5 Gew.-% und besonders bevorzugt ein Bereich von 0,05 bis 2,5 Gew.-%. Bei einer üblichen Dosierung der Aromen von 1 - 2 Gew.-%, bezogen auf das gebrauchsfertige Kaugummi beträgt dann der Gehalt der erfindungsgemäß zu verwendenden Stoffe der Tabelle 0 0,00005 bis 0,2 Gew-% bezogen auf das Fertigprodukt; bevorzugt ist entsprechend ein Bereich von 0,0001 bis 0,1 Gew.-% und besonders bevorzugt entsprechend ein Gehalt von 0,0005 bis 0,05 Gew.-%.

In Zubereitungen(Aromamischungen), die zu Aromatisierungen von Mundwässern und - spülungen eingesetzt werden, beträgt der Gehalt der erfindungsgemäß zu verwendenden Stoffe der Tabelle 0 0,01 bis 10 Gew.-%; bevorzugt ist ein Bereich von 0,05 bis 5 Gew.-% und besonders bevorzugt ein Bereich von 0,1 bis 2,5 Gew.-%. Bei einer üblichen Dosierung des Aromas von 2 - 4 Gew.-%, bezogen auf ein gebrauchsfertiges Mundwasserkonzentrat beträgt dann der Gehalt der erfindungsgemäß zu verwendenden Stoffe der Tabelle 0 0,0002 bis 0,4 Gew-% bezogen auf das Fertigprodukt; bevorzugt ist entsprechend ein Bereich von 0,001 bis 0,2 Gew.-% und besonders bevorzugt entsprechend ein Gehalt von 0,002 bis 0,1 Gew.-%. In gebrauchsfertigen Mundwässern und -spülungen beträgt dann bei einer üblichen Dosierung der Aromamischung von 0,1 - 0,3 Gew.-% der Gehalt der erfindungsgemäß zu verwendenden Stoffe der Tabelle 0 0,00001 bis 0,03 Gew-% bezogen auf das Fertigprodukt; bevorzugt ist entsprechend ein Bereich von 0,00005 bis 0,015 Gew.-% und besonders bevorzugt entsprechend ein Gehalt von 0,0001 bis 0,0075 Gew.-%.

Als Aromastoffe eignen sich sowohl komplexe natürliche Rohstoffe wie aus Pflanzen gewonnene Extrakte und etherische Öle, bzw. daraus gewonnene Fraktionen und einheitliche Stoffe, als auch einheitliche synthetisch oder biotechnologisch gewonnene Aromastoffe.

### Beispiele für natürliche Rohstoffe sind z.B.:

Pfefferminzöle, Krauseminzöle, Mentha-Arvensis-Öle, Anisöle, Nelkenöle, Citrusöle, Zimtrindenöle, Wintergrünöle, Cassiaöle, Davanaöle, Fichtennadelöle, Eucalyptusöle, Fenchelöle, Galbanumöle, Ingweröle, Kamillenöle, Kümmelöle, Rosenöle, Geraniumöle, Salbeiöle, Scharfgarbenöle, Sternanisöle, Thymianöle, Wacholderbeeröle, Rosmarinöle, Angelikawurzelöle, und die Fraktionen dieser Öle.

### Beispiele für einheitliche Aromastoffe sind z.B.:

Anethol, Menthol, Menthon, Isomenthon, Menthylacetat, Menthofuran, Menthylmethylether, Mintlacton, Eucalyptol, Limonen, Eugenol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Hexanol, Hexanal, cis-3-Hexenol, Linalool, alpha-Terpineol, cis und trans Carvylacetat, p-Cymol, Thymol, 4,8-Dimethyl-3,7-nonadien-2-on, Damascenon, Damascone, Rosenoxid, Dimethylsulfid, Fenchol, Acetaldehyddiethylacetal, cis-4-Heptenal, Isobutyraldehyd, Isovaleraldehyd, cis-Jasmon, Anisaldehyd, Methylsalicylat, Myrtenylacetat, 8-Ocimenylacetat, 2-Phenylethylalkohol, 2-Phenylethylisobutyrat, 2-Phenylethylisovalerat, Zimtaldehyd, Geraniol, Nerol. Bei chiralen Verbindungen können die genannten Aromastoffe als Racemat, als einzelnes Enantiomer oder als enantiomerenangereicherte Mischungen vorliegen.

In der Ernährung oder dem Genuss dienenden Zubereitungen beträgt der Gehalt der erfindungsgemäß zu verwendenden Stoffe der Tabelle 0 0,000005 bis 0,1 Gew.-%; bevorzugt ist ein Bereich von 0,00005 bis 0,05 Gew.-% und besonders bevorzugt ein Bereich von 0,0001 bis 0,02 Gew.-%.

In kosmetischen Zubereitungen beträgt der Gehalt der erfindungsgemäß zu verwendenden Stoffe der Tabelle 0 0,001 bis 10 Gew.-%; bevorzugt ist ein Bereich von 0,005 bis 5 Gew.-% und besonders bevorzugt ein Bereich von 0,01 bis 2 Gew.-%.

Erfindungsgemäß besonders bevorzugt ist, dass das gemäß dem speziellen Aspekt der Erfindung erfindungsgemäße Mittel eine Zahnpasta ist.

Von Interesse ist auch eine Verbindung ausgewählt aus der Gruppe bestehend aus

**(Tabelle N)**

| LN | Struktur |
|---|---|
| 7 | 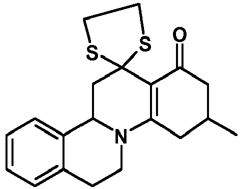 |
| 8 | 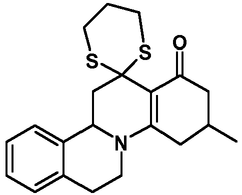 |
| 20 | 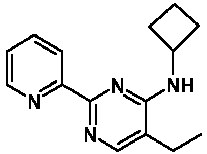 |
| 21 | 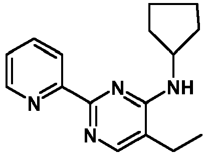 |
| 22 | 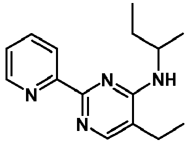 |

Diese in Tabelle N aufgeführten Verbindungen waren noch nicht vorbeschrieben.

Weitere Aspekte der vorliegenden Erfindung ergeben sich aus den nachfolgenden Beispielen sowie den beigefügten Patentansprüchen.

### Beispiele

Die Beispiele dienen zur Verdeutlichung der Erfindung.

Sofern nicht anders angegeben, beziehen sich alle Angaben auf das Gewicht.

### 7. Wirkstoffherstellung

Die oben beschriebenen Wirkstoffe der Strukturtypen 1, 2 und 3 sind entweder an sich bekannte Verbindungen oder können in Anlehnung an bekannte Synthesemethoden vom Fachmann auf dem Gebiet der organischen Synthese hergestellt werden.

Im folgenden experimentellen Teil werden verschiedene Synthesemethoden für einen repräsentativen Querschnitt oben beschriebener Wirkstoffe beschrieben.

Die Erfindung wird nun anhand folgender Ausführungsbeispiele beschrieben.

### 8. Experimenteller Teil, Beispiele

Die nachfolgenden Beispiele dienen der Erläuterung der Erfindung. Sofern Menganangaben angegeben sind, handelt es sich im Zweifelsfall um Gew.-%.

### Referenzbeispiel 1 - Klonierung von humanem TRPM8

Ausgangspunkt für die Klonierung des humanen TRPM8 Rezeptors ist eine LnCaP cDNA-Bank. Diese ist beispielsweise kommerziell erhältlich (z. B. Firma BioChain, Hayward, USA) oder aus der androgensensitiven humanen Prostata-Adenokarzinomzelllinie LnCaP (z.B. ATCC, CRL1740 oder ECACC, 89110211) unter Verwendung von Standardkits herstellbar.

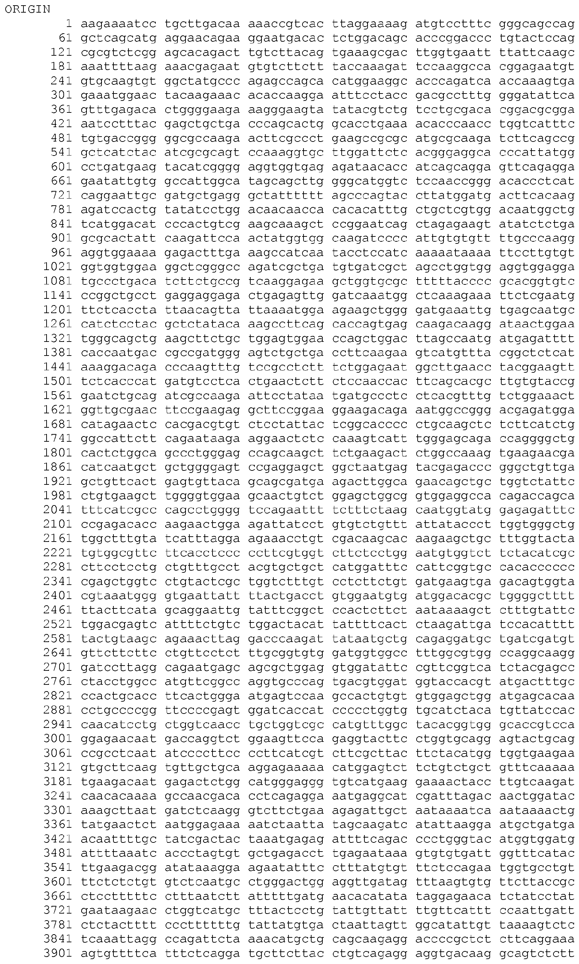

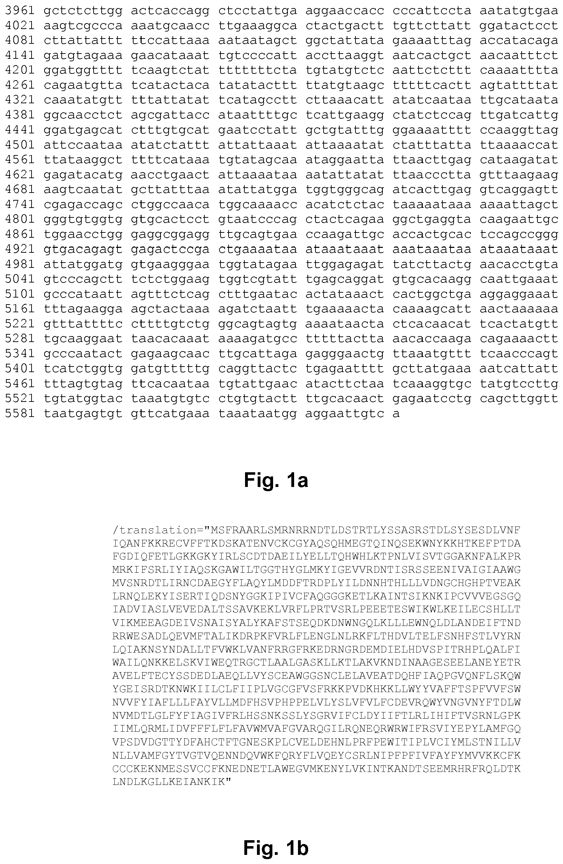

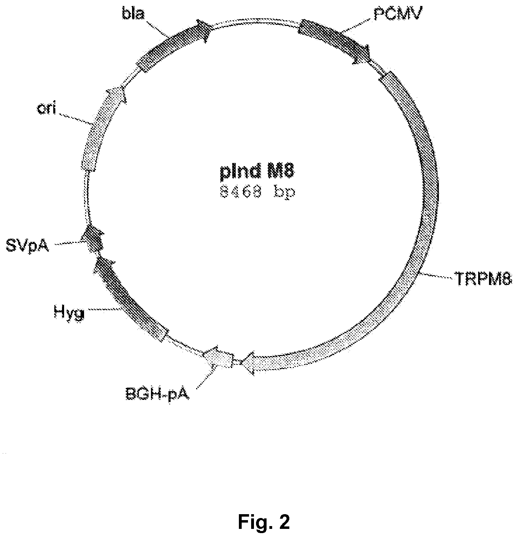

Die kodierende TRPM8 Sequenz (vgl Fig. 1A; sowie **http://www.ncbi.nlm.nih.gov/entrez/viewer.fcgi?db=nuccore&id=109689694)** kann unter Verwendung von Standardmethoden PCR-amplifiziert und kloniert werden. Das so isolierte humane TRPM8 Gen wurde zur Herstellung des Plasmids plnd_M8 verwendet, dessen Konstruktion durch die Plasmidkarte gemäß Figur 2 veranschaulicht wird.

Alternativ dazu kann das TRPM8 Gen auch synthetisch hergestellt werden.

### Referenzbeispiel 2 - Generierung der HEK293-Testzellen

Als Testzellsystem wurde mit der humanen TRPM8 DNA (vgl obiges Plasmid plnd-M8) stabil transfizierte HEK293 Zelllinie hergestellt. Bevorzugt wird dabei HEK293 die über das eingebrachte Plasmid die Möglichkeit zur Induktion der TRPM8-Expression mittels Tetrazyklin bietet.

Methoden zur Herstellung geeigneter Testzellsysteme sind dem Fachmann bekannt. So kann man die Herstellung der erfindungsgemäß verwendeten Zellen den Angaben in Behrendt H.J. et al.,, Br. J. Pharmacol. 141, 2004, 737-745 oder der Dissertation von Behrendt "Vergleichende funktionale Untersuchungen des Hitze-Capsaicin-Rezeptors (TRPV1) und des Kälte-Menthol-Rezeptors (TRPM8) in rekombinanten und nativen Zellssystemen", zugänglich unter http://www-brs.ub.ruhr-uni-bochum.de/netahtml/HSS/Diss/BehrendtHansJoerg/diss.pdf entnehmen. Auf die Offenbarung dieser Druckschriften wird ausdrücklich Bezug genommen.

### Referenzbeispiel 3- Assay auf TRPM8 Modulatoren

Es wird ein Test, vergleichbar mit dem bereits in der Literatur beschrieben Test von Behrendt H.J. et al., Br. J. Pharmacol. 141, 2004, 737-745, durchgeführt. Die Agonisierung bzw. Antagonisierung des Rezeptors kann mittels eines Ca²⁺-sensitiven Farbstoffs (z.B. FURA, Fluo-4 etc.) quantifiziert werden. Agonisten bewirken alleine eine Zunahme des Ca²⁺-Signals; Antagonisten bewirken in Gegenwart von z.B. Menthol eine Reduzierung des Ca²⁺-Signals (jeweils detektiert über den Farbstoff Fluo-4, der durch Ca²⁺ andere Fluoreszenzeigenschaften hat).

Zunächst wird in an sich bekannter Weise in Zellkulturflaschen eine frische Kultur transformierter HEK-Zellen hergestellt. Die Testzellen HEK293-TRPM8 werden mittels Trypsin von den Zellkulturflaschen abgelöst und 40.000 Zellen/well werden mit 100 µl Medium in 96-Lochplatten (Greiner # 655948 Poly-D-Lysin-beschichtet) ausgesät. Zur Induktion des Rezeptors TRPM8 wird dem Wachstumsmedium Tetrazyklin beigemischt (DMEM/HG, 10% FCS tetrazyklinfrei, 4 mM L-Glutamin, 15 µg/ml Blasticidin, 100 µg/ml Hygromycin B, 1 µg/ml Tetrazyklin). Am darauffolgenden Tag werden die Zellen mit Fluo-4AM Farbstoff beladen und der Test wird durchgeführt. Dazu wird wie folgt vorgegangen:
- Zugabe von je 100 µl/well Färbelösung Ca-4 Kit (RB 141, Molecular Devices) zu je 100 µl Medium (DMEM/HG, 10% FCS tetrazyklinfrei, 4 mM L-Glutamin, 15 µg/ml Blasticidin, 100 µg/ml Hygromycin B, 1 µg/ml Tetrazyklin)
- Inkubation im Brutschrank, 30 Minuten / 37°C / 5% CO₂, 30 Minuten / RT
- Vorbereitung der Testsubstanzen (unterschiedliche Konzentrationen in 200 µl HBSS-Puffer), sowie von Positivkontrollen (unterschiedliche Konzentrationen von Menthol, Icilin bzw. lonomycin in 200 µl HBSS-Puffer) und Negativkontrollen (nur 200 µl HBSS-Puffer)
- Zugabe der Testsubstanzen in Mengen von 50 µl/well und Messung der Fluoreszenzänderung (z.B. im Assaygerät FLIPR, Molecular Devices oder NovoStar, BMG) bei 485 nm Anregung, 520 nm Emission, und Auswertung der Wirkstärke der verschiedenen Substanzen/Konzentrationen und Bestimmung der EC50-Werte

Die Testsubstanzen werden in Triplikaten in Konzentrationen von 0,1 - 200 µM im Assay eingesetzt. Normalerweise werden die Verbindungen in DMSO-Lösungen bereitgehalten und für den Assay auf eine maximale DMSO-Konzentration von 2% herunterverdünnt.

Die Auswertung zeigt überraschenderweise, dass erfindungsgemäß erstmalig Agomisten von TRPM8 bereitgestellt werden konnten, welche sich strukturell von bisher bekannten Agonisten, wie (-) Menthol, Icilin und anderen von Behrendt H.J. et al.,in Br. J. Pharmacol. 141, 2004, 737-745 (vgl dortige Tabelle 1) beschriebenen Modulatoren, signifikant unterscheiden und zudem teilweise bessere Aktivitäten als (-) Menthol zeigen, bzw. vergleichbar stark wirken wie Icilin.

### Herstellungsbeispiele

### a) Herstellungsbeispiele für Verbindungen gemäß Strukturtyp 1 (nicht erfindungsgemäß)

Die Herstellung repräsentativer Verbindungen der Formel I ist in folgendem Abschnitt beschrieben.

Grundsätzlich sind die Verbindungen der Formel I aus der Umsetzung von Keto-Vorstufen der Formel V-I mit der ketoreaktiven Verbindung der Formel Y-I zugänglich (siehe Akhrem et al. ,Kimiya Geteotsiklicheskikh Soedinenii, 1995, 187-194, Akhrem et al., Journal of Organic Chemistry of the USSR, 1985, 21 (6), 1227-1232)

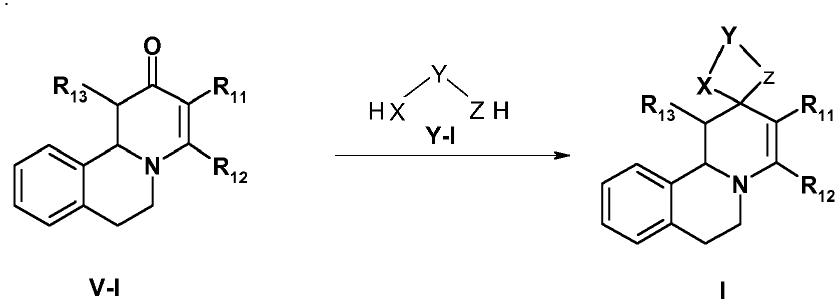

Die verschiedenen dazu erforderlichen Ausgangsstoffe sind ebenfalls bekannt oder nach bekannten Verfahren zugänglich.

(Zur Herstellung von Verbindungen vom Typ V-1 siehe Akhrem et al. in Journal of Organic Chemistry of the USSR, 1979, 1247-1252, Akhrem et al., Izvestia Akademii Nauk SSSR Seria Himiceskaa, 1969, (10), 2338-2339, Akhrem et al., Doklady Akademii Nauk SSSR, 1972, 203 (1), 95-98.)

### Herstellungsbeispiel 1-1: Herstellung von Verbindung 1-1

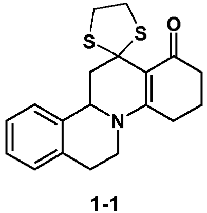

Die Herstellung erfolgt dabei nach folgendem Schema:

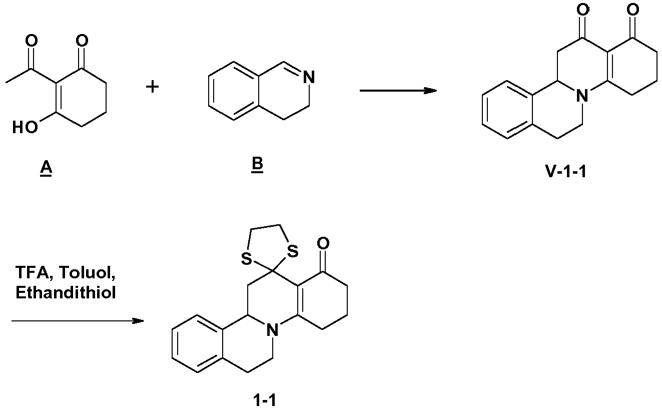

### (1) Herstellung der Vorstufe V-1-1

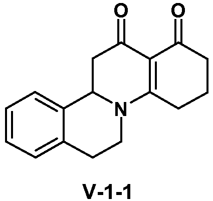

Eine Mischung aus 40,1 g (0,259 mol) 2-Acetyl-1,3-hexandion (A), 34,1 g (0,26 mol) 1,2-Dihydroisochinolin (B) und 254 ml EtOH werden 2 h unter Rückfluß erhitzt. Es wird langsam abgekühlt und das Reaktionsgemisch am Rotationsverdampfer bei 50°C eingeengt. Der Rückstand wird in 230 ml Toluol und 31 ml n-Heptan aufgenommen, die Mischung auf Rückfluß erhitzt und langsam auf Raumtemperatur abgekühlt. Die entstandene Suspension wird filtriert. Der Filterrückstand wird mit 50 ml Toluol gewaschen und trockengesaugt. Man erhält so das Dion **V-1-1.**
Ausbeute: 57 g Produkt (82 %), HPLC: 97,4 FI-%.
¹H-NMR (CDCl₃): m 7,12-7,34; d 4,86; m 4,16-4,26; m 3,35-3,46; m 3,04-3,18; m 2,78-3,01; m 2,55-3,72; m 2,40-2,50; m 2,26-2,40; m 1,90-2,14 [ppm]

### (2) Herstellung von Verbindung 1-1

In einem Reaktor werden 21,4 g Wasser vorgelegt und mit 75,9 g (0,79 mol) Methansulfonsäure versetzt. Nach Abklingen der Exothermie werden 9,3 g (0,099 mol) Ethandithiol zugegeben. Anschließend werden portionsweise 17,6 g (0,066 mol) Verbindung **V-1-1** zudosiert. Es wird 17 h bei Raumtemperatur nachgerührt. Das Reaktionsgemisch wird mit 202 ml Toluol und anschließend mit 158 g 25 % NaOH versetzt. Es wird filtriert, die wäßrige Phase abgetrennt und die organische Phase noch einmal mit 107 g Wasser gewaschen. Die organische Phase wird über Aktivkohle fiiltriert, das Filtrat aufkonzentriert und der Rückstand auf 5°C abgekühlt. Die Suspension wird filtriert und der Filterkuchen mit 15 ml Toluol gewaschen. Nach 2 h Trocknen im Vakuumtrockenschrank bei 50°C werden 12,4 g Feststoff isoliert.

Der Feststoff wird in 115 ml Toluol gelöst und heiß über einen Sand/Kieselgur/Aktivkohlefilter filtriert. Der Filter wird mit 15 ml heißem Toluol nachgespült. 80 ml Toluol werden abdestilliert und die aufkonzentrierte Reaktionsmischung wird auf -10°C abgekühlt. Die Suspension wird filtriert und der Filterkuchen mit wenig kaltem Toluol nachgewaschen. Es wird über Nacht im Vakuumtrockenschrank bei 50°C getrocknet, wobei man Verbindung **1-1** erhält
Ausbeute: 10,5 g (46 %), HPLC 99 FI-%

### Herstellungsbeispiel H1-2: Herstellung von Verbindung 1-4

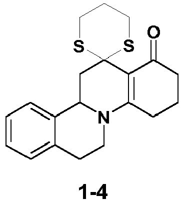

4,5 g der Ausgangsverbindung **(V-1-1)** werden in 45 ml Toluol vorgelegt und mit 7,3 g Propandithiol versetzt. Es werden 9,7 g Trifluoressigsäure zugegeben und es wird 53 h bei 50°C gerührt. Das Reaktionsgemisch wird auf RT abgekühlt und mit 12,9 g 25 % Natronlauge versetzt. Das Gemisch wird 5 min bei RT gerührt, die organische Phase wird abgetrennt und noch zweimal mit Wasser gewaschen. Die organische Phase wird eingeengt und mehrmals mit Isopropanol versetzt und wieder aufkonzentriert. Anschließend wird über eine Kieselgelsäule mit Heptan/EtOAc als Laufmittel chromatographisch gereinigt, wobei man die gewünschte Verbindung erhält.
Ausbeute: 1,5 g (25 %)

### Herstellungsbeispiel H1-3: Herstellung von 1-2

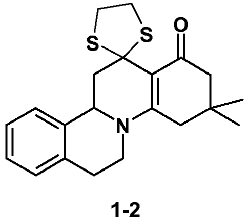

### (1) Herstellung der Vorstufe V-1-2

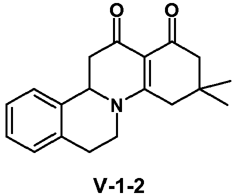

19,7 g (0,106 mol) 2-Acetyldimedon und 78 ml Ethanol werden vorgelegt und mit 14,6 g 1,2-Dihydroisochinolin versetzt. Die Mischung wird 2 h auf Rückfluß erhitzt. Anschließend wird auf Raumtemperatur abgekühlt und bei 50°C im Vakuum am Rotationsverdampfer einrotiert. Es werden 98 ml Toluol zugesetzt und auf Rückfluss erhitzt. Es wird langsam auf 10°C abgekühlt, 2h bei 10°C nachgerührt und filtriert. Der Filterkuchen wird mit 25 ml Toluol gewaschen und anschließend im Stickstoffstrom getrocknet, wobei man die gewünschte Verbindung erhält.
Ausbeute: 30 g (96 %), HPLC: 100 FI-%
¹H-NMR (CDCl₃) : m 7,20-7,32; dd 4,98; s 4,83; dtr 4,38; dtr 3,43; m 3,28-3,32; m 3,07-3,19; dtr 2,96; d 2,88; m 2,56-2,76; d 2,29; d 2,17; s 2,14; s 1,08 [ppm]

### (2) Herstellung von Verbindung 1-2

26,4 g (89 mmol) Verbindung **V-1-2** und 161 ml Toluol werden vorgelegt und nacheinander mit 25,3 g (0,27 mol) Ethandithiol und 51 g (0,45 mol) Trifluoressigsäure versetzt. Es wird auf 50°C erwärmt und 121 h bei gleicher Temperatur nachgerührt. Es werden 8,4 g (89 mmol) Ethandithiol und 10,2 g (89 mmol) Trifluoressigsäure nachgegeben und es werden weitere 21 h bei 50°C gerührt. Es wird auf Raumtemperatur abgekühlt und es werden 78 ml 20 % Natronlauge und 50 g Wasser zugegeben. Die wäßrige Phase wird wird abgetrennt und die organische Phase wird noch einmal mit 16 g Wasser gewaschen. Die organische Phase wird einrotiert und das Rohprodukt mit 50 ml Isopropanol versetzt. Das Gemisch wird auf 60°C erwärmt, angeimpft und langsam auf 0°C abgekühlt. Es wird filtriert und der Filterkuchen mit 10 ml Isopropanol gewaschen. Der Filterkuchen wird im Stickstoffstrom getrocknet.

Zur Aufreinigung werden 14,6 g des Produkts in 145 ml Isopropanol aufgenommen und die Mischung wird auf Rückfluss erhitzt. Es werden portionsweise 132 ml Isopropanol in den Sumpf gefahren und anschließend wieder abdestilliert. Der Sumpf wird langsam auf 0°C abgekühlt und filtriert. Der Filterkuchen wird zweimal mit je 13 ml Isopropanol gewaschen und über Nacht bei 50°C im Vakuum getrocknet, wobei man die gewünschte Verbindung erhält.
Ausbeute: 13,1 g (42 %).

### Herstellungsbeispiel H1-4: Herstellung von Verbindung 1-5

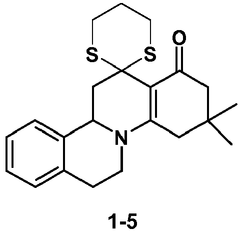

4,5 g der Ausgangsverbindung **V-1-2** werden in 40 ml Toluol vorgelegt und mit 6,6 g Propandithiol versetzt. Es werden 8,8 g Trifluoressigsäure zugegeben und es wird 50 h bei 50°C gerührt. Es werden 3,3 g 1,3 Propandithiol und 3,5 g Trifluoressigsäure nachdosiert und weitere 74 h bei 50°C gerührt. Das Reaktionsgemisch wird mit 12,7 g 25 % Natronlauge versetzt. Die organische Phase wird abgetrennt und noch zweimal mit Wasser gewaschen. Die organische Phase wird einrotiert und anschließend mehrmals mit Isopropanol versetzt und wieder aufkonzentriert. Das Rohprodukt wird über eine Kieselgelsäule mit Heptan/EtOAc als Laufmittel gereinigt, wobei man die gewünschte Verbindung erhält.
Ausbeute: 2,9 g (49 %)

### Herstellungsbeispiel H1-5: Herstellung von Verbindung 1-8

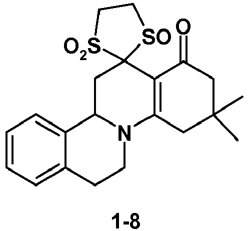

1 g Verbindung **1-2** werden in 4,5 ml Dichlormethan vorgelegt und innerhalb von 50 min bei 20-23°C mit einer Lösung von 2,65 g meta-Chlorperbenzoesäure in 40 ml Dichlormethan versetzt. Es wird 72 h nachgerührt und es wird mit 20 ml wässriger Natriumbisulfitlösung (38-40 %) und 30 ml Wasser gequencht. Die organische Phase wird abgetrennt und mit 50 ml Natriumhydrogencarbonat-Lösung und anschließend mit 50 ml Wasser gewaschen. Die organische Phase wird eingeengt und chromatographisch über Kieselgel mit DCM/THF als Laufmittel gereinigt, wobei man die gewünschte Verbindung erhält.
Ausbeute: 0,75 g (66 %, Diastereomerengemisch)

### Herstellungsbeispiel H1-6: Herstellung von Verbindung 1-3

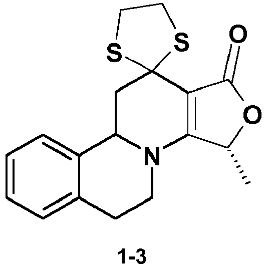

### (1) Herstellung der Vorstufe V-1-3 bzw. der aktiven Verbindung 1-9

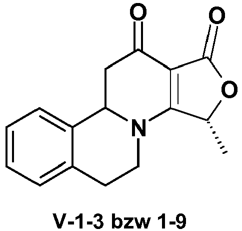

In einem Reaktor werden 10,3 g (R)-3-Acetyl-5-methyl-furan-2,4-dion in 207 ml Toluol vorgelegt und nacheinander mit 7,5 g Trifluoressigsäure und mit 8,7 g 1,2-Dihydroisochinolin versetzt. Die Mischung wird 24 h am Rückfluss erhitzt, auf Raumtemperatur abgekühlt und mit 67,5 ml Wasser und 4,2 ml 50 % NaOH versetzt. Die entstandene Suspension wird filtriert und der Filterkuchen 2 x mit je 10 ml Toluol gewaschen. Der Filterkuchen wird im Stickstoffstrom getrocknet, wobei man die gewünschte Verbindung erhält.
Ausbeute: 13 g (73 %, HPLC: 98,9 FI-%).
¹H-NMR (CDCl₃): m 7,20-7,39; q 5,50; q 5,39; m 5,14-5,22; m 5,06-5,14; d 3,98; m 3,78-3,86; m 3,42-3,58; m 3,23-3,41; m 3,00-3,13; m 2,86-3,00; m 2,42-2,72; d 1,50 [ppm]

### (2) Herstellung von Verbindung 1-3:

10,8 g Verbindung **V-1-3** werden in 127 ml Toluol vorgelegt und bei Raumtemperatur nacheinander mit 15,1 g Ethandithiol und 22,9 g Trifluoressigsäure versetzt. Es wird 40 h bei Raumtemperatur nachgerührt. Anschließend werden 1,1g Aktivkohle zugesetzt, es wird eine Stunde gerührt und es wird filtriert. Das Filtrat wird mit 77 g 10 % Natronlauge versetzt. Dabei fällt eine Festoff aus, der abfiltriert wird. Der Feststoff wird in 51 ml Toluol und 51 ml 1 N NaOH aufgenommen, 30 min bei 0°C gerührt und wieder filtriert. Der Filterkuchen wird mit Toluol und MeOH gewaschen und im Stickstoffstrom getrocknet, wobei man die gewünschte Verbindung erhält.
Ausbeute: 74 % (HPLC-Reinheit: 97 FI-%)

### Herstellungsbeispiel H1-7: Herstellung von Verbindung 1-6

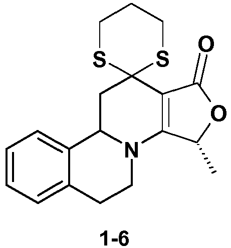

7,7 g Verbindung **V-1-3** werden in 91 ml Toluol vorgelegt und mit 9,3 g 1,3 Propandithiol versetzt. Es werden 16,4 g (0,143 mol) Trifluoressigsäure zugesetzt und es wird 24 h bei RT gerührt. Es werden 57 g 10 % NaOH zugesetzt. Der ausgefallene Feststoff wird abgesaugt. Vom Filtrat wird die wässrige Phase abgetrennt und die organische Phase wird am Rotationsverdampfer aufkonzentriert. Der Rückstand wird mit 51 ml Essigsäureethylester versetzt, zum Rückfluss erhitzt und anschließend langsam auf 0°C abgekühlt. Es wird 30 min bei 0°C nachgerührt. Der ausgefallene Feststoff wird abfiltriert, mit Essigsäurethylester gewaschen und im Vakuum bei 50°C getrocknet.

Der Feststoff wird mit 100 ml Isopropanol versetzt und es werden 70 ml Isopropanol über eine Vigreux-Kolonne wieder abdestilliert. Die Suspension wird auf 0°C abgekühlt. Der Feststoff wird abfiltriert, mit wenig kaltem Isopropanol nachgewaschen und getrocknet, wobei man die gewünschte Verbindung erhält.

### Ausbeute: 6,9 g (67 %)

### Herstellungsbeispiel H1-8: Herstellung von Verbindung 1-7

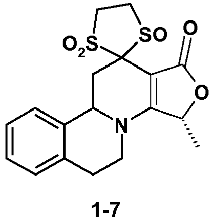

1,0 g Verbindung **1-3** in 4,5 ml Dichlormethan werden vorgelegt und innerhalb von 50 min bei 20-24°C mit einer Lösung von 2,85 g meta-Chlorperbenzoesäure in 40 ml Dichlormethan versetzt. Es wird 72 h nachgerührt und mit 20 ml wässriger Natriumbisulfitlösung (38-40 %) und 30 ml Wasser gequencht. Die organische Phase wird abgetrennt und nacheinander mit 50 ml wässriger Natriumhydrogencarbonat-Lösung und 50 ml Wasser gewaschen. Die organische Phase wird eingeengt und chromatographisch über Kieselgel mit DCM/THF als Laufmittel gesäult, wobei man die gewünschte Verbindung erhält.
Ausbeute: 0,88 g (74 %)

### b) Herstellungsbeispiele für Verbindungen gemäß Strukturtyp 2 (nicht erfindungsgemäß)

Grundsätzlich sind erfindungsgemäße Verbindungen der Formel II aus den Amidin-Vorstufen C-II und Enolaten der Formel D-II und der daraus resultierende Hydroxyl-funktionalen Verbindung B-II, die dann zum gewünschten Endprodukt weiter umgesetzt wird, zugänglich.

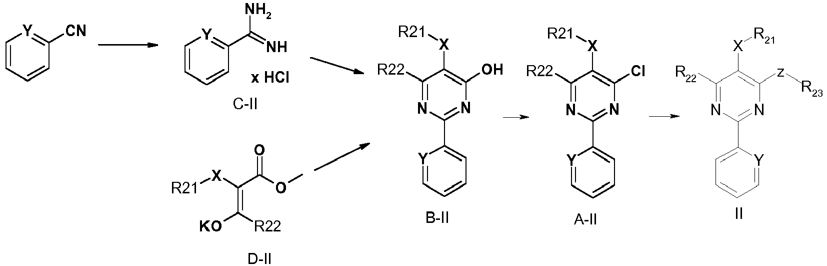

Die verschiedenen dazu erforderlichen Ausgangsstoffe bzw. Zwischenverbindungensind ebenfalls bekannt oder nach bekannten Verfahren zugänglich. (Medwid et al., J. Med. Chem., 1990, 33 (4), 1230-1241), Chesterfield et al., J. Chem. Soc., 1960, 4590-4594; W. Gienke et al, EP 407899B1)

### Herstellungsbeispiel H2-1: Herstellung von Verbindung 2-1

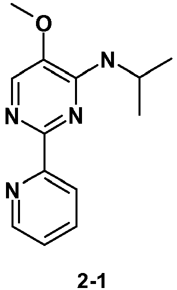

### (1) Herstellung von Vorstufe B-2-1

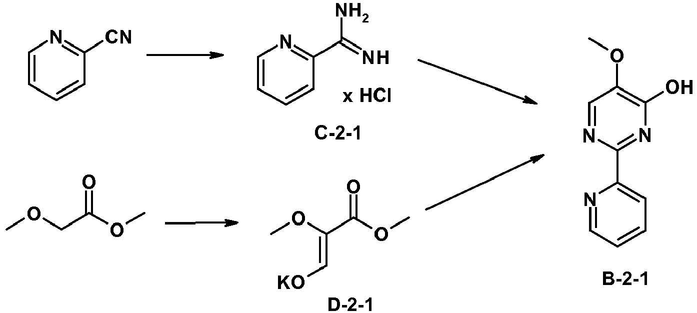

Zur Herstellung des Amidins **C-2-1** wird Picolinnitril (90 mmol) in Methanol vorgelegt und bei Raumtemperatur mit 0,1 eq % Natriummethylat (30 % in MeOH) versetzt. Es wird über Nacht bei Raumtemperatur nachgerührt. Es werden 1,13 eq Ammouniumchlorid zugefügt und es wird 5 h bei Rückfluss erhitzt.

Zur Herstellung des Enolat **D-2-1** werden 23 g (2,23 eq) Kalium-tert-butylat in THF vorgelegt und innerhalb von 90 min mit einer Lösung aus (11,9 g) 2,16 eq Ameisensäuremethylester und 9,6 g (1,0 eq) Methoxyessigsäuremethylester versetzt. Es wird über Nacht bei Raumtemperatur nachgerührt und es werden 120 ml THF unter Vakuum abdestilliert. Bei 10-20°C wird innerhalb von 30 min das Amidiniumsalz **2-C** in Methanol zugefahren. Es wird 5 h bei 63°C nachgerührt. Es wird abgekühlt und mit Wasser hydrolysiert. Es wird andestilliert, die wäßrige Phase mit 32 % aq. HCl auf pH 4-5 eingestellt und mit Dichlormethan extrahiert. Die vereinigten wäßrigen Phasen werden am Rotationsverdampfer aufkonzentriert und der Rückstand mit Diisopropylether ausgerührt. Es wird filtriert und das Kristallisat im Stickstoffstrom getrocknet, wobei man die gewünschte Verbindung erhält.
Ausbeute: 78 %
1H-NMR (DMSO): d 8,71; d 8,25; tr 8,03; br s 7,68; m 7,56-7,63; s 3,84 [ppm]

### (2) Herstellung von Vorstufe A-2-1

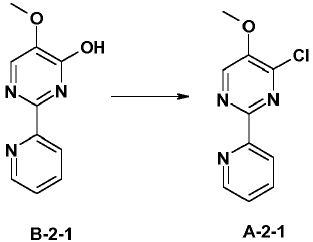

5 g (25 mmol) Verbindung **B-2-1** werden mit 40 g Phosphorylchlorid versetzt und die Mischung wird für 90 min auf Rückfluss erhitzt. Der Überschuss POCl₃ wird destillativ entfernt. Es wird abgekühlt und der Rückstand mit Dichlormethan versetzt. Es wird mit Wasser hydrolysiert und der Ansatz mit NaOH basisch gestellt. Die organische Phase wird abgetrennt und die wäßrige Phase noch zweimal mit Dichlormethan extrahiert. Die vereinigten organischen Phasen werden über Natriumsulfat getrocknet, filtriert und am Rotationsverdampfer einrotiert.
Ausbeute: 94 %
¹H-NMR (CDCl₃): d 8,82; s 8,47; d 8,42; tr 7,85; m 7,36-7,41; s 4,08 [ppm]

### (3) Herstellung von Verbindung 2-1

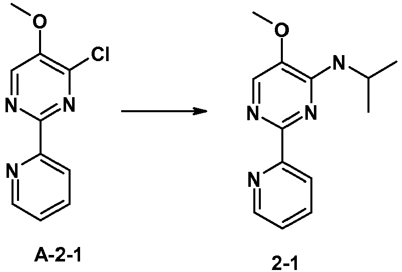

10,5 g (47 mmol) Verbindung **A-2-1** werden mit 25 eq. Isopropylamin versetzt und über Nacht bei 32°C gerührt. Es werden Wasser und Toluol zugesetzt und bei 50°C die Phasen getrennt. Die wäßrige Phase wird 2 x mit Toluol nachextrahiert und die vereinigten organischen Phase 2 x mit wenig Wasser gewaschen. Die organische Phase wird über Natriumsulfat getrocknet, filtriert und am Rotationsverdampfer eingeengt. Das Produkt wird aus MTBE umkristallisiert, wobei man die gewünschte Verbindung erhält.
Ausbeute: 78 %

### Herstellungsbeispiel H2-2: Herstellung von Verbindung 2-2

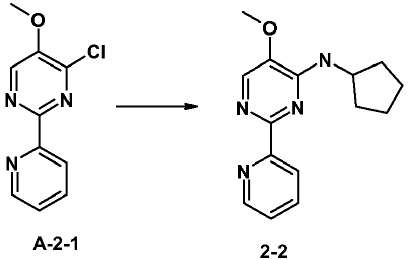

Eine Lösung von 4 g (47 mmol) Cyclopentylamin in 2-Methyltetrahydrofuran wird mit 5 g Verbindung **A-2-1** versetzt und 4 h auf Rückfluss erhitzt. Es wird auf Raumtemperatur abgekühlt und der Ansatz hydrolysiert. Die wäßrige Phase wird mit wäßriger NaOH basisch gestellt und die organische Phase abgetrennt. Die organische Phase wird noch einmal mit Wasser gewaschen und anschließend am Rotationsverdampfer eingeengt. Das Rohprodukt wird über Kieselgel chromatographiert, wobei man die gewünschte Verbindung erhält..
Ausbeute: 56 %

### Herstellungsbeispiel H2-3: Herstellung von Verbindung 2-8

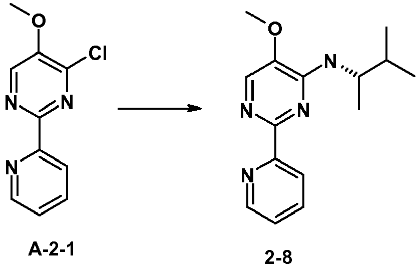

Eine Lösung von 1,7 g (19 mmol) (S)-2-Amino-3-methylbutan in 2-Methyltetrahydrofuran wird mit 2 g Verbindung **A-2-1** versetzt und 20 h auf Rückfluss erhitzt. Es wird auf Raumtemperatur abgekühlt und der Ansatz hydrolysiert. Die wäßrige Phase wird mit wäßriger NaOH basisch gestellt und die organische Phase abgetrennt. Die organische Phase wird noch einmal mit Wasser gewaschen und anschließend am Rotationsverdampfer eingeengt. Das Rohprodukt wird über Kieselgel chromatographiert, wobei man die gewünschte Verbindung erhält.
Ausbeute: 27 %

### Herstellungsbeispiel H2-4: Herstellung von Verbindung 2-5

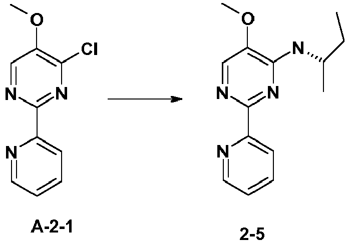

Eine Lösung von 2,1 g (28,4 mmol) (S)-2-Aminobutan in 2-Methyltetrahydrofuran wird mit 3 g Verbindung **A-2-1** versetzt und 28 h auf Rückfluss erhitzt. Es wird auf Raumtemperatur abgekühlt und der Ansatz hydrolysiert. Die wäßrige Phase wird mit wäßriger NaOH basisch gestellt und die organische Phase abgetrennt. Die organische Phase wird noch einmal mit Wasser gewaschen und anschließend am Rotationsverdampfer eingeengt, wobei man die gewünschte Verbindung erhält.
Ausbeute: 92 %

### Herstellungsbeispiel H2-5: Herstellung von Verbindung 2-12

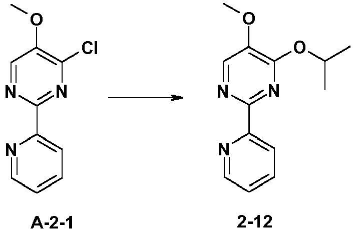

1,0 g Kalium-tert-Butylat und 28 ml Isopropanol werden auf 50°C erwärmt und mit 2,0 g Verbindung **A-2-1** versetzt. Die Heizung wird ausgeschaltet und es wird 68 h gerührt. Der Ansatz wird am Rotationsverdampfer einrotiert und es werden 50 g Wasser und 0,54 g Essigsäure zugesetzt. Die wäßrige Phase wird 2 x mit Dichlormethan extrahiert, die vereinigten organischen Phasen werden einrotiert und der Rückstand wird aus einem Gemisch von 5 ml MTBE und 15 ml n-Heptan kristallisiert. Die Kristalle werden abgesaugt, mit n-Heptan gewaschen und im Vakuumtrockenschrank bei 30°C getrocknet, wobei man die gewünschte Verbindung erhält.
Ausbeute: 41 % (HPLC-Reinheit: 100 FI-%)

### Herstellungsbeispiel H2-6: Herstellung von Verbindung 2-14

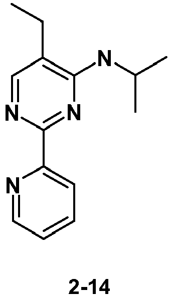

### (1) Herstellung der Vorstufe B-2-2

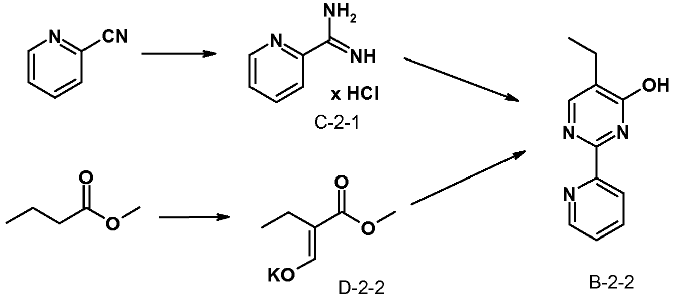

Zur Herstellung des Amidins **C-2-1** wird Picolinnitril (90 mmol) in Methanol vorgelegt und bei Raumtemperatur mit 0,1 eq Natriummethylat gelöst in Methanol versetzt. Es wird über Nacht bei Raumtemperatur nachgerührt. Es werden 1,13 eq Ammouniumchlorid zugefügt und es wird 5 h bei Rückfluss erhitzt.

Zur Herstellung des Enolat **D-2-2** werden 12,8 g (2,23 eq) Kalium-tert-butylat in THF vorgelegt und innerhalb von 35 min bei 18-22°C mit einer Lösung aus 6,6 g (2,16 eq) Ameisensäuremethylester und 5,9 g (1,0 eq) Buttersäureethylester in 23,2 ml THF versetzt. Es wird über Nacht bei Raumtemperatur nachgerührt und der Ansatz einrotiert. Bei 10-20°C wird das Amidiniumsalz **C-2-1** in Methanol zugefahren. Es wird 5 h bei 63°C nachgerührt. Es wird abgekühlt und mit Wasser hydrolysiert. Es werden 100 ml MTBE zugegeben, es wird kurz aufgerührt und die Phasen werden getrennt. Die organische Phase wird verworfen und die wäßrige Phase wird mit 6,5 ml Essigsäure versetzt. Die wäßrige Phase wird dreimal mit je 67 ml Dichlormethan extrahiert. Die organische Phase wird einrotiert und wieder in MTBE aufgenommen. Die MTBE-Phase wird mit Wasser gewaschen und mit 1,2 eq wäßriger NaOH versetzt. Die wäßrige Phase wird abgetrennt, mit Essigsäure auf pH 7 gebracht und mit MTBE extrahiert. Die MTBE-Phase wird über Natriumsulfat getrocknet, filtriert und einrotiert. Es wird aus n-Heptan kristallisiert.
Ausbeute: 18,5 % (HPLC-Reinheit: 100 FI-%)
¹H-NMR (CDCl₃): 1br s 11,05; d 8,64; d 8,38; m 7,83-7,95; m 7,41-7,50; q 2,59; tr 1,25 [ppm]

### (2) Herstellung von Vorstufe A-2-2

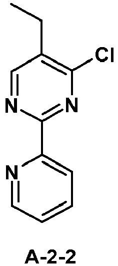

1,9 g Verbindung **B-2-2** werden in 19 ml Phosphorylchlorid dosiert. Der Ansatz wird 1 h auf Rückfluss erhitzt. Das überschüssige Phosphoroxychlorid wird unter Vakuum abdestilliert und der Rückstand in Dichlormethan aufgenommen. Es werden 42 ml Wasser zugegeben und mit NaOH auf pH 7 eingestellt. Die Phasen werden getrennt und die organischen Phase über Na₂SO₄ getrocknet. Es wird filtriert und das Filtrat einrotiert, wobei man die gewünschte Verbindung erhält.
Ausbeute: 72 % (HPLC-Reinheit: 100 FI-%)
¹H-NMR (CDCl₃): d 8,84; s 8,70; d 8,48; tr 7,87; m 7,39-7,45; q 2,81; tr 1,33 [ppm]

### (3) Herstellung von Verbindung 2-14

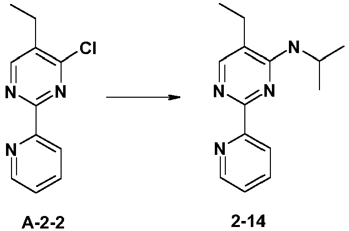

Eine Mischung aus 1,6 g (7,8 mmol) 2-E und 11,04 g (187 mmol) Isopropylamin werden 42 h bei Raumtemperatur gerührt. Der Ansatz wird einrotiert und mit Wasser, 1,33 eq NaOH und Dichlormethan versetzt. Die wäßrige Phase wird abgetrennt und noch einmal mit Dichlormethan extrahiert. Die vereinigten organischen Phasen werden über Natriumsulfat getrocknet, filtriert und einrotiert. Der Rückstand wird mit heißem MTBE ausgerührt und die Suspension anschließend auf Raumtemperatur abgekühlt. Es wird filtriert, der Rückstand mit wenig MTBE gewaschen und bei 50°C im Vakuumtrockenschrank getrocknet, wobei man die gewünschte Verbindung erhält.
Ausbeute: 0,92 g (49 %)

### Herstellungsbeispiel H2-7: Herstellung von Verbindung 2-15

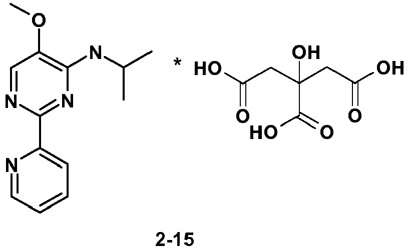

0,865 g (4,5 mmol) Zitronensäure und 1,11 g (4,09 mmol) Verbindung **2-1** werden mit 20 ml iPrOH versetzt und zum Rückfluss erhitzt. Es wird auf 30°C abgekühlt und die ausgefallenen Kristalle werden abgesaugt. Der Filterkuchen wird mit wenig Isopropanol gewaschen und bei 50°C im Vakuumtrockenschrank getrocknet, wobei man die gewünschte Verbindung erhält.
Ausbeute: 1,74 g (97 %)

### Herstellungsbeispiel H2-8: Herstellung von Verbindung 2-16

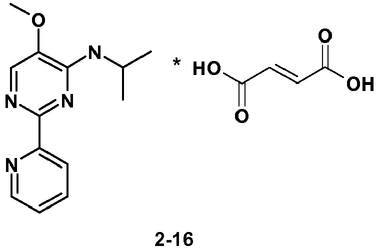

0,520 g (4,5 mmol) Fumarsäure und 1,11 g (4,09 mmol) Verbindung **2-1** werden mit 20 ml iPrOH versetzt und zum Rückfluss erhitzt. Es wird soviel Wasser zugesetzt, dass eine klare Lösung ensteht. Es wird auf Raumtemperatur abgekühlt und die ausgefallenen Kristalle werden abgesaugt. Der Filterkuchen wird mit wenig iPrOH und MTBE gewaschen und bei 50°C im Vakuumtrockenschrank getrocknet, wobei man die gewünschte Verbindung erhält..
Ausbeute: 0,88 g (60 %)

### Herstellungsbeispiel H2-9: Herstellung von Verbindung 2-17

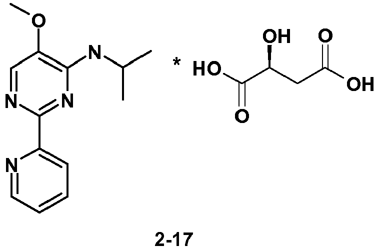

0,600 g (4,5 mmol) Äpfelsäure und 1,1 g (4,1 mmol) Verbindung **2-1** werden mit 20 ml iPrOH versetzt und zum Rückfluss erhitzt. Es wird soviel Wasser zugesetzt, dass eine klare Lösung ensteht. Es wird auf Raumtemperatur abgekühlt und die ausgefallenen Kristalle werden abgesaugt. Der Filterkuchen wird mit wenig iPrOH und MTBE gewaschen und bei 50°C im Vakuumtrockenschrank getrocknet.
Auswaage: 0,77 g (50 %)

### Herstellungsbeispiel H2-10: Herstellung von Verbindung 2-18

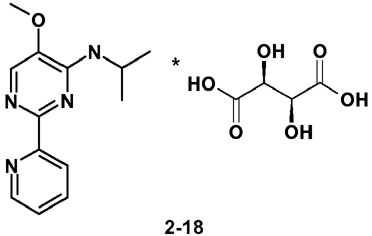

0,675 g (4,5 mmol) Weinsäure und 1,11 g (4,1 mmol) Verbindung **2-1** werden mit 20 ml iPrOH versetzt und zum Rückfluss erhitzt. Es wird soviel Wasser zugesetzt, dass eine klare Lösung ensteht. Es wird auf Raumtemperatur abgekühlt und die ausgefallenen Kristalle werden abgesaugt. Der Filterkuchen wird mit wenig iPrOH und MTBE gewaschen und bei 50°C im Vakuumtrockenschrank getrocknet, wobei man die gewünschte Verbindung erhält..
Ausbeute: 1,32 g (85 %)

### Herstellungsbeispiel H2-11: Herstellung von Verbindung 2-19

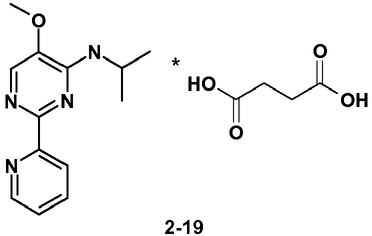

0,675 g (5,7 mmol) Bernsteinsäure und 1,41 g (5,2 mmol) Verbindung **2-1** werden mit 26 ml iPrOH versetzt und zum Rückfluss erhitzt. Es wird auf Raumtemperatur abgekühlt und die Lösung wird einrotiert. Das einrotierte Öl wird mit 5 ml EtOAc verrührt und das gebildetet Kristallisat wird abgesaugt. Der Filterkuchen wird mit wenig EtOAc gewaschen und bei 50°C im Vakuumtrockenschrank getrocknet, wobei man die gewünschte Verbindung erhält.
Ausbeute: 0,77 g (41 %)

### Herstellungsbeispiel H2-12: Herstellung von Verbindung 2-21

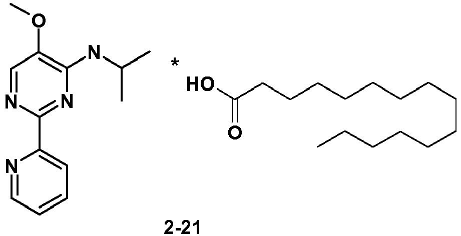

1,1 g (4,3 mmol) Palmitinsäure und 1,0 g (4,1 mmol) Verbindung **2-1** werden mit 5 ml Heptan versetzt und auf 50°C erwärmt. Es wird auf 0°C abgekühlt und filtriert. Das Kristallisat wird abgesaugt und im Vakuumtrockenschrank getrocknet, wobei man die gewünschte Verbindung erhält.
Ausbeute: 1,29 g (63%)

### c) Herstellungsbeispiele für Verbindungen gemäß Strukturtyp 3

Die Herstellung repräsentativer Verbindungen des Strukturtyps 2 der Formel III ist in folgendem Abschnitt beschrieben. Die verschiedenen dazu erforderlichen Ausgangsstoffe der allgemeinen Formel C-III und B-III sind ebenfalls bekannt oder nach bekannten Verfahren zugänglich.

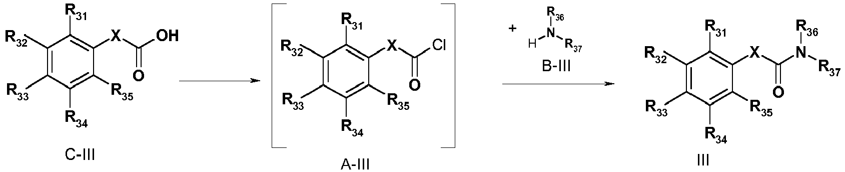

Die Reaktion wird beispielsweise in einem apolaren organischen Lösungsmittel in Gegenwart eines Säurefängers durchgeführt..

### Herstellungsbeispiel H3-1: Herstellung von (E)-N-Cyclohexyl-N-pyridin-2-yl-3-m-tolyl-acrylamid, Verbindung 3-31

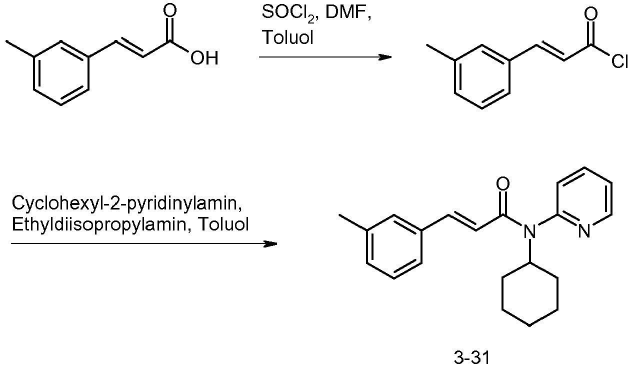

4,87 g (0,03 mol) 3-Methylzimtsäure, 49 ml Toluol und 0,19 g (2,6 mmol) g N,N-Dimethyformamid werden vorgelegt und mit 4,3 g (0,036 mol) Thionylchlorid versetzt. Es wird 1 h nachgerührt und anschließend auf 60°C erwärmt. Toluol und überschüssiges Thionylchlorid werden unter Vakuum abgezogen. Anschließend werden bei 60°C 24 ml Toluol und 4,3 g (0,033 mol) Ethyldiisopropylamin zugesetzt. Es wird eine Lösung von 5,8 g (0,033 mol) Cyclohexyl-2-pyridinylamin (Herstellung siehe E. H. Mørkved, Journal f. prakt.Chemie, 1986, 328 (3), 401-406) in Toluol zudosiert und 2 h bei 60-65°C nachgerührt. Es wird mit Wasser hydrolyiert und die organische Phase mit Wasser, 1 N NaOH und nochmals mit Wasser gewaschen. Die organische Phase wird einrotiert und der Rückstand aus MTBE kristallisiert. Der auskristallisierte Feststoff wird abgesaugt, mit MTBE gewaschen und getrocknet, wobei man die gewünschte Verbindung erhält.
Ausbeute: 74 %

### Herstellungsbeispiel H3-2: Herstellung von (E)-3-Benzo[1,3]dioxol-5-yl-N,N-diphenyl-acrylamid, Verbindung 3-6

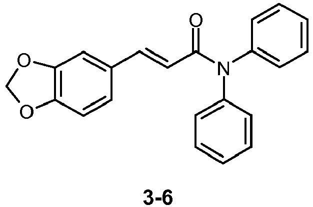

4,8 g (0,025 mol) 3,4-Methylendioxyzimtsäure, 48 ml Toluol und 0,16 g (2,16 mmol) N,N-Dimethylformamid werden vorgelegt, auf 60°C aufgeheizt und bei dieser Temperatur mit 3,57 g (0,030 mol) Thionylchlorid versetzt. Es wird 1,5 h bei 60-65°C nachgerührt. Anschließend wird auf Siedetemperatur erhitzt und es werden überschüssiges Thionylchlorid und Toluol abdestilliert. Bei 60-65°C werden nacheinander 2,78 g Triethylamin und eine Lösung von von 4,2 g (0,025 mol) Diphenylamin in 24 ml Toluol zudosiert. Anschließend wird 16 h bei 60-65°C nachgerührt. Es wird mit Wasser hydrolysiert und die organische Phase nach Phasentrennung noch einmal bei 60°C mit Wasser gewaschen. Es wird heiß filtriert und einrotiert. Der Rückstand wird aus Isopropanol kristallisiert. Der auskristallisierte Feststoff wird bei 0°C abgesaugt, mit iPrOH gewaschen und getrocknet, wobei man die gewünschte Verbindung erhält.
Ausbeute: 81 %

### Herstellungsbeispiel H3-3: Herstellung von (E)-N,N-Dicyclohexyl-3-(4-methoxy-phenyl)-acrylamid, Verbindung 3-18

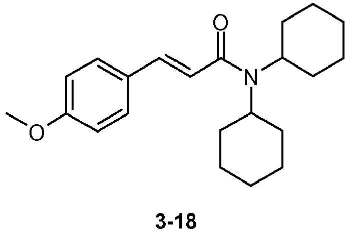

5,0 g (0,028 mol) 3-Methoxyzimtsäure, 50 ml Toluol und 0,23 g (3,0 mmol) N,N-Dimethylformamid werden vorgelegt und mit 4,01 g (0,034 mol) Thionylchlorid versetzt. Es wird über Nacht nachgerührt. Anschließend wird auf 50°C erwärmt und unter Vakuum überschüssiges Thionylchlorid und Toluol abdestilliert. Unter Normaldruck werden bei 60°C Toluol und 4,0 g (0,031 mol) Diisopropylamin zugesetzt. Anschließend wird eine Lösung von 5,6 g (0,031 mol) Dicyclohexylamin in Toluol zudosiert. Es wird 30 min bei 60°C nachgerührt und auf RT abgekühlt. Das Reaktionsgemisch wird mit Wasser und 2-Methyltetrahydrofuran versetzt und auf 70°C erwärmt. Nach Phasentrennung wird die organische Phase 2 x mit Wasser, 1 x mit 1 N Natronlauge und noch 2 x mit Wasser bei 50°C gewaschen. Die organische Phase wird einrotiert und aus n-Heptan umkristallisiert. Der auskristallisierte Feststoff wird bei 0°C abgesaugt, mit n-Heptan gewaschen und getrocknet, wobei man die gewünschte Verbindung erhält.
Ausbeute: 50 % (HPLC: 92 FI-%)

### Herstellungsbeispiel H3-4: Herstellung von (E)-3-Benzo[1,3]dioxol-5-yl-N-cyclohexyl-N-isopropyl-acrylamid, Verbindung 3-5

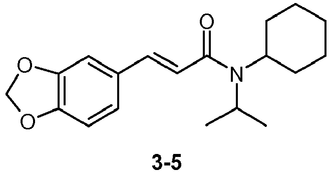

5,77 g (0,030 mol) trans-3,4-(Methylendioxy)-zimtsäure, 58 ml Toluol und 0,19 g (3,6 mmol) N,N-Dimethylformamid werden vorgelegt und mit 4,28 g (0,036 mol) Thionylchlorid versetzt. Es wird 1 h bei RT nachgerührt. Anschließend wird auf 50-60°C erwärmt und unter Vakuum überschüssiges Thionylchlorid und Toluol abdestilliert. Unter Normaldruck werden bei 60-70°C Toluol und 4,27 g (0,033 mol) Ethyldiisopropylamin zugesetzt. Anschließend wird eine Lösung von 4,66 g (0,033 mol) Cyclohexyl-isopropylamin in Toluol zudosiert. Es wird 2 h bei 60-70°C nachgerührt und anschließend mit Wasser hydrolysiert. Nach Phasentrennung wird die organische Phase bei 50-70°C mit Wasser, 2 N HCl, Wasser, 1 N NaOH, Wasser gewaschen. Die organische Phase wird einrotiert und aus iPrOH/n-Heptan umkristallisiert. Der auskristallisierte Feststoff wird bei RT abgesaugt, mit n-Heptan gewaschen und getrocknet, wobei man die gewünschte Verbindung erhält.
Ausbeute: 46 % (Reinheit: 82 %)

### Herstellungsbeispiel H3-5: Herstellung von (E)-N-Cyclohexyl-N-pyridin-2-yl-3-p-tolyl-acrylamid, Verbindung 3-32

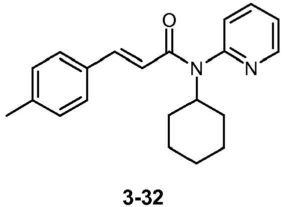

5,50 g (0,034 mol) 4-Methylzimtsäure, 61 ml Toluol und 0,27 g (3,7 mmol) g N,N-Dimethyformamid werden vorgelegt und mit 4,8 g (0,041 mol) Thionylchlorid versetzt. Es wird 1 h nachgerührt und anschließend auf 50°C erwärmt. Toluol und überschüssiges Thionylchlorid werden unter Vakuum abgezogen. Anschließend werden bei 60°C 18 ml Toluol und 4,8 g (0,037 mol) Ethyldiisopropylamin zugesetzt. Es wird eine Lösung von 6,8 g (0,037 mol) Cyclohexyl-2-pyridylamin in Toluol zudosiert und 1 h bei 60°C nachgerührt. Es wird mit Wasser hydrolyiert und 2-Methyltetrahydrofuran zugesetzt. Die organische Phase wird abgetrennt und mit Wasser, 1 N NaOH und nochmals mit Wasser gewaschen. Die organische Phase wird einrotiert und der Rückstand aus MTBE kristallisiert. Der auskristallisierte Feststoff wird abgesaugt, mit MTBE gewaschen und getrocknet, wobei man die gewünschte Verbindung erhält.
Ausbeute: 62 %

### Herstellungsbeispiel H3-6: Herstellung von (E)-N-Cyclohexyl-3,N-diphenyl-acrylamid, Verbindung 3-26

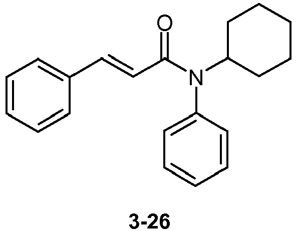

7,60 g (0,051 mol) Zimtsäure, 92 ml Toluol und 0,41 g (5,6 mmol) g N,N-Dimethyformamid werden vorgelegt und mit 7,3 g (0,062 mol) Thionylchlorid versetzt. Es wird 1 h nachgerührt und anschließend auf 50°C erwärmt. Toluol und überschüssiges Thionylchlorid werden unter Vakuum abgezogen. Anschließend werden bei 60°C 22 ml Toluol und 7,3 g (0,056 mol) Ethyldiisopropylamin zugesetzt. Es wird eine Lösung von 9,1 g (0,056 mol) N-Cyclohexylanilin in Toluol zudosiert und 1 h bei 60°C nachgerührt. Es wird mit Wasser bei 50°C hydrolyiert und 2-Methyltetrahydrofuran zugesetzt. Die organische Phase wird abgetrennt und mit Wasser, 1N Salzsäure, 1N NaOH und nochmals mit Wasser gewaschen. Die organische Phase wird einrotiert und der Rückstand aus n-Heptan/MTBE kristallisiert. Der auskristallisierte Feststoff wird abgesaugt, mit n-Heptan gewaschen und getrocknet, wobei man die gewünschte Verbindung erhält.
Ausbeute: 68 %

### Herstellungsbeispiel H3-7: Herstellung von (E)-3-Benzo[1,3]dioxol-5-yl-N-cyclohexyl-N-pyridin-2-yl-acrylamid, Verbindung 3-1

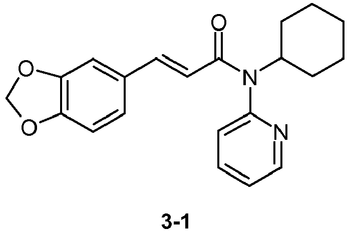

5,18 g (0,027 mol) trans-3,4-(Methylendioxy)zimtsäure, 52 ml Toluol und 0,24 g (3,3 mmol) N,N-Dimethyformamid werden vorgelegt und mit 3,85 g (0,032 mol) Thionylchlorid versetzt. Es wird 1,5 h nachgerührt und anschließend auf 50°C erwärmt. Toluol und überschüssiges Thionylchlorid werden unter Vakuum abgezogen. Anschließend werden unter Normaldruck bei 50-60°C 18 ml Toluol und 3,83 g (0,030 mol) Ethyldiisopropylamin zugesetzt. Es wird eine Lösung von 5,2 g (0,030 mol) N-Cyclohexyl-2-pyridylamin in Toluol zudosiert und 1 h bei 50-60°C nachgerührt. Es wird mit Wasser bei 50-60°C hydrolyiert. Die organische Phase wird abgetrennt und mit Wasser, 1 N NaOH und nochmals mit Wasser gewaschen. Die organische Phase wird filtriert, einrotiert und der Rückstand aus Toluol kristallisiert. Der auskristallisierte Feststoff wird bei 0°C abgesaugt, mit Toluol gewaschen und getrocknet, wobei man die gewünschte Verbindung erhält.
Ausbeute: 76 %

### Formulierungsbeispiele (erfindungsgemäß soweit sie unter die Ansprüche fallen)

### a) Mundpflege

### Formulierungsbeispiel FM-1: Mundwasser

Geeignete Mundwässer können nach folgender Basisrezeptur hergestellt werden:

| Gew.-% | Inhaltsstoff-Typ | Inhaltsstoff-Beispiele |
|---|---|---|
| 0,01-0,1% | antibakterielle Mittel | beta-Naphthol, Thymol, Chlorthymol und Hexylresorcin |
| 5-25% | Feuchthaltemittel | Glycerin, Sorbit, Propylenglykol und Polyalkylenglykol |
| 0,01-0,2% | etherische Öle | Nelkenöl, Pfefferminzöl und Krauseminzöl |
| 0-30% | Ethanol | |
| 0-5 % | Polymer | Polyoxyalkylenblockcopoymere Mw 5000-30000 |
| 40-80% | Wasser | |
| | | |
| | | |
| 0,001-10% | TRPM8-Agonist | |
| 0-10% | Weitere Zusätze | |

Ein Mundwasser folgender Zusammensetzung wird hergestellt:

| Anteil | Inhaltsstoff |
|---|---|
| 177mL | Ethanol 95% |
| 250 g | Sorbit 70% |
| 50mL | TRPM8 Agonist gemäß Herstellungsbeispiel H 1-1..... |
| | als 1% Lösung im Ethanol |
| 0.30 g | Pfefferminzöl, |
| 0.64 g | Methylsalicylat |
| 0.922 g | Eucalyptol |
| 0.639 g | Thymol |
| 1.50 g | Benzoesäure |
| 5.00 g | Pluronic ® F127 |
| | nichtionisches Tensid |
| 0.60 g | Natrium-Saccharin |
| 0.30 g | Natriumcitrat |
| 0.10 g | Zitronensäure |
| q.s. 1 Liter | Wasser |

Zur Herstellung eines Mundwassers werden die oben beschriebenen Komponenten in den angegebenen Mengen miteinander vermischt.

### Formulierungsbeispiel FM-2: Zahnpasta

Geeignete Zahnpasten können nach folgender Basisrezeptur hergestellt werden:

| Gew.-% | Inhaltsstoff-Typ | Inhaltsstoff-Beispiele |
|---|---|---|
| 0,05-0,2% | Fluoride | Natriumfluorid, Zinn(II)-fluorid, Natriummonofluorophosphat; |
| 10-55% | Feuchthaltemittel | Glycerin, Sorbit, Propylenglykol, Polyalkylenglykol |
| 0-50% | Polymer | Polyoxyalkylenblockcopoymere Mw 5000-30000 |
| 10-50% | Wasser | |
| 10-55% | Abrasiva | Calciumpyrophosphat, Dicalciumphosphat, Siliziumoxidhydrat; |
| 2-10% | Bindemittel | Karayagummi, Tragant USP, Natriumalginat, irländisches Moos, Methylcellulose |
| 2-8% | Tensid | Natriumlaurylsulphat, Natrium-N-laurylsarcosinat, Dioctlnatriumsulphosuccinat, Natriumlaurylsulphoacetat |
| 0-10% | Persauerstoffverbindung | Wasserstoffperoxid, anorganischen Peroxiden |
| 0,001-10% | TRPM8-Agonist | |
| 0-10% s.o. | Weitere Zusätze | |

### Formulierungsbeispiel FM-3: Kaugummi

Geeignete Kaugummis können nach folgender Basisrezeptur hergestellt werden:

| Gew.-% | Inhaltsstoff |
|---|---|
| 15 - 25% | Kaugummi-Grundmasse ("gum-base") |
| 20 - 30% | Glucosesirup |
| 50 - 60% | Puderzucker |
| 0,001 - 10% | TRPM8-Agonist gemäß Beispiel .H 2-1... |
| 1 - 2% | Weichmacher (z.B. Glycerin) |
| 3 - 6% | Wasser |

Anstelle des Glucosesirups und des Puderzuckers können für "zuckerfreie" Rezepturen auch die Zuckeralkohole Mannit, Xylit und Sorbit, "Palatinit" und andere sowie künstliche Süßstoffe, wie Saccharin, Cyclamat, Acesulfam-K und Aspartame, eingesetzt werden.

### b) Körperpflege

### Formulierungsbeispiel FK-1: Haarwasser

| | % | Inhaltsstoff (INCI) |
|---|---|---|
| A | q.s. | Parfümöl |
| | 1,00 | PEG-40 Hydrogenated Castor Oil |
| B | 65,0 | Alkohol |
| | 1,0 | Panthenol |
| | 0,5 | Polyquarternium-16 |
| | 0,1 | Menthol |
| | 27,4 | Aqua dem. |
| | 5,00 | wässrige Lösung mit ca. x 0,001-10% % TRPM8 Agonist gemäß .Beispiel..H 3-1.... |

| | | |
|---|---|---|
| Herstellung: Phase A mischen. Phase B zugeben und rühren bis alles gelöst ist, pH-Wert einstellen auf pH 7,0. | | |

### Formulierungsbeispiel FK-2: Haargel

| | % | Inhaltsstoff (INCI) |
|---|---|---|
| A | 45,00 | Carbopol 940 1% in Wasser |
| | 0,70 | Aminomethyl Propanol |
| B | 7,50 | VP/Methacrylamide/Vinyl Imidazole Copolymer |
| | 0,10 | Parfümöl |
| | 0,30 | PEG-40 Hydrogenated Castor Oil |
| | 0,30 | Konservierungsmittel |
| | 0,05 | Disodium EDTA |
| | 0,30 | Panthenol |
| | 8,00 | Alcohol |
| | 5,00 | wässrige Lösung mit ca. 0,001-10 % TRPM8 Agonist gemäß Beispiel.H 1-3... |
| | 32,75 | Aqua dem. |

| | | |
|---|---|---|
| Herstellung: Die Komponenten der Phase A einwiegen und homogenisieren. Phase B lösen und in Phase A einrühren. pH Wert einstellen auf pH 6,9. | | |

### Formulierungsbeispiel FK-3: Kosmetische Sonnenschutzzubereitung

In den folgenden Rezepturen wird eine kosmetische Sonnenschutzzubereitung, enthaltend eine Kombination aus mindestens anorganischem Pigment und organische m UV-Filter beschrieben.

Die Herstellung der nachfolgend genannten Formulierungen erfolgt auf übliche, dem Fachmann bekannte Art und Weise.

| | | |
|---|---|---|
| A | 7,50 Uvinul MC 80 | Ethylhexylcinnamat |
| | 2,00 Uvinul M 40 | Benzophenon-3 |
| | 0,80 Rylo PG 11 | Polyglyceryldimersoyat |
| | 1,00 Span 60 | Sorbitanstearat |
| | 0,50 Vitamin E-Acetat | Tocopherylacetat |
| | 3,00 Dracorin 100 SE | Glycerylstearat, PEG-100 Stearat |
| | 1,00 Cremophor CO 410 | PEG-40-hydriertes Rizinusöl |
| B | 3,00 T-Lite SF | Titandioxid, Aluminiumoxidhydrat, Dimethicon-/Methicon Copolymer |
| | 1,00 Cetiol SB 45 | *Butyrospermum parkii* (Shea Butter) |
| | 6,50 Finsolv TN | C₁₂₋₁₅-Alkylbenzoat |
| C | 5,00 Butylenglykol | Butylenglycol |
| | 0,30 Keltrol | Xanthangummi |
| | 0,10 Edeta BD | Dinatrium-EDTA |
| | 0,10 Allantoin | Allantoin |
| | 66,20 Wasser dem. | Aqua dem. |
| D | 1,00 Sepigel 305 | Polyacrylamid, C₁₃₋₁₄-Isoparaffin, Laureth-7 |
| 0,001-10 % TRPM8 Agonist gemäßBeispiel. H2-3 | | |
| | q.s. | Konservierungsmittel |

### Formulierungsbeispiel FK-4: Feuchtigkeitsspendende Körperpflegecreme

| | % | Inhaltsstoff (INCI) |
|---|---|---|
| A | 6,0 | PEG-7-hydriertes Rizinusöl |
| | 10,0 | Cetearylethylhexanoat |
| | 5,0 | Isopropylmyristat |
| | 7,0 | Mineralöl |
| | 0,5 | Shea Butter (*Butyrospermum parkii*) |
| | 0,5 | Aluminumstearat |
| | 0,5 | Magnesiumstearat |
| | 0,2 | Bisabolol |
| | 0,7 | Quaternium-18-Hectorit |
| B | 5,0 | Dipropylenglykol |
| | 0,7 | Magnesiumsulfat |
| | q.s. | Konservierungsmittel |
| | 62,9 | Aqua dem. |
| | q.s. | Parfümöl |
| C | 1,0 | wässrige Lösung mit 0,001-10 % TRPM8 Agonist gemäß Beispiel H 3-2... |

| | | |
|---|---|---|
| Herstellung: Die Phasen A und B getrennt auf ca. 80 °C erwärmen. Phase B in Phase A einrühren und homogenisieren. Unter Rühren auf ca. 40 °C abkühlen, Phase C zugeben und nochmals homogenisieren. Unter Rühren auf Raumtemperatur abkühlen lassen. | | |

### Formulierungsbeispiel FK-5: Pflegeshampoo

| | % | Inhaltsstoff (INCI) |
|---|---|---|
| A | 30,0 | Natriumlaurethsulfat |
| | 6,0 | Natriu mocoam phoacetat |
| | 6,0 | Cocamidopropylbetain |
| | 3,0 | Natriumlaurethsulfat, Glykoldistearat, Cocamid-MEA, Laureth-10 |
| | 1,0 | wässrige Lösung mit 0,001 - 10 % TRPM8 Agonist gemäß Beispiel H 1-6... |
| | 7,7 | Polyquaternium-44 |
| | 2,0 | Amodimethicon |
| | q.s. | Parfümöl |
| | q.s. | Konservierungsmittel |
| | 1,0 | Natriumchlorid |
| | 43,3 | Aqua dem. |
| B | q.s. | Zitronensäure |

| | | |
|---|---|---|
| Herstellung: Die Komponenten der Phase A mischen und lösen. Den pH-Wert mit Zitronensäure auf 6-7 einstellen. | | |

### Formulierungsbeispiel FK-6: Duschgel

| | % | Inhaltsstoff (INCI) |
|---|---|---|
| A | 40,0 | Natriumlaurethsulfat |
| | 5,0 | Decylglukosid |
| | 5,0 | Cocamidopropylbetain |
| | 1,0 | wässrige Lösung mit 0,001 - 10 % TRPM8 Agonist gemäßBeispiel H 2-5... |
| | 1,0 | Panthenol |
| | q.s. | Parfümöl |
| | q.s. | Konservierungsmittel |
| | 2,0 | Natriumchlorid |
| | 46,0 | Aqua dem. |
| B | q.s. | Zitronensäure |

| | | |
|---|---|---|
| Herstellung: Die Komponenten der Phase A mischen und lösen. Den pH-Wert mit Zitronensäure auf 6-7 einstellen. | | |

### Formulierungsbeispiel FK-7: Shampoo

| | % | Inhaltsstoff (INCI) |
|---|---|---|
| A | 40,0 | Natriumlaurethsulfat |
| | 5,0 | Natrium-C₁₂₋₁₅-Pareth-15-sulfonat |
| | 5,0 | Decylglukosid |
| | q.s. | Parfümöl |
| | 0,1 | Phytantriol |
| | 44,6 | Aqua dem. |
| | 1,0 | wässrige Lösung mit 0,001 -10 % TRPM8 Agonist gemäß H 2-7... |
| | 0,3 | Polyquaternium-10 |
| | 1,0 | Panthenol |
| | q.s. | Konservierungsmittel |
| | 1,0 | Lau reth-3 |
| | 2,0 | Natriumchlorid |

| | | |
|---|---|---|
| Herstellung: Die Komponenten der Phase A mischen und lösen. Den pH-Wert mit Zitronensäure auf 6-7 einstellen. | | |

### Formulierungsbeispiel FK-8: Fußbalsam

| | % | Inhaltsstoff (INCI) |
|---|---|---|
| A | 2,0 | Ceteareth-6, Stearylalkohol |
| | 2,0 | Ceteareth-25 |
| | 5,0 | Cetearylethylhexanoat |
| | 4,0 | Cetylalkohol |
| | 4,0 | Glycerylstearat |
| | 5,0 | Mineralöl |
| | 0,2 | Menthol |
| | 0,5 | Kampfer |
| B | 69,3 | Aqua dem. |
| | q.s. | Konservierungsmittel |
| C | 1,0 | Bisabolol |
| | 1,0 | Tocopherylacetat |
| D | 1,0 | wässrige Lösung mit 0,001 - 10 % TRPM8 Agonist gemäß H 3-3... |
| | 5,0 | Zaubernussextrakt |

| | | |
|---|---|---|
| Herstellung: Die Komponenten der Phasen A und B getrennt voneinander auf ca. 80 °C erwärmen. Phase B in Phase A unter Homogenisieren einrühren. Unter Rühren abkühlen auf ca. 40 °C, die Phasen C und D hinzugeben und kurz nachhomogenisieren. Unter Rühren auf Raumtemperatur abkühlen. | | |

### Formulierungsbeispiel FK-9: Gesichtsreinigungslotion - Typ O/W

:

| | % | Inhaltsstoff (INCI) |
|---|---|---|
| A | 10,0 | Cetearylethylhexanoat |
| | 10,0 | Capryl-/Caprintriglycerid |
| | 1,5 | Cyclopentasiloxan, Cyclohexasilosan |
| | 2,0 | PEG-40-hydriertes Rizinusöl |
| B | 3,5 | Capryl-/Caprintriglycerid, Natriumacrylat-Copolymer |
| C | 1,0 | Tocopherylacetat |
| | 0,2 | Bisabolol |
| | q.s. | Konservierungsmittel |
| | q.s. | Parfümöl |
| D | 3,0 | Polyquaternium-44 |
| | 0,5 | Cocotrimoniummethosulfat |
| | 0,5 | Ceteareth-25 |
| | 2,0 | Panthenol, Propylenglykol |
| | 4,0 | Propylenglykol |
| | 0,1 | Dinatrium-EDTA |
| | 1,0 | wässrige Lösung mit 0,001 - 10 % TRPM8 Agonist gemäß H 3-4... |
| | 60,7 | Aqua dem. |

| | | |
|---|---|---|
| Herstellung: Phase A lösen. Phase B in Phase A einrühren, Phase C in die kombinierten Phasen A und B einarbeiten. Phase D lösen, in die kombinierten Phasen A, B und C einrühren und homogenisieren. 15 min nachrühren. | | |

### Formulierungsbeispiel FK-10: Body-Spray

| | % | Inhaltsstoff (INCI) |
|---|---|---|
| A | 3,0 | Ethylhexylmethoxycinnamat |
| | 2,0 | Diethylaminohydroxybenzoylhexylbenzoat |
| | 1,0 | Polyquaternium-44 |
| | 3,0 | Propylenglykol |
| | 2,0 | Panthenol, Propylenglykol |
| | 1,0 | Cyclopentasiloxan, Cyclohexasilosan |
| | 10,0 | Octyldodecanol |
| | 0,5 | PVP |
| | 10,0 | Capryl-/Caprintriglycerid |
| | 3,0 | C₁₂₋₁₅-Alkylbenzoat |
| | 3,0 | Glycerin |
| | 1,0 | Tocopherylacetat |
| | 0,3 | Bisabolol |
| | 1,0 | wässrige Lösung mit 0,001 - 10 % TRPM8 Agonist gemäß H 2-4... |
| | 59,2 | Alkohol |

| | | |
|---|---|---|
| Herstellung: Die Komponenten der Phase A einwiegen und klar lösen. | | |

### Formulierungsbeispiel FK-11: Hautpflegegel

| | % | Inhaltsstoff (INCI) |
|---|---|---|
| A | 3,6 | PEG-40-hydriertes Rizinusöl |
| | 15,0 | Alkohol |
| | 0,1 | Bisabolol |
| | 0,5 | Tocopherylacetat |
| | q.s. | Parfümöl |
| B | 3,0 | Panthenol |
| | 0,6 | Carbomer |
| | 1,0 | wässrige Lösung mit 0,001 - 10 % TRPM8 Agonist gemäßH 3-5... |
| | 75,4 | Aqua dem. |
| C | 0,8 | Triethanolamin |

### Formulierungsbeispiel FK-12: After-Shave-Lotion

| | % | Inhaltsstoff (INCI) |
|---|---|---|
| A | 10,0 | Cetearylethylhexanoat |
| | 5,0 | Tocopherylacetat |
| | 1,0 | Bisabolol |
| | 0,1 | Parfümöl |
| | 0,3 | Acrylate/C₁₀₋₃₀ Alkylacrylat-Crosspolymer |
| B | 15,0 | Alkohol |
| | 1,0 | Panthenol |
| | 3,0 | Glycerin |
| | 1,0 | wässrige Lösung mit 0,001 - 10 % TRPM8 Agonist gemäß...H 2-7 |
| | 0,1 | Triethanolamin |
| | 63,5 | Aqua dem. |

| | | |
|---|---|---|
| Herstellung: Die Komponenten der Phase A mischen. Phase B lösen, in Phase A einarbeiten und homogenisieren. | | |

### Formulierungsbeispiel FK-13: After-Sun-Lotion

| | % | Inhaltsstoff (INCI) |
|---|---|---|
| A | 0,4 | Acrylate/C10-30-Alkylacrylat-Crosspolymer |
| | 15,0 | Cetearylethylhexanoat |
| | 0,2 | Bisabolol |
| | 1,0 | Tocopherylacetat |
| | q.s. | Parfümöl |
| B | 1,0 | Panthenol |
| | 15,0 | Alkohol |
| | 3,0 | Glycerin |
| | 1,0 | wässrige Lösung mit 0,001 - 10 % TRPM8 Agonist gemäß H 3-6... |
| | 63,2 | Aqua dem. |
| C | 0,2 | Triethanolamin |

| | | |
|---|---|---|
| Herstellung: Die Komponenten der Phase A mischen. Phase B unter Homogenisieren in Phase A einrühren. Mit Phase C neutralisieren und erneut homogenisieren. | | |

### Formulierungsbeispiel FK-14: Sonnenschutzlotion

| | % | Inhaltsstoff (INCI) |
|---|---|---|
| A | 4,5 | Ethylhexylmethoxycinnamat |
| | 2,0 | Diethylaminohydroxybenzoylhexylbenzoat |
| | 3,0 | Octocrylen |
| | 2,5 | Di-C12-13-Alkylmalat |
| | 0,5 | Tocopherylacetat |
| | 4,0 | Polyglyceryl-3-methylglucosedistearat |
| B | 3,5 | Cetearylisononanoat |
| | 1,0 | VP-/EicosenCopolymer |
| | 5,0 | Isohexadecan |
| | 2,5 | Di-C12-13-Alkylmalat |
| | 3,0 | Titaniumdioxid, Trimethoxycaprylylsilan |
| C | 5,0 | Glycerin |
| | 1,0 | Natriumcetearylsulfat |
| | 0,5 | Xanthangummi |
| | 59,7 | Aqua dem. |
| D | 1,0 | wässrige Lösung mit 0,001 - 10 % TRPM8 Agonist gemäß H 2-9... |
| | 1,0 | Phenoxyethanol, Methylparaben, Ethylparaben, Butylparaben, Propyl-paraben, Isobutylparaben |
| | 0,3 | Bisabolol |

| | | |
|---|---|---|
| Herstellung: Die Komponenten der Phasen A und B getrennt voneinander auf ca. 80 °C erwärmen. Phase B in Phase A einrühren und homogenisieren. Phase C auf ca. 80 °C erwärmen und unter Homogenisieren in die kombinierten Phasen A und B einrühren. Unter Rühren auf ca. 40 °C abkühlen, Phase D zugeben und nochmals homogenisieren. | | |

### Formulierungsbeispiel FK-15: Pflaster

50 Teile Wirkstoff gemäß Herstellungsbeispiel H 3-7 wurden in 100 Teilen einer 10 %igen Natriumlaurylsulfatlösung unter starkem Rühren und Erwärmen auf 50°C dispergiert. In die entstandene Emulsion wurden 880 Teile einer 50 %igen Butylacrylatdispersion eingerührt und die erhaltene wirkstoffhaltige Polymerdispersion mittels einer geeigneten Ausstreichrakel auf einer Polyesterfolie mit 15 µm Dicke (Fa. Kalle, D-Wiesbaden) ausgestrichen und bei 35 bis 40 °C unter kontrollierter Luftfeuchtigkeit getrocknet. Je nach Rakeleinstellung resultierten Flächengewichte von 5 mg/cm², die durch Mehrfachauftrag weiter erhöht werden konnten. Der so hergestellte selbstklebende Film mit einem Wirkstoffgehalt von 5 % wurde mit einer silikonisierten Abziehfolie aus Polyester (Scotch Pak 75 mu m, 3M) versehen und in die gewünschten Abmessungen geschnitten.

Die Mengenangabe betreffen jeweils Gewichtsteile.

### c) Nahrungsmittel

### Formulierungsbeispiel FN-1: Pudding

**Rezept (für 100 ml)**

| Inhaltsstoff | Menge |
|---|---|
| Fettfreie Trockenmilch | 10,715 g |
| Saccharose | 5 g |
| Novelose Stärke, National Starch | 7 g |
| Pflanzenölgemisch | 2,2 g |
| Carrageenan | 0,016 g |
| Vanillearoma | 0,5 g |
| Natriumstearoyl-2-lactylat | 0,095 g |
| Gelber Farbstoff | 0,189 g |
| Magnesiumphosphat | 0,165 g |
| Vitaminvormischung | 1,84 g |
| Spurenelementvormischung | 0,015 g |
| Wirkstoff gemäß Herstellungsbeispiel H 2-11 | 0,5 g |
| Wasser | 81,94 g |

| | |
|---|---|
| Herstellung: Neun Zehntel des Wassers auf 43,3 °C erhitzen. Magermilchpulver im Wasser auflösen. Öl auf 60 °C erhitzen und Carrageenan und öllösliche Vitamine zum Öl geben. Öl in das Erzeugnis einmischen. Die übrigen Bestandteile außer der modifizierten Stärke, Vanillearoma und Vitaminvormischung hinzufügen. Das Gemisch homogenisieren. Stärke langsam hinzufügen. Wirkstoff, Vitamine und Aroma hinzufügen. Feststoffgehalt standardisieren. In sterilen Einheiten erhitzen und in Dosen verpacken. | |

### d) Formulierungsbeispiel FT-1: Textilausrüstung mit erfindungemäßen Wirkstoffen

Zunächst stellt man eine wässrige Aufschlämmung amylosehaltiger Stärke her, indem man 570 g entionisiertes Wasser wurden mit 10 g eines handelsüblichen Konservierungsmittels versetzt. Hierin löste man 20 g Carboxymethylcellulose, gab anschließend 400 g einer amylosehaltigen Stärke mit einem Amylosegehalt von 50 Gew.-% zu und stellte unter Rühren eine Aufschlämmung her.

Anschließend erfolgt die Herstellung wässriger Flotten mit amylosehaltiger Stärke nach einer der beiden folgenden Methoden:
Methode 1: Die jeweilige Aufschlämmung wird durch Verdünnen mit Wasser auf einen Stärke-gehalt von 5 oder 15 Gew.-% eingestellt.
Methode 2: Die jeweilige Aufschlämmung werden zunächst mit Wasser auf einen Stärke-Gehalt von 5 oder 15 Gew.-% verdünnt und anschließend mit 30 g/l einer 30 Gew.-%igen, wässrigen Polyurethandispersion (nicht-ionogen) versetzt.

Anschießend erfolgt die Ausrüstung eines Gewebes mit amylosehaltiger Stärke und erfindungsgemäßem Wirkstoff:
Baumwollgewebemuster mit einem Flächengewicht von 124 g/m² wird mit einer der obe hergestellten Flotten mittels eines Foulards bis zu einer Flottenaufnahme von 80 Gew.-%, bezogen auf das Gewicht des Gewebes behandelt. Anschließend trocknet man 2 min bei 120°C.

Anschließend werden die so ausgerüsteten Gewebemuster mit einer wässrigen Wirkstoff-Formulierung behandelt, indem man eine wässrige Emulsion/Suspension eines erfindungsgemäßen Wirkstoffs mit einem Wirkstoffgehalt von 1 bis 7 Gew.-% bis zu einer Flottenaufnahme von 79 - 80 Gew.-% auf das Gewebemuster auffoulardiert. Anschließend werden die so behandelten Gewebemuster in einem Haushaltstrockner bis zu einer Restfeuchte von 15 % getrocknet.

Die so hergestellten wirkstoffbeladenen Gewebe können dann weiter untersucht werden, wie z.B. auf deren Kühlende Wirkung bei Hautkontakt oder deren Repellent-Wirkung auf Insekten.

### Beispiele, insbesondere zum speziellen Aspekt der Erfindung (Kapitel 6.)

Die nachfolgend aufgeführten Beispiele S-X betreffen insbesondere den speziellen Aspekt der Erfindung (Kapitel 6). Sie sind allerdings nicht auf diesen begrenzt und können genauso zur Erläuterung des allgemeinen Aspektes der Erfindung dienen. Umgekehrt sind die mit S-X bezeichneten Beispiele nicht die ausschließlichen Beispiele für den speziellen Aspekt der Erfindung (Kapitel 6), sondern auch die zuvor beschriebenen Beispiele können selbstverständlich zur Erläuterung des speziellen Aspektes der Erfindung hinzugezogen werden. Dementsprechend werden in den nachfolgenden Beispielen die gemäß dem speziellen Aspekt der Erfindung zu verwendenden Verbindungen (Verbindungen aus der Tabelle 0) auch als erfindungsgemäße Verbindungen bezeichnet.

### Beispiel S-1

Herstellung von Aromen mit Kühlwirkung vom Eukalyptus-Menthol-Typ unter Verwendung der erfindungsgemäßen Kühlsubstanzen:
Es wurden vermischt (alle Angaben, soweit nicht anders vermerkt, in Gew-%):

| **Ansatz** | a | b | c (ne) | d (ne) | e (ne) | f (ne) | g (ne) | h (ne) |
|---|---|---|---|---|---|---|---|---|
| **Komponente** | | | | | | | | |
| Anethol | 10 | 10 | 10 | 10 | 10 | 10 | 10 | 10 |
| Pfefferminzöl Mentha piperita Typ Willamette | 20 | 20 | 20 | 20 | 20 | 20 | 20 | 20 |
| Pfefferminzöl Mentha arvensis, rektifiziert | 20 | 20 | 20 | 20 | 20 | 20 | 20 | 20 |
| I-Menthyllactat | 1 | 1 | 1 | 1 | 1 | 1 | 1 | 1 |
| 2-Hydroxyethylmenthylcarbonat | 2 | 2 | 2 | 2 | 2 | 2 | 2 | 2 |
| 2-Hydroxypropylmenthylcarbonat | 2 | 2 | 2 | 2 | 2 | 2 | 2 | 2 |
| 1,8-Cineol (Eucalyptol) | 5 | 5 | 5 | 5 | 5 | 5 | 5 | 5 |
| *I*-Menthol | 39,4 | 39,5 | 39,7 | 39,7 | 39,8 | 39,4 | 39,5 | 39,4 |
| 3,4-Methylendioxyzimtsäure-N-cyclohexyl-N-2-pyridylamid | 0,6 | | | | | | | |
| 3,4-Methylendioxyzimtsäure-N,N-diphenylamid | | 0,5 | | | | | | |
| 2,3,4,5,6,10b,11,12-Octahydro-spiro[4b-azachrysen-12,2'-[1,3]dithiolan-]-1-on | | | 0,2 | | | | | |
| 2,3,4,5,6,10b,11,12-octahydro-3,3-dimethyl-spiro[4b-azachrysen-12,2'-[1,3]dithiolan]-1-on | | | 0,1 | 0,3 | | | | |
| 2,3,4,5,6,10b,11,12-octahydro-3,3-dimethyl-spiro[4b-azachrysen-12,2'-[1,3]dithian]-1-on | | | | | 0,2 | | | |
| sec-Butyl-(5-methoxy-2-pyridin-2-yl-pyrimidin-4-yl)-amin | | | | | | 0,3 | | |
| Cyclopentyl-(5-methoxy-2-pyridin-2-yl-pyrimidin-4-yl)-amin | | | | | | | 0,5 | |
| Isopropyl-(5-methoxy-2-pyridin-2-yl-pyrimidin-4-yl)-amin | | | | | | 0,3 | | 0,6 |
| | | | | | | | | |
| Total | 100 | 100 | 100 | 100 | 100 | 100 | 100 | 100 |

| | | | | | | | | |
|---|---|---|---|---|---|---|---|---|
| (ne) = nicht erfindungsgemäß | | | | | | | | |

Die so erhaltenen Aromen wurden in eine Standard-Zahnpasta-Masse auf Kieselsäure-Basis in einer Konzentration von 1,2 Gew.-% eingearbeitet. Die Zahnpasten wurden von einem sensorisch geschulten Experten- Panel unter Praxisbedingungen getestet. Die sensorischen Bewertungen ergaben: sehr schöne, reiche und voluminöse minzige Frische mit einem vergleichsweise schnell einsetzenden, starken und ausgeprägtem und vergleichsweise sehr lang anhaltendem kühlendem Frischeeindruck, wobei auffallend war, dass sich das Kälteempfinden schnell im gesamten Mundraum wahrzunehmen war. Zudem wurde das Aroma als weicher und neutraler und weniger scharf beurteilt als ohne die erfindungsgemäßen Verbindungen. Wenn anstelle der erfindungsgemäßen Verbindungen eine gleiche Menge von 3-Menthancarbonsäure-N-ethylamid ("WS-3"), das im allgemeinen als Standard-Kühlsubstanz gilt, verwendet wurde, zeigte sich, dass der kühlende Frischeeindruck als nicht so kräftig, nicht so voluminös und nicht so lange anhaltend empfunden wurde.

### Beispiel S-2

Herstellung von Aromen mit Kühlwirkung vom Krauseminz-Typ unter Verwendung der erfindungsgemäßen Kühlsubstanzen.

Es wurden vermischt (alle Angaben, soweit nicht anders vermerkt, in Gew-%):

| Ansatz | a | b | c | d (ne) | e (ne) | f (ne) | g (ne) | h (ne) |
|---|---|---|---|---|---|---|---|---|
| **Komponente** | | | | | | | | |
| Menthol | 29,25 | 29,25 | 29 | 29,6 | 29,6 | 29,5 | 29,2 | 29,5 |
| Carvon | 20 | 20 | 20 | 20 | 20 | 20 | 20 | 20 |
| Krauseminzöl Typ Native | 20 | 20 | 20 | 20 | 20 | 20 | 20 | 20 |
| Anethol | 5 | 5 | 5 | 5 | 5 | 5 | 5 | 5 |
| Pfefferminzöl Mentha arvensis rektifiziert | 10 | 10 | 10 | 10 | 10 | 10 | 10 | 10 |
| Pfefferminzöl Mentha piperita Typ Willamette | 15 | 15 | 15 | 15 | 15 | 15 | 15 | 15 |
| 3,4-Methylendioxyzimtsäure-N,N-diphenylamid | 0,75 | | | | | | | |
| 3,4-Methylendioxyzimtsäure-N-cyclohexyl-N-2-pyridylamid | | 0,75 | | | | | | |
| 4-Methoxyzimtsäure-N,N-diphenylamid | | | 1 | | | | | |
| 2,3,4,5,6,10b,11,12-Octahydro-spiro[4b-azachrysen-12,2'-[1,3]dithiolan-]-1-on | | | | 0,4 | | | | 0,2 |
| 2,3,4,5,6,10b,11,12-Octahydro-3-methyl-spiro[4b-azachrysen-12,2'-[1,3]dithiolan]-1-on | | | | | 0,4 | | | |
| 5,6,10b,11-Tetrahydro-3-methyl-spiro[12H-benzo[a]furo[3,4-f]chinolizin-12,2'-[1,3]dithiolan]-1(3H)on | | | | | | 0,5 | | |
| Isopropyl-(5-methoxy-2-pyridin-2-yl-pyrimidin-4-yl)-amin | | | | | | | 0,6 | |
| Cyclobutyl-(5-methoxy-2-pyridin-2-yl-pyrimidin-4-yl)-amine | | | | | | | 0,2 | 0,3 |
| | | | | | | | | |
| Total | 100 | 100 | 100 | 100 | 100 | 100 | 100 | 100 |

| | | | | | | | | |
|---|---|---|---|---|---|---|---|---|
| (ne) = nicht erfindungsgemäß | | | | | | | | |

Die erhaltenen Aromen wurden mit einer Konzentration von 1,2 % in eine Zahnpasta-Masse eingearbeitet , die zu einem Anteil von 65% aus Natriumbicarbonat besteht. Die Zahnpasten wurden unter Praxisbedingungen getestet und von einem sensorisch geschulten Experten-Panel bewertet. Es wurde jeweils ein schöner, kräftiger Krauseminzgeschmack in Verbindung mit einem voluminösen und lang anhaltenden kühlenden Frischegeschmack festgestellt.

### Beispiel S-3

Herstellung von Aromen mit Kühlwirkung und einem würzig-aromatischen Geschmackseindruck unter Verwendung der erfindungsgemäßen Kühlsubstanzen.

Es wurden vermischt (alle Angaben, soweit nicht anders vermerkt, in Gew-%):

| Ansatz | a | b | c (ne) | d | e (ne) | f | g (ne) | h (ne) |
|---|---|---|---|---|---|---|---|---|
| Komponente | | | | | | | | |
| *I*-Menthol | 29,2 | 29 | 29,5 | 29,4 | 29,4 | 29 | 29,3 | 29 |
| Pfefferminzöl Mentha arvensis, rektifiziert | 25 | 25 | 25 | 25 | 25 | 25 | 25 | 25 |
| Pfefferminzöl Mentha piperita Typ Willamette | 15 | 15 | 15 | 15 | 15 | 15 | 15 | 15 |
| Anethol | 10 | 10 | 10 | 10 | 10 | 10 | 10 | 10 |
| Krauseminzöl Typ Native | 10 | 10 | 10 | 10 | 10 | 10 | 10 | 10 |
| Zimtaldehyd | 5 | 5 | 5 | 5 | 5 | 5 | 5 | 5 |
| Eugenol | 5 | 5 | 5 | 5 | 5 | 5 | 5 | 5 |
| 3,4-Methylendioxyzimtsäure-N,N-diphenylamid | 0,8 | | | 0,3 | | 0,3 | | |
| 4-Methylzimtsäure-N,N-diphenylamid | | 1 | | | | | | |
| 2,3,4,5,6,10b,11,12-Octahydro-spiro[4b-azachrysen-12,2'-[1,3]dithiolan-]-1-on | | | 0,5 | | | | 0,2 | |
| 2,3,4,5,6,10b,11,12-octahydro-3,3-dimethyl-spiro[4b-azachrysen-12,2'-[1,3]dithiolan]-1-on | | | | 0,3 | | | | |
| 2,3,4,5,6,10b,11,12-octahydro-3-methyl-spiro[4b-azachrysen-12,2'-[1,3]dithian]-1-on | | | | | 0,4 | | | |
| 5-Ethyl-2-pyridin-2-yl-pyrimidin-4-yl)-isopropyl-amin | | | | | | 0,7 | | 1 |
| sec-Butyl-(5-methoxy-2-pyridin-2-yl-pyrimidin-4-yl)-amin | | | | | 0,2 | | | |
| Cyclopentyl-(5-ethyl-2-pyridin-2-yl-pyrimidin-4-yl)-amine | | | | | | | 0,5 | |
| | | | | | | | | |
| Total | 100 | 100 | 100 | 100 | 100 | 100 | 100 | 100 |

| | | | | | | | | |
|---|---|---|---|---|---|---|---|---|
| (ne) = nicht erfindungsgemäß | | | | | | | | |

Die so erhaltenen Aromen wurden jeweils in eine Standard-Zahnpasta-Masse auf Kieselsäure-Basis in einer Konzentration von 1,2 Gew.-% eingearbeitet. Die Zahnpasten wurden von einem sensorisch geschulten Experten-Panel unter Praxisbedingungen getestet. Die sensorische Bewertung ergab jeweils: sehr schöne, minzige, aromatisch-würzige Frische mit einem sehr starken und ausgeprägten, sehr lang anhaltenden kühlenden Frischeeindruck.

### Beispiel S-4

Herstellung von Aromen mit Kühlwirkung und Wintergrün-Geschmack unter Verwendung der erfindungsgemäßen Kühlsubstanzen:
Es wurden vermischt (alle Angaben, soweit nicht anders vermerkt, in Gew-%):

| Ansatz | a | b | c | d (ne) | e (ne) | f (ne) | g | h (ne) |
|---|---|---|---|---|---|---|---|---|
| Komponente | | | | | | | | |
| Anethol | 10 | 10 | 10 | 10 | 10 | 10 | 10 | 10 |
| Pfefferminzöl Mentha arvensis | 12 | 12 | 12 | 12 | 12 | 12 | 12 | 12 |
| Pfefferminzöl Mentha piperita Typ Willamette | 12 | 12 | 12 | 12 | 12 | 12 | 12 | 12 |
| Methylsalicylat | 25 | 25 | 25 | 25 | 25 | 25 | 25 | 25 |
| I-Menthol | 40,5 | 40,25 | 40,25 | 40,5 | 40,5 | 40,4 | 40,5 | 40,4 |
| 3,4-Methylendioxyzimtsäure-N-cyclohexyl-N-2-pyridylamid | 0,5 | | | | | | 0,3 | |
| 4-Methoxyzimtsäure-N-cyclohexyl-N-2-pyridylamid | | 0,75 | 0,5 | | | | | |
| Zimtsäure-N,N-diphenylamid | | | 0,25 | | | | | |
| 2,3,4,5,6,10b,11,12-Octahydro-spiro[4b-azachrysen-12,2'-[1,3]dithian]-1-on | | | | 0,4 | | | | |
| 2,3,4,5,6,10b,11,12-octahydro-3,3-dimethyl-spiro[4b-azachrysen-12,2'-[1,3]dithiolan]-1-on | | | | 0,1 | 0,1 | | | |
| 5-Ethyl-2-pyridin-2-yl-pyrimidin-4-yl)-isopropyl-amin | | | | | 0,4 | 0,6 | | |
| (1,2-Dimethyl-propyl)-(5-methoxy-2-pyridin-2-yl-pyrimidin-4-yl)-amin | | | | | | | 0,2 | |
| Cyclopentyl-(5-methoxy-2-pyridin-2-yl-pyrimidin-4-yl)-amin | | | | | | | | 0,6 |
| | | | | | | | | |
| Total | 100 | 100 | 100 | 100 | 100 | 100 | 100 | 100 |

| | | | | | | | | |
|---|---|---|---|---|---|---|---|---|
| (ne) = nicht erfindungsgemäß | | | | | | | | |

Die so erhaltenen Aromen wurden in eine Standard-Zahnpasta-Masse auf Kieselsäure-Basis in einer Konzentration von 1,2 Gew.-% eingearbeitet. Die Zahnpasten wurden von einem sensorisch geschulten Expertenpanel unter Praxisbedingungen getestet. Die sensorische Bewertung ergab jeweils: ausgeprägte frisch-minzige Wintergrün-Note, mit einem sehr starken und ausgeprägten, sehr lang anhaltenden kühlenden Frischeeindruck.

### Beispiel S-5

Herstellung von Aromen mit Kühlwirkung und Pfefferminzgeschmack unter Verwendung von der erfindungsgemäßen Kühlsubstanzen:
Es wurden vermischt (alle Angaben, soweit nicht anders vermerkt, in Gew-%):

| Ansatz | a | b | c (ne) | d (ne) | e | f (ne) | g (ne) | h (ne) |
|---|---|---|---|---|---|---|---|---|
| Komponente | | | | | | | | |
| Pfefferminzöl Mentha arvensis | 59 | 59,2 | 59,5 | 59,5 | 59 | 59 | 59 | 59 |
| I-Menthon | 20 | 20 | 20 | 20 | 20 | 20 | 20 | 20 |
| I-Menthol | 20 | 20 | 20 | 20 | 20 | 20 | 20 | 20 |
| 3,4-Methylendioxyzimtsäure-N-cyclohexyl-N-2-pyridylamid | 1 | | | | 0,2 | | | |
| 3,4-Methylendioxyzimtsäure-N,N-diphenylamid | | 0,8 | | | | | | |
| 2,3,4,5,6,10b,11,12-Octahydro-spiro[4b-azachrysen-12,2'-[1,3]dithiolan-]-1-on | | | 0,5 | | | | 0,2 | |
| 2,3,4,5,6,10b,11,12-Octahydro-3-methyl-spiro[4b-azachrysen-12,2'-[1,3]dithiolan]-1-on | | | | 0,3 | | | | |
| 2,3,4,5,6,10b,11,12-octahydro-3,3-dimethyl-spiro[4b-azachrysen-12,2'-[1,3]dithiolan]-1-on | | | | 0,2 | | | | |
| Isopropyl-(5-methoxy-2-pyridin-2-yl-pyrimidin-4-yl)-amin | | | | | 0,8 | | | 0,5 |
| sec-Butyl-(5-methoxy-2-pyridin-2-yl-pyrimidin-4-yl)-amin | | | | | | 1 | | |
| Cyclopentyl-(5-methoxy-2-pyridin-2-yl-pyrimidin-4-yl)-amin | | | | | | | 0,8 | 0,5 |
| | | | | | | | | |
| Total | 100 | 100 | 100 | 100 | 100 | 100 | 100 | 100 |

| | | | | | | | | |
|---|---|---|---|---|---|---|---|---|
| (ne) = nicht erfindungsgemäß | | | | | | | | |

Die so erhaltenen Aromen wurden jeweils in eine zuckerfreie Standard-Kaugummi-Masse in einer Konzentration von 1,5 Gew.-% eingearbeitet. Die Kaugummis wurden von einem geschulten Experten-Panel auf ihre sensorische Qualität getestet. Es zeigte sich, dass die Aromen durch das Hinzufügen der erfindungsgemäßen Substanzen eine ausgeprägte Frischenote enthalten, die den Pfefferminzgeschmack verstärkt und für ein lang anhaltendes Frischeempfinden sorgt, das auch nach dem Kauen des Kaugummis für eine lange Zeit noch deutlich wahrgenommen wird.

### Beispiel S-6

Herstellung von Aromen mit Kühlwirkung und Krauseminzgeschmack unter Verwendung der erfindungsgemäßen Kühlsubstanzen:
Es wurden vermischt (alle Angaben, soweit nicht anders vermerkt, in Gew-%):

| Ansatz | a | b | c | d (ne) | e | f (ne) | g (ne) | h |
|---|---|---|---|---|---|---|---|---|
| Komponente | | | | | | | | |
| Pfefferminzöl Mentha piperita Typ Madras | 50 | 50,3 | 50,3 | 51 | 50,5 | 51 | 50 | 50 |
| Eucalyptol | 20 | 20 | 20 | 20 | 20 | 20 | 20 | 20 |
| I-Menthol | 13,5 | 13,5 | 13,5 | 13,5 | 13,5 | 13,5 | 13,5 | 13,5 |
| I-Menthon | 10 | 10 | 10 | 10 | 10 | 10 | 10 | 10 |
| Krauseminzöl Typ Midwest Scotch | 5 | 5 | 5 | 5 | 5 | 5 | 5 | 5 |
| 4-Methylzimtsäure N-cyclohexyl-N-2-pyridylamid | 1,5 | | | | | | | |
| 4-Methoxyzimtsäure-N, N-diphenylamid | | 0,6 | | | 0,8 | | | 1 |
| 3,4-Methylendioxyzimtsäure-N,N-diphenylamid | | | 1,2 | | | | | |
| Zimtsäure-N-cyclohexyl-N-2-pyridylamid | | 0,6 | | | | | | |
| 2,3,4,5,6,10b,11,12-octahydro-3,3-dimethyl-spiro[4b-azachrysen-12,2'-[1,3]dithiolan]-1-on | | | | 0,5 | 0,2 | | | |
| 2,3,4,5,6,10b,11,12-octahydro-3,3-dimethyl-spiro[4b-azachrysen-12,2'-[1,3]dithian]-1-on | | | | | | 0,5 | | |
| sec-Butyl-(5-methoxy-2-pyridin-2-yl-pyrimidin-4-yl)-amin | | | | | | | 1,2 | |
| sec-Butyl-(5-ethyl-2-pyridin-2-yl-pyrimidin-4-yl)-amin | | | | | | | 0,3 | 0,5 |
| Total | 100 | 100 | 100 | 100 | 100 | 100 | 100 | 100 |

| | | | | | | | | |
|---|---|---|---|---|---|---|---|---|
| (ne) = nicht erfindungsgemäß | | | | | | | | |

Die so erhaltenen Aromen wurden jeweils in eine zuckerfreie Standard-Kaugummi-Masse in einer Konzentration von 1,5 Gew.-% eingearbeitet. Die Kaugummis wurden von einem geschulten Experten-Panel auf seine sensorische Qualität getestet. Es zeigte sich, dass die Aromen durch das Hinzufügen der erfindungsgemäßen Substanzen eine ausgeprägte Frischenote erhalten, die mit dem typischen Krauseminzgeschmack sehr gut harmoniert und für ein ausgeprägtes, lang anhaltendes Frischeempfinden sorgt, das auch nach dem Kauen des Kaugummis für eine lange Zeit noch deutlich wahrnehmbar anhält.

### Beispiel S-7

Herstellung von Aromen mit Kühlwirkung und einem aromatisch-würzigen Zimtgeschmack unter Verwendung der erfindungsgemäßen Kühlsubstanzen:
Es wurden vermischt (alle Angaben, soweit nicht anders vermerkt, in Gew-%):

| Ansatz | a | b | c | d (ne) | e (ne) | f (ne) | g (ne) | h |
|---|---|---|---|---|---|---|---|---|
| Komponente | | | | | | | | |
| Menthylmethylether | 3 | 3 | 3 | 3 | 3 | 3 | 3 | 3 |
| Zimtaldehyd | 10 | 10 | 10 | 10 | 10 | 10 | 10 | 10 |
| Anethol | 10 | 10 | 10 | 10 | 10 | 10 | 10 | 10 |
| Eugenol | 2 | 2 | 2 | 2 | 2 | 2 | 2 | 2 |
| Pfefferminzöl Mentha piperita Typ Madras | 10 | 10 | 10 | 10 | 10 | 10 | 10 | 10 |
| Pfefferminzöl mentha arvensis | 10 | 10 | 10 | 10 | 10 | 10 | 10 | 10 |
| Krauseminzöl Typ Midwest Scotch | 10 | 10 | 10 | 10 | 10 | 10 | 10 | 10 |
| I-Menthol | 40 | 39,8 | 40,4 | 40,4 | 41 | 40,5 | 40 | 40,2 |
| 2-Hydroxyethylmenthylcarbonat | 2 | 2 | 2 | 2 | 2 | 2 | 2 | 2 |
| 2-Hydroxypropylmenthylcarbonat | 2 | 2 | 2 | 2 | 2 | 2 | 2 | 2 |
| Zimtsäure-N,N-diphenylamid | | 0,6 | | | | | | |
| 4-Methoxyzimtsäure-N-cyclohexyl-N-2-pyridylamid | | 0,6 | | | | | | |
| 3,4-Methylendioxyzimtsäure-N,N-diphenylamid | 1 | | 0,4 | | | | | 0,1 |
| 5,6,10b,11-Tetrahydro-3-methyl-spiro[12H-benzo[a]furo[3,4-f]chinolizin-12,2'-[1,3]dithian]-1(3H)on | | | | 0,3 | | | | |
| 2,3,4,5,6,10b,11,12-Octahydro-spiro[4b-azachrysen-12,2'-[1,3]dithian]-1-on | | | | 0,3 | | | | |
| 2,3,4,5,6,10b,11,12-Octahydro-spiro[4b-azachrysen-12,2'-[1,3]dithiolan-]-1-on ( | | | 0,2 | | | 0,5 | | 0,2 |
| Isopropyl-(5-methoxy-2-pyridin-2-yl-pyrimidin-4-yl)-amin | | | | | | | 1 | |
| Cyclobutyl-(5-ethyl-2-pyridin-2-yl-pyrimidin-4-yl)-amin | | | | | | | | 0,5 |
| | | | | | | | | |
| Total | 100 | 100 | 100 | 100 | 100 | 100 | 100 | 100 |

| | | | | | | | | |
|---|---|---|---|---|---|---|---|---|
| (ne) = nicht erfindungsgemäß | | | | | | | | |

Die so erhaltenen Aromen wurden jeweils in eine zuckerfreie Standard-Kaugummi-Masse in einer Konzentration von 1,5 Gew.-% eingearbeitet. Die Kaugummis wurden von einem geschulten Experten-Panel auf seine sensorische Qualität getestet. Es zeigte sich, dass die Aromen durch das Hinzufügen der erfindungsgemäßen Kühlsubstanzen eine ausgeprägte Frischenote erhalten, die mit dem würzig-aromatischen Geschmack sehr gut harmoniert und für ein schnell einsetzendes im gesamten Mundraum wahrnehmbares und dabei lang anhaltendes Frischeempfinden sorgt, das auch nach dem Kauen des Kaugummis für eine längere Zeit noch deutlich wahrnehmbar anhält. Insgesamt wirkt das Aroma durch den Zusatz von erfindungsgemäßen Kühlsubstanzen harmonischer und deutlich weniger scharf.

### Beispiel S-8

Herstellung von Mundwasser-Aromen mit Kühlwirkung unter Verwendung der erfindungsgemäßen Kühlsubstanzen:
Es wurden vermischt (alle Angaben, soweit nicht anders vermerkt, in Gew-%):

| Ansatz | a | b | c (ne) | d (ne) | e (ne) | f (ne) | g (ne) | h |
|---|---|---|---|---|---|---|---|---|
| Komponente | | | | | | | | |
| Anethol | 30 | 30 | 30 | 30 | 30 | 30 | 30 | 30 |
| Eucalyptol | 25 | 25 | 25 | 25 | 25 | 25 | 25 | 25 |
| I-Menthol | 44,4 | 44,2 | 44,9 | 44,8 | 44,4 | 44,5 | 44,4 | 44,4 |
| 3,4-Methylendioxyzimtsäure-N,N-diphenylamid | 0,6 | | | | | | 0,4 | |
| 3,4-Methylendioxyzimtsäure-N-cyclohexyl-N-2-pyridylamid | | 0,8 | | | | | | 0,4 |
| 2,3,4,5,6,10b,11,12-Octahydro-spiro[4b-azachrysen-12,2'-[1,3]dithiolan-]-1-on | | | 0,1 | 0,1 | | 0,1 | 0,1 | |
| 2,3,4,5,6,10b,11,12-octahydro-3,3-dimethyl-spiro[4b-azachrysen-12,2'-[1,3]dithiolan]-1-on | | | | 0,1 | | | | |
| Isopropyl-(5-methoxy-2-pyridin-2-yl-pyrimidin-4-yl)-amin | | | | | 0,6 | 0,2 | 0,1 | |
| sec-Butyl-(5-methoxy-2-pyridin-2-yl-pyrimidin-4-yl)-amin | | | | | | 0,2 | | 0,2 |
| | | | | | | | | |
| Total | 100 | 100 | 100 | 100 | 100 | 100 | 100 | 100 |

| | | | | | | | | |
|---|---|---|---|---|---|---|---|---|
| (ne) = nicht erfindungsgemäß | | | | | | | | |

Die Aromen wurden jeweils mit einer Konzentration von 0,15 Gew.% in ein gebrauchsfertiges Mundwasser, bzw. mit einer Konzentration von 3 Gew.% in ein Mundwasserkonzentrat eingearbeitet. Die sensorische Bewertung durch ein geschultes Experten-Panel zeigte, dass die Aromen zu einer schnell einsetzenden, sehr lang anhaltenden Frischewirkung führten, die auch nach Verwendung der Mundwässer noch über eine Zeit von fast 1 h anhielt.

Die unter den Beispielen S-1 bis S-8 genannten Aromakompositionen eignen sich für einen Einsatz in einer ganzen Reihe von unterschiedlichen Fertigprodukten, wobei der Einsatz nicht nur auf Zahnpasten beschränkt ist. Bei allen nachfolgend aufgeführten Beispielen konnte ein vorteilhaftes schnell einsetzendes, aber gleichzeitig sehr lang andauerndes Frischegefühl wahrgenommen werden, ohne dass dieser Frischeeindruck durch scharfe und bittere Noten beeinträchtigt wurde.

Nachfolgend sind weitere Anwendungsbeispiele für oben genannte Aromakompositionen in weiteren Fertigprodukten aufgeführt:

### Beispiel S-9

### Zahnpasta (,Silica opaque')

Alle Angaben, soweit nicht anders vermerkt, in Gew-%.

| Bestandteil | 1 | 2 | 3 | 4 | 5 | 6 |
|---|---|---|---|---|---|---|
| | | | (ne) | (ne) | (ne) | (ne) |
| Entionisiertes Wasser | 26,53 | 26,53 | 26,53 | 26,53 | 26,53 | 26,53 |
| Sorbitol 70% | 45 | Ad 100 | 45 | Ad 100 | 45 | Ad 100 |
| Solbrol M Na-Salz | 0,15 | 0,15 | 0,15 | 0,15 | 0,15 | 0,15 |
| Trinatriumphosphat | 0,1 | 0,1 | 0,1 | 0,1 | 0,1 | 0,1 |
| Saccharin | 0,2 | 0,2 | 0,2 | 0,2 | 0,2 | 0,2 |
| Natriummonofluorphosphat | 1,12 | 1,12 | 1,12 | 1,12 | 1,12 | 1,12 |
| PEG 1500 | 5 | 5 | 5 | 5 | 5 | 5 |
| Sident 9 (Abrasive Silica) | 10 | 10 | 10 | 10 | 10 | 10 |
| Sident 22 S (Dickende Silica) | 8 | 8 | 8 | 8 | 8 | 8 |
| Natriumcarboxymethylcellulose | 0,9 | 0,9 | 0,9 | 0,9 | 0,9 | 0,9 |
| Titan (IV) oxid | 0,5 | 0,5 | 0,5 | 0,5 | 0,5 | 0,5 |
| Natriumlaurylsulfat (SLS) | 1,5 | 1,5 | 1,5 | 1,5 | 1,5 | 1,5 |
| Pellitorin-Lösung PLM (enthaltend 10% Pellitorin) | - | 0,025 | - | 0,025 | - | 0,025 |
| Aroma Typ Eucalyptus-Menthol (Beispiel S-1b) | 1 | 1 | | | | |
| Aroma Typ Eucalyptus-Menthol (Beispiel S-1c) | | | 1 | 1 | | |
| Aroma Typ Eucalyptus-Menthol (Beispiel S-1f) | | | | | 1 | 1 |

| | | | | | | |
|---|---|---|---|---|---|---|
| (ne) = nicht erfindungsgemäß | | | | | | |

### Beispiel S-10

### Zahnpasta (Calciumcarbonat-Base)

Alle Angaben, soweit nicht anders vermerkt, in Gew-%.

| Bestandteil | 1 | 2 | 3 | 4 | 5 | 6 |
|---|---|---|---|---|---|---|
| | | | (ne) | (ne) | (ne) | (ne) |
| Entionisiertes Wasser | 27,5 | Ad 100 | 27,5 | Ad 100 | 27,5 | Ad 100 |
| Saccharin | 0,2 | 0,2 | 0,2 | 0,2 | 0,2 | 0,2 |
| Solbrol M Natriumsalz | 0,2 | 0,2 | 0,2 | 0,2 | 0,2 | 0,2 |
| Natriummonofluorphosphat | 0,8 | 0,8 | 0,8 | 0,8 | 0,8 | 0,8 |
| Sorbitol 70% | 29 | 29 | 29 | 29 | 29 | 29 |
| Calciumcarbonat | 35 | 35 | 35 | 35 | 35 | 35 |
| Sident 22 S (Verdickende Silica) | 2,5 | 2,5 | 2,5 | 2,5 | 2,5 | 2,5 |
| Natriumcarboxymethylcellulose | 1,3 | 1,3 | 1,3 | 1,3 | 1,3 | 1,3 |
| Titandioxid | 0,5 | 0,5 | 0,5 | 0,5 | 0,5 | 0,5 |
| Natriumlaurylsulfat | 2 | 2 | 2 | 2 | 2 | 2 |
| Pellitorin-Lösung PLM (enthaltend 10% Pellitorin) | - | 0,02 | - | 0,02 | - | 0,02 |
| Aroma Typ Eucalyptus-Menthol (Beispiel S-1a) | 1 | 1 | | | | |
| Aroma Typ Eucalyptus-Menthol (Beispiel S-1d) | | | 1 | 1 | | |
| Aroma Typ Eucalyptus-Menthol (Beispiel S-1h) | | | | | 1 | 1 |

| | | | | | | |
|---|---|---|---|---|---|---|
| (ne) = nicht erfindungsgemäß | | | | | | |

### Beispiel S-11

### Zahnpasta mit Bleichwirkung

Alle Angaben, soweit nicht anders vermerkt, in Gew-%.

| Bestandteil | 1 | 2 | 3 | 4 | 5 | 6 |
|---|---|---|---|---|---|---|
| | (ne) | | (ne) | (ne) | | (ne) |
| Polyphosphat (Glass H, (n ≈ 21), Astaris) | 7 | 7 | 7 | 7 | 7 | 7 |
| Calciumperoxid | 1 | - | 2,5 | 1 | - | 2,5 |
| Na-percarbonat | - | 11 | - | - | 11 | - |
| Poloxamer 407 | 5 | 2 | 5 | 5 | 2 | 5 |
| Polyethylenglycol | 3 | - | 3 | 3 | - | 3 |
| Sorbitol, 70 %ig in Wasser | - | 22 | - | - | 22 | - |
| Glycerin | 43,8 | 12,5 | 28,6 | 43,8 | 12,5 | 28,6 |
| 1,2-Propylenglycol | 4 | - | 2,5 | 4 | - | 2,5 |
| Na-Saccharin | 0,4 | 0,2 | 0,5 | 0,4 | 0,2 | 0,5 |
| Natriumbicarbonat | - | 5 | 15 | - | 5 | 15 |
| Natriumcarbonat | 2 | 2 | 2 | 2 | 2 | 2 |
| Silica | 20 | 22 | 20 | 20 | 22 | 20 |
| Na-Carboxymethylcellu lose | 0,6 | 0,55 | 0,3 | 0,6 | 0,55 | 0,3 |
| Natriumlaurylsulfat | 1 | 4 | 2 | 1 | 4 | 2 |
| Xanthan Gum | 0,2 | 0,2 | 0,2 | 0,2 | 0,2 | 0,2 |
| Titandioxid (Anatas) | 0,5 | 0,5 | 0,5 | 0,5 | 0,5 | 0,5 |
| Aroma Typ Eucalyptus-Menthol (Beispiel S-1d) | 1 | | | | | |
| Aroma Typ Eucalyptus-Menthol (Beispiel S-1g) | | | | 1 | | |
| Aroma Typ würzig-aromatisch (Beispiel S-3a) | | 1,25 | | | | |
| Aroma Typ würzig-aromatisch (Beispiel S-3d) | | | | | 1,25 | |
| Aroma Typ würzig-aromatisch (Beispiel S-3e) | | | 1,5 | | | |
| Aroma Typ würzig-aromatisch (Beispiel S-3g) | | | | | | 1,5 |
| Wasser dest. | Ad 100 | Ad 100 | Ad 100 | Ad 100 | Ad 100 | Ad 100 |

| | | | | | | |
|---|---|---|---|---|---|---|
| (ne) = nicht erfindungsgemäß | | | | | | |

### Beispiel S-12

### Zahnpasten mit Zinn- und Zinksalzen

Alle Angaben, soweit nicht anders vermerkt, in Gew-%.

| Bestandteil | 1 | 2 | 3 | 4 | 5 | 6 |
|---|---|---|---|---|---|---|
| | | (ne) | | (ne) | | (ne) |
| Natriumfluorid NaF | 0,42 | 0,5 | - | 0,42 | 0,5 | - |
| Zinnfluorid SnF2 | - | 0,9 | 0,95 | - | 0,9 | 0,95 |
| Zinnchlorid SnCl2 | 1,5 | - | 2 | 1,5 | - | 2 |
| Zinklactat | 2 | 2 | - | 2 | 2 | - |
| Zinkcarbonat ZnCO3 | - | 1 | 1,5 | - | 1 | 1,5 |
| Na-gluconat | - | 0,67 | 1,5 | - | 0,67 | 1,5 |
| Poloxamer 407 | 14,5 | - | - | 14,5 | - | - |
| Polyethylenglycol | 1 | 3 | - | 1 | 3 | - |
| Sorbitol, 70 %ig in Wasser | - | 38 | 37,5 | - | 38 | 37,5 |
| Glycerin | 37,5 | 5 | 14,4 | 37,5 | 5 | 14,4 |
| 1,2-Propylenglycol | 7 | 5 | - | 7 | 5 | - |
| Na-Saccharin | 0,3 | 0,5 | 0,5 | 0,3 | 0,5 | 0,5 |
| Abrasiv-Silica | 20 | 22,5 | 25 | 20 | 22,5 | 25 |
| Natriumhydroxid | - | 0,1 | 0,2 | - | 0,1 | 0,2 |
| Natriumlaurylsulfat | - | 2 | 1,5 | - | 2 | 1,5 |
| Na-polyphosphat | - | - | 4 | - | - | 4 |
| Tetranatriumpyrophosphat | 1 | 2,5 | - | 1 | 2,5 | - |
| Farbstoff (1%ig in Wasser) | 0,4 | 0,5 | 0,5 | 0,4 | 0,5 | 0,5 |
| Aroma Eucalyptus-Menthol-Typ (Beispiel S-1b) | 0,95 | - | - | | - | - |
| Aroma Eucalyptus-Menthol-Typ (Beispiel S-1c) | | | | 0,95 | | |
| Aroma Typ würzig-aromatisch (Beispiel S-3c) | - | 1,2 | - | - | | - |
| Aroma Typ würzig-aromatisch (Beispiel S-3f) | | | | | 1,2 | |
| Aroma Typ Wintergrün (Beispiel S-4a) | - | - | 1,15 | - | - | |
| Aroma Typ Wintergrün (Beispiel S-4e) | | | | | | 1,15 |
| Wasser dest. | Ad 100 | Ad 100 | Ad 100 | Ad 100 | Ad 100 | Ad 100 |

| | | | | | | |
|---|---|---|---|---|---|---|
| (ne) = nicht erfindungsgemäß. | | | | | | |

### Beispiel S-13

### Zahnpasta auf Phosphatbasis

Alle Angaben, soweit nicht anders vermerkt, in Gew-%.

| | 1 | 2 | 3 | 4 | 5 | 6 |
|---|---|---|---|---|---|---|
| Bestandteil | | | (ne) | (ne) | (ne) | (ne) |
| Entionisiertes Wasser | 36,39 | 36,39 | 36,59 | 36,59 | 36,39 | 36,39 |
| Glycerin | 20 | 20 | 20 | 20 | 20 | 20 |
| Solbrol M (Natriumsalz) | 0,15 | 0,15 | 0,15 | 0,15 | 0,15 | 0,15 |
| Natriummonofluorphosphat | 0,76 | 0,76 | 0,76 | 0,76 | 0,76 | 0,76 |
| Saccharin | 0,2 | 0,2 | 0,2 | 0,2 | 0,2 | 0,2 |
| Dicalciumphosphat-Dihydrat | 36 | 36 | 36 | 36 | 36 | 36 |
| Aerosil® 200 (Silica) | 3 | 3 | 3 | 3 | 3 | 3 |
| Natriumcarboxymethylcellulose | 1,2 | 1,2 | 1,2 | 1,2 | 1,2 | 1,2 |
| Natriumlaurylsulfat (Texapon) | 1,3 | 1,3 | 1,3 | 1,3 | 1,3 | 1,3 |
| Aroma Typ Krauseminze (Beispiel S-2a) | 1 | | | | | |
| Aroma Typ Krauseminze (Beispiel S-2b) | | 1 | | | | |
| Aroma Typ Krauseminze (Beispiel S-2d) | | | 0,8 | | | |
| Aroma Typ Krauseminze (Beispiel S-2f) | | | | 0,8 | | |
| Aroma Typ Krauseminze (Beispiel S-2g) | | | | | 1 | |
| Aroma Typ Krauseminze (Beispiel S-2h) | | | | | | 1 |

| | | | | | | |
|---|---|---|---|---|---|---|
| (ne) = nicht erfindungsgemäß | | | | | | |

### Beispiel S-14

### Zahnpasta (transparente Gelformulierung)

Alle Angaben, soweit nicht anders vermerkt, in Gew-%.

| Bestandteil | 1 | 2 | 3 | 4 | 5 | 6 |
|---|---|---|---|---|---|---|
| | (ne) | (ne) | | (ne) | (ne) | |
| Sorbitol 70% | 63 | Ad 100 | 63 | Ad 100 | 63 | Ad 100 |
| Entionisiertes Wasser | 11,31 | 11,31 | 11,31 | 11,31 | 11,31 | 11,31 |
| Saccharin | 0,2 | 0,2 | 0,2 | 0,2 | 0,2 | 0,2 |
| Natriummonofluorphosphat | 1,14 | 1,14 | 1,14 | 1,14 | 1,14 | 1,14 |
| Solbrol | 0,15 | 0,15 | 0,15 | 0,15 | 0,15 | 0,15 |
| Trinatriumphosphat | 0,1 | 0,1 | 0,1 | 0,1 | 0,1 | 0,1 |
| PEG 1500 ( PEG 32) | 5 | 5 | 5 | 5 | 5 | 5 |
| Sident 9 (Abrasive Silica) | 8 | 8 | 8 | 8 | 8 | 8 |
| Sident 22 S (Verdickende Silica) | 8 | 8 | 8 | 8 | 8 | 8 |
| Natriumcarboxymethylcellulose | 0,6 | 0,6 | 0,6 | 0,6 | 0,6 | 0,6 |
| Natriumlaurylsulfat | 1,5 | 1,5 | 1,5 | 1,5 | 1,5 | 1,5 |
| Pellitorin-Lösung PLM (enthaltend 10% Pellitorin) | - | 0,025 | - | 0,025 | - | 0,025 |
| Aroma Typ würzig-aromatisch (Menthol-Zimt) (Beispiel S-3c) | 1 | - | | | | |
| Aroma Typ würzig-aromatisch (Menthol-Zimt) (Beispiel S-3e) | | 1 | | | | |
| Aroma Typ würzig-aromatisch (Menthol-Zimt) (Beispiel S-3h) | | | 1 | | | |
| Aroma Typ Wintergrün (Beispiel S-4b) | - | | | 1 | | |
| Aroma Typ Wintergrün (Beispiel S-4d) | | | | | 1 | |
| Aroma Typ Wintergrün (Beispiel S-4g) | | | | | | 1 |

| | | | | | | |
|---|---|---|---|---|---|---|
| (ne) = nicht erfindungsgemäß | | | | | | |

### Beispiel S-15

### Mundwasserkonzentrat mit Aroma vom Wintergrüntyp

Alle Angaben, soweit nicht anders vermerkt, in Gew-%.

| Bestandteil | 1 | 2 | 3 | 4 | 5 | 6 |
|---|---|---|---|---|---|---|
| | | | (ne) | (ne) | (ne) | (ne) |
| Ethylalkohol 96% | 42 | 42 | 42 | 42 | 42 | 42 |
| Cremophor RH 455 | 5 | 5 | 5 | 5 | 5 | 5 |
| Entionisiertes Wasser | 48,67 | 48,67 | 50,67 | 49,67 | 48,67 | 48,67 |
| Allantoin | 0,2 | 0,2 | 0,2 | 0,2 | 0,2 | 0,2 |
| Natriumsaccharin 450 | 0,1 | 0,1 | 0,1 | 0,1 | 0,1 | 0,1 |
| Colour L-Blue 5000 (1% in Wasser) | 0,03 | 0,03 | 0,03 | 0,03 | 0,03 | 0,03 |
| Aroma Typ Wintergrün (Beispiel S-4b) | 4 | | | | | |
| Aroma Typ Wintergrün (Beispiel S-4c) | | 4 | | | | |
| Aroma Typ Wintergrün (Beispiel S-4d) | | | 2 | | | |
| Aroma Typ Wintergrün (Beispiel S-4e) | | | | 3 | | |
| Aroma Typ Wintergrün (Beispiel S-4f) | | | | | 4 | |
| Aroma Typ Wintergrün (Beispiel S-4h) | | | | | | 4 |

| | | | | | | |
|---|---|---|---|---|---|---|
| (ne) = nicht erfindungsgemäß | | | | | | |

### Beispiel S-16

### Mundwasser ('ready to use' ohne Alkohol)

Alle Angaben, soweit nicht anders vermerkt, in Gew-%.

| Bestandteil | 1 | 2 | 3 | 4 | 5 | 6 |
|---|---|---|---|---|---|---|
| | | | (ne) | (ne) | (ne) | (ne) |
| Cremophor RH 455 | 1,8 | 1,8 | 1,8 | 1,8 | 1,8 | 1,8 |
| Entionisiertes Wasser | 87,57 | Ad 100 | 87,57 | Ad 100 | 87,57 | Ad 100 |
| Sorbitol 70% | 10 | 10 | 10 | 10 | 10 | 10 |
| Natriumfluorid | 0,18 | 0,18 | 0,18 | 0,18 | 0,18 | 0,18 |
| Natriumsaccharin 450 | 0,1 | 0,1 | 0,1 | 0,1 | 0,1 | 0,1 |
| Solbrol M Natriumsalz | 0,15 | 0,15 | 0,15 | 0,15 | 0,15 | 0,15 |
| Pellitorin-Lösung PLM (enthaltend 10% Pellitorin) | - | 0,0125 | - | 0,0125 | - | 0,0125 |
| Mundwasser Aroma (Beispiel S-8a) | 0,2 | 0,2 | | | | |
| Mundwasser Aroma (Beispiel S-8d) | | | 0,2 | 0,2 | | |
| Mundwasser Aroma (Beispiel S-8g) | | | | | 0,2 | 0,2 |

| | | | | | | |
|---|---|---|---|---|---|---|
| (ne) = nicht erfindungsgemäß | | | | | | |

### Beispiel S-17

### Mundwasser ('ready to use' mit Alkohol)

Alle Angaben, soweit nicht anders vermerkt, in Gew-%.

| Bestandteil | 1 | 2 | 3 | 4 | 5 | 6 |
|---|---|---|---|---|---|---|
| | (ne) | | | (ne) | (ne) | (ne) |
| Ethylalkohol 96% | 10 | 5 | 7 | 10 | 5 | 7 |
| Cremophor CO 40 | 1 | 1 | 1 | 1 | 1 | 1 |
| Benzoesäure | 0,1 | 0,12 | 0,1 | 0,1 | 0,12 | 0,1 |
| Entionisiertes Wasser | 83,46 | Ad 100 | Ad 100 | 83,46 | Ad 100 | Ad 100 |
| Sorbitol 70% | 5 | 1 | 5 | 5 | 1 | 5 |
| Natriumsaccharin 450 | 0,07 | 0,05 | 0,05 | 0,07 | 0,05 | 0,05 |
| L-Blue 5000 (1% in Wasser) | 0,1 | 0,1 | 0,1 | 0,1 | 0,1 | 0,1 |
| Glycerin | - | 8 | - | - | 8 | - |
| 1,2-Propylenglycol | - | 2 | 3 | - | 2 | 3 |
| Cetylpyridiniumchlorid | - | - | 0,07 | - | - | 0,07 |
| Wasserstoffperoxid (35% H2O2 in Wasser) | - | 3 | 4 | - | 3 | 4 |
| Aroma Wintergrün (Beispiel S-4d) | 0,25 | - | - | | - | - |
| Aroma Wintergrün (Beispiel S-4h) | | | | 0,25 | | |
| Mundwasser Aroma (Beispiel S-8b) | - | 0,25 | 0,25 | | | |
| Mundwasser Aroma (Beispiel S-8c) | | | | | 0,25 | 0,25 |

| | | | | | | |
|---|---|---|---|---|---|---|
| (ne) = nicht erfindungsgemäß | | | | | | |

### Beispiel S-18

### Zahncreme und Mundwasser als 2-in-1 Produkt

Alle Angaben, soweit nicht anders vermerkt, in Gew-%.

| Bestandteil | 1 | 2 | 3 | 4 | 5 | 6 |
|---|---|---|---|---|---|---|
| | | | (ne) | (ne) | (ne) | (ne) |
| Ethanol, 96%ig | 5 | 5 | 5 | 5 | 5 | 5 |
| Sorbitol, 70 %ig in Wasser | 40 | 40 | 40 | 40 | 40 | 40 |
| Glycerin | 20 | 20 | 20 | 20 | 20 | 20 |
| Saccharin | 0,2 | 0,2 | 0,2 | 0,2 | 0,2 | 0,2 |
| Na-Monofluorphosphat | 0,76 | 0,76 | 0,76 | 0,76 | 0,76 | 0,76 |
| Solbrol M, Na-Salz | 0,15 | 0,15 | 0,15 | 0,15 | 0,15 | 0,15 |
| Abrasivkieselsäure (Sident 9) | 20 | 20 | 20 | 20 | 20 | 20 |
| Verdickungskieselsäure (Sident 22S) | 2 | 2 | 2 | 2 | 2 | 2 |
| Na-Carboxymethylcellu lose | 0,3 | 0,3 | 0,3 | 0,3 | 0,3 | 0,3 |
| Natriumlaurylsulfat | 1,2 | 1,2 | 1,2 | 1,2 | 1,2 | 1,2 |
| Grüner Farbstoff (1%ig in Wasser) | 0,5 | 0,5 | 0,5 | 0,5 | 0,5 | 0,5 |
| Aroma Typ Eucalyptus-Menthol (Beispiel S-1a) | 1 | | | | | |
| Aroma Typ Eucalyptus-Menthol (Beispiel S-1b) | | 1 | | | | |
| Aroma Typ Eucalyptus-Menthol (Beispiel S-1c) | | | 1 | | | |
| Aroma Typ Eucalyptus-Menthol (Beispiel S-1e) | | | | 1 | | |
| Aroma Typ Eucalyptus-Menthol (Beispiel S-1f) | | | | | 1 | |
| Aroma Typ Eucalyptus-Menthol (Beispiel S-1h) | | | | | | 1 |
| Wasser dest. | Ad 100 | Ad 100 | Ad 100 | Ad 100 | Ad 100 | Ad 100 |

| | | | | | | |
|---|---|---|---|---|---|---|
| (ne) = nicht erfindungsgemäß | | | | | | |

### Beispiel S-19

### Standard Kaugummi

Alle Angaben, soweit nicht anders vermerkt, in Gew-%.

| Bestandteil | 1 | 2 | 3 | 4 | 5 | 6 |
|---|---|---|---|---|---|---|
| | | (ne) | (ne) | | (ne) | (ne) |
| Gum Base (Kaugummibase) | 21 | 21 | 21 | 21 | 21 | 21 |
| Glucosesirup | 16,5 | 17 | 16,5 | 16,5 | 17 | 16,5 |
| Glycerin | 0,5 | 0,5 | 0,5 | 0,5 | 0,5 | 0,5 |
| Zucker gepulvert | 60 | 60 | 60 | 60 | 60 | 60 |
| Aroma Eucalyptus Typ Pfefferminz (Beispiel S-5b) | 2 | - | | | | |
| Aroma Eucalyptus Typ Pfefferminz (Beispiel S-5c) | | 1,5 | | | | |
| Aroma Eucalyptus Typ Pfefferminz (Beispiel S-5h) | | | 2 | | | |
| Aroma Typ Krauseminz (Beispiel S-6c) | - | | | 2 | | |
| Aroma Typ Krauseminz (Beispiel S-6d) | | | | | 1,5 | |
| Aroma Typ Krauseminz (Beispiel S-6g) | | | | | | 2 |

| | | | | | | |
|---|---|---|---|---|---|---|
| (ne) = nicht erfindungsgemäß | | | | | | |

### Beispiel S-20

### Zuckerfreier Kaugummi

Alle Angaben, soweit nicht anders vermerkt, in Gew-%.

| Bestandteil | 1 | 2 | 3 | 4 | 5 | 6 |
|---|---|---|---|---|---|---|
| | | | (ne) | (ne) | (ne) | (ne) |
| Gum Base (Kaugummibase) | 30 | 30 | 30 | 30 | 30 | 30 |
| Sorbit gepulvert | 40 | Ad 100 | 40,2 | Ad 100 | 40 | Ad 100 |
| Isomalt gepulvert | 9,5 | 9,5 | 9,5 | 9,5 | 9,5 | 9,5 |
| Xylitol | 2 | 2 | 2 | 2 | 2 | 2 |
| Mannit D | 3 | 3 | 3 | 3 | 3 | 3 |
| Aspartam | 0,1 | 0,1 | 0,1 | 0,1 | 0,1 | 0,1 |
| Acesulfam K | 0,1 | 0,1 | 0,1 | 0,1 | 0,1 | 0,1 |
| EmulgumTM (Soja-Lecithine mit hohem Gehalt an Phospholipiden) | 0,3 | 0,3 | 0,3 | 0,3 | 0,3 | 0,3 |
| Sorbitol (70% in Wasser) | 13 | 13 | 13 | 13 | 13 | 13 |
| 1,2-Propylenglyol | - | 1 | - | 1 | - | 1 |
| Glycerin | 1 | - | 1 | - | 1 | - |
| Pellitorin-Lösung PLM (enthaltend 10% Pellitorin) | - | 0,035 | - | 0,035 | - | 0,035 |
| Aroma Eucalyptus Typ Pfefferminz (Beispiel S-5a) | 1 | 1 | | | | |
| Aroma Eucalyptus Typ Pfefferminz (Beispiel S-5d) | | | 0,8 | 0,8 | | |
| Aroma Eucalyptus Typ Pfefferminz (Beispiel S-5f) | | | | | 1 | 1 |

| | | | | | | |
|---|---|---|---|---|---|---|
| (ne) = nicht erfindungsgemäß | | | | | | |

### Beispiel S-21

### Kaugummis (mit Zucker und zuckerfrei)

Alle Angaben, soweit nicht anders vermerkt, in Gew-%.

| Bestandteil | 1 | 2 | 3 | 4 | 5 | 6 |
|---|---|---|---|---|---|---|
| | | | (ne) | (ne) | | |
| Gum Base (Kaugummibase) | 21 | 30 | 21 | 30 | 21 | 30 |
| Glycerin | 0,5 | 1 | 0,5 | 1 | 0,5 | 1 |
| Glucosesirup | 16,5 | - | 16,5 | - | 16,5 | - |
| Puderzucker | Ad 100 | - | Ad 100 | - | Ad 100 | - |
| Sorbitol (in Pulverform) | - | Ad 100 | - | Ad 100 | - | Ad 100 |
| Palatinit | - | 9,5 | - | 9,5 | - | 9,5 |
| Xylitol | - | 2 | - | 2 | - | 2 |
| Mannitol | - | 3 | - | 3 | - | 3 |
| Aspartam | - | 0,1 | - | 0,1 | - | 0,1 |
| Acesulfam K | - | 0,1 | - | 0,1 | - | 0,1 |
| EmulgumTM (Emulgator) | - | 0,3 | - | 0,3 | - | 0,3 |
| Sorbitol 70%, in Wasser | - | 14 | - | 14 | - | 14 |
| Aroma Typ Krauseminz (Beispiel S-6a) | 1 | 1,4 | | | | |
| Aroma Typ Krauseminz (Beispiel S-6f) | | | 0,8 | 1,2 | | |
| Aroma Typ Krauseminz (Beispiel S-6h) | | | | | 1 | 1,4 |

| | | | | | | |
|---|---|---|---|---|---|---|
| (ne) = nicht erfindungsgemäß | | | | | | |

### Beispiel S-22

### Zuckerfreie Kaugummis

Die Kaugummibase K1 bestand aus 2,0% Butyl Rubber (Isobuten-Isopren-Copolymer, MW = 400000, 6,0% Polyisobuten (MW = 43.800), 43,5% Polyvinylacetat (MW = 12.000), 31,5% Polyvinylacetat (MW = 47.000), 6,75% Triacetin und 10,25% Calciumcarbonat. Die Herstellung der Kaugummibase K1 und der Kaugummis kann analog zu US 5,601,858 erfolgen.

Alle Angaben, soweit nicht anders vermerkt, in Gew-%.

| Bestandteil | 1 | 2 | 3 | 4 | 5 | 6 |
|---|---|---|---|---|---|---|
| | | | | (ne) | | (ne) |
| Kaugummibase K1 | 26 | 27 | 26 | 26 | 27 | 26 |
| Triacetin | 0,25 | 0,25 | 0,25 | 0,25 | 0,25 | 0,25 |
| Lecithin | 0,5 | 0,5 | 0,5 | 0,5 | 0,5 | 0,5 |
| Sorbitol, kristallin | Ad 100 | Ad 100 | Ad 100 | Ad 100 | Ad 100 | Ad 100 |
| Mannitol | 15,3 | 15,2 | 15,1 | 15,3 | 15,2 | 15,1 |
| Glycerin | 12,1 | 12 | 11,8 | 12,1 | 12 | 11,8 |
| Saccharin-Na | 0,17 | - | 0,1 | 0,17 | - | 0,1 |
| Verkapseltes Aspartam | 1,08 | 1,18 | 1,08 | 1,08 | 1,18 | 1,08 |
| Amorphes Silica | 1 | 1 | 1 | 1 | 1 | 1 |
| Baumwollsamenöl | 0,5 | 0,5 | 0,5 | 0,5 | 0,5 | 0,5 |
| Polyoxyethylen-sorbitanmonolaurat (E-432) | 1 | 1 | 1 | 1 | 1 | 1 |
| Verkapseltes I-Carvon (Beladung: 30%) | - | 0,2 | - | - | 0,2 | - |
| I-Menthyl-I-lactat | - | - | 0,2 | - | - | 0,2 |
| Aroma Typ Krauseminz (Beispiel S-6c) | 1 | - | 1,7 | | | |
| Aroma Typ Krauseminz (Beispiel S-6d) | | | | 0,8 | - | 1,4 |
| Aroma Typ Pfefferminz (Beispiel S-5b) | 0,5 | 1,4 | - | | | |
| Aroma Typ Pfefferminz (Beispiel S-5e) | | | | 0,5 | 1,4 | - |

| | | | | | | |
|---|---|---|---|---|---|---|
| (ne) = nicht erfindungsgemäß | | | | | | |

### Beispiel S-23

### Zuckerfreie Kaugummis

Alle Angaben, soweit nicht anders vermerkt, in Gew-%.

Die Kaugummibase K2 bestand aus 28,5% Terpenharz, 33,9% Polyvinylacetat (MW = 14.000), 16,25% hydriertes Pflanzenöl, 5,5% Mono- und Diglyceride, 0,5% Polyisobuten (MW 75.000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis kann analog zu US 6,986,907 erfolgen.

| Bestandteil | 1 | 2 | 3 | 4 | 5 | 6 |
|---|---|---|---|---|---|---|
| | | | | | (ne) | |
| Kaugummibase K2 | 25,3 | 27,3 | 26,3 | 25,3 | 27,3 | 26,3 |
| Sorbitol | Ad 100 | Ad 100 | Ad 100 | Ad 100 | Ad 100 | Ad 100 |
| Glycerin | 2,4 | 2,4 | 2,4 | 2,4 | 2,4 | 2,4 |
| Lecithin | 7 | 7 | 7 | 7 | 7 | 7 |
| Aspartam | 0,14 | 0,14 | 0,14 | 0,14 | 0,14 | 0,14 |
| Verkapseltes Aspartam | 0,68 | 0,68 | 0,68 | 0,68 | 0,68 | 0,68 |
| Menthol, sprühgetrocknet (Beladung: 25%) | 0,5 | - | 0,5 | 0,5 | - | 0,5 |
| Kirscharoma, sprühgetrocknet (enthält Benzaldehyd) | - | 1 | - | - | 1 | - |
| Aroma Typ Pfefferminz (Beispiel S-5b), sprühgetrocknet, Aromagehalt 30% | 1,5 | 1,7 | - | | | |
| Aroma Typ Pfefferminz (Beispiel S-5c), sprühgetrocknet, Aromagehalt 30% | | | | 1,5 | 1,7 | - |
| Aroma Typ würzig-aromatisch (Beispiel S-7c) | 1 | - | 1,5 | | | |
| Aroma Typ würzig-aromatisch (Beispiel S-7h) | | | | 1 | - | 1,5 |

| | | | | | | |
|---|---|---|---|---|---|---|
| (ne) = nicht erfindungsgemäß | | | | | | |

Die Kaugummis der Rezeptur (1) und (2) wurden als Streifen, die der Rezeptur (3) als Kompaktate in Kissenform hergestellt und anschließend mit Xylit dragiert.

### Beispiel S-24

Herstellung von Aromen mit Kühlwirkung vom Geschmackstyp "Eisbonbon" unter Verwendung der erfindungsgemäßen Kühlsubstanzen.

Es wurden vermischt:
(Alle Angaben, soweit nicht anders vermerkt, in Gew-%.)

| Komponente | a | b | c | d | e | f | g | h |
|---|---|---|---|---|---|---|---|---|
| | | | (ne) | (ne) | (ne) | (ne) | (ne) | (ne) |
| Isoamylacetat | 2 | 2 | 2 | 2 | 2 | 2 | 2 | 2 |
| Ethylbutyrat | 0,5 | - | 0,5 | - | 0,5 | - | 0,5 | - |
| Butylbutyrat | - | 0,5 | - | 0,5 | - | 0,5 | - | 0,5 |
| Ethylvanillin | 2 | - | 2 | - | 2 | - | 2 | - |
| Vanillin | - | 1 | - | 1 | - | 1 | - | 1 |
| FrambinonTM [4-(4-Hydroxyphenyl)-2-butanon] | 0,5 | 0,5 | 0,5 | 0,5 | 0,5 | 0,5 | 0,5 | 0,5 |
| I-Menthol | 5 | 5 | 5 | 5 | 5 | 5 | 5 | 5 |
| Pfefferminzöl Typ piperita | 6 | 6 | 6 | 6 | 6 | 6 | 6 | 6 |
| Triacetin | - | 84 | - | 84,5 | - | 84,5 | - | 84 |
| 1,2-Propylenglycol | 83 | - | 83,5 | - | 83,5 | - | 83 | - |
| 3,4-Methylendioxyzimtsäure-N, N-diphenylamid | 0,5 | 0,5 | | | | | | |
| 3,4-Methylendioxyzimtsäure-N-cyclohexyl-N-2-pyridylamid | 0,5 | 0,5 | | | | | | |
| 2,3,4,5,6,10b,11,12-Octahydro-spiro[4b-azachrysen-12,2'-[1,3]dithiolan-]-1-on | | | 0,5 | 0,5 | 0,2 | 0,2 | | |
| 2,3,4,5,6,10b,11, 12-octahydro-3,3-dimethyl-spiro[4b-azachrysen-12,2'-[1,3]dithiolan]-1-on | | | | | 0,3 | 0,3 | | |
| Isopropyl-(5-methoxy-2-pyridin-2-yl-pyrimidin-4-yl)-amin | | | | | | | 0,5 | 0,5 |
| sec-Butyl-(5-methoxy-2-pyridin-2-yl-pyrimidin-4-yl)-amin | | | | | | | 0,5 | 0,5 |

| | | | | | | | | |
|---|---|---|---|---|---|---|---|---|
| (ne) = nicht erfindungsgemäß | | | | | | | | |

Die Aromen wurden mit Konzentrationen von 0,15 bis 0,2 Gew-% in verschiedene Bonbonmassen eingearbeitet. Die sensorische Bewertung durch ein geschultes Experten-Panel zeigte, dass das Aroma zu einer starken, schnell einsetzenden, sehr lang anhaltenden Frischewirkung führte, die auch nach dem Verzehr der Bonbons noch über eine Zeit von weit über 30 min anhielt.

### Beispiel S-25

### Bonbon ('Hardboiled candy'), zuckerfrei

Alle Angaben, soweit nicht anders vermerkt, in Gew-%.

| Bestandteil | 1 | 2 | 3 | 4 | 5 | 6 |
|---|---|---|---|---|---|---|
| | (ne) | (ne) | | (ne) | (ne) | (ne) |
| Wasser | 2,24 | 2,24 | 2,24 | 2,24 | 2,24 | 2,24 |
| Isomalt | 94,98 | Ad 100 | Ad 100 | 94,98 | Ad 100 | Ad 100 |
| Xylitol | 2,4 | 2,4 | 2,4 | 2,4 | 2,4 | 2,4 |
| Sucralose | 0,03 | 0,03 | 0,03 | 0,03 | 0,03 | 0,03 |
| Acesulfam K | 0,05 | 0,05 | 0,05 | 0,05 | 0,05 | 0,05 |
| Citronensäure | 0,05 | 0,05 | 0,05 | 0,05 | 0,05 | 0,05 |
| Pellitorin-Lösung PLM (enthaltend 10% Pellitorin) | - | 0,0075 | 0,01 | - | 0,0075 | 0,01 |
| Aroma Eucalyptus-Menthol-Typ (Beispiel S-1c) | 0,25 | 0,2 | | | | |
| Aroma Eucalyptus-Menthol-Typ (Beispiel S-1d) | | | | 0,25 | 0,2 | |
| Aroma Typ Eisbonbon (Beispiel S-24a) | | | 0,25 | | | |
| Aroma Typ Eisbonbon (Beispiel S-24e) | | | | | | 0,2 |

| | | | | | | |
|---|---|---|---|---|---|---|
| (ne) = nicht erfindungsgemäß | | | | | | |

### Beispiel S-26

### Bonbons ('Hardboiled candy')

Alle Angaben, soweit nicht anders vermerkt, in Gew-%.

| Bestandteil | 1 | 2 | 3 | 4 | 5 | 6 |
|---|---|---|---|---|---|---|
| | | | | (ne) | (ne) | (ne) |
| Wasser | 2,75 | 2,5 | 2,5 | 2,75 | 2,5 | 2,5 |
| Zucker | 60,1 | Ad 100 | Ad 100 | 60,1 | Ad 100 | Ad 100 |
| Glucosesirup | 36,9 | 36 | 36 | 36,9 | 36 | 36 |
| Maltose | - | 2 | 2 | - | 2 | 2 |
| Palmkernöl | - | 0,8 | 0,8 | - | 0,8 | 0,8 |
| Citronensäure | - | 0,25 | 0,25 | - | 0,25 | 0,25 |
| Ginseng Extrakt | - | 0,4 | 0,4 | - | 0,4 | 0,4 |
| Blauer Farbstoff | - | 0,01 | 0,01 | - | 0,01 | 0,01 |
| Aroma Krauseminz-Typ (Beispiel S-2a) | 0,25 | 0,35 | - | | | |
| Aroma Krauseminz-Typ (Beispiel S-2d) | | | | 0,25 | 0,35 | - |
| Aroma Typ Eisbonbon (Beispiel S-24b) | | | 0,175 | | | |
| Aroma Typ Eisbonbon (Beispiel S-24d) | | | | | | 0,175 |

| | | | | | | |
|---|---|---|---|---|---|---|
| (ne) = nicht erfindungsgemäß | | | | | | |

### Beispiel S-27

### Instant-Getränkepulver

Alle Angaben, soweit nicht anders vermerkt, in Gew-%.

| Bestandteil | 1 | 2 | 3 | 4 | 5 | 6 |
|---|---|---|---|---|---|---|
| | | | (ne) | | (ne) | (ne) |
| Zucker (Saccharose) | Ad 100 | Ad 100 | Ad 100 | Ad 100 | Ad 100 | Ad 100 |
| Citronensäure | 11,58 | 11,58 | 11,58 | 11,58 | 11,58 | 11,58 |
| Trinatriumcitrat | 0,7 | 0,7 | 0,7 | 0,7 | 0,7 | 0,7 |
| Tricalciumphosphat | 0,6 | 0,6 | 0,6 | 0,6 | 0,6 | 0,6 |
| Vitamin C | 0,66 | 0,66 | 0,66 | 0,66 | 0,66 | 0,66 |
| Grindsted® JU 543 Stabilizer System (Danisco) | 0,9 | 0,9 | 0,9 | 0,9 | 0,9 | 0,9 |
| Saccharin | 0,561 | 0,561 | 0,561 | 0,561 | 0,561 | 0,561 |
| Citronenaroma, sprühgetrocknet | 1,75 | - | 1,75 | - | 1,75 | - |
| Orangenaroma, sprühgetrocknet | | 1,85 | | 1,85 | | 1,85 |
| Aroma Eucalyptus-Menthol-Typ (Beispiel S-1a), sprühgetrocknet auf Maltodextrin (DE 15-19) und Gummi Arabicum, Aromabeladung 40% | 1,75 | | | | | |
| Aroma Eucalyptus-Menthol-Typ (Beispiel S-1c), sprühgetrocknet auf Maltodextrin (DE 15-19) und Gummi Arabicum, Aromabeladung 40% | | | 1,75 | | | |
| Aroma Eucalyptus-Menthol-Typ (Beispiel S-1h), sprühgetrocknet auf Maltodextrin (DE 15-19) und Gummi Arabicum, Aromabeladung 40% | | | | | 1,75 | |
| Aroma Typ würzig-aromatisch (Menthol-Zimt) (Beispiel S-3b); sprühgetrocknet auf Maltodextrin (DE 15-19) und Gummi Arabicum, Aromabeladung 40% | | 1,2 | | | | |
| Aroma Typ würzig-aromatisch (Menthol-Zimt) (Beispiel S-3d); sprühgetrocknet auf Maltodextrin (DE 15-19) und Gummi Arabicum, Aromabeladung 40% | | | | 1,2 | | |
| Aroma Typ würzig-aromatisch (Menthol-Zimt) (Beispiel S-3e); sprühgetrocknet auf Maltodextrin (DE 15-19) und Gummi Arabicum, Aromabeladung 40% | | | | | | 1,2 |

| | | | | | | |
|---|---|---|---|---|---|---|
| (ne) = nicht erfindungsgemäß | | | | | | |

45 g dieser Instant-Getränkepulver wurden jeweils in 1000 mL unter Rühren gelöst. Das erhaltenen Getränke hatten einen erfrischenden, kühlenden Geschmack von Citrus, Zimt und Minze.

### Beispiel S-28

### Halsbonbons mit flüssig-viskoser Kernfüllung (center-filled hard candy)

Alle Angaben, soweit nicht anders vermerkt, in Gew-%.

| Bestandteil | 1 | 2 | 3 | 4 | 5 | 6 |
|---|---|---|---|---|---|---|
| | | | | | | |
| **Mischung A (Hülle)** (80% der Bonbons) | | | | | | |
| Zucker (Saccharose) | 58,1 | 58,1 | 58,1 | 49,36 | 49,36 | 49,36 |
| Glucosesirup (Feststoffgehalt 80%) | 41,51 | 41,51 | 41,51 | 49,36 | 49,36 | 49,36 |
| Aroma Typ würzig-aromatisch (Menthol-Zimt) (Beispiel S-3a) | 0,17 | 0,17 | 0,17 | 0,25 | 0,25 | 0,25 |
| Aroma Typ würzig-aromatisch (Menthol-Zimt) (Beispiel S-3c) | 0,17 | 0,17 | 0,17 | 0,25 | 0,25 | 0,25 |
| Aroma Typ würzig-aromatisch (Menthol-Zimt) (Beispiel S-3f) | 0,17 | 0,17 | 0,17 | 0,25 | 0,25 | 0,25 |
| tr.-Pellitorin 10% in Propylenglycol/Pfefferminzöl (1:1) | 0,02 | 0,02 | 0,02 | 0,03 | 0,03 | 0,03 |
| I-Menthol | 0,1 | 0,1 | 0,1 | - | - | - |
| Citronenöl | 0,1 | 0,1 | 0,1 | 0,1 | 0,1 | 0,1 |
| Citronensäure | - | - | - | 0,9 | 0,9 | 0,9 |
| Total: | 100 | 100 | 100 | 100 | 100 | 100 |

| **Mischung B (Kern)** (20% der Bonbons) | | | | | | |
|---|---|---|---|---|---|---|
| High Fructose Maissirup (Gehalt an festen Zuckern 85%, knapp 15% Wasser) | 84,355 | 84,355 | 84,355 | 84,31 | 84,31 | 84,31 |
| Glycerin | 15 | 15 | 15 | 15 | 15 | 15 |
| Lecithin | 0,02 | 0,02 | 0,02 | 0,02 | 0,02 | 0,02 |
| Zimtöl | - | - | - | 0,27 | 0,27 | 0,27 |
| Aroma Typ Krauseminze (Beispiel S-2b) | 0,28 | 0,28 | 0,28 | - | - | - |
| Aroma Typ Krauseminze (Beispiel S-2d) | 0,28 | 0,28 | 0,28 | - | - | - |
| Aroma Typ Krauseminze (Beispiel S-2g) | 0,28 | 0,28 | 0,28 | - | - | - |
| Capsaicin | 0,025 | 0,025 | 0,025 | - | - | - |
| Piperin | 0,05 | 0,05 | 0,05 | 0,05 | 0,05 | 0,05 |
| Vanillylalkohol-n-butylether | - | - | - | 0,1 | 0,1 | 0,1 |
| Roter Farbstoff, als 2,5%ige wässrige Lösung | 0,2 | 0,2 | 0,2 | 0,2 | 0,2 | 0,2 |
| Vanillin | 0,07 | 0,07 | 0,07 | - | - | - |

In Anlehnung an die in US 6,432,441 (dort Beispiel 1) sowie die in US 5,458,894 bzw. US 5,002,791 beschriebenen Verfahren wurden Bonbons mit flüssig-viskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

### Beispiel S-29

### Zum Direktverzehr geeignete Gelatinekapseln

Alle Angaben, soweit nicht anders vermerkt, in Gew-%.

| Bestandteile | 1 | 2 |
|---|---|---|
| **Gelatinehülle:** | | |
| Glycerin | 2,014 | 2,014 |
| Gelatine 240 Bloom | 7,91 | 7,91 |
| Sucralose | 0,070 | 0,070 |
| Allura Red (roter Farbstoff) | 0,006 | 0,006 |
| Brillant Blue (blauer Farbstoff) | 0,005 | 0,005 |

| **Kernzusammensetzung:** | | |
|---|---|---|
| Aroma Eucalyptus-Menthol-Typ (Beispiel S-1a) | 15 | |
| Aroma Eucalyptus-Menthol-Typ (Beispiel S-1b) | | 15 |
| Pflanzenöltriglyceride (Kokonussölfraktion; coconut oil fraction) | Ad 100 | Ad 100 |

Die zum Direktverzehr geeigneten Gelatinekapseln wurden gemäß WO 2004/050069 hergestellt und hatten einen Durchmesser von 5 mm; das Gewichtsverhältnis von Kernmaterial zu Hüllmaterial lag bei 90 : 10. Die Kapseln öffneten sich jeweils im Mund innerhalb von weniger als 10 Sekunden und lösten sich vollständig innerhalb von weniger als 50 Sekunden auf.

### Beispiel S-30

Herstellung eines Kaubonbons mit kühlendem Himbeergeschmack unter Verwendung der erfindungsgemäßen Kühlsubstanzen.

Alle Angaben, soweit nicht anders vermerkt, in Gew-%.

| Bestandteile | 1 | 2 | 3 | 4 | 5 | 6 |
|---|---|---|---|---|---|---|
| | | | (ne) | (ne) | (ne) | (ne) |
| Wasser | 7,8 | 7,8 | 7,8 | 7,8 | 7,8 | 7,8 |
| Zucker Raffinade C4 | 42,1 | 42,1 | 42,1 | 42,1 | 42,1 | 42,1 |
| Glucose Sirup Dextrose 40 | 37,3 | 37,3 | 37,3 | 37,3 | 37,3 | 37,3 |
| gehärtetes Pflanzenfett Schmelzpunkt 32-36°C | 6,6 | 6,6 | 6,6 | 6,6 | 6,6 | 6,6 |
| Lecithin Emulgator (Sojalecithin) | 0,3 | 0,3 | 0,3 | 0,3 | 0,3 | 0,3 |
| Gelatine (Schweinegelatine) | 0,8 | 0,8 | 0,8 | 0,8 | 0,8 | 0,8 |
| Fondant Typ - S30 | 4,8 | 4,8 | 4,8 | 4,8 | 4,8 | 4,8 |
| Himbeeraroma | 0,22 | 0,22 | 0,22 | 0,22 | 0,22 | 0,22 |
| Menthyllactat | 0,06 | 0,06 | 0,06 | 0,06 | 0,06 | 0,06 |
| 3,4-Methylendioxyzimtsäure-N,N-diphenylamid | 0,02 | | | | | |
| 3,4-Methylendioxyzimtsäure-N-cyclohexyl-N-2-pyridylamid | | 0,03 | | | | |
| Isopropyl-(5-methoxy-2-pyridin-2-yl-pyrimidin-4-yl)-amin | | | 0,015 | | | |
| (5-Ethyl-2-pyridin-2-yl-pyrimidin-4-yl)-isopropyl-amin | | | | 0,02 | | |
| 2,3,4,5,6,10b,11,12-Octahydro-spiro[4b-azachrysen-12,2'-[1,3]dithiolan-]-1-on | | | | | 0,005 | |
| 2,3,4,5,6,10b,11,12-octahydro-3,3-dimethyl-spiro[4b-azachrysen-12,2'-[1,3]dithiolan]-1-on | | | | | | 0,01 |

| | | | | | | |
|---|---|---|---|---|---|---|
| (ne) = nicht erfindungsgemäß | | | | | | |

### Herstellungshinweise:

a) Gelatine mit Wasser (1,8 fache Menge der Gelatine) bei 70°C 2 Stunden quellen lassen;
b) Zucker, Sirup, Wasser, Fett und Lecithin auf 123°C kochen;
c) Gelatinelösung langsam mit dem Kochansatz vermischen;
d) Himbeeraroma, Menthyllactat und die erfindungsgemäßen Kühlsubstanzen vermischen und optional Farbe unterrühren;
e) resultierende Masse auf einem Kühltisch auf ca. 70°C temperieren, danach Fondant zugeben und auf einer Ziehmaschine ca. 3 Minuten belüften;
f) Kaubonbonmasse anschließend schneiden und verpacken.

Beim Verzehr der Kaubonbons wird während des Kauens ein frischer, kühlender Himbeergeschmack wahrgenommen.

### Beispiel S-31

### Herstellung eines Extrudates für die Bereitung von Getränkemischungen mit Kühlwirkung

Alle Angaben, soweit nicht anders vermerkt, in Gew-%.

| Bestandteil | | | |
|---|---|---|---|
| Glukosesirup, sprühgetrocknet (DE-Wert: 31-34) [Glucidex IT33W (Firma Roquette)] | 62,0 | 62,0 | 62,0 |
| Maltodextrin (DE-Wert: 17-20), Firma Cerestar | 28,4 | 28,4 | 28,4 |
| Emulgator Monomuls, Emulgator auf der Basis von gehärtetem Palmöl; Schmelzpunkt: 64°C, (Firma Grünau) | 1,8 | 1,8 | 1,8 |
| Dextrosemonohydrat (DE-Wert: 99,5), Firma Cerestar | 1,8 | 1,8 | 1,8 |
| Wasser | 2,0 | 2,0 | 2,0 |
| Orangen-Vanillearoma | 3,2 | 3,2 | 3,2 |
| Aroma Eucalyptus-Menthol-Typ (Beispiel S-1a) | 0,8 | | |
| Aroma Eucalyptus-Menthol-Typ (Beispiel S-1b) | | 0,8 | |
| Aroma Eucalyptus-Menthol-Typ (Beispiel S-1f) | | | 0,8 (ne) |

| | | | |
|---|---|---|---|
| (ne) = nicht erfindungsgemäß | | | |

### Herstellungshinweis (siehe auch WO 03/092412):

Alle Bestandteile wurden gemischt und per Einpunktdosierung in einen Doppelschneckenextruder gefördert. Die Extrusionstemperaturen lagen zwischen 100 und 120°C, der spezifische Energieeintrag lag bei 0,2 kWh/kg. Die aus der mit 1 mm Bohrungen versehene Düsenplatte des Extruders austretenden Stränge wurden unmittelbar nach Austritt aus den Düsen durch rotierende Messer auf Partikel mit ca. 1 mm Durchmesser geschnitten.

### Beispiel S-32

### Herstellung von Wirbelschichtgranulaten für die Bereitung von Getränkemischungen mit Kühlwirkung

In einer Granulierapparatur des in EP 163 836 dargestellten Typs (mit den folgenden Merkmalen: Durchmesser Anströmboden: 225 mm, Sprühdüse: Zweistoffdüse; Sichtender Austrag: Zick-Zack-Sichter; Filter: internes Schlauchfilter) wird eine Lösung bestehend aus 44 Gew.-% Wasser, 8 Gew.% Citronenaroma, 3 Gew.-% Aroma Eucalyptus-Menthol-Typ (s. Beispiel S-1a bis S-1h), 13 Gew.-% Gummi arabicum und 32 Gew.-% hydrolysierter Stärke (Maltodextrin DE 15-19) sowie etwas grünem Farbstoff granuliert. Die Lösung wird bei einer Temperatur von 32°C in den Wirbelschichtgranulator gesprüht. Zur Fluidisierung des Bettinhaltes wird Stickstoff in einer Menge von 140 kg/h eingeblasen. Die Eintrittstemperatur des Fluidisiergases beträgt 140°C. Die Temperatur des Abgases beträgt 76°C. Als Sichtgas wird ebenfalls Stickstoff in einer Menge von 15 kg/h mit einer Temperatur von 50°C zugeführt. Der Inhalt des Wirbelbettes beträgt ca. 500 g. Die Granulierleistung beträgt ca. 2,5 kg pro Stunde. Man erhält ein frei fließendes Granulat mit einem mittleren Teilchendurchmesser von 360 Mikrometern. Die Granulate sind rund und weisen eine glatte Oberfläche auf. Aufgrund des konstanten Druckverlustes des Filters und des ebenfalls konstant bleibenden Bettinhalts ist von stationären Bedingungen hinsichtlich des Granulationsprozesses auszugehen.

### Beispiel S-33

Herstellung von Teebeuteln mit Rooibos- bzw. schwarzem Tee und Extrudaten aus Beispiel S-31 bzw. Granulaten aus Beispiel S-32 für die Bereitung von Teegetränken mit kühlernder Wirkung.

Jeweils 800 g Rotbuschtee (Rooibos-Tee) wurden einmal mit 33 g der Extrudate aus Beispiel S-31 und einmal mit 30 g Granulaten aus Anwendungsbeispiel 32 gemischt, portioniert und anschließend in Teebeutel abgefüllt.

Jeweils 800 g schwarzer Tee (Blattgrad Fannings) wurden einmal mit 33 g der Extrudate aus Beispiel S-31 und einmal mit 30 g Granulaten aus Beispiel S-32 gemischt, portioniert und anschließend in Teebeutel abgefüllt.

### Beispiel S-34

Herstellung einer zuckerhaltigen, bzw. zuckerreduzierten Eiscreme mit lang anhaltender Kühlwirkung unter Verwendung der erfindugsgemäßen Kühlsubstanzen

Alle Angaben, soweit nicht anders vermerkt, in Gew-%.

| Bestandteil | 1 | 2 | 3 | 4 | 5 | 6 |
|---|---|---|---|---|---|---|
| | | | | | | |
| Magermilch | 57,15 | 60,95 | 57,15 | 60,95 | 57,15 | 60,95 |
| Pflanzenfett, Schmelzbereich 35 - 40 °C | 20 | 20 | 20 | 20 | 20 | 20 |
| Zucker (Saccharose) | 12 | 8 | 12 | 8 | 12 | 8 |
| Magermilchpulver | 5 | 5 | 5 | 5 | 5 | 5 |
| Glucosesirup 72 % Trockensubstanz | 5 | 5 | 5 | 5 | 5 | 5 |
| Emulgator SE 30 (Grindstedt Products, Dänemark) | 0,65 | 0,65 | 0,65 | 0,65 | 0,65 | 0,65 |
| Vanillearoma, enthaltend 1 % Vanillin und 2,5% 3,4-Methylendioxyzimtsäure-N,N-diphenylamid | 0,2 | 0,2 | 0,2 | 0,2 | 0,2 | 0,2 |
| Vanillearoma, enthaltend 1 % Vanillin und 1% 2,3,4,5,6,10b,11,12-Octahydro-spiro[4b-azachrysen-12,2'-[1,3]dithiolan-]-1-on | 0,2 | 0,2 | 0,2 | 0,2 | 0,2 | 0,2 |
| Vanillearoma, enthaltend 1 % Vanillin und 2% Isopropyl-(5-methoxy-2-pyridin-2-yl-pyrimidin-4-yl)-amin | 0,2 | 0,2 | 0,2 | 0,2 | 0,2 | 0,2 |
| Hesperitin, 2,5% in 1,2-Propylenglycol | 0 | 0,2 | 0 | 0,2 | 0 | 0,2 |

Magermilch und Glucosesirup wurden auf 55° erhitzt und Zucker, Magermilchpulver sowie Emulgator hinzugefügt. Das Pflanzenfett wurde vorerhitzt und die gesamte Masse auf 58°C erwärmt. Nach Zugabe des Aromas wurde mit Hilfe eines Durchflusshochdruckhomogenisators homogenisiert (180 / 50 bar). Die erhaltene Masse wurde 1 min lang bei 78°C temperiert, anschließend auf 2 - 4 °C abgekühlt und 10 h zur Reifung bei dieser Temperatur inkubiert. Danach wurde die gereifte Masse abgefüllt und bei -18°C eingefroren gelagert.

### Beispiel S-35

Herstellung von zuckerhaltigen und zuckerreduzierten Erfrischungsgetränken verschiedener Geschmacksrichtungen und einem lang anhaltenden erfrischenden Kühlempfinden unter Verwendung der erfindungsgemäßen Kühlsubstanzen.

| Komponente | Gehalt | Zubereitung | | | | | | | |
|---|---|---|---|---|---|---|---|---|---|
| | Gewichtsanteil | 1 | 2 | 3 | 4 | 5 | 6 | 7 | 8 |
| Saccharose | % | 10,5 | 8 | 8 | 8 | 8 | 8 | 8 | 8 |
| Citronensäure | % | 0,2 | 0,2 | 0,2 | 0,2 | 0,2 | 0,2 | 0,2 | 0,2 |
| Hesperetin 1 % in 1,2-Propylenglycol | % | | 0,05 | 0,05 | 0,05 | 0,05 | 0,05 | 0,05 | 0,05 |
| Phloretin 1 % in 1,2-Propylenglycol | % | | 0,05 | 0,05 | 0,05 | 0,05 | 0,1 | 0,1 | 0,1 |
| Ethylhydroxymethylfuranon | ppb | 0,01 | 0,01 | | | | | | |
| Vanillin | ppb | 15 | 15 | | | | | | |
| Diethylmalonat | ppb | | | 70 | | | | | |
| Phenylethylacetat | ppb | | | 1 | | | | | |
| 2-Methylbutanal | ppb | | | | 0,3 | | 0,3 | | |
| Isovaleraldehyd | ppb | | | | 0,2 | | 0,2 | | |
| Furfurylacetat | ppb | | | | 0,3 | | | | |
| Massoilacton | ppb | | | | | 5 | 5 | | 5 |
| γ-Octalacton | ppb | | | | | 5 | 5 | | 5 |
| Ethylbutyrat | ppb | | | | 0,5 | | 0,5 | | 0,5 |
| Maltol | ppb | 350 | 350 | | | | 350 | | 350 |
| 2,5-Dimethyl-4-hydroxy-2H-furan-3-on | ppb | 3 | 3 | | | | 3 | | 3 |
| Ethylisobutyrat | ppb | | | 0,1 | | | 0,1 | | 0,1 |
| Ethyl-2-methylbutyrat | ppb | | | 0,1 | | | 0,1 | | 0,1 |
| 3,4-Methylendioxyzimtsäure-N,N-diphenylamid oder Isopropyl-(5-methoxy-2-pyridin-2-yl-pyrimidin-4-yl)-amin, (ne) jeweils 1% in 1,2-Propylenglycol oder 2,3,4,5,6,10b,11,12-Octahydro-spiro[4b-azachrysen-12,2'-[1,3]dithiolan-]-1-on, (ne) 0,5 % in 1,2-Propylenglycol | % | 0,5 | 0,5 | 0,5 | 0,5 | 0,5 | 0,5 | 0,5 | 0,5 |
| Butylphenyacetat | ppb | | | | | | 10 | | |
| Acetanisol | ppb | | | | | | 20 | | |
| Methylsorbat | ppb | | | | | | 100 | | |
| L-Lysin | ppm | | | | | | | 100 | 30 |
| Äpfelsäure | ppm | | | | | | | 80 | |
| L-Arginin | ppm | | | | | | | 5 | 20 |
| L-Asparaginsäure | ppm | | | | | | | 0,5 | |
| Calciumchlorid | ppm | | | | | | | 20 | |
| Glutamin | ppm | | | | | | | 2 | |
| Kaliumhydrogenphosphat | ppm | | | | | | | 6 | |
| Magnesiumchlorid | ppm | | | | | | | 20 | |
| L-Valin | ppm | | | | | | | 0,5 | |
| Glycin | ppm | | | | | | | | 40 |
| L-Alanin | ppm | | | | | | | | 20 |
| L-Serin | ppm | | | | | | | | 50 |
| Wasser | Ad 100 | | | | | | | | |

| | | | | | | | | | |
|---|---|---|---|---|---|---|---|---|---|
| (ne) = nicht erfindungsgemäß | | | | | | | | | |

Die Stoffe wurden vorgelegt und mit Wasser auf 100 % aufgefüllt und gelöst. Das Produkt wurde im Bedarfsfall in Flaschen abgefüllt und carbonisiert.

### Beispiel S-36

Herstellung eines Fruchtgummis mit einem lang anhaltenden frischen kühlenden Geschmack unter Verwendung der erfindungsgemäßen Kühlsubstanzen.

Alle Angaben, soweit nicht anders vermerkt, in Gew-%.

| Komponente | 1 | 2 | 3 | 4 | 5 | 6 |
|---|---|---|---|---|---|---|
| | | | (ne) | (ne) | (ne) | (ne) |
| Wasser | 23,6 | 23,6 | 23,6 | 23,6 | 23,6 | 23,6 |
| Saccharose | 34,5 | 34,5 | 34,5 | 34,5 | 34,5 | 34,5 |
| Glucosesirup, DE 40 | 31,89 | 31,89 | 31,89 | 31,89 | 31,89 | 31,89 |
| Iso Syrup C* Tru Sweet 01750 (Cerestar GmbH) | 1,5 | 1,5 | 1,5 | 1,5 | 1,5 | 1,5 |
| Gelatine 240 Bloom | 8,2 | 8,2 | 8,2 | 8,2 | 8,2 | 8,2 |
| Gelber und roter Farbstoff | 0,01 | 0,01 | 0,01 | 0,01 | 0,01 | 0,01 |
| Citronensäure | 0,2 | 0,2 | 0,2 | 0,2 | 0,2 | 0,2 |
| Kirscharoma, enthaltend 5 Gew.-% an 3,4-Methylendioxyzimtsäure-N,N-diphenylamid, bezogen auf das Aroma | 0,1 | | | | | |
| Kirscharoma, enthaltend 5 Gew.-% an 3,4-Methylendioxyzimtsäure-N-cyclohexyl-N-2-pyridylamid bezogen auf das Aroma | | 0,1 | | | | |
| Kirscharoma, enthaltend 2 Gew.-% an 2,3,4,5,6,10b,11,12-Octahydro-spiro[4b-azachrysen-12,2'-[1,3]dithiolan-]-1-on bezogen auf das Aroma | | | 0,1 | | | |
| Kirscharoma, enthaltend 2,5 Gew.-% an 2,3,4,5,6,10b,11,12-octahydro-3,3-dimethyl-spiro[4b-azachrysen-12,2'-[1,3]dithiolan]-1-on bezogen auf das Aroma | | | | 0,1 | | |
| Kirscharoma, enthaltend 4 Gew.-% an Isopropyl-(5-methoxy-2-pyridin-2-yl-pyrimidin-4-yl)-amin bezogen auf das Aroma | | | | | 0,1 | |
| Kirscharoma, enthaltend 5 Gew.-% an Cyclopentyl-(5-methoxy-2-pyridin-2-yl-pyrimidin-4-yl)-amin bezogen auf das Aroma | | | | | | 0,1 |

| | | | | | | |
|---|---|---|---|---|---|---|
| (ne) = nicht erfindungsgemäß | | | | | | |

### Beispiel S-37

Herstellung von zuckerhaltigen und zuckerreduzierten kohlensäurehaltigen Erfrischungsgetränken der Geschmacksrichtung "Cola" mit erfrischenden lang anhaltendem Kühleffekt unter Verwendung der erfindungsgemäßen Kühlsubstanzen.

Alle Angaben, soweit nicht anders vermerkt, in Gew-%.

| Komponente | 1 | 2 | 3 | 4 | 5 |
|---|---|---|---|---|---|
| | | | (ne) | (ne) | |
| Phosphorsäure 85% | 0,635 | 0,635 | 0,635 | 0,635 | 0,635 |
| Zitronensäure, wasserfrei | 0,064 | 0,064 | 0,064 | 0,064 | 0,064 |
| Koffein | 0,064 | 0,064 | 0,064 | 0,064 | 0,064 |
| Succrose | 63,600 | - | - | - | 12,9 |
| Sucralose | - | 0,126 | - | - | - |
| Erythritol | - | - | 6,000 | - | - |
| Aspartam | - | - | 0,350 | - | 0,07 |
| Steviosid | - | - | - | 0,300 | - |
| Acesulfam K | - | - | - | - | 0,07 |
| Zuckercoloeur | 0,800 | 0,800 | 0,800 | 0,800 | 0,800 |
| Getränke-Emulsion Typ: Cola | 1,445 | 1,445 | 1,445 | 1,445 | 1,445 |
| Natriumbenzoat | 0,106 | 0,106 | 0,106 | 0,106 | 0,106 |
| 3,4-Methylendioxyzimtsäure-N,N-diphenylamid | 0,030 | 0,015 | | | 0,030 |
| 3,4-Methylendioxyzimtsäure-N-cyclohexyl-N-2-pyridylamid | | 0,015 | | | |
| Isopropyl-(5-methoxy-2-pyridin-2-yl-pyrimidin-4-yl)-amin | | | 0,030 | | |
| 2,3,4,5,6,10b,11,12-Octahydro-spiro[4b-azachrysen-12,2'-[1,3]dithiolan-]-1-on | | | | 0,015 | |
| Wasser | ad 100 | ad 100 | ad 100 | ad 100 | ad 100 |

| | | | | | |
|---|---|---|---|---|---|
| (ne) = nicht erfindungsgemäß | | | | | |

Die festen Bestandteile bzw. Inhaltsstoffe werden einzeln mit Wasser vermischt, zusammengegeben und mit Wasser auf 100 g aufgefüllt. Anschließend lässt man das erhaltene Konzentrat über Nacht bei Raumtemperatur altern. Schließlich wird 1 Teil Konzentrat mit 5 Teilen kohlensäurehaltigem Wasser gemischt, in Flaschen gefüllt und verschlossen.

### Beispiel S-38

Herstellung von Schokoladen mit einem lang anhaltenden kühlenden Geschmack unter Verwendung der erfindungsgemäßen Kühlsubstanzen.

Alle Angaben, soweit nicht anders vermerkt, in Gew-%.
1 = dunkle Schokolade
2 = kalorienreduzierte dunkle Schokolade
3 = kalorienreduzierte dunkle Schokolade
4 = kalorienreduzierte dunkle Schokolade
5 = kalorienreduzierte Vollmilchschokolade

| Komponente | 1 | 2 | 3 | 4 | 5 |
|---|---|---|---|---|---|
| | | | (ne) | (ne) | (ne) |
| Kakaobutter | 13,50 | 13,00 | 13,50 | 9,48 | 14,00 |
| Cacaomasse | 42,0 | 39,00 | 42,00 | 44,00 | 23,00 |
| Erythritol | - | 47,45 | - | - | - |
| Maltitol, kristallin | - | - | - | 23,00 | |
| Inulin | - | - | - | 23,00 | |
| Sorbitol | - | - | 44,00 | - | - |
| Lactitol | - | - | - | - | 38,55 |
| Polydextrose | - | - | - | - | 9,70 |
| Vollmilchpulver | - | - | - | - | 14,0 |
| Sucrose | 43,9 | - | - | - | - |
| Lecitin | 0,48 | 0,48 | 0,40 | 0,48 | 0,50 |
| Vanillin | 0,02 | 0,02 | 0,02 | 0,02 | 0,20 |
| Aspartam | - | 0,03 | 0,06 | - | 0,03 |
| 3,4-Methylendioxy-zimtsäure-N,N-diphenylamid | 0,01 | 0,01 | | | |
| 2,3,4,5,6,10b,11,12-Octahydro spiro[4b-azachrysen-12,2'-[1,3]dithiolan-]-1-on | | | 0,005 | | |
| Isopropyl-(5-methoxy-2-pyridin-2-yl-pyrimidin-4-yl)-amin | | | | 0,0075 | 0,01 |

| | | | | | |
|---|---|---|---|---|---|
| (ne) = nicht erfindungsgemäß | | | | | |

### Beispiel S-39

Herstellung eines Biermix-Getränkes mit einem lang anhaltenden frischen kühlenden Geschmack: unter Verwendung der erfindungsgemäßen Kühlsubstanzen.

Alle Angaben, soweit nicht anders vermerkt, in Gew-%.

Es wurden vermischt:

| Komponente | 1 | 2 | 3 | 4 | 5 | 6 |
|---|---|---|---|---|---|---|
| | | | (ne) | (ne) | (ne) | (ne) |
| Zuckersirup | 4 | 4 | 4 | 4 | 4 | 4 |
| Bier | 50 | 50 | 50 | 50 | 50 | 50 |
| Ethylalkohol | 4 | 4 | 4 | 4 | 4 | 4 |
| Zitronensäure | 0,15 | 0,15 | 0,15 | 0,15 | 0,15 | 0,15 |
| Ascorbinsäure | 0,01 | 0,01 | 0,01 | 0,01 | 0,01 | 0,01 |
| Grapefruitsaft | 6 | 6 | 6 | 6 | 6 | 6 |
| Grapefruitaroma, enthaltend 2,5% 3,4-Methylendioxyzimtsäure-N,N-diphenylamid | 0,2 | | | | | |
| Grapefruitaroma, enthaltend 1% 2,3,4,5,6,10b,11,12-octahydro-3,3-dimethyl-spiro[4b-azachrysen-12,2'-[1,3]dithiolan]-1-on | | 0,2 | | | | |
| Grapefruitaroma, enthaltend 1% 2,3,4,5,6,10b,11,12-Octahydro-3-methyl-spiro[4b-azachrysen-12,2'-[1,3]dithiolan]-1-on | | | 0,2 | | | |
| Grapefruitaroma, enthaltend 2% sec-Butyl-(5-methoxy-2-pyridin-2-yl-pyrimidin-4-yl)-amin | | | | 0,2 | | |
| Grapefruitaroma, enthaltend 2,5% (5-Ethyl-2-pyridin-2-yl-pyrimidin-4-yl)-isopropyl-amin | | | | | 0,2 | |
| Grapefruitaroma, enthaltend 2% (1,2-Dimethyl-propyl)-(5-methoxy-2-pyridin-2-yl-pyrimidin-4-yl)-amin | | | | | | 0,2 |
| Wasser | ad 100 | ad 100 | ad 100 | ad 100 | ad 100 | ad 100 |
| Kohlensäure | 0,7 | 0,7 | 0,7 | 0,7 | 0,7 | 0,7 |

| | | | | | | |
|---|---|---|---|---|---|---|
| (ne) = nicht erfindungsgemäß | | | | | | |

Die in den vorstehenden Anwendungsbeispielen gefundenen Effekte lassen sich - gegebenenfalls durch vom Fachmann unschwer vorzunehmende Modifikationen - auf alle Produkte der jeweiligen Produktgruppe, d.h. insbesondere auf Zahnpasten, Kaugummis, Mundwässer, Halsbonbons, Gelatinekapseln, Kaubonbons und Tee in Beuteln übertragen. Dabei ist es für den Fachmann aufgrund der vorliegenden Beschreibung unschwer erkennbar, dass ohne großen Aufwand die erfindungsgemäßen Verbindungen und Mischungen - eventuell unter geringfügigen Modifikationen - untereinander austauschbar sind. Das bedeutet, dass die in den Produkten der Anwendungsbeispiele verwendete erfindungsgemäße Verbindung als Platzhalter auch für die anderen erfindungsgemäßen Verbindungen und Mischungen aufgefasst werden muss. Auch die Konzentration der verwendeten erfindungsgemäßen Verbindung oder Mischung ist für den Fachmann leicht erkenntlich variierbar. Außerdem sind die produktspezifischen weiteren Bestandteile in dem jeweiligen Anwendungsbeispiel für den Fachmann leicht nachvollziehbar ebenfalls gegen weitere produkttypische Bestandteile austauschbar bzw. durch solche ergänzbar. Eine Vielzahl solcher produktspezifischen Bestandteile sind in der oben stehenden Beschreibung offenbart.

Die folgenden Bespiele verdeutlichen die Einsatzmöglichkeiten der erfindungsgemäß zu verwendenden Kühlsubstanzen in kosmetischen Formulierungen, durch deren Verwendung auf der Haut ein als angenehm empfundenes Kälteempfinden und eine Hautberuhigung erzielt werden können.

### Beispiele S-40 - S-46

S-40 = Aerosol Deo-Spray
S-41 = Sport Shower Gel
S-42= After Shave Balm
S-43 = Eau de Toilette
S-44 = Fuß Spray
S-45 = Deo Stick
S-46 = Deo APP Roll on Emulsion

| Rohstoff | INCI-Name | | | | Beispiel | | | |
|---|---|---|---|---|---|---|---|---|
| | | S-40 (ne) | S-41 | S-42 | S-43 | S-44 (ne) | S-45 | S-46 |
| | | Gew.-% | | | | | | |
| 3,4-Methylendioxyzimtsäure-N,N-diphenylamid | | | | 0,1 | 0,1 | | 0,1 | |
| 3,4-Methylendioxyzimtsäure-N-cyclohexyl-N-2-pyridylamid | | | 0,15 | | | | | 0,2 |
| Cyclopentyl-(5-methoxy-2-pyridin-2-yl-pyrimidin-4-yl)-amin | | 0,15 | | | | 0,1 | | |
| sec-Butyl-(5-methoxy-2-pyridin-2-yl-pyrimidin-4-yl)-amin | | | 0,05 | 0,1 | | | 0,1 | |
| 2,3,4,5,6,10b,11,12-octahydro-3,3-dimethyl-spiro[4b-azachrysen-12,2'-[1,3]dithiolan]-1-on | | 0,05 | | | | | | 0,05 |
| 2,3,4,5,6,10b,11,12-Octahydro-spiro[4b-azachrysen-12,2'-[1,3]dithian]-1-on | | | | | 0,05 | 0,05 | | |
| Allantoin | Allantoin | | | 0,1 | | | | |
| (-) alpha Bisabolol Natural | Bisabolol | 0,1 | | | | | | |
| Abil 350 | Dimethicone | | | 3,0 | | | | |
| Akyposoft 100 BVC | Sodium Laureth-11 Carboxylate, Laureth-10 | | 8,5 | | | | | |
| Aloe Vera Gel Konzentrat 10:1 | Aloe Barbadensis Leaf Juice | | | | | | 1,0 | |
| Arlypon F | Laureth-2 | | 2,5 | | | | | |
| Carbopol Ultrez-21 | Acrylates/C10-30 Alkyl Acrylate Crosspolymer | | | 0,4 | | | | |
| Covi-Ox T-70 | Tocopherol | | | 0,1 | | | | |
| Dehyton K | Cocoamidopropyl Betaine | | 7,0 | | | | | |
| Deolite | Dimethyl Phenylpropanol Pentylene Glycol | | | | | | 0,5 | 0,5 |
| Dow Corning 246 fluid | Cyclohexasiloxane | | | | | | | 1,0 |
| D-Panthenol 75 L | Panthenol | | | 1,0 | | | | |
| Dracorin® 100 S.E.P. | Glyceryl Stearate, PEG-100 Stearate | | | | | | | 0,5 |
| Dracorin® GOC | Glyceryl Oleate Citrate | | | | | | | 2,0 |
| | Caprylic Capric Triglyceride | | | | | | | |
| Dragocide® Liquid | Phenoxyethanol, Methylparaben, Ethylparaben, Butylparaben, Propylparaben, Isobutylparaben | | 0,5 | 0,8 | | | | 0,8 |
| Dragosantol® 100 | Bisabolol | | | 0,2 | | 0,2 | | 0,2 |
| Dragoxat® 89 | Ethylhexyl Isononanoate | | | | | | 1,0 | |
| EDTA BD | Disodium EDTA | | | 0,1 | | | | |
| Ethanol 96% | Ethanol | 27,5 | | | 81,0 | 45,0 | | |
| Extrapone®Ginkgo Biloba | Propylene Glycol, Water (Aqua), Ginkgo Biloba Leaf Extract, Glucose, Lactic Acid | | 1,0 | | | | | |
| Farnesol | Farnesol | | | | | 0,5 | | |
| Fragrance | Perfum | 1,0 | 1,5 | 1,0 | 10,0 | 0,5 | 0,5 | 0,4 |
| Frescolat® MGA | Menthone Glycerin Acetal | | | | | | 0,8 | |
| Frescolat® ML | Menthyl Lactate | | 0,4 | 0,8 | | 0,2 | | 0,3 |
| Genapol LRO Liquid | Sodium Laureth Sulfate | | 40,0 | | | | | |
| Glycerin 99,5% | Glycerin | | | 2,5 | | | | 4,0 |
| Isodragol® | Triisononanoin | | | | | | | 1,0 |
| Jojoba Oil | Simmondsia Chinensis (Jojoba) Seed Oil | | | 2,0 | | | | |
| Natrium Hydroxid 10% Lsg. | Sodium Hydroxide | | 0,1 | 0,8 | | | | 0,6 |
| Natrium Stearat | Sodium Stearate | | | | | | 9,0 | |
| Neutral Oil | Caprylic/Ca pric Triglyceride | | | | | | | 3,5 |
| PCL -Liquid 100 | Cetearyl Ethylhexanoate | | | 3,0 | | 1,0 | | |
| Pemulen TR-2 | Acrylates/C10-30 Alkyl Acrylate Crosspolymer | | | | | | | 0,3 |
| Polymer JR400 | Polyquaternium-10 | | 0,3 | | | | | |
| Propane Butane 2,7 bar | Propane, Butane | 70,2 | | | | 49,5 | | |
| Propylene Glycol | Propylene Glycol | | | | | | 36,5 | |
| Rezal 36 GP | Aluminium Zirconium Tetrachlorohydrex GLY | | | | | | | 5,0 |
| Solubilizer | PEG-40Hydrogenated Castor Oil, Trideceth-9, Propylene Glycol, Water (Aqua) | | 0,5 | | 1,0 | 1,0 | | |
| SymAmide UDA | Undecylenamide DEA, Diethanolamine | | | | | 1,0 | | |
| SymCalmin® | Pentylene Glycol, Butylene Glycol, Hydroxyphenyl Propamidobenzoic Acid | | | 0,5 | | | | |
| SymClariol® | Decylene Glycol | 0,5 | | | | 0,5 | | |
| SymDeo® MPP | Dimethyl Phenylbutanol | 0,5 | | | | | | 0,5 |
| SymMollient® W/S | Trideceth-9, PEG-5 Isononanoate | | | | 1,0 | 0,5 | | |
| SymRelief® | Bisabolol, Zingiber Officinale (Ginger) Root Extract | | 0,2 | 0,2 | | | | |
| SymVital™ | Aloe Barbadensis Leaf Juice Powder, Magnesium Ascorbyl Phosphate, Rubus Idaeus (Raspberry) Leaf Extract | | | 0,1 | | | | |
| Vitamin E acetat | Tocopherol Acetate | | | 0,5 | | | | |
| Wasser | Water (Aqua) | | ad 100 | ad 100 | ad 100 | | ad 10 | ad 100 |

| | | | | | | | | |
|---|---|---|---|---|---|---|---|---|
| (ne) = nicht erfindungsgemäß | | | | | | | | |

### Beispiele S-47 - S-52

S-47 = Tagescreme O/W, ca. SPF 15
S-48 = Sonnenschutzemulsion ca. SPF 25
S-49 = After Sun Spray
S-50 = After Shave
S-51 = Creme W/O
S-52 = Haar Conditioner

| Rohstoff | INCI-Name | Beispiele | | | | | |
|---|---|---|---|---|---|---|---|
| | | S-47 | S-48 (ne) | S-49 | S-50 (ne) | S-51 (ne) | S-52 (ne) |
| | | Gew-% | | | | | |
| 3,4-Methylendioxyzimtsäure-N-cyclohexyl-N-2-pyridylamid | | 0;1 | | 0,1 | | | |
| 2,3,4,5,6,10b,11,12-octahydro-3,3-dimethyl-spiro[4b-azachrysen-12,2'-[1,3]dithiolan]-1-on | | 0,05 | 0,05 | | | 0,05 | 0,1 |
| Isopropyl-(5-methoxy-2-pyridin-2-yl-pyrimidin-4-yl)-amin | | | 0,25 | 0,2 | | | |
| Cyclopentyl-(5-ethyl-2-pyridin-2-yl-pyrimidin-4-yl)-amin | | | | | 0,1 | 0,1 | 0,2 |
| Allantoin | Allantoin | | | 0,1 | | | |
| (-) alpha Bisabolol Natural | Bisabolol | | | 0,2 | | 0,3 | |
| Abil 350 | Dimethicone | 2,0 | | | | | |
| Aluminium Stearate | Aluminium Stearate | | | | | 1,2 | |
| Arlypon F | Laureth-2 | | | | | | |
| Biotive® L-Arginine | Arginine | | 0,5 | | | | |
| Carbopol Ultrez-10 | Carbomer | 0,2 | | 0,2 | | | |
| Covi-Ox T-70 | Tocopherol | | | | 0,1 | | |
| Cutina GMS V | Glyceryl Stearate | 2,0 | | 2,0 | | | |
| Dehyquart A CA | Cetrimonium Chloride | | | | | | 4,0 |
| Dow Corning 246 fluid | Cyclohexasiloxane | | | | 2,0 | | |
| D-Panthenol 75 L | Panthenol | | | | 1,0 | | 1,0 |
| Dracorin® CE | Glyceryl Stearate/Citrate | | 2,0 | | | | |
| Dracorin® GOC | Glyceryl Oleate Citrate | | | | 2,0 | | |
| | Caprylic Capric Triglyceride | | | | | | |
| Drago-Beta-Glucan | Water (Aqua), Butylene Glycol, Glycerin, Avena Sativa (Oat) Kernel Extract | | | 2,0 | | | |
| DragoCalm® | Water, Glycerin, Avena Sativa (Oat Kernel Extract) | | | 1,0 | | | |
| Dragocide® Liquid | Phenoxyethanol, Methylparaben, Ethylparaben, Butylparaben, Propylparaben, Isobutylparaben | 0,8 | | | | 0,8 | 0,8 |
| Dragoderm® | Glycerin, Triticum Vulgare (Wheat) Gluten, Water (Aqua) | | | 2,0 | 2,0 | | 2,0 |
| Dragosan W/O P | Sorbitan Isostearate, Hydrogenated Castor Oil, Ceresin, Beeswax (Cera Alba) | | | | | 8,0 | |
| Dragosine® | Carnosine | | | 0,2 | | | |
| Dragoxat® 89 | Ethylhexyl Isononanoate | | 3,0 | 4,0 | 1,0 | 5,0 | |
| EDTA BD | Disodium EDTA | | 0,1 | 0,1 | | | |
| Emulsiphos® | Potassium Cetyl Phosphate, Hydrogenated Palm Glycerides | 2,0 | | 2,0 | | | |
| Ethanol 96% | Ethanol | | | | 65,0 | | |
| Farnesol | Farnesol | | | | | | |
| Fragrance | Perfum | 0,3 | 0,4 | 0,3 | 1,0 | 0,3 | 0,3 |
| Frescolat® ML | Menthyl Lactate | 0,2 | | | 0,3 | | |
| Fruitapone® Orange B | Propylene Glycol, Water (Aqua), Citric Acid, Citrus Aurantium Dulcis (Orange) Juice, Trideceth-9, Bisabolol | 1,0 | | | | | |
| Glycerin 99,5% | Glycerin | 2,0 | | 3,0 | 4,0 | 3,0 | |
| Hydrolite®-5 | Pentylene Glycol | | 5,0 | | 5,0 | | |
| Hydroviton®-24 | Water, Pentylene Glycol, Glycerin, Lactic Acid, Sodium Lactate, Serine, Urea, Sorbitol, Sodium Chloride, Allantoin | | | 1,0 | | 2,0 | |
| Iso Adipat | Diisopropyl Adipate | | | 1,0 | 5,0 | | |
| Jojoba Oil | Simmondsia Chinensis (Jojoba) Seed Oil | | | | | 2,0 | |
| Keltrol CG RD | Xanthan Gum | 0,1 | 0,1 | 0,2 | | | |
| Lanette O | Cetearyl Alcohol | 3,0 | 2,0 | 3,0 | | | 3,5 |
| Mineral Oil | Mineral Oil | | | | | 8,0 | |
| Natrium Chlorid | Sodium Chloride | | | | | 1,0 | 2,0 |
| Natrium Hydroxid 10% Lsg. | Sodium Hydroxide | 0,5 | | | | 0,4 | |
| Neo Heliopan® 303 | Octocrylene | 5,0 | 8,0 | | | | |
| Neo Heliopan® 357 | Butylmethoxydibenzoylmethane | 1,1 | 3,0 | | | | |
| Neo Heliopan® HMS | Homosalate | | 5,0 | | | | |
| Neo Heliopan® Hydro, 25% Lsg. neutralisiert mit Biotive L-Arginin | Phenylbenzimidazole Sulfonic Acid | 3,0 | 8,0 | | | | |
| Neo Heliopan®AP, 10% Lsg., neutralisiert mit NAOH | Disodium Phenyl Dibenzimidazole Tetrasulfonate | 3,0 | 13,3 | | | | |
| Neo Heliopan® OS | Ethylhexyl Salicylate | 5,0 | | | | | |
| Neutral Oil | Caprylic/Capric Triglyceride | | | | 5,0 | | |
| Ozokerite Wax 2389 | Ozokerite | | | | | 2,0 | |
| Pemulen TR-2 | Acrylates/C10-30 Alkyl Acrylate Cross polymer | | | | 0,3 | | |
| Polyquart H81 | PEG-15 Coco Polyamine | | | | | | 3,0 |
| Propylene Glycol | Propylene Glycol | 3,0 | 4,0 | | | | |
| Softisan 100 | Hydrogenated Coco Glycerides | | 1,5 | | | | |
| Squalan, Vegetable Based | Squalane | | | 3,0 | | | |
| SymCalmin® | Pentylene Glycol, Butylene Glycol, Hydroxyphenyl Propamidobenzoic Acid | | | 1,0 | | | |
| SymDiol® 68 | 1,2 Hexanediol, Caprylyl Glycol | | | 1,0 | | | |
| SymGlucan® | Water (Aqua) Glycerin, Beta Glucan | | | | 1,0 | | |
| SymMollient® W/S | Trideceth-9, PEG-5 Isononanoate | | | | 0,5 | | |
| SymRelief® | Bisabolol, Zingiber Officinale (Ginger) Root Extract | | | | 0,2 | | |
| SymRepair® | Hexyldecanol, Bisabolol, Cetylhydroxyproline Palmitamide, Stearic Acid, Brassica Campestris (Rapeseed Sterols) | | | 2,0 | 3,0 | | |
| SymVital™ | Aloe Barbadensis Leaf Juice Powder, Magnesium Ascorbyl Phosphate, Rubus Idaeus (Raspberry) Leaf Extract | 0,3 | | | | | |
| Triethanolamine 99% | Triethanolamine | | | 0,4 | 0,3 | | |
| Vitamin E acetat | Tocopherol Acetate | | 0,5 | | | 0,2 | |
| Wasser | Water (Aqua) | ad 100 | ad 100 | ad 100 | ad 100 | ad 100 | ad 100 |

| | | | | | | | |
|---|---|---|---|---|---|---|---|
| (ne) = nicht erfindungsgemäß | | | | | | | |

### Testbeispiel 1: Untersuchung erfindungsgemäßer Wirkstoffe im Assay auf TRPM8 Modulatoren

Verschiedene erfindungsgemäße Wirkstoffe wurden in dem Assay gemäß Referenzbeispiel 3 untersucht.

Die dabei ermittelten EC50-Werte der Modulatoren sind in den folgenden Tabellen A, B und C (erfindungsgemäß) zusammengefasst.

**Tabelle A: (nicht erfindungsgemäß)**

| Nr. | Struktur-Typ1 | Physikalische Daten | EC₅₀ |
|---|---|---|---|
| 1-1 | 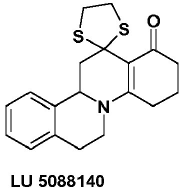 | ¹H-NMR (CDCl₃): m 7,12-7,33; d 4,70; m 4,10-4,20: m 4,00-4,10; m 3,60-3,69; m 3,38-3,48; m 3,14-3,23; m 2,96-3,04; m 2,76-2,85; m 2,59-2,68; m 2,36-2,48; m 2,23-2,34; m 1,95-2,04; m 1,84-1,95 [ppm] | 0,1 µM |
| 1-2 | 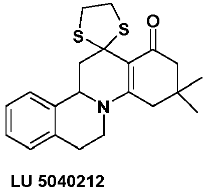 | ¹H-NMR (CDCl₃): m 7,10-7,32; d 4,68; m 4,08-4,20; dtr 4,04; m 3,56-3,65; m 3,35-3,50; dtr 3,19; m 2,93-3,05; m 2,74-2,86; m 2,34-2,46; s 2,23; s 1,10; s 1,08 [ppm] | 0,6 µM |
| 1-3 | 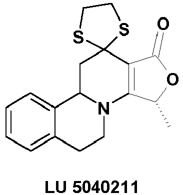 | ¹H-NMR (CDCl₃): m 7,14-7,34; m 4,90-5,02; m 4,63-4,74; m 3,87-3,99; m 3,56-3,68; m 3,30-3,48; 2,96-3,08; m 2,78-2,92; d 2,66; m 2,26-2,43; d 1,54 [ppm] | 0,7 µM |
| 1-4 | 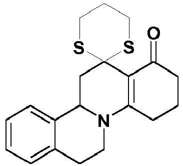 | ¹H-NMR (CDCl₃): m 7,12-7,34; d 4,90; m 4,04-4,16; m 2,94-3,32; m 2,78-2,88; m 2,62-2,64; m 2,40-2,50; m 2,24-2,34; m 1,81-2,20 [ppm] | 3,1 µM |
| 1-5 | 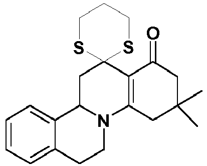 | ¹H-NMR (CDCl₃): m 7,28-7,32; m 7,20-7,26; d 7,17; d 4,86; m 4,02-4,09; m 3,14-3,30; m 2,94-3,09; m 2,79-2,86; m 2,63-2,71; m 2,35-2,44; s 2,24; tr 2,17; m 2,00-2,14; d 1,08 [ppm] | 1,4 µM |
| 1-6 | 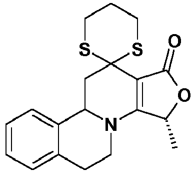 | ¹H-NMR (CDCl₃): m 7,16-7,34; q 5,06; q 5,00; d 4,86; m 3,62-3,74; m 3,35-3,46; m 3,13-3,31; m 2,94-3,12; m 2,70-2,92; m 1,98-2,14; d 1,54 [ppm] | 0,2 µM |
| 1-7 | 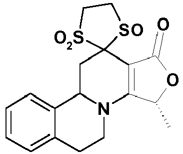 | Diastereomere: ¹H-NMR (CDCl₃): m 7,14-7,42; m 5,18-5,27; d 4,99; d 4,74; m 4,30-4,46; m 4,16-4,25; m 3,99-4,08; m 3,67-3,91; tr 3,49; m 3,19-3,33; m 2,98-3,14; m 2,84-2,93; tr 2,18; tr 1,98; m 1,44-1,69 [ppm] | 0,7 µM |
| 1-8 | 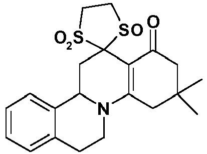 | Diastereomere: ¹H-NMR (CDCl₃): m 7,40-7,46; m 7,20-7,33; m 7,12-7,19; d 4,97; m 4,22-4,44; m 4,09-4,17; m 3,87-3,97; m 3,80-3,87; m 3,60-3,67; tr 3,35; tr 3,22; m 2,93-3,11; m 2,72-2,92; d 2,68; dd 2,54; d 2,39; m 2,14-2,34; tr 1,98; s 1,44; m 1,10-1,25 [ppm] | 9,8 µM |
| 1-9 | 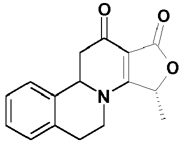 | Diastereomere: 1H-NMR (CDCl₃): m 7,20-7,40; q 5,50; q 5,40; m 5,06-5,22; m 3,93-4,03; m 3,77-3,86; m 3,43-3,58; m 3,28-3,43; m 3,00-3,14; m 2,86-3,00; m 2,43-2,72; tr 1,50 [ppm] | 10 µM |

**Tabelle B: (nicht erfindungsgemäß)**

| Nr. | Struktur-Typ 2 | Physikalische Daten | EC₅₀ |
|---|---|---|---|
| 2-1 | 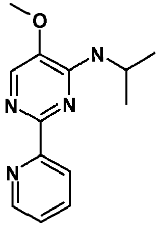 | ¹H-NMR (CDCl₃): d 8,77; d 8,39; s 7,91; m 7,26-7,32; d 5,17; m 5,42-5,54; s 3,94 d 1,32 [ppm] | 0,1 µM |
| 2-2 | 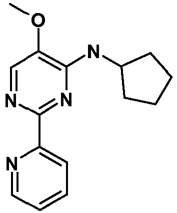 | ¹H-NMR (CDCl₃): s 8,74; d 8,40; s 7,88; tr 7,74; m 7,20-7,32; s 5,32; m 4,48-4,60; s 3,88; m 2,08-2,24; m 1,46-1,84 [ppm] | 0,3 µM |
| 2-3 | 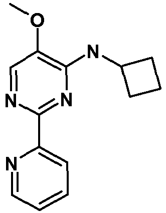 | Retentionszeit (HPLC): 2,645 min; | 0,8 µM |
| | | Methode 1 im Anhang | |
| 2-4 | 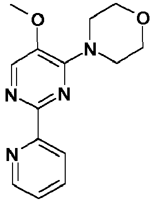 | Retentionszeit (HPLC): 4,02 min; | 1,0 µM |
| | | Methode 2 im Anhang | |
| 2-5 | 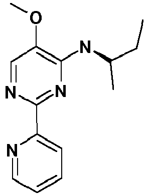 | ¹H-NMR (CDCl₃): s 8,74; d 8,38; s 7,90; tr 7,75; d 5,16; m 4,27-4,38; s 3,90; m 1,57-1,72; d 1,28; tr 0,98 [ppm] | 0,1 µM |
| 2-7 | 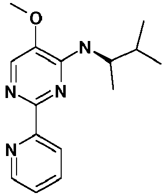 | ¹H-NMR (CDCl₃): d 8,74; d 8,37; s 7,90; tr 7,76; m 7,24-7,30; d 5,20; m 4,25-4,36; s 3,92; m 1,88-1,98; d 1,22; d 1,00; d 0,97 [ppm] | 0,1 µM |
| 2-8 | 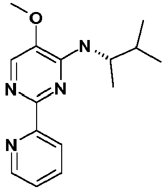 | ¹H-NMR (CDCl₃): d 8,74; d 8,37; s 7,90; tr 7,76; m 7,24-7,30; d 5,20; m 4,25-4,36; s 3,92; m 1,88-1,98; d 1,22; d 1,00; d 0,97 [ppm] | 0,5 µM |
| 2-9 | 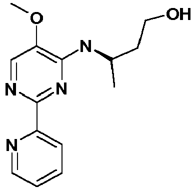 | ¹H-NMR (CDCl₃): s 8,56; d 8,24; s 7,73; tr 7,67; m 7,15-7,22; m 5,50-5,61; d 5,50; m 4,48-4,60, s 3,83; br s 3,49; m 1,78-1,88; m 1,40-1,49; d 1,24 [ppm] | 1,0 µM |
| 2-10 | 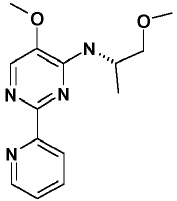 | ¹H-NMR (CDCl₃): d 8,76; d 8,38; s 7,91; tr 7,76; m 7,24-7,30; d 5,54; m 4,55-4,64; s 3,89; d 3,51; s 3,39; d 1,35 [ppm] | 2,2 µM |
| 2-11 | 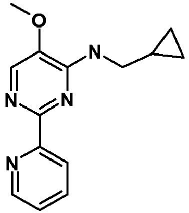 | HPLC-Retentionszeit: 2,41 min | 1,2 µM |
| | | HPLC-Methode 1: siehe Anhang | |
| 2-12 | 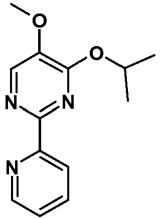 | 1H-NMR (CDCl₃): d 8,79; m 8,32-8,38; tr 7,82; m 7,31-7,36; m 5,62-5,72; s 3,98; dd 1,50 [ppm] | 3,2 µM |
| 2-13 | 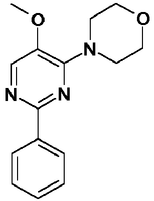 | Retentionszeit (HPLC): 4,92 min; | 11,7 µM |
| | | Methode 2 im Anhang | |
| 2-14 | 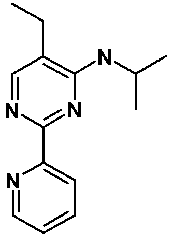 | ¹H-NMR (CDCl₃): d 8,77; d 8,43; s 8,20; tr 7,78; tr 7,30; m 4,49-4,60; q 2,41; d 1,30; tr 1,25 [ppm] | 0,4 µM |
| 2-15 | 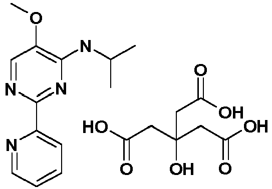 | ¹H-NMR (CDCl₃): s 8,71; d 8,30; tr 7,95; s 7,91; tr 7,49; d 7,20; m 4,46-4,56; s 3,94; dd 2,72; d 1,26 [ppm] | 0,8 µM |
| 2-16 | 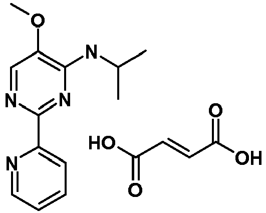 | ¹H-NMR (CDCl₃): s 8,70; d 8,30; m 7,90-7,97; tr 7,46; d 7,00; s 6,68; m 4,46-4,56; s 3,94; d 1,26 [ppm] | 1,6 µM |
| 2-17 | 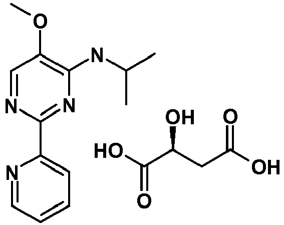 | ¹H-NMR (CDCl₃): s 8,70; d 8,29; m 7,90-7,97; tr 7,46; d 6,98; m 4,46-4,56; tr 4,28; s 3,93; dd 2,66; m 2,44-2,57; d 1,25 [ppm] | 0,4 µM |
| 2-18 | 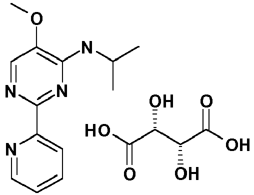 | ¹H-NMR (CDCl₃): s 8,71; d 8,30; m 7,90-8,00; tr 7,48; d 7,04; br s 5,54; m 4,46-4,57; s 4,33; s 3,95; d 1,26 [ppm] | 0,5 µM |
| 2-19 | 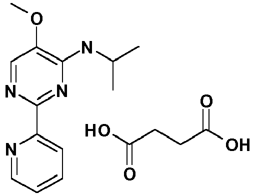 | ¹H-NMR (CDCl₃): s 8,70; d 8,30; s 7,96; tr 7,91; tr 7,43; d 6,78; m 4,46-4,56; s 3,94; s 2,49; d 1,25 [ppm] | 0,3 µM |
| 2-20 | 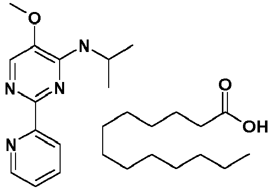 | ¹H-NMR (CDCl₃): br s 9,55; s 8,80; d 8,35; s 7,97; tr 7,78; tr 7,31; d 5,26; m 4,49-4,58; s 3,91; tr 2,34; m 1,61-1,70; m 1,20-1,41; tr 0,88 [ppm] | 0,9 µM |
| 2-21 | 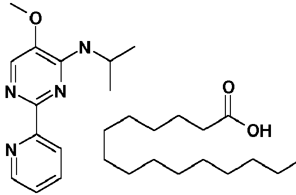 | ¹H-NMR (CDCl₃): s 8,82; d 8,35; s 7,98; tr 7,77; tr 7,30; d 5,28; m 4,49-4,59; s 3,88; tr 2,35; m 1,62-1,71; m 1,20-1,41; m 0,85-0,93 [ppm] | 0,3 µM |
| 2-22 | 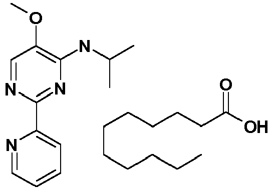 | ¹H-NMR (CDCl₃): br s 10,58; s 8,81; d 8,35; s 7,97; tr 7,78; tr 7,31; d 5,29; m 4,49-4,59; s 3,90; tr 2,35; m 1,60-1,70; m 1,20-1,42; tr 0,88 [ppm] | 0,7 µM |

**Tabelle C:**

| Nr. | Struktur-Typ 3 | Physik. Daten | EC₅₀ |
|---|---|---|---|
| 3-1 | 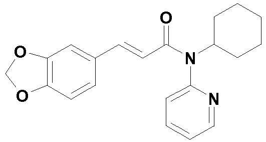 | ¹H-NMR (CDCl₃): s 8,62; tr 7,80; d 7,57; 7,32-7,38; d 7,17; d 6,80; m m 6,65-6,72; s 5,91; d 5,80; m 4,62-4,71; d 1,96; d 1,75; d 1,60; m 1,34-1,46; m 1,22-1,34; m 0,93-1,06 [ppm] | 0,1 µM |
| 3-2 | 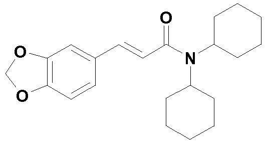 | ¹H-NMR (CDCl₃): d 7,50; s 7,00; d 6,97; d 6,78; d 6,68; s 5,96; br s 3,56; m 1,06-2,48 [ppm] | 11,8 µM |
| 3-3 | 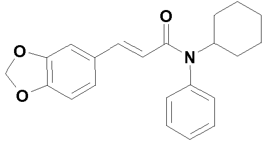 | ¹H-NMR (CDCl₃): d 7,55; m 7,37-7,47; m 7,12-7,18; d 6,78; m 6,62-6,71; tr 4,72; d 1,88; d 1,74; d 1,58; q 1,43; q 1,11 [ppm] | 0,9 µM |
| 3-4 | 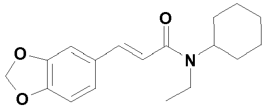 | ¹H-NMR (CDCl₃): m 7,53-7,68; m 6,96-7,08; d 6,79; m 6,60-6,76; s 5,96; br s 4,50; m 3,72-3,82; m 3,33-3,46; m 1,04-1,92 [ppm] | 0,8 µM |
| 3-5 | 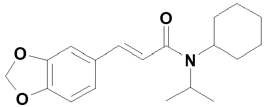 | ¹H-NMR (CDCl₃): d 7,51; s 7,03; d 6,97; d 6,80; d 6,67; s 5,98; m 3,33-4,17; br s 2,22; m 1,03-1,94 [ppm] | 0,2 µM |
| 3-6 | 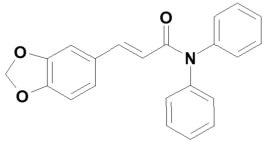 | ¹H-NMR (CDCl₃): d 7,68; m 7,16-7,48; d 6,90; s 6,78; d 6,75; d 6,30; s 5,95 [ppm] | 0,8 µM |
| 3-7 | 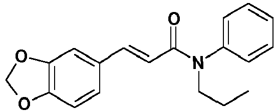 | ¹H-NMR (CDCl₃): d 7,59; m 7,34-7,48; d 7,21; m 6,80-6,88; m 6,63-6,77; d 6,11; s 5,91; tr 3,80; m 1,54-1,66; tr 0,92 [ppm] | 1,1 µM |
| 3-8 | 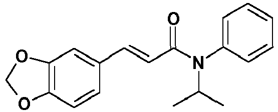 | ¹H-NMR (CDCl₃): d 7,56; m 7,39-7,50; m 7,12-7,20; d 6,80; d 6,70; s 6,66; m 5,86-5,97; m 5,06-5,19; d 1,12 [ppm] | 0,6 µM |
| 3-9 | 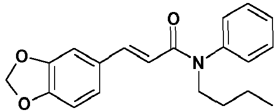 | ¹H-NMR (CDCl₃): d 7,58; m 7,34-7,47; d 7,20; d 6,83; m 6,67-6,76; d 6,11; s 5,90; tr 3,83; m 1,50-1,60; m 1,30-1,40; tr 0,90 [ppm] | 0,7 µM |
| 3-10 | 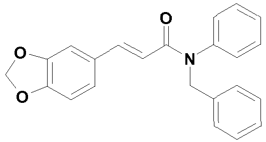 | ¹H-NMR (CDCl₃): d 7,65; m 7,18-7,36; d 7,06; d 6,84; m 6,67-6,74; d 6,16; s, 5,88; s 5,02 [ppm] | 1,2 µM |
| 3-11 | 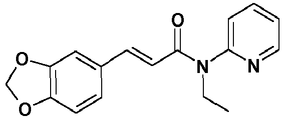 | ¹H-NMR (CDCl₃): s 8,54; tr 7,73; d 7,64; m 7,18-7,24; d 6,86; s 6,80; d 6,72; d 6,27; s 5,90; q 4,07; tr 1,22 [ppm] | 6,2 µM |
| 3-12 | 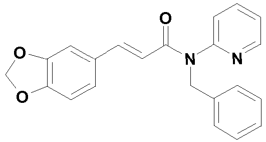 | Retentionszeit (HPLC): 8,99 min; | 0,7 µM |
| | | Methode 2 im Anhang | |
| 3-13 | 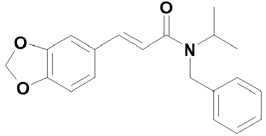 | ¹H-NMR (CDCl₃): m 7,61-7,74; m 6,94-7,39; m 6,65-6,94; d 6,48; m 5,81-6,00; m 4,89-5,03; m 4,31-4,72; m 1,06-1,28 [ppm] | 3,9 µM |
| 3-14 | 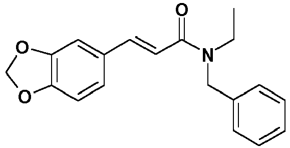 | Retentionszeit (HPLC): 8,93 min; | 1,5 µM |
| | | Methode 2 im Anhang | |
| 3-15 | 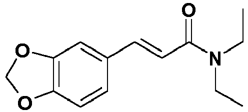 | ¹H-NMR (CDCl₃): d 7,62; s 7,04; m 6,96-7,01; d 6,78; d 6,67; s 5,97; m 3,40-3,52; m 1,13-1,30 | 12,3 µM |
| 3-16 | 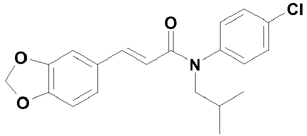 | Retentionszeit (HPLC): 12,31 min; | 8,2 µM |
| | | Methode 2 im Anhang | |
| 3-17 | 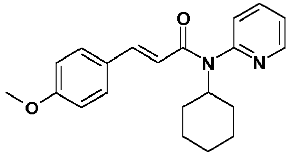 | ¹H-NMR (CDCl₃): s 8,62; tr 7,80; d 7,65; m 7,35-7,42; m 7,12-7,28; d 6,78; d 5,90; tr 4,69; s 3,72; d 1,98; d 1,75; d 1,59; m 1,24-1,50; m 0,92-1,06 [ppm] | 0,4 µM |
| 3-18 | 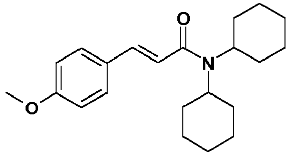 | ¹H-NMR (CDCl₃): d 7,55; d 7,46; d 6,90; d 6,72; s 3,82; br s 3,59; br s 2,28; m 1,08-1,93 [ppm] | 0,1 µM |
| 3-19 | 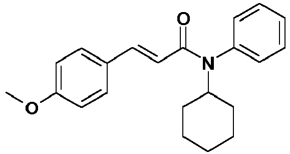 | ¹H-NMR (CDCl₃): d 7,61; s 7,40; m 7,10-7,20; d 6,76; d 5,95; m 4,68-4,78; s 3,72; d 1,90; d 1,74; d 1,58; q 1,43; q 1,11; q 0,92 [ppm] | 0,7 µM |
| 3-20 | 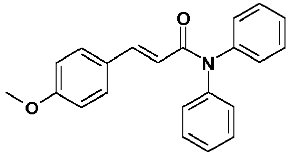 | ¹H-NMR (CDCl₃): d 7,74; m 7,15-7,39; d 6,79; d 6,36; s 3,81 [ppm] | 1,1 µM |
| 3-21 | 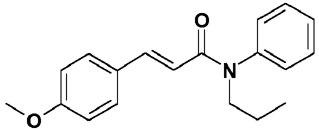 | ¹H-NMR: (CDCl₃) d 7,64; m 7,33-7,46; tr 7,21; d 6,76; d 6,18; d 3,80; s 3,72; m 1,55-1,66; tr 0,92 [ppm] | 1,7 µM |
| 3-22 | 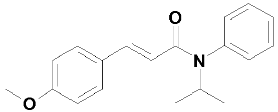 | ¹H-NMR (CDCl₃): d 7,61; m 7,38-7,46; m 7,12-7,22; d 6,76; d 5,97; m 5,08-5,18; s 3,74; d 1,12 [ppm] | 1,3 µM |
| 3-23 | 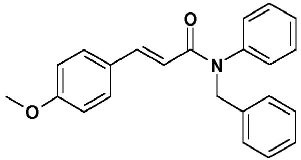 | ¹H-NMR (CDCl₃): d 7,71; m 7,18-7,36; d 7,06; d 6,78; d 6,22; s 5,03; s 3,73 | 3,2 µM |
| 3-24 | 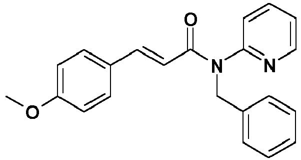 | ¹H-NMR (CDCl₃): m 8,46-8,54; d 7,74; tr 7,60; m 7,02-7,32; d 6,80; d 6,38; s 5,27; s 3,73 [ppm] | 2,2 µM |
| 3-25 | 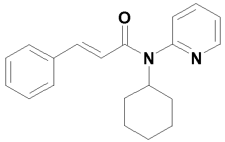 | ¹H-NMR (CDCl₃): m 8,60-8,66; m 7,75-7,83; d 7,67; m 7,31-7,39; s 7,25; d 7,16; d 6,0; m 4,64-4,73; d, 1,97; d 1,75; d 1,60; m 1,24-1,48; m 0,94-1,06 [ppm] | 2,3 µM |
| 3-26 | 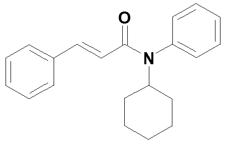 | ¹H-NMR (CDCl₃): d 7,64; m 7,38-7,49; m 7,20-7,30; m 7,12-7,20; d 6,06; m 4,67-4,78; d 1,90; d 1,75; d 1,60; m 1,38-1,50; m 1,07-1,18; 0,87-1,01 [ppm] | 0,6 µM |
| 3-27 | 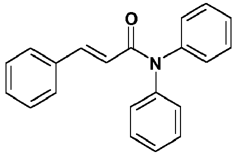 | ¹H-NMR (CDCl₃): d 7,78; m 7,21-7,47; d 6,49 [ppm] | 2,9 µM |
| 3-28 | 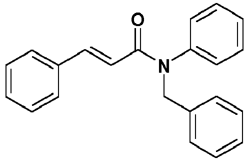 | ¹H-NMR (CDCl₃): d 7,78; m 6,91-7,50; d 6,36; s 5,03 [ppm] | 2,4 µM |
| 3-29 | 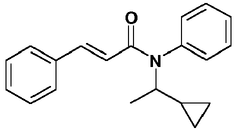 | Retentionszeit (HPLC): 11,56 min; | 0,8 µM |
| | | Methode 2 im Anhang | |
| 3-30 | 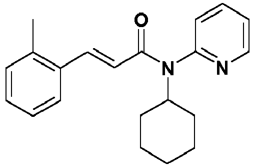 | ¹H-NMR (CDCl₃): s 8,62; d 7,96; tr 7,79; m 7,30-7,36; m 7,08-7,20; tr 7,04; d 5,90; m 4,64-4,72; s 2,36; d 1,98; d 1,76; d 1,60; q 1,42; q 1,30; m 0,95-1,06 [ppm] | 0,4 µM |
| 3-31 | 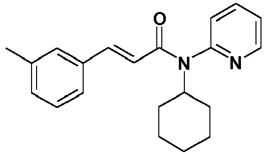 | ¹H-NMR (CDCl₃): s 8,62; tr 7,78; d 7,64; m 7,32-7,36; m 7,10-7,19; m 7,02-7,10; d 5,96; m 4,62-4,71; s 2,28; d 1,97; d 1,77; d 1,61; q 1,42; q 1,29; m 0,95-1,06 [ppm] | 0,3 µM |
| 3-32 | 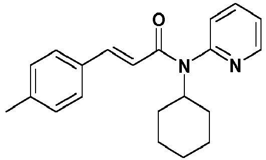 | ¹H-NMR (CDCl₃): s 8,62; tr 7,78; d 7,64; m 7,31-7,37; m 7,11-7,19; d 7,06; d 5,94; m 4,62-4,72; s 2,28; d 1,96; d 1,76; d 1,60; m 1,35-1,48; m 1,23-1,35; m 0,93-1,06 [ppm] | 0,3 µM |
| 3-33 | 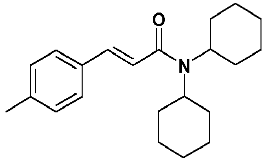 | ¹H-NMR (CDCl₃): d 7,55; d 7,40; d 7,16; d 6,80; br s 3,57; s 2,36; br s 2,29; m 1,04-1,96 [ppm] | 2,1 µM |
| 3-34 | 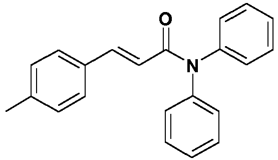 | ¹H-NMR (CDCl₃): d 7,76; m 7,12-7,42; d 7,07; d 6,44; s 2,29 [ppm] | 4,0 µM |
| 3-25 | 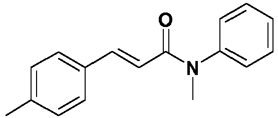 | ¹H-NMR (CDCl₃): d 7,67; m 7,30-7,45; tr 7,20; d 7,06; d 6,34; s 3,40, s 2,28 [ppm] | 4,2 µM |
| 3-36 | 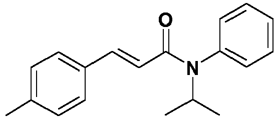 | ¹H-NMR (CDCl₃): d 7,65; 7,38-7,49; m 7,09-7,20; d 7,03; d 6,06; m 5,07-5,19; s 2,26; d 1,13 | 1,8 µM |
| 3-37 | 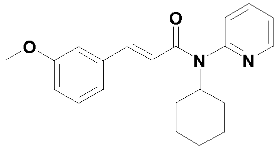 | ¹H-NMR (CDCl₃): s 8,62; tr 7,78; d 7,64; m 7,31-7,36; m 7,13-7,19; m 6,76-6,88; d 5,98; m 4,63-4,72; s 3,71; d 1,96; d 1,76; d 1,59; q 1,41; q 1,29; m 0,94-1,06 [ppm] | 3,0 µM |
| 3-38 | 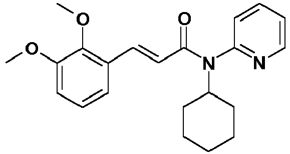 | ¹H-NMR (CDCl₃): s 8,62; d 7,87; tr 7,80; tr 7,33; d 7,17; tr 6,92; d 6,83; d 6,77; d 6,15; m 4,62-4,72; s 3,82; s 3,69; d 1,98; d 1,77; d 1,61; q 1,42; q 1,30; m 0,95-1,07 [ppm] | 0,7 µM |
| 3-39 | 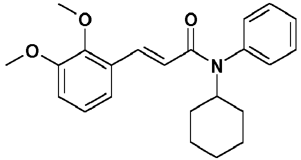 | ¹H-NMR (CDCl₃): d 7,85; m 7,37-7,46; d 7,15; tr 6,90; d 6,80; d 6,74; d 6,25; m 4,68-4,78; s 3,80; s 3,66; d 1,91; d 1,76; d 1,60; q 1,44; q 1,14; m 0,89-1,00 [ppm] | 2,6 µM |
| 3-40 | 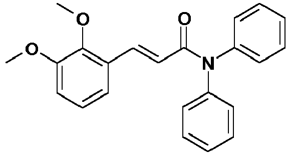 | ¹H-NMR (CDCl₃): d 8,00; m 7,05-7,68; tr 6,94; m 6,70-6,90; d 6,64; s 3,82; s 3,73 [ppm] | 5,9 µM |
| 3-41 | 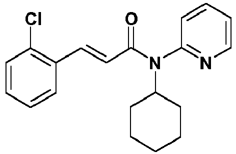 | Retentionszeit (HPLC): 10,43 min; | 0,7 µM |
| | | Methode 2 im Anhang | |
| 3-42 | 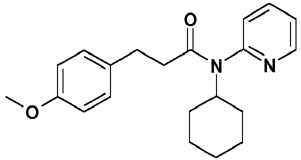 | ¹H-NMR (CDCl₃): d 8,56; tr 7,70; m 7,24-7,30; d 6,96; d 6,90; d 6,76; m 4,50-4,60; s 3,73; tr 2,86; tr 2,18; d 1,90; d 1,72; 1,57; m 1,30-1,44; m 1,02-1,14; m 0,86-1,00 [ppm] | 3,3 µM |
| 3-43 | 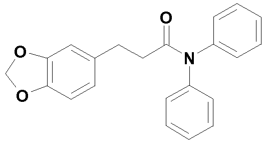 | ¹H-NMR (CDCl₃): m 7,00-7,43; d 6,69; m 6,55-6,60; s 5,87; tr 2,90; tr 2,52 [ppm] | 6,8 µM |
| 3-44 | 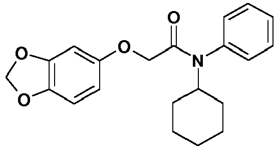 | Retentionszeit (HPLC): 10,96 min; | 1,0 µM |
| | | Methode 2 im Anhang | |
| 3-45 | 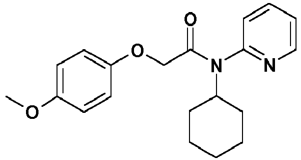 | ¹H-NMR (CDCl₃): s 8,56; tr 7,78; m m 7,28-7,36; d 7,17; d 6,76; d 6,70; d 1,92; d 1,73; d 1,58; m 1,29-1,42; m 1,14- 1,29; m 0,90-1,02 [ppm] | 4,0µM |
| 3-46 | 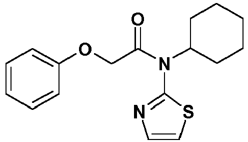 | Retentionszeit (HPLC): 9,86 min; | 3,0 µM |
| | | Methode 2 im Anhang | |
| 3-47 | 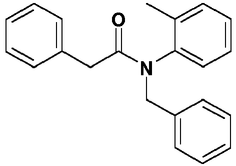 | Retentionszeit (HPLC): 11,63 min; | 1,7 µM |
| | | Methode 2 im Anhang | |
| 3-48 | 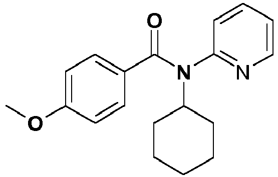 | ¹H-NMR (CDCl₃): s 8,47; tr 7,45; d 7,23; m 7,05-7,10; d 6,78; d 6,62; m 4,64-4,72; s 3,68; d 1,97; d 1,78; m 1,49-1,66; q 1,42; m 1,02-1,13 [ppm] | 10,9 µM |
| 3-49 | 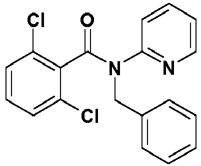 | Retentionszeit (HPLC): 9,40 min; | 5,5 µM |
| | | Methode 2 im Anhang | |

**HPLC-Methode 1**

| **Meth.** | **CBMPPA-A** | | | |
|---|---|---|---|---|
| Säule: | Luna C8(2), 150*3,0mm (Phenomenex) | | | |
| Vorsäule: | C18 ODS | | | |
| Temperatur: | 40 °C | | | |
| Flußrate: | 1,00 ml/min | | | |
| Injektionsvolumen: | 1,0 µl | | | |
| Detektion: | UV 264nm | | | |
| Stopzeit: | 4,0 Minuten | | | |
| Nachlaufzeit: | 0,0 Minuten | | | |
| Maximaldruck: | 300 bar | | | |
| Eluent A: | 10mM KH2PO4, pH2,5 | | | |
| Eluent B: | Acetonitril | | | |
| Gradient: | Zeit [min] | A [%] | B [%] | Fluß[ml/min] |
| | 0,0 | 75,0 | 25,0 | 1,00 |
| | 4,0 | 75,0 | 25,0 | 1,00 |
| Matrix: | Probe wird durch 0,22 µm filtriert. | | | |
| Kalibrierung: | mit Standard 10mmol/L in DMSO | | | |

**HPLC-Methode 2**

| **Meth.** | **TRPM8-A** | | | |
|---|---|---|---|---|
| Säule: | Luna C8(2), 150*3,0mm (Phenomenex) | | | |
| Vorsäule: | C18 ODS | | | |
| Temperatur: | 40 °C | | | |
| Flußrate: | 1,00 ml/min | | | |
| Injektionsvolumen: | 1,0 µl | | | |
| Detektion: | UV 210nm | | | |
| Stopzeit: | 17,0 Minuten | | | |
| Nachlaufzeit: | 3,0 Minuten | | | |
| Maximaldruck: | 300 bar | | | |
| Eluent A: | 10mM KH2PO4, pH2,5 | | | |
| Eluent B: | Acetonitril | | | |
| Gradient: | Zeit [min] | A [%] | B [%] | Fluß[ml/min] |
| | 0,0 | 95,0 | 5,0 | 1,00 |
| | 17,0 | 40,0 | 60,0 | 1,00 |
| Matrix: | Probe wird durch 0,22 µm filtriert. | | | |

### Testbeispiel 2

Evaluierung des zeitlichen Verlaufs der Kühlintensitäten der erfindungsgemäß einzusetzenden Verbindungen.

Verfahren: Die erfindungsgemäß einzusetzenden Kühlsubstanzen wurden in Zahnpasta gemäß Tabelle Z (siehe unten) eingearbeitet.

Die sensorischen Eigenschaften der resultierenden Zahnpasta wurden durch ein trainiertes Panel (von 6 Personen) evaluiert. Dazu wurden die Zähne mit der die erfindungsgemäßen Verbindungen enthaltenden Zahnpasta zunächst 30 sec geputzt, dann der Zahnpastaschaum ausgespuckt und der Mund einmal mit Wasser ausgespült. Die Testpersonen beurteilten die Stärke des Kältegefühls auf einer Scala von 0 (kein Kältegefühl) bis 9 (extrem starkes Kältegefühl). Die Beurteilung des Kältegefühls erfolgte jeweils nach 30 sec, 1, 5, 10, 20, 30, 45 und 60 min.

Als Verbindungen aus den Strukturklassen 1, 2 und 3 wurden die Verbindungen *2,3,4,5,6,10b,11,12-Octahydro-spiro[4b-azachrysen-12,2'-[1,3]dithiolan-] -1-on* ((**LN 1**)); *Isopropyl-(5-methoxy-2-pyridin-2-yl-pyrimidin-4-yl)-amin* ((**LN 9**)) und 3,4-*Methylendioxyzimtsäure-N,N-diphenylamid* ((**LN 24**))(erfindungsgemäß) getestet.

Zum Vergleich wurden Zahnpasten mit gleicher Zusammensetzung getestet, die als konventionelle Kühlsubstanz Menthan-3-carbonsäure-N-ethylamid ("WS 3", s.a. US 4.150.052) enthielten.

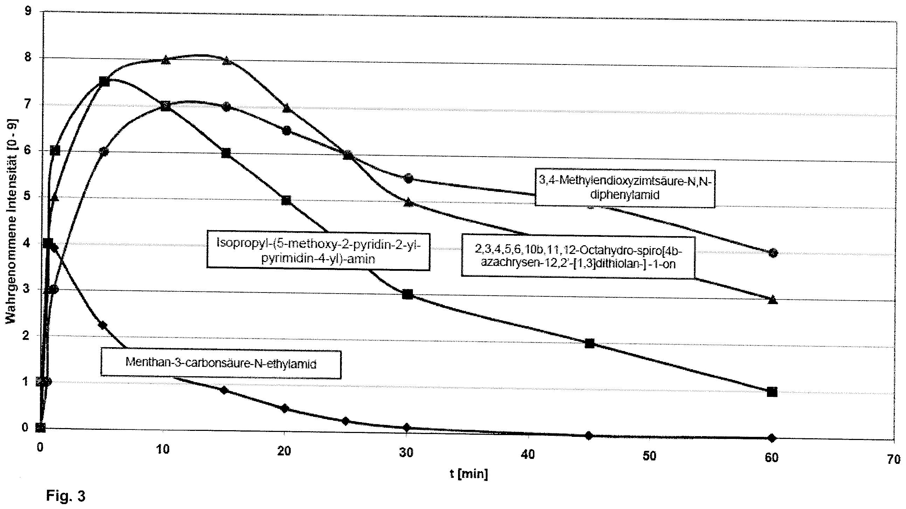

Das Ergebnis ist in Figur 3 dargestellt und zeigt, dass die Kühlsubstanzen den konventionell verwendeten sowohl von einer schnell wahrnehmbaren Intensität her als auch von der Dauer der Kühlwahrnehmung her deutlich überlegen sind (die Figur 1 stellt dabei wie wahrgenommene Kühlintensitäten der jeweiligen Kühlsubstanz bei Verwendung in einer aromatisierten Zahnpasta bei einer Dosierung von 100 ppm bezogen auf die gesamte Zahnpasta dar). So vermitteln die erfindungsgemäßen Kühlsubstanzen auch noch 1 h nach dem Zähneputzen ein sehr deutlich wahrnehmbares Kältegefühl, während es bei den konventionellen Vergleichsverbindung nach dieser Zeit schon ganz verschwunden ist.

**Tabelle Z**

| | | |
|---|---|---|
| **Entionisiertes Wasser** | **27,52** | |
| **Sorbitol 700/0** | **45** | |
| **Solbrol M Na-Salz** | **0.15** | |
| **Trinatriumphosphat** | **0,1** | |
| **Saccharin** | **0.2** | |
| **Natriummonofluorphosphat** | **1.12** | |
| **PEG 1500** | 5 | |
| **Sident 9 (Abrasive Silica)** | **10** | |
| **Sident 22 S (Dickende Silica)** | 8 | |
| **Natriumcarboxymethylcellulose** | **0.9** | |
| **Titan (IV) oxid** | **0.5** | |
| **Natriumlaurylsulfat (SLS)** | **1,5** | |
| **jeweilige Kühlsubstanz** | **0,01** | |
| | | |
| **Total** | | **100** |

| | | |
|---|---|---|
| Alle Angaben in Gew.-% | | |

## Patentansprüche

1. Nicht-therapeutisches Verfahren zur in-vitro Modulation des Kälte-Menthol-Rezeptors TRPM8, wobei man den Rezeptor mit wenigstens einem Modulator in Kontakt bringt, der ausgewählt ist unter Verbindungen des folgenden Strukturtyps 3:
Strukturtyp 3: 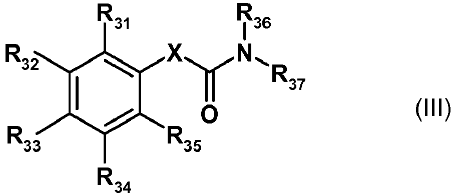 worin
R₃₁, R₃₂, R₃₃, R₃₄ und R₃₅ gleich oder verschieden sind und ausgewählt sind unter
H;
Halogen;
geradkettigen oder verzweigten C₁-C₆-Alkylgruppen die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen;
geradkettigen oder verzweigten C₁-C₆-Alkoxygruppen die gegebenenfalls 1, 2, 3 oder 4 gleiche oder verschiedene Substituenten tragen, die ausgewählt sind unter NH₂, OH, SH, Halogen oder C₁-C₆-Alkoxygruppen;
ein- oder mehrkernige Aryl-, Arylalkyl- und Heteroarylgruppen, die gegebenenfalls 1, 2, 3 oder 4 gleiche oder verschiedene Substituenten tragen, die ausgewählt sind unter NH₂, OH, SH, Halogen, geradkettigen oder verzweigten C₁-C₆-Alkylgruppen und geradkettigen oder verzweigten C₁-C₆-Alkyloxygruppen; wobei die Heteroarylgruppen 1, 2, 3 oder 4 Ring-Heteroatome aufweisen, die gleich oder verschieden sind und ausgewählt sind unter O, N und S; oder
zwei benachbarte Reste R₃₁, R₃₂, R₃₃, R₃₄ und R₃₅ zusammen mit den Kohlenstoffatomen, an die sie gebunden sind, einen 4-, 5-, 6- oder 7-gliedrigen, ein- oder mehrfach ungesättigten heterocyclischen Ring bilden, der gegebenenfalls 1, 2, 3, 4 oder 5 gleiche oder verschiedene Substituenten trägt, die ausgewählt sind unter geradkettigen oder verzweigten C₁-C₆-Alkylgruppen, und der 1, 2 oder 3 Ring-Heteroatome aufweist, die gleich oder verschieden sind und ausgewählt sind unter O, N und S;
R₃₆ und R₃₇ gleich oder verschieden sind und ausgewählt ist unter:
geradkettigen oder verzweigten C₁-C₆-Alkylgruppen die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen;
ein- oder mehrkernige Aryl-, Arylalkyl-, Aryloxy-, Heteroaryl- und Heteroaryloxygruppen, die gegebenenfalls 1, 2, 3 oder 4 gleiche oder verschiedene Substituenten tragen, die ausgewählt sind unter NH₂, OH, SH, Halogen, geradkettigen oder verzweigten C₁-C₆-Alkylgruppen und geradkettigen oder verzweigten C₁-C₆-Alkoxygruppen; wobei die Heteroarylgruppen 1, 2, 3 oder 4 Ring-Heteroatome aufweisen, die gleich oder verschieden sind und ausgewählt sind unter O, N und S;
und C₃-C₇-Cycloalkylgruppen, die gegebenenfalls 1, 2, 3 oder 4 gleiche oder verschiedene Substituenten tragen, die ausgewählt sind unter NH₂, OH, SH, Halogen, geradkettigen oder verzweigten C₁-C₆-Alkylgruppen, oder geradkettigen oder verzweigten C₁-C₆-Alkoxygruppen; wobei die Cycloalkylgruppe gegebenenfalls über eine C₁-C₄-Alkylengruppe gebunden ist; und wobei gegebenenfalls 1, 2 oder 3 Ring-Kohlenstoffatome durch gleiche oder verschiedene Heteroatome, ausgewählt unter O, N und S ersetzt sein können;
X ausgewählt ist unter
-C₁-C₄-Alkylengruppen; -C₂-C₄-Alkenylengruppen, sowie -Z-C₁-C₄- oder -C₁-C₄-Z- Alkylengruppen oder -Z-C₂-C₄- oder - C₂-C₄-Z-Alkenylengruppen, worin Z für O, S oder NH steht; oder für eine chemische Einfachbindung steht;
sowie Salze dieser Verbindungen, insbesondere Säureadditionssalze mit anorganischen oder insbesondere organischen, ein- oder insbesondere mehrwertigen Carbonsäuren;
und gegebenenfalls in Stereoisomeren-reiner Form oder als Gemisch von Stereoisomeren,
wobei die Maßgabe gilt, dass die Verbindung der Formel (III) nicht 1-(2-Aminoethyl)-1-(1,3-benzothiazol-2-yl)-3-[(1S)-1-phenylethyl]urea hydrochlorid, 1-(2-Aminoethyl)-1-(1,3-benzothiazol-2-yl)-3-[(1R)-1-phenylethyl]urea hydrochlorid, 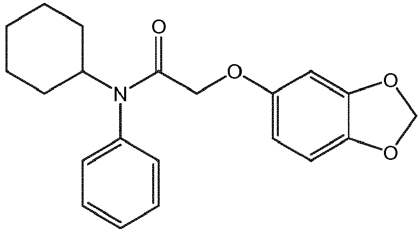 und 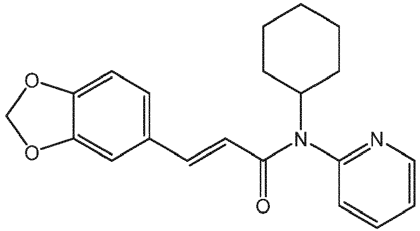 ist.

2. Verfahren nach Anspruch 1, wobei man den Rezeptor mit wenigstens einer Verbindung in Kontakt bringt, welche in einem zellulären Aktivitätstest unter Verwendung von Zellen, welche den humanen TRPM8-Rezeptor rekombinant exprimieren, die Permeabilität dieser Zellen für Ca²⁺ Ionen modulieren.

3. Verfahren nach Anspruch 1, wobei die modulierende Verbindung auf die zelluläre Ca2+-Ionen-Permeabilität agonistisch oder antagonistisch wirkt.

4. Verfahren nach einem der Ansprüche 1 bis 3, wobei die modulierende Verbindung ein TRPM8-Rezeptor-Agonist ist.

5. Verwendung einer Verbindung gemäß der Definition in einem der Ansprüche 1 bis 4, wobei zusätzlich die Maßgabe nicht gilt, dass die Verbindung der Formel (III) nicht 1-(2-Aminoethyl)-1-(1,3-benzothiazol-2-yl)-3-[(1S)-1-phenylethyl]urea hydrochlorid und 1-(2-Aminoethyl)-1-(1,3-benzothiazol-2-yl)-3-[(1R)-1-phenylethyl]urea hydrochlorid ist, zur nicht-therapeutischen Induktion von Kältegefühl bei Mensch und/oder Tier.

6. Verwendung einer Verbindung gemäß der Definition in einem der Ansprüche 1 bis 4, wobei zusätzlich die Maßgabe nicht gilt, dass die Verbindung der Formel (III) nicht 1-(2-Aminoethyl)-1-(1,3-benzothiazol-2-yl)-3-[(1S)-1-phenylethyl]urea hydrochlorid und 1-(2-Aminoethyl)-1-(1,3-benzothiazol-2-yl)-3-[(1R)-1-phenylethyl]urea hydrochlorid ist, zur nicht-therapeutischen Induktion eines Kältegefühls durch eine Verpackung oder zur nicht-therapeutischen Induktion eines Kältegefühls durch ein Textil.

7. Verwendung nach Anspruch 5, wobei ein Mittel eingesetzt wird, umfassend wenigstens eine, zwei, drei oder mehr der Verbindungen gemäß der Definitionen aus einem der Ansprüche 1 bis 4 und ausgewählt aus der Gruppe A bestehend aus
| | |
|---|---|
| | |
| 24 | 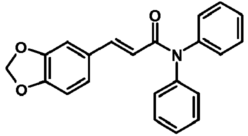 |
| 25 | 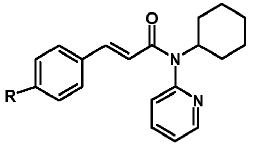 mit R = H |
| 26 | 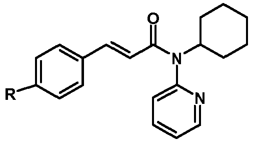 mit R = Me |
| 27 | 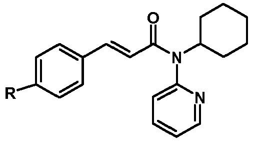 mit R = Meo |
| 28 | 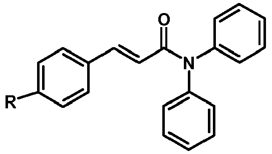 mit R = H |
| 29 | 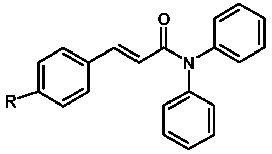 mit R = Me |
| 30 | 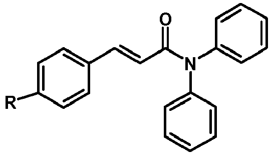 mit R = MeO |
in einer Konzentration von 0,1 ppm bis 10 Gew-% bezogen auf das Gesamtgewicht des Mittels zum Erzielen einer Kühlwirkung auf Haut oder Schleimhaut, die verglichen mit der Kühlwirkung eines Mittels gleicher Zusammensetzung, bei dem lediglich die Verbindung oder die Verbindungen ausgewählt aus der Gruppe A gegen Menthancarbonsäure-N-ethylamid in gleicher Konzentration ausgetauscht sind, um wenigstens 10 Minuten verlängert ist, zu nicht-therapeutischen Zwecken.

8. Verbindung gemäß der Definition nach einem der Ansprüche 1 bis 4, mit der Maßgabe, dass die Verbindung nicht Diethyltoluamid, 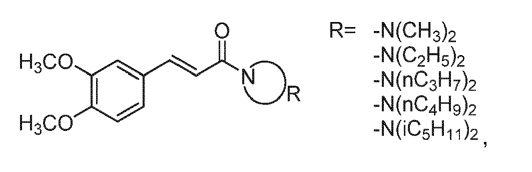 S-4-Chlorobenzyl-diethyl-thiocarbamat 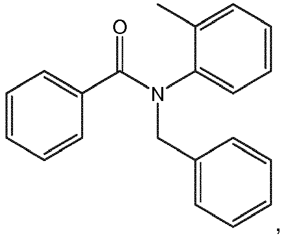 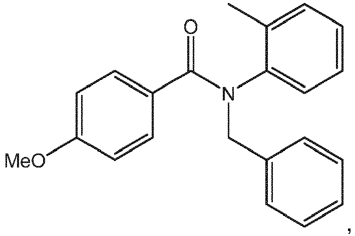 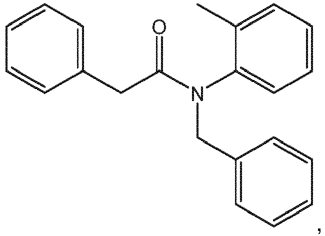 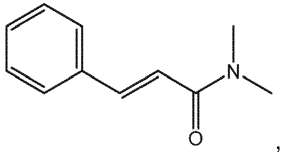 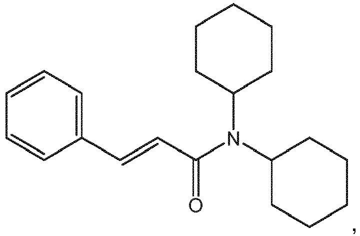 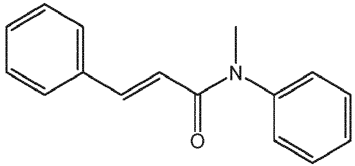 und 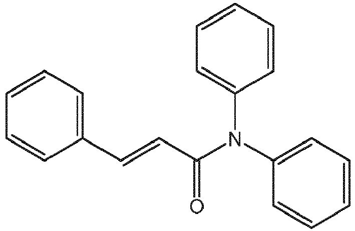 ist.

9. Mittel enthaltend wenigstens eine Verbindung nach Anspruch 8.

10. Mittel enthaltend wenigstens eine Verbindung, gemäß der Definition nach einem der Ansprüche 1 bis 4, wobei zusätzlich die Maßgabe nicht gilt, dass die Verbindung der Formel (III) nicht 1-(2-Aminoethyl)-1-(1,3-benzothiazol-2-yl)-3-[(1S)-1-phenylethyl]urea hydrochlorid und 1-(2-Aminoethyl)-1-(1,3-benzothiazol-2-yl)-3-[(1R)-1-phenylethyl]urea hydrochlorid ist,
zur Verwendung als Mediator des TRPM8-Rezeptors ausgewählt unter
a) Nahrungsmitteln, wie Speiseeis, Mousse, Creme, Getränke, Süßwaren,
b) Mundpflegemitteln, wie Zahnpasta, Mundwasser, Kaugummi,
c) Körperpflegemitteln, wie Haut- oder Haarpflegemitteln, wie Sonnencreme, Sonnenbrandcreme, Lotionen, Shampoos, Pflaster,
d) Schäumen und Gelen.

11. Mittel nach Anspruch 10 oder Mittel enthaltend 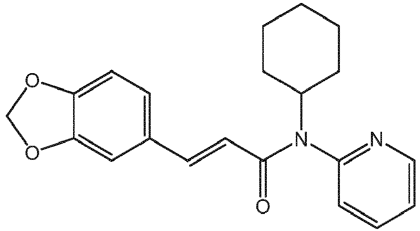 zur Verwendung als Mediator des TRPM8-Rezeptors
ausgewählt aus der Gruppe bestehend aus Aromamischung und der Ernährung, der Mundhygiene oder dem Genuss dienende kosmetische Zubereitung, umfassend eine, zwei, drei oder mehr der Verbindungen gemäß der Definitionen aus einem der Ansprüche 1-4, die ausgewählt ist oder ausgewählt sind (i) aus der Gruppe B bestehend aus
| | |
|---|---|
| 24 | 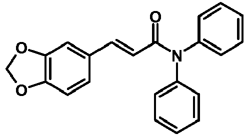 |
| 25 | 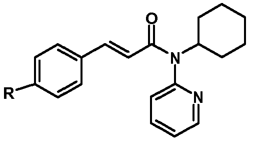 mit R = H |
| 26 | 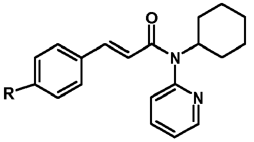 mit R = Me |
| 27 | 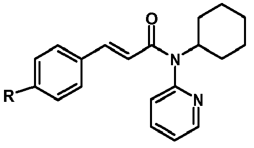 mit R = MeO |
| 28 | 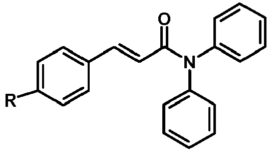 mit R = H |
| 29 | 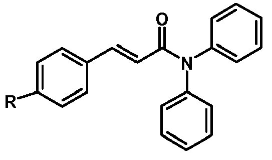 mit R = Me |
| 30 | 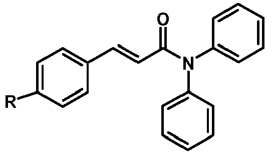 mit R = MeO |
und/oder die ausgewählt ist (ii) aus der Gruppe C bestehend aus
| LN | Struktur |
|---|---|
| 23 | 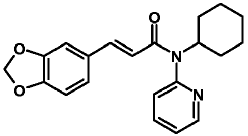 |
, wobei die Verbindung der Gruppe C in einer Konzentration von 0,05 bis < 0,1 ppm oder 0,1 ppm bis 10 Gew-% bezogen auf das Gesamtgewicht der Zubereitung enthalten ist, mit der Maßgabe, dass im Falle (ii) die Zubereitung kein Mundwasser mit der Zusammensetzung von jeweils bezogen auf einen Liter
| | |
|---|---|
| Ethanol 95% | 177mL |
| Sorbit 70% | 250 g |
| Verbindung der Formel 23 als 1%ige Lösung in Ethanol | 50mL |
| Pfefferminzöl, | 0.30 g |
| Methylsalicylat | 0.64 g |
| Eucalyptol | 0.922 g |
| Thymol | 0.639 g |
| Benzoesäure | 1.50 g |
| Pluronic ® F127 nichtionisches Tensid | 5.00 g |
| Natrium-Saccharin | 0.60 g |
| Natriumcitrat | 0.30 g |
| Zitronensäure | 0.10 g |
| Wasser q.s. | 1 Liter |
ist.

12. Mittel nach Anspruch 11, umfassend
(1) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
und/oder
(2) einen oder mehrere Aromastoffe ohne physiologische Kühlwirkung und/oder
(3) einen oder mehrere trigeminal oder mundwässernd wirksame Stoffe ohne physiologische Kühlwirkung
und/oder
(4) (iii) eine oder (iv) mehrere Verbindungen, die im Falle (iv) unabhängig voneinander oder gemeinsam zusätzlich einen geschmacksmodulierenden Effekt und/oder einen trigeminalen und/oder einen mundwässernden Reiz verursachen.

13. Produkt enthaltend wenigstens eine Verbindung gemäß der Definition nach einem der Ansprüche 1 bis 4, wobei zusätzlich die Maßgabe nicht gilt, dass die Verbindung der Formel (III) nicht 1-(2-Aminoethyl)-1-(1,3-benzothiazol-2-yl)-3-[(1S)-1-phenylethyl]urea hydrochlorid und 1-(2-Aminoethyl)-1-(1,3-benzothiazol-2-yl)-3-[(1R)-1-phenylethyl]urea hydrochlorid ist,
zur Verwendung als Mediator des TRPM8-Rezeptors
ausgewählt unter
a) Textilprodukten,
b) Verpackungsmaterialien,
c) Tabakprodukten;
d) Hygieneprodukten, oder
e) Erfrischungstüchern.

14. Verbindung nach Anspruch 8, ausgewählt unter
| Nr. | Struktur-Typ 3 |
|---|---|
| 3-2 | 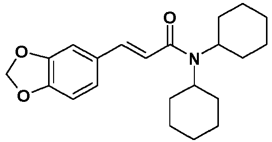 |
| 3-3 | 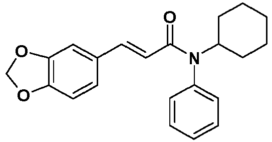 |
| 3-4 | 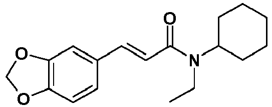 |
| 3-5 | 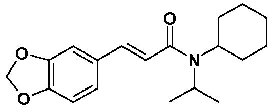 |
| 3-6 | 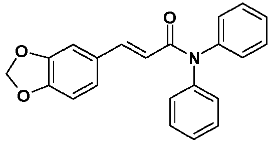 |
| 3-7 | 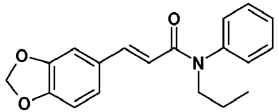 |
| 3-8 | 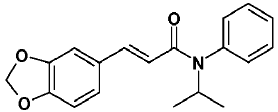 |
| 3-9 | 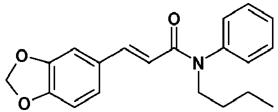 |
| 3-10 | 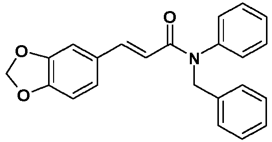 |
| 3-11 | 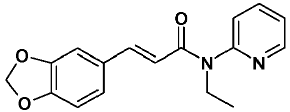 |
| 3-12 | 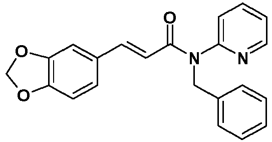 |
| 3-13 | 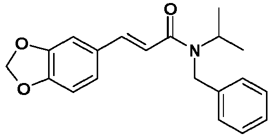 |
| 3-14 | 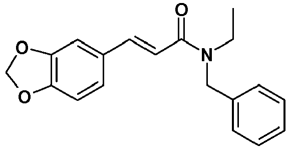 |
| 3-15 | 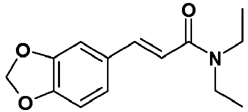 |
| 3-16 | 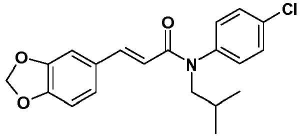 |
| 3-17 | 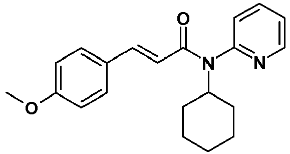 |
| 3-18 | 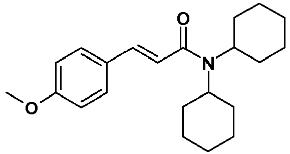 |
| 3-19 | 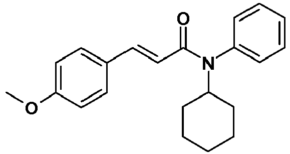 |
| 3-20 | 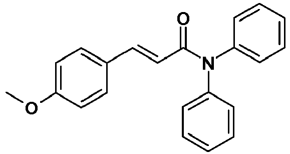 |
| 3-21 | 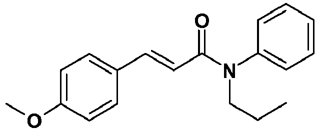 |
| 3-22 | 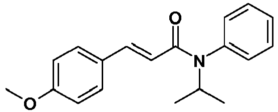 |
| 3-23 | 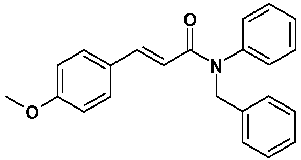 |
| 3-24 | 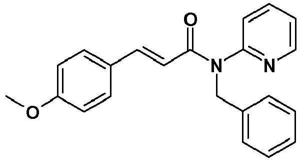 |
| 3-25 | 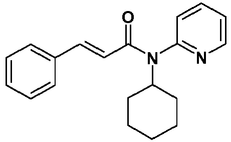 |
| 3-26 | 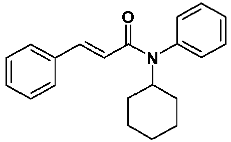 |
| 3-29 | 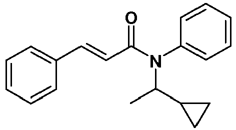 |
| 3-30 | 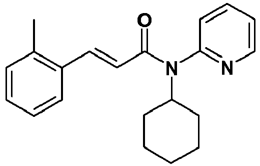 |
| 3-31 | 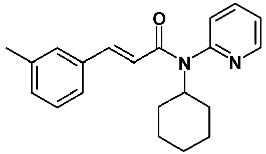 |
| 3-32 | 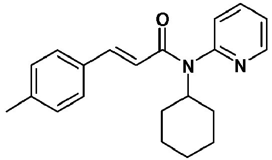 |
| 3-33 | 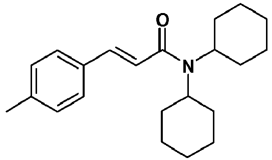 |
| 3-34 | 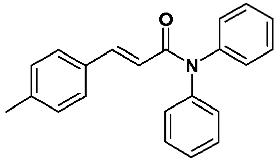 |
| 3-25 | 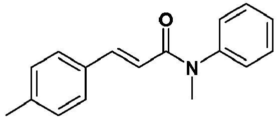 |
| 3-36 | 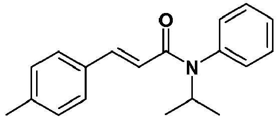 |
| 3-37 | 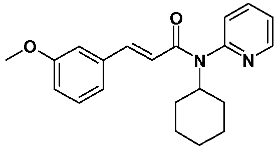 |
| 3-38 | 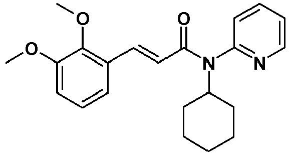 |
| 3-39 | 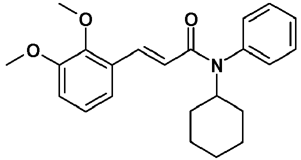 |
| 3-40 | 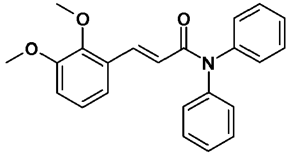 |
| 3-41 | 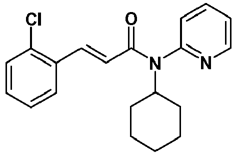 |
| 3-42 | 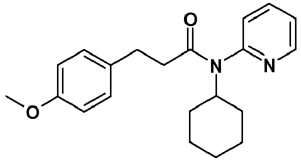 |
| 3-43 | 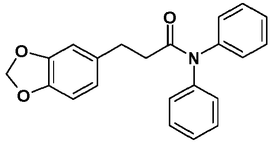 |
| 3-45 | 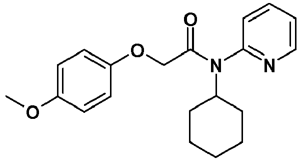 |
| 3-46 | 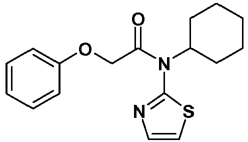 |
| 3-48 | 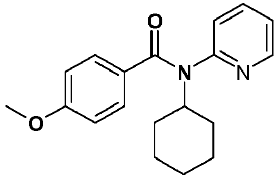 |
| 3-49 | 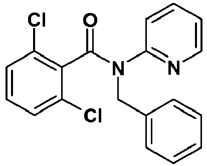 |
gegebenenfalls in stereoisomerenreiner Form, als Gemisch von Stereoisomeren, sowie Salze dieser Verbindungen.

## Claims

1. Non-therapeutic method for *in vitro* modulation of the cold and menthol receptor TRPM8, in which the receptor is brought into contact with at least one modulator chosen from compounds of the following structural type 3:
structural type 3: 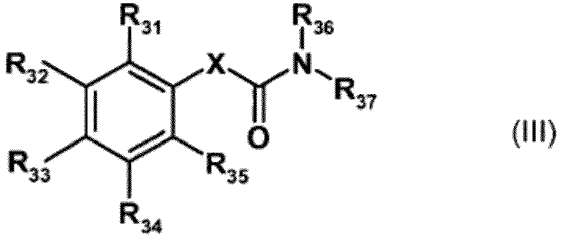 in which:
R₃₁, R₃₂, R₃₃, R₃₄ and R₃₅ are identical or different and chosen from:
H;
halogen;
straight-chain or branched C₁-C₆-alkyl groups which optionally carry 1, 2, 3 or 4 identical or different substituents chosen from NH₂, OH, SH, halogen or straight-chain or branched C₁-C₆-alkoxy groups;
straight-chain or branched C₁-C₆-alkoxy groups which optionally carry 1, 2, 3 or 4 identical or different substituents chosen from NH₂, OH, SH, halogen or C₁-C₆-alkoxy groups;
mono- or polynuclear aryl, arylalkyl or heteroaryl groups which optionally carry 1, 2, 3 or 4 identical or different substituents chosen from NH₂, OH, SH, halogen, straight-chain or branched C₁-C₆-alkyl groups and straight-chain or branched C₁-C₆-alkyloxy groups; the heteroaryl groups exhibiting 1, 2, 3 or 4 ring heteroatoms which are identical or different and chosen from O, N and S; or
two neighbouring radicals R₃₁, R₃₂, R₃₃, R₃₄ and R₃₅, together with the carbon atoms to which they are bonded, form a 4-, 5-, 6- or 7-membered mono- or polyunsaturated heterocyclic ring which optionally carries 1, 2, 3, 4 or 5 identical or different substituents chosen from straight-chain or branched C₁-C₆-alkyl groups and which exhibits 1, 2 or 3 ring heteroatoms which are identical or different and chosen from O, N and S;
R₃₆ and R₃₇ are identical or different and chosen from:
straight-chain or branched C₁-C₆-alkyl groups which optionally carry 1, 2, 3 or 4 identical or different substituents chosen from NH₂, OH, SH, halogen or straight-chain or branched C₁-C₆-alkoxy groups;
mono- or polynuclear aryl, arylalkyl, aryloxy, heteroaryl and heteroaryloxy groups which optionally carry 1, 2, 3 or 4 identical or different substituents chosen from NH₂, OH, SH, halogen, straight-chain or branched C₁-C₆-alkyl groups and straight-chain or branched C₁-C₆-alkoxy groups; the heteroaryl groups exhibiting 1, 2, 3 or 4 ring heteroatoms which are identical or different and chosen from O, N and S;
and C₃-C₇-cycloalkyl groups which optionally carry 1, 2, 3 or 4 identical or different substituents chosen from NH₂, OH, SH, halogen, straight-chain or branched C₁-C₆-alkyl groups or straight-chain or branched C₁-C₆-alkoxy groups; the cycloalkyl group optionally being bonded via a C₁-C₄-alkylene group; and it optionally being possible for 1, 2 or 3 ring carbon atoms to be replaced by identical or different heteroatoms chosen from O, N and S;
X is chosen from:
-C₁-C₄-alkylene groups; -C₂-C₄-alkenylene groups, and also -Z-C₁-C₄- or -C₁-C₄-Z-alkylene groups or -Z-C₂-C₄- or -C₂-C₄-Z-alkenylene groups, in which Z stands for O, S or NH; or stands for a single chemical bond;
and also salts of these compounds, in particular acid addition salts with inorganic or in particular organic mono- or in particular polyvalent carboxylic acids;
and optionally in stereoisomerically pure form or as a mixture of stereoisomers,
with the proviso that the compound of the formula (III) is not 1-(2-aminoethyl)-1-(1,3-benzothiazol-2-yl)-3-[(1S)-1-phenylethyl]urea hydrochloride, 1-(2-aminoethyl)-1-(1,3-benzothiazol-2-yl)-3-[(1R)-1-phenylethyl]urea hydrochloride, 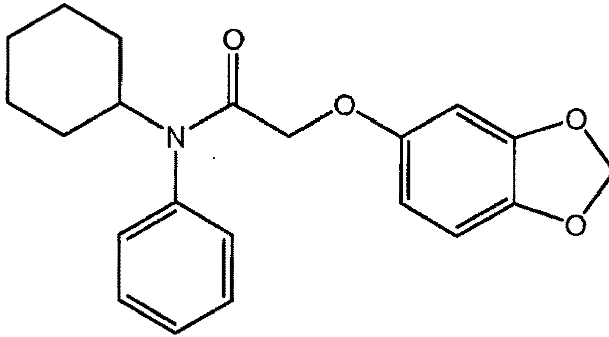 and

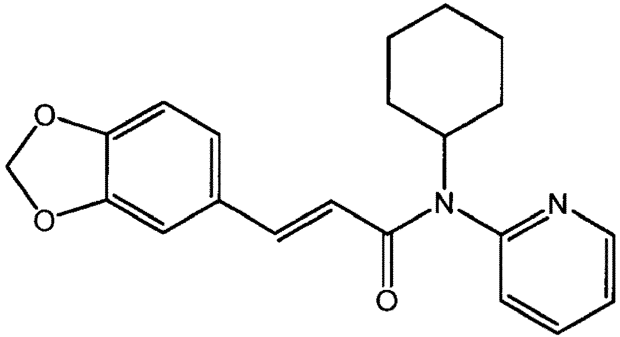

2. Method according to Claim 1, in which the receptor is brought into contact with at least one compound which modulates, in a cellular activity test using cells which recombinantly express the human TRPM8 receptor, the permeability of these cells for Ca²⁺ ions.

3. Method according to Claim 1, in which the modulating compound acts agonistically or antagonistically on the cellular Ca²⁺-ion permeability.

4. Method according to one of Claims 1 to 3, wherein the modulating compound is a TRPM8 receptor agonist.

5. Use of a compound according to the definition in one of Claims 1 to 4, in which in addition the proviso, that the compound of the formula (III) is not 1-(2-aminoethyl)-1-(1,3-benzothiazol-2-yl)-3-[(1S)-1-phenylethyl]urea hydrochloride and 1-(2-aminoethyl)-1-(1,3-benzothiazol-2-yl)-3-[(1R)-1-phenylethyl]urea hydrochloride, is not valid for the non-therapeutic induction of cold sensation in man and/or animals.

6. Use of a compound according to the definition in one of Claims 1 to 4, in which in addition the proviso, that the compound of the formula (III) is not 1-(2-aminoethyl)-1-(1,3-benzothiazol-2-yl)-3-[(1S)-1-phenylethyl]urea hydrochloride and 1-(2-aminoethyl)-1-(1,3-benzothiazol-2-yl)-3-[(1R)-1-phenylethyl]urea hydrochloride, is not valid for the non-therapeutic induction of a cold sensation through a packaging or for the non-therapeutic induction of a cold sensation through a textile.

7. Use according to Claim 5, in which use is made of a composition comprising at least one, two, three or more of the compounds according to the definitions from one of Claims 1 to 4 and chosen from the group A consisting of:
| | |
|---|---|
| 24 | 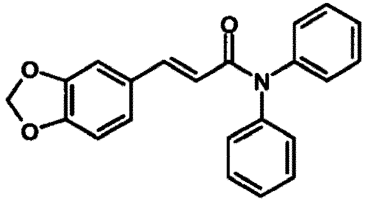 |
| 25 | 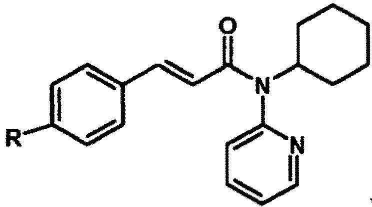 with R = M |
| 26 | 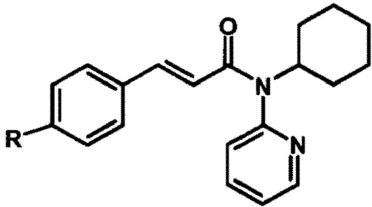 with R = Me |
| 27 | 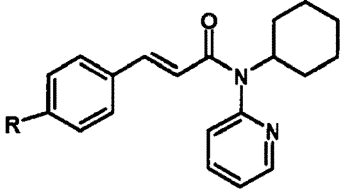 with R = Meo |
| 28 | 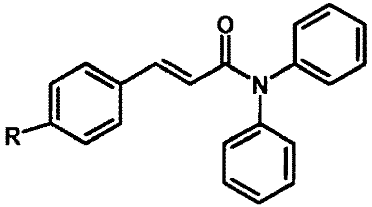 with R = H |
| 29 | 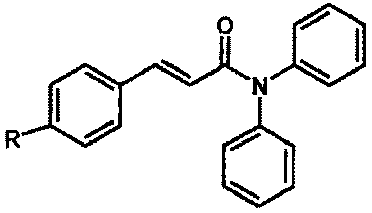 with R = Me |
| 30 | 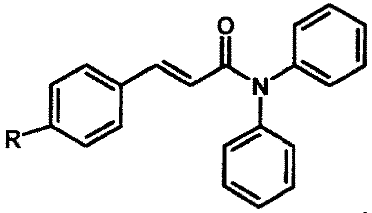 with R = Meo |
in a concentration of 0.1 ppm to 10% by weight, based on the total weight of the composition, for the targeting of a cooling effect on the skin or mucous membrane which, compared with the cooling effect of a composition of the same composition in which the compound or the compounds chosen from the group A are simply exchanged for N-ethylmenthane-carboxamide in an identical concentration, is lengthened by at least 10 minutes, for non-therapeutic purposes.

8. Compound according to the definition according to one of Claims 1 to 4, with the proviso that the compound is not diethyltoluamide, 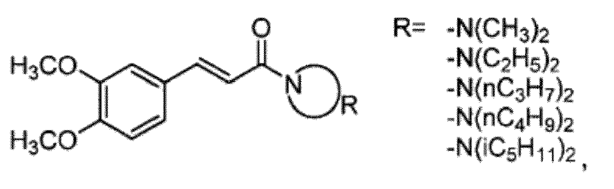 S-4-chlorobenzyl diethylthiocarbamate 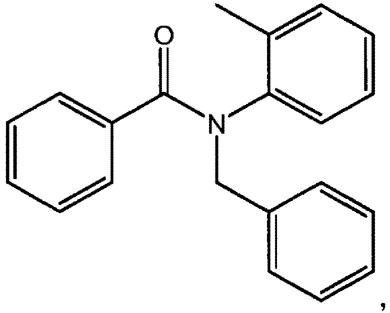 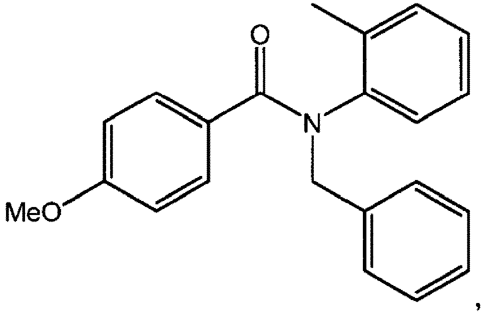 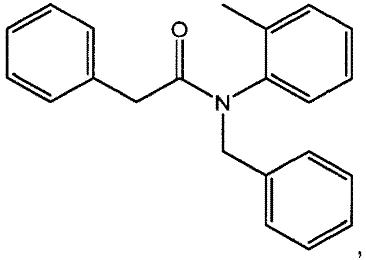 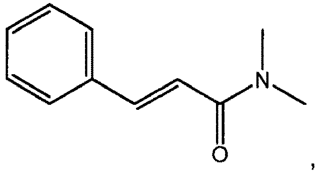 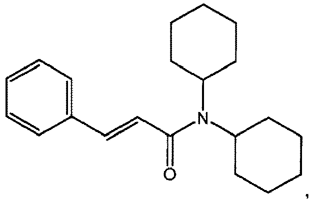 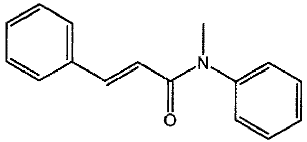 and

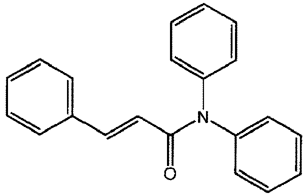

9. Composition comprising at least one compound according to Claim 8.

10. Composition comprising at least one compound according to the definition according to one of Claims 1 to 4, in which in addition the proviso, that the compound of the formula (III) is not 1-(2-aminoethyl)-1-(1,3-benzothiazol-2-yl)-3-[(1S)-1-phenylethyl]urea hydrochloride and 1-(2-aminoethyl)-1-(1,3-benzothiazol-2-yl)-3-[(1R)-1-phenylethyl]urea hydrochloride, is not valid,
for use as mediator of the TRPM8 receptor chosen from:
a) foods, such as ice cream, mousse, cream, beverages, confectionary;
b) oral hygiene compositions, such as toothpaste, mouthwash, chewing gum,
c) body care compositions, such as skin or hair care compositions, such as sun cream, sunburn cream, lotions, shampoos, plasters,
d) foams and gels.

11. Composition according to Claim 10 or composition comprising 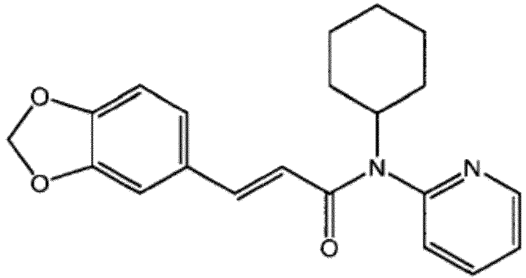 for use as mediator of the TRPM8 receptor,
chosen from the group consisting of aroma mixture and cosmetic preparation used for nutrition, oral hygiene or enjoyment comprising one, two, three or more of the compounds according to the definitions from one of Claims 1-4 which is chosen or are chosen (i) from the group B consisting of:
| | |
|---|---|
| 24 | 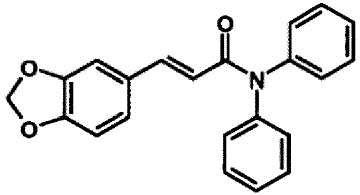 |
| 25 | 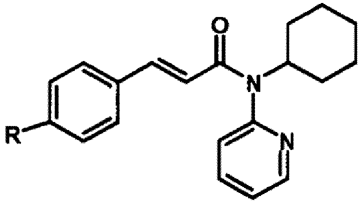 with R = H |
| 26 | 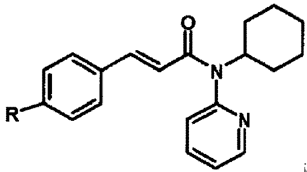 with R = Me |
| 27 | 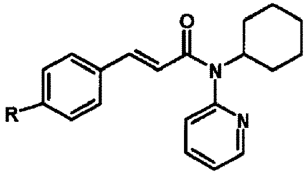 with R = MeO |
| 28 | 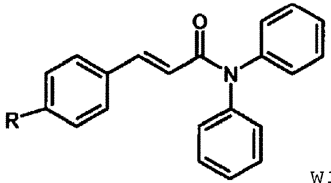 with R = H |
| 29 | 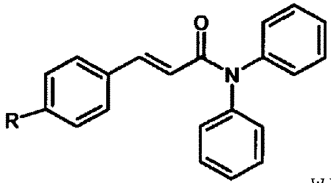 with R = Me |
| 30 | 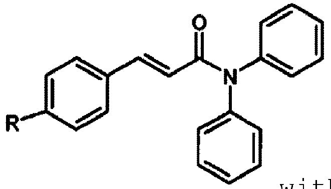 with R = MeO |
and/or which is chosen (ii) from the group C consisting of:
| LN | Structure |
|---|---|
| 23 | 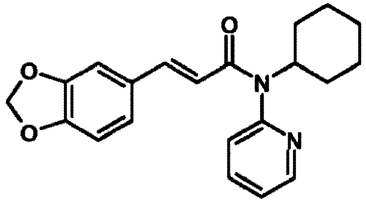 |
, the compound of group C being present in a concentration of 0.05 to < 0.1 ppm or 0.1 ppm to 10% by weight, based on the total weight of the preparation, with the proviso that, in the case (ii), the preparation is not a mouthwash with the ingredients of, in each case based on a litre:
| | |
|---|---|
| 95% Ethanol | 177 ml |
| 70% Sorbitol | 250 g |
| Compound of the formula 23 as 1% solution in ethanol | 50 ml |
| Peppermint oil | 0.30 g |
| Methyl salicylate | 0.64 g |
| Eucalyptol | 0.922 g |
| Thymol | 0.639 g |
| Benzoic acid | 1.50 g |
| Pluronic® F127, Non-ionic surfactant | 5.00 g |
| Sodium saccharin | 0.60 g |
| Sodium citrate | 0.30 g |
| Citric acid | 0.10 g |
| Water q.s. | 1 litre. |

12. Composition according to Claim 11, comprising:
(1) one or more additional substances with a physiological cooling effect, the additional substance or one, more or all of the additional substances (i) causing an effect with respect to taste or (ii) not causing an effect with respect to taste,
and/or
(2) one or more aroma substances without physiological cooling effect,
and/or
(3) one or more substances which are effective trigeminally or with respect to mouth washing without physiological cooling effect,
and/or
(4) (iii) one or (iv) more compounds which, in the case (iv), independently of one another or together, in addition cause a taste-modulating effect and/or a trigeminal and/or a mouth-washing stimulus.

13. Product comprising at least one compound according to the definition according to one of Claims 1 to 4, in which in addition the proviso, that the compound of the formula (III) is not 1-(2-aminoethyl)-1-(1,3-benzothiazol-2-yl)-3-[(1S)-1-phenylethyl]urea hydrochloride and 1-(2-aminoethyl)-1-(1,3-benzothiazol-2-yl)-3-[(1R)-1-phenylethyl]urea hydrochloride, is not valid,
for use as mediator of the TRPM8 receptor,
chosen from:
a) textile products,
b) packaging materials,
c) tobacco products,
d) hygiene products, or
e) tissue wipes.

14. Compound according to Claim 8, chosen from:
| No. | Structural type 3 |
|---|---|
| 3-2 | 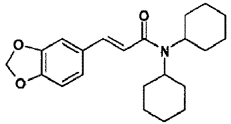 |
| 3-3 | 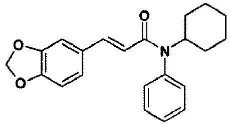 |
| 3-4 | 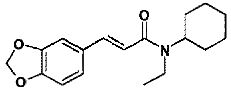 |
| 3-5 | 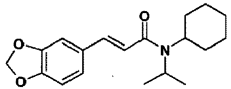 |
| 3-6 | 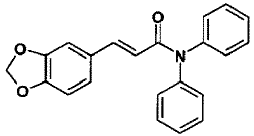 |
| 3-7 | 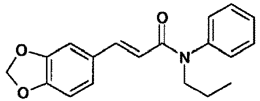 |
| 3-8 | 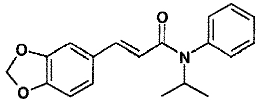 |
| 3-9 | 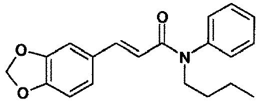 |
| 3-10 | 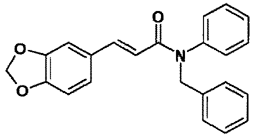 |
| 3-11 | 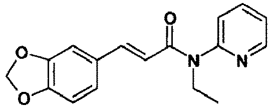 |
| 3-12 | 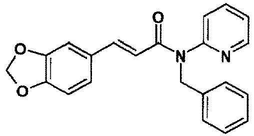 |
| 3-13 | 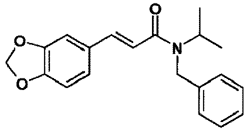 |
| 3-14 | 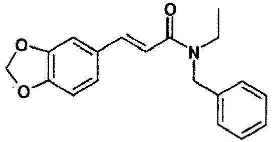 |
| 3-15 | 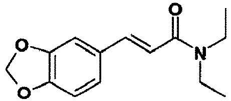 |
| 3-16 | 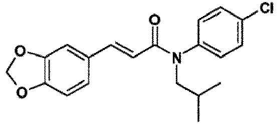 |
| 3-17 | 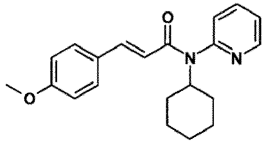 |
| 3-18 | 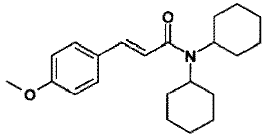 |
| 3-19 | 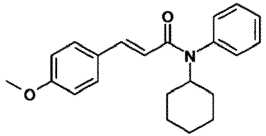 |
| 3-20 | 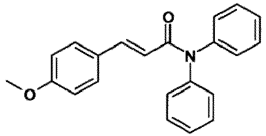 |
| 3-21 | 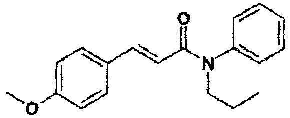 |
| 3-22 | 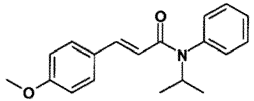 |
| 3-23 | 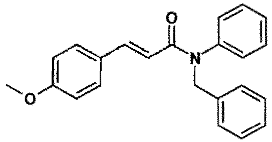 |
| 3-24 | 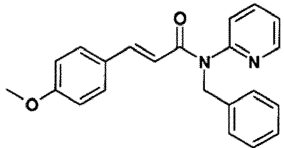 |
| 3-25 | 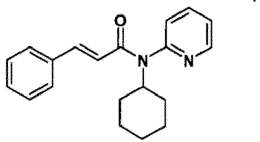 |
| 3-26 | 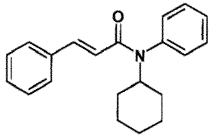 |
| 3-29 | 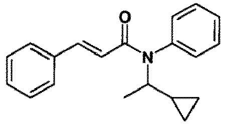 |
| 3-30 | 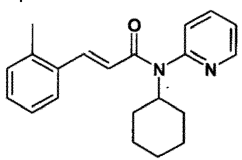 |
| 3-31 | 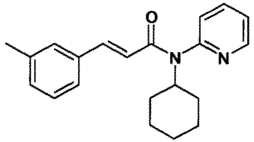 |
| 3-32 | 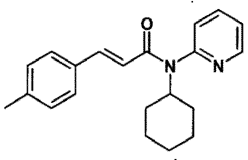 |
| 3-33 | 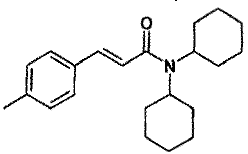 |
| 3-34 | 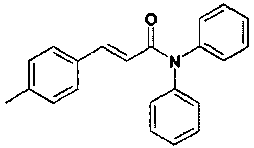 |
| 3-25 | 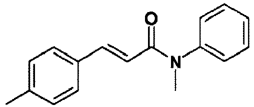 |
| 3-36 | 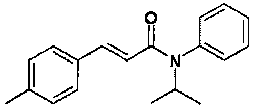 |
| 3-37 | 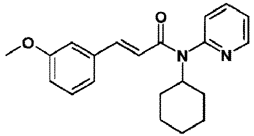 |
| 3-38 | 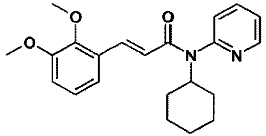 |
| 3-39 | 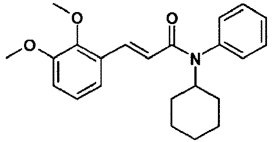 |
| 3-40 | 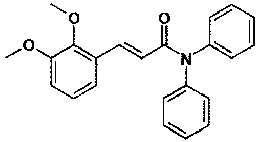 |
| 3-41 | 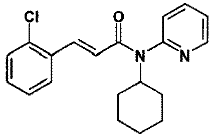 |
| 3-42 | 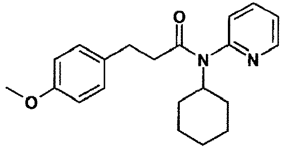 |
| 3-43 | 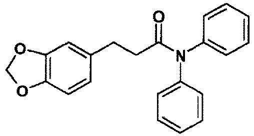 |
| 3-45 | 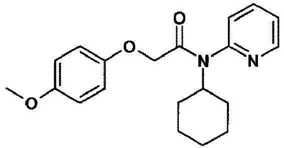 |
| 3-46 | 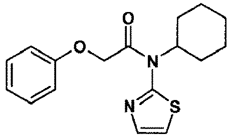 |
| 3-48 | 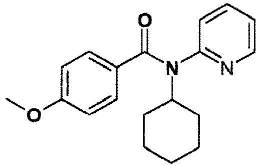 |
| 3-49 | 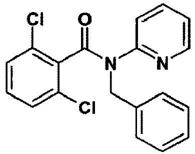 |
optionally in stereoisomerically pure form, as mixture of stereoisomers, and also salts of these compounds.

## Revendications

1. Procédé non thérapeutique pour la modulation in vitro du récepteur sensible au froid et au menthol TRPM8, selon lequel le récepteur est mis en contact avec au moins un modulateur, qui est choisi parmi les composés du type structural 3 suivant :
type structural 3 : 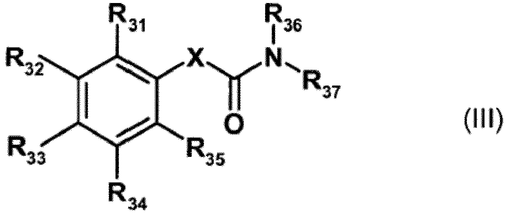 dans lequel
R₃₁, R₃₂, R₃₃, R₃₄ et R₃₅ sont identiques ou différents, et sont choisis parmi :
H ;
halogène ;
les groupes alkyle en C₁-C₆ linéaires ou ramifiés, qui portent éventuellement 1, 2, 3 ou 4 substituants identiques ou différents, qui sont choisis parmi NH₂, OH, SH, halogène ou les groupes alcoxy en C₁-C₆ linéaires ou ramifiés ;
les groupes alcoxy en C₁-C₆ linéaires ou ramifiés, qui portent éventuellement 1, 2, 3 ou 4 substituants identiques ou différents, qui sont choisis parmi NH₂, OH, SH, halogène ou les groupes alcoxy en C₁-C₆ ;
les groupes aryle, arylalkyle et hétéroaryle mono- ou polynucléaires, qui portent éventuellement 1, 2, 3 ou 4 substituants identiques ou différents, qui sont choisis parmi NH₂, OH, SH, halogène, les groupes alkyle en C₁-C₆ linéaires ou ramifiés, et les groupes alkyloxy en C₁-C₆ linéaires ou ramifiés ; les groupes hétéroaryle comprenant 1, 2, 3 ou 4 hétéroatomes de cycle, qui sont identiques ou différents, et sont choisis parmi O, N et S ;
ou
deux radicaux R₃₁, R₃₂, R₃₃, R₃₄ et R₃₅ voisins forment conjointement avec les atomes de carbone auxquels ils sont reliés un cycle hétérocyclique mono- ou polyinsaturé à 4, 5, 6 ou 7 chaînons, qui porte éventuellement 1, 2, 3, 4 ou 5 substituants identiques ou différents, qui sont choisis parmi les groupes alkyle en C₁-C₆ linéaires ou ramifiés, et qui comprend 1, 2 ou 3 hétéroatomes de cycle, qui sont identiques ou différents, et qui sont choisis parmi O, N et S ;
R₃₆ et R₃₇ sont identiques ou différents, et sont choisis parmi :
les groupes alkyle en C₁-C₆ linéaires ou ramifiés, qui portent éventuellement 1, 2, 3 ou 4 substituants identiques ou différents, qui sont choisis parmi NH₂, OH, SH, halogène ou les groupes alcoxy en C₁-C₆ linéaires ou ramifiés ;
les groupes aryle, arylalkyle, aryloxy, hétéroaryle et hétéroaryloxy mono- ou polynucléaires, qui portent éventuellement 1, 2, 3 ou 4 substituants identiques ou différents, qui sont choisis parmi NH₂, OH, SH, halogène, les groupes alkyle en C₁-C₆ linéaires ou ramifiés, et les groupes alcoxy en C₁-C₆ linéaires ou ramifiés ; les groupes hétéroaryle comprenant 1, 2, 3 ou 4 hétéroatomes de cycle, qui sont identiques ou différents, et sont choisis parmi O, N et S ;
et les groupes cycloalkyle en C₃-C₇, qui portent éventuellement 1, 2, 3 ou 4 substituants identiques ou différents, qui sont choisis parmi NH₂, OH, SH, halogène, les groupes alkyle en C₁-C₆ linéaires ou ramifiés, ou les groupes alcoxy en C₁-C₆ linéaires ou ramifiés ; le groupe cycloalkyle étant éventuellement relié par un groupe alkylène en C₁-C₄, et 1, 2 ou 3 atomes de carbone de cycle pouvant éventuellement être remplacés par des hétéroatomes identiques ou différents, choisis parmi O, N et S ;
X est choisi parmi :
les groupes alkylène en C₁-C₄ ; les groupes alcénylène en C₂-C₄, ainsi que les groupes -Z-alkylène en C₁-C₄ ou alkylène en C₁-C₄-Z, ou les groupes -Z-alcénylène en C₂-C₄ ou alcénylène en C₂-C₄-Z, Z représentant O, S ou NH ; ou une simple liaison chimique ;
ainsi que les sels de ces composés, notamment les sels d'addition acide avec des acides carboxyliques inorganique ou notamment organiques, mono- ou notamment polyvalents ;
et éventuellement sous forme stéréoisomériquement pure ou sous la forme d'un mélange de stéréoisomères,
à condition que le composé de formule (III) ne soit pas le chlorhydrate de 1-(2-aminoéthyl)-1-(1,3-benzothiazol-2-yl)-3-[(1S)-1-phényléthyl]urée, ni le chlorhydrate de 1-(2-aminoéthyl)-1-(1,3-benzothiazol-2-yl)-3-[(1R)-1-phényléthyl]urée, 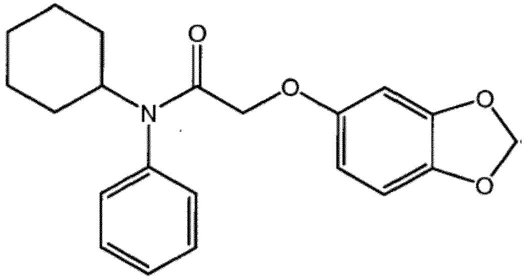 et

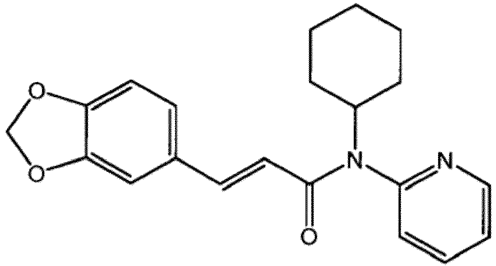

2. Procédé selon la revendication 1, selon lequel le récepteur est mis en contact avec au moins un composé qui, lors d'un test d'activité cellulaire utilisant des cellules qui expriment le récepteur TRPM8 humain de manière recombinante, module la perméabilité de ces cellules pour les ions Ca²⁺.

3. Procédé selon la revendication 1, selon lequel le composé modulateur agit sur la perméabilité cellulaire des ions Ca²⁺ de manière agonistique ou antagonistique.

4. Procédé selon l'une quelconque des revendications 1 à 3, selon lequel le composé modulateur est un agoniste du récepteur TRPM8.

5. Utilisation d'un composé selon la définition dans l'une quelconque des revendications 1 à 4, la condition selon laquelle le composé de formule (III) n'est pas le chlorhydrate de 1-(2-aminoéthyl)-1-(1,3-benzothiazol-2-yl)-3-[(1S)-1-phényléthyl]urée, ni le chlorhydrate de 1-(2-aminoéthyl)-1-(1,3-benzothiazol-2-yl)-3-[(1R)-1-phényléthyl]urée ne s'appliquant pas, pour l'induction non thérapeutique d'une sensation de froid chez un être humain et/ou un animal.

6. Utilisation d'un composé selon la définition dans l'une quelconque des revendications 1 à 4, la condition selon laquelle le composé de formule (III) n'est pas le chlorhydrate de 1-(2-aminoéthyl)-1-(1,3-benzothiazol-2-yl)-3-[(1S)-1-phényléthyl]urée, ni le chlorhydrate de 1-(2-aminoéthyl)-1-(1,3-benzothiazol-2-yl)-3-[(1R)-1-phényléthyl]urée ne s'appliquant en outre pas, pour l'induction non thérapeutique d'une sensation de froid au travers d'un emballage ou pour l'induction non thérapeutique d'une sensation de froid au travers d'un textile.

7. Utilisation selon la revendication 5, dans laquelle un agent est utilisé, comprenant au moins un, deux, trois ou plus des composés selon les définitions de l'une quelconque des revendications 1 à 4, et choisis dans le groupe A constitué par :
| | |
|---|---|
| | |
| 24 | 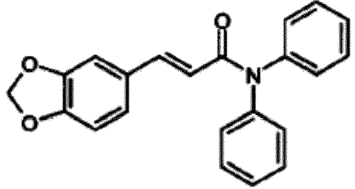 |
| 25 | 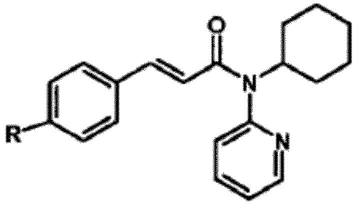 avec R = H |
| 26 | 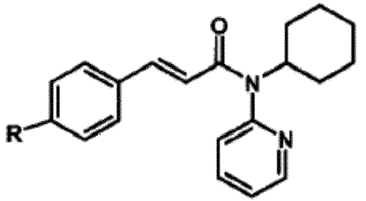 avec R = Me |
| 27 | 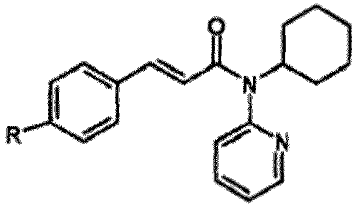 avec R = MeO |
| 28 | 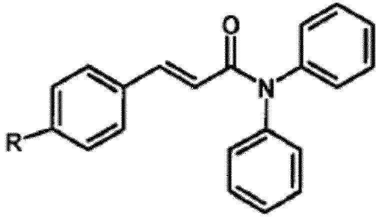 avec R = H |
| 29 | 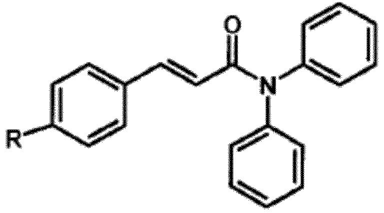 avec R = Me |
| 30 | 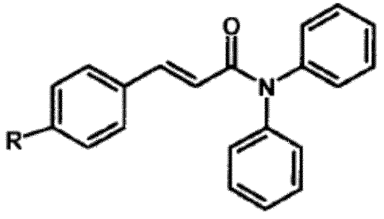 avec R = MeO |
en une concentration de 0,1 ppm à 10 % en poids, par rapport au poids total de l'agent, pour obtenir un effet de refroidissement sur la peau ou une muqueuse, qui, en comparaison de l'effet de refroidissement d'un agent de même composition, dans lequel uniquement le composé ou les composés choisis dans le groupe A sont remplacés par du N-éthylamide de l'acide menthane-carboxylique en la même concentration, est prolongé d'au moins 10 minutes, à des fins non thérapeutiques.

8. Composé selon la définition selon l'une quelconque des revendications 1 à 4, à condition que le composé ne soit pas le diéthyltoluamide, 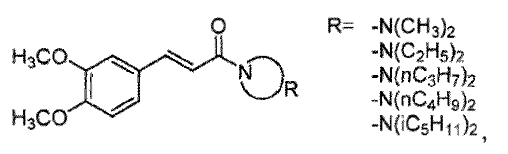 le thiocarbamate de S-4-chlorobenzyl-diéthyle 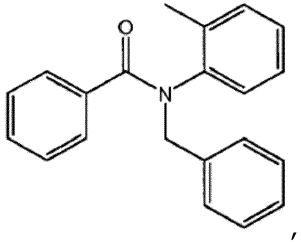 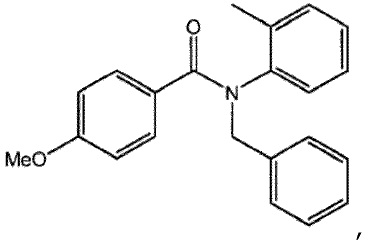 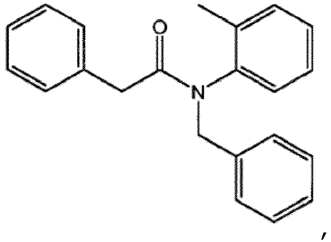 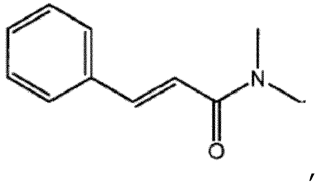 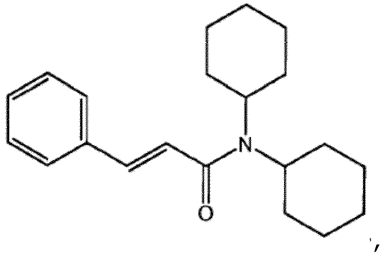 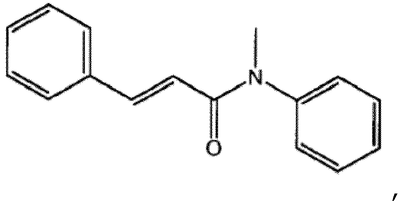 ni

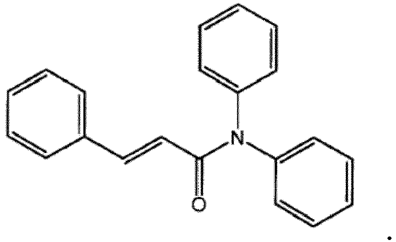

9. Agent contenant au moins un composé selon la revendication 8.

10. Agent contenant au moins un composé selon la définition selon l'une quelconque des revendications 1 à 4, la condition selon laquelle le composé de formule (III) n'est pas le chlorhydrate de 1-(2-aminoéthyl)-1-(1,3-benzothiazol-2-yl)-3-[(1S)-1-phényléthyl]urée, ni le chlorhydrate de 1-(2-aminoéthyl)-1-(1,3-benzothiazol-2-yl)-3-[(1R)-1-phényléthyl]urée ne s'appliquant en outre pas,
destiné à une utilisation en tant que médiateur du récepteur TRPM8, choisi parmi :
a) les produits alimentaires, tels que les crèmes glacées, les mousses, les crèmes, les boissons, les confiseries,
b) les produits de soin de la bouche, tels que les dentifrices, les bains de bouche, les gommes à mâcher,
c) les produits de soin du corps, tels que les produits de soin de la peau ou des cheveux, tels que les crèmes solaires, les crèmes pour les coups de soleil, les lotions, les shampoings, les pansements,
d) les mousses et les gels.

11. Agent selon la revendication 10 ou agent contenant 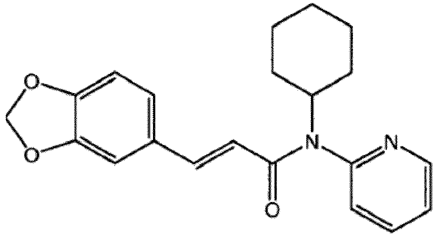 destiné à une utilisation en tant que médiateur du récepteur TRPM8,
choisi dans le groupe constitué par un mélange d'arômes et une préparation cosmétique servant pour l'alimentation, l'hygiène buccale ou le plaisir, comprenant un, deux, trois ou plus des composés selon les définition de l'une quelconque des revendications 1 à 4, qui est ou sont choisis (i) dans le groupe (B) constitué par :
| | |
|---|---|
| 24 | 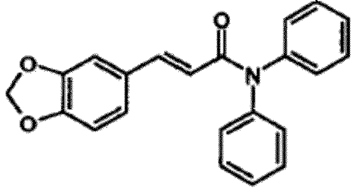 |
| 25 | 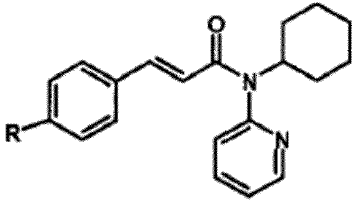 avec R = H |
| 26 | 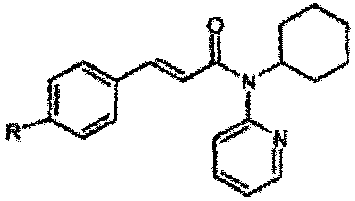 avec R = Me |
| 27 | 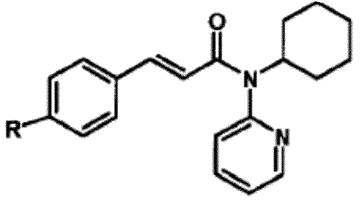 avec R = MeO |
| 28 | 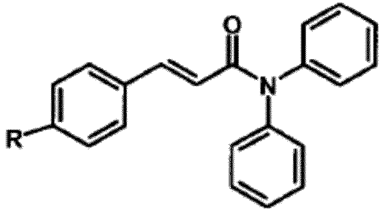 avec R = H |
| 29 | 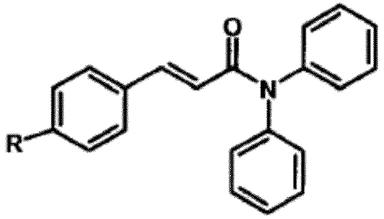 avec R = Me |
| 30 | 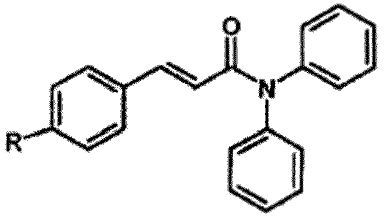 avec R = MeO |
et/ou qui est choisi (ii) dans le groupe C constitué par :
| LN | Structure |
|---|---|
| 23 | 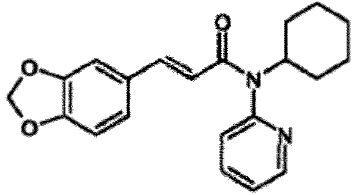 |
le composé du groupe C étant contenu en une concentration de 0,05 à < 0,1 ppm ou de 0,1 ppm à 10 % en poids, par rapport au poids total de la préparation, à condition que, dans le cas (ii), la préparation ne soit pas un bain de bouche ayant la composition suivante, à chaque fois par rapport à un litre :
| | |
|---|---|
| éthanol 95 % | 177 ml |
| sorbitol 70 % | 250 g |
| composé de formule 23 en solution à 1 % dans de l'éthanol | 50 ml |
| huile de menthe poivrée | 0,30 g |
| salicylate de méthyle | 0,64 g |
| eucalyptol | 0,922 g |
| thymol | 0,639 g |
| acide benzoïque | 1,50 g |
| Pluronic® F127 tensioactif non ionique | 5,00 g |
| saccharine de sodium | 0,60 g |
| citrate de sodium | 0,30 g |
| acide citrique | 0,10 g |
| eau q.s.p | 1 litre. |

12. Agent selon la revendication 11, comprenant :
(1) une ou plusieurs substances supplémentaires à effet de refroidissement physiologique, la substance supplémentaire ou une des, plusieurs des ou toutes les substances supplémentaires (i) ayant un effet de goût ou (ii) n'ayant pas d'effet de goût,
et/ou
(2) un ou plusieurs arômes sans effet de refroidissement physiologique,
et/ou
(3) une ou plusieurs substances à effet trigéminal ou de bain de bouche sans effet de refroidissement physiologique,
et/ou
(4) (iii) un ou (iv) plusieurs composés qui, dans le cas de (iv), ont indépendamment les uns des autres ou ensemble en outre un effet de modulation du goût et/ou un effet trigéminal et/ou de bain de bouche.

13. Produit contenant au moins un composé selon la définition selon l'une quelconque des revendications 1 à 4, la condition selon laquelle le composé de formule (III) n'est pas le chlorhydrate de 1-(2-aminoéthyl)-1-(1,3-benzothiazol-2-yl)-3-[(1S)-1-phényléthyl]urée, ni le chlorhydrate de 1-(2-aminoéthyl)-1-(1,3-benzothiazol-2-yl)-3-[(1R)-1-phényléthyl]urée ne s'appliquant en outre pas,
destiné à une utilisation en tant que médiateur du récepteur TRPM8,
choisi parmi :
a) les produits textiles,
b) les matériaux d'emballage,
c) les produits de tabac,
d) les produits d'hygiène ou
e) les lingettes rafraîchissantes.

14. Composé selon la revendication 8, choisi parmi :
| N° | Type structural 3 |
|---|---|
| 3-2 | 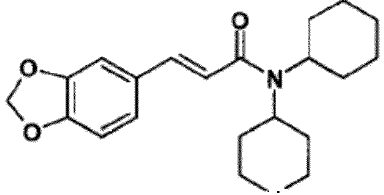 |
| 3-3 | 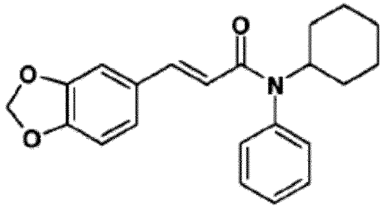 |
| 3-4 | 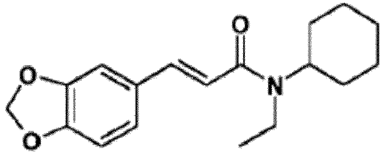 |
| 3-5 | 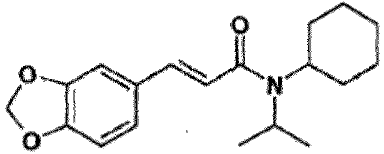 |
| 3-6 | 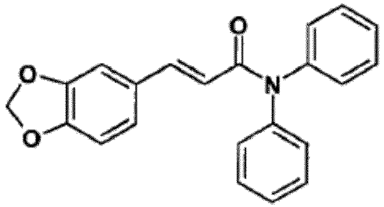 |
| 3-7 | 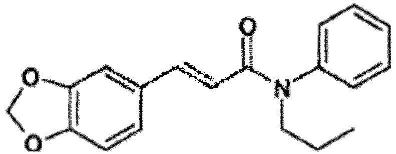 |
| 3-8 | 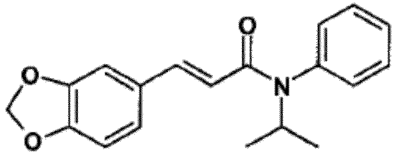 |
| 3-9 | 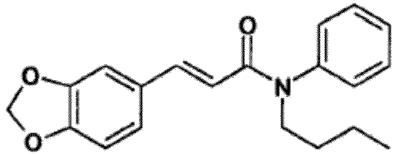 |
| 3-10 | 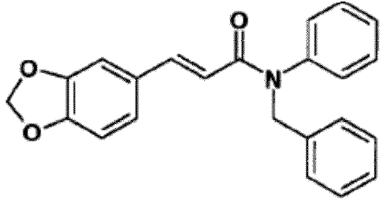 |
| 3-11 | 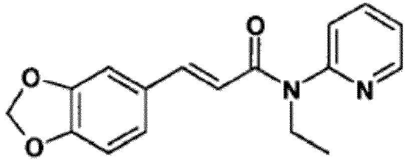 |
| 3-12 | 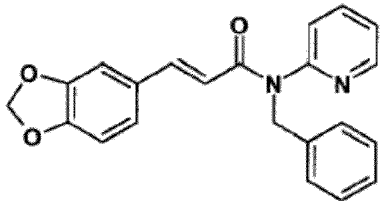 |
| 3-13 | 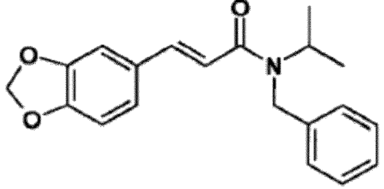 |
| 3-14 | 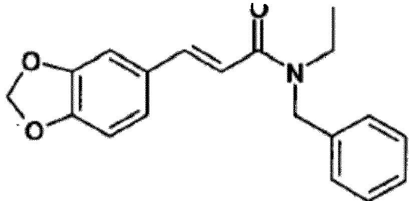 |
| 3-15 | 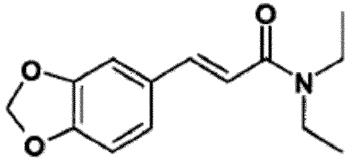 |
| 3-16 | 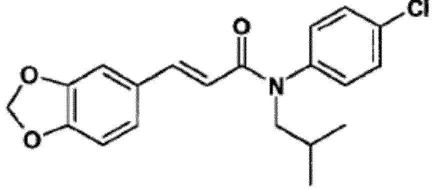 |
| 3-17 | 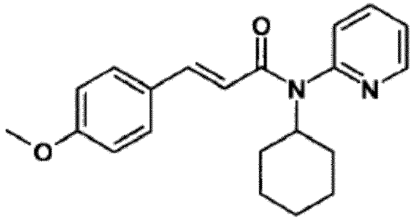 |
| 3-18 | 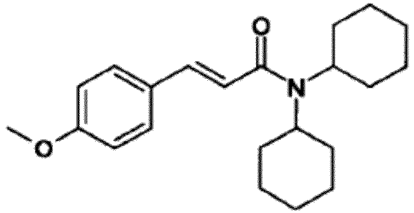 |
| 3-19 | 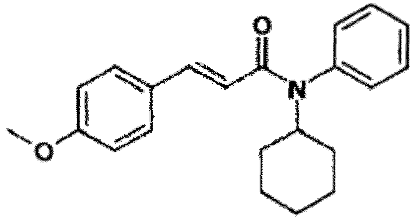 |
| 3-20 | 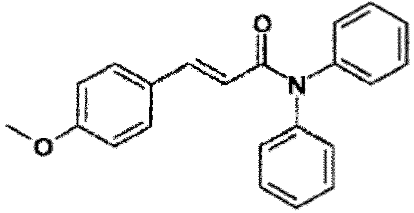 |
| 3-21 | 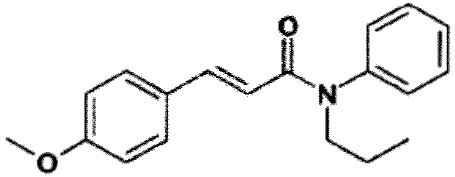 |
| 3-22 | 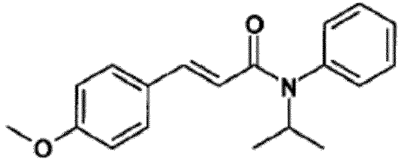 |
| 3-23 | 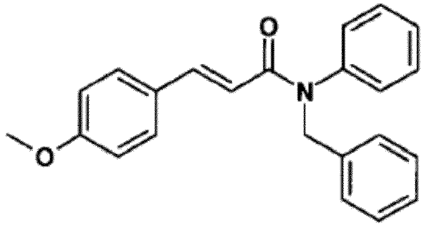 |
| 3-24 | 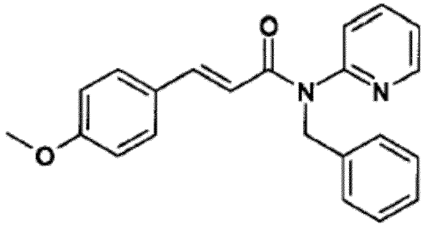 |
| 3-25 | 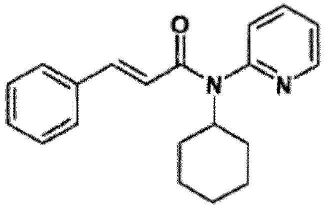 |
| 3-26 | 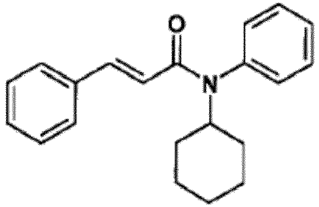 |
| 3-29 | 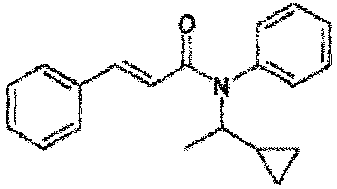 |
| 3-30 | 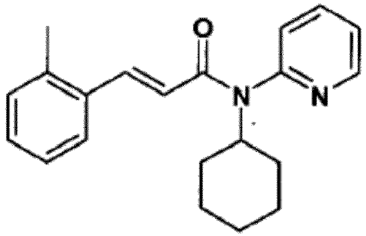 |
| 3-31 | 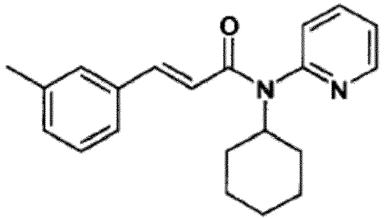 |
| 3-32 | 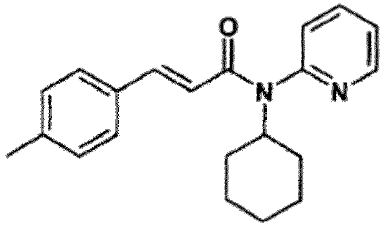 |
| 3-33 | 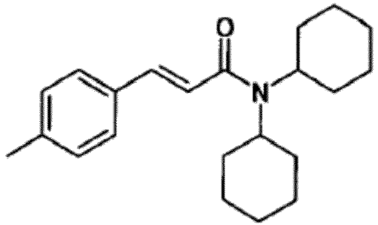 |
| 3-34 | 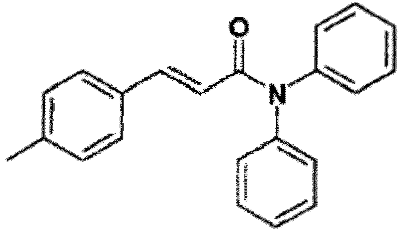 |
| 3-25 | 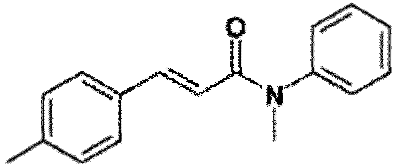 |
| 3-36 | 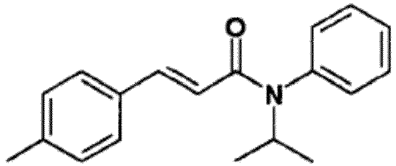 |
| 3-37 | 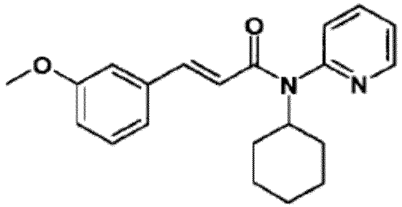 |
| 3-38 | 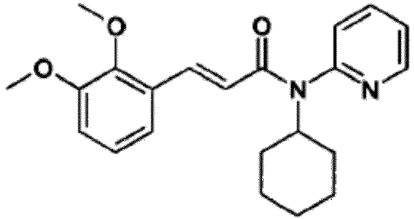 |
| 3-39 | 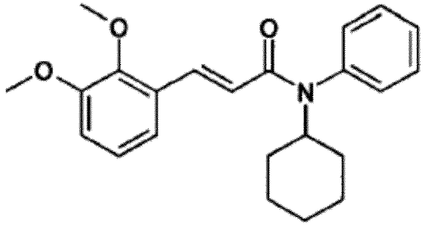 |
| 3-40 | 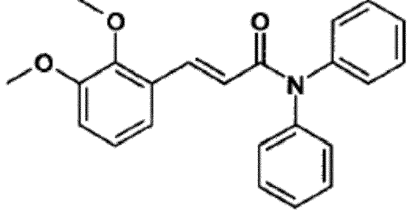 |
| 3-41 | 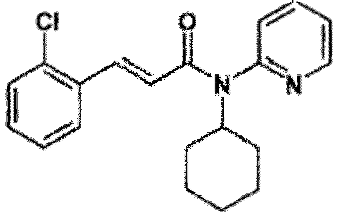 |
| 3-42 | 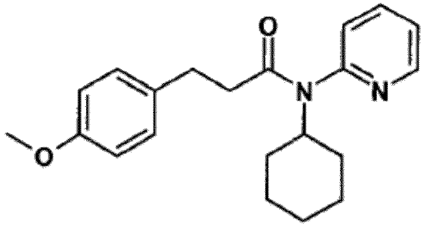 |
| 3-43 | 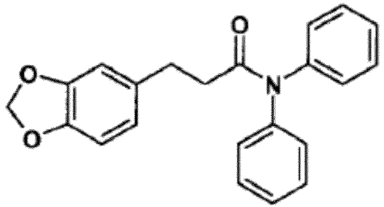 |
| 3-45 | 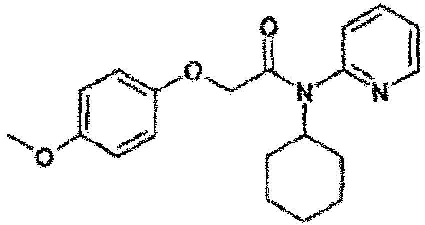 |
| 3-46 | 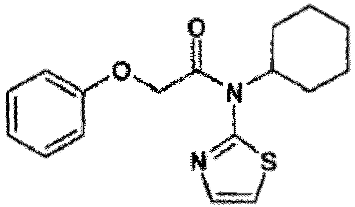 |
| 3-48 | 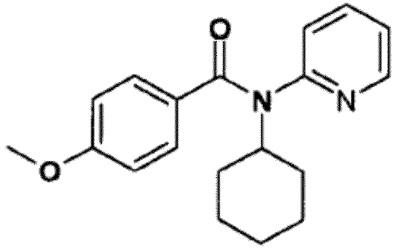 |
| 3-49 | 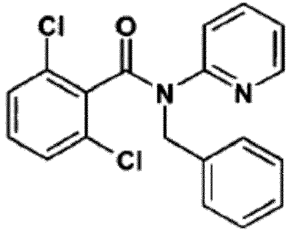 |
éventuellement sous forme stéréoisomériquement pure, sous la forme d'un mélange de stéréoisomères, ainsi que les sels de ces composés.
